(19) Europäisches Patentamt
European Patent Office
Office européen des brevets

(11) EP 1 661 896 A1

(12) **EUROPEAN PATENT APPLICATION**
published in accordance with Art. 158(3) EPC

(43) Date of publication:
**31.05.2006 Bulletin 2006/22**

(21) Application number: **04772646.8**

(22) Date of filing: **26.08.2004**

(51) Int Cl.:
*C07D 487/04* (1985.01) *A61K 31/519* (2000.01)
*A61P 3/10* (2000.01) *A61P 17/14* (2000.01)
*A61P 25/24* (2000.01) *A61P 25/28* (2000.01)
*A61P 29/00* (2000.01) *A61P 35/00* (2000.01)
*A61P 37/02* (2000.01) *A61P 43/00* (2000.01)

(86) International application number:
**PCT/JP2004/012690**

(87) International publication number:
**WO 2005/019218 (03.03.2005 Gazette 2005/09)**

(84) Designated Contracting States:
**AT BE BG CH CY CZ DE DK EE ES FI FR GB GR HU IE IT LI LU MC NL PL PT RO SE SI SK TR**

(30) Priority: **26.08.2003 JP 2003301022**
**30.03.2004 JP 2004100022**

(71) Applicant: **Teijin Pharma Limited**
**Tokyo 100-8585 (JP)**

(72) Inventors:
- **TSUTSUMI, Takaharu**
  **c/o Teijin Pharma Limited**
  **Hino-shi Tokyo 1910065 (JP)**
- **SUGIURA, Satoshi**
  **c/o Teijin Pharma Limited**
  **Hino-shi, Tokyo 1910065 (JP)**
- **KOGA, Masahiro**
  **c/o Teijin Pharma Limited Tokyo Res**
  **Hino-shi Tokyo 1910065 (JP)**
- **MATSUMOTO, Yoshiyuki**
  **c/o Teijin Pharma Limited**
  **Hino-shi, Tokyo 1910065 (JP)**
- **ISHII, Toshihiro**
  **c/o Teijin Pharma Limited**
  **Hino-shi Tokyo 1910065 (JP)**
- **NAKANO, Akira**
  **c/o Teijin Pharma Limited**
  **Hino-shi Tokyo 1910065 (JP)**
- **UNOKI, Gen**
  **c/o Teijin Pharma Limited**
  **Hino-shi Tokyo 1910065 (JP)**
- **SAKAI, Yuri**
  **c/o Teijin Pharma Limited**
  **Hino-shi, Tokyo 1910065 (JP)**
- **TAKARADA, Reiko**
  **c/o Teijin Pharma Limited**
  **Hino-shi, Tokyo 1910065 (JP)**
- **OGAWA, Hiroko**
  **c/o Teijin Pharma Limited**
  **Hino-shi, Tokyo 1910065 (JP)**

(74) Representative: **Hallybone, Huw George et al**
**Carpmaels and Ransford,**
**43-45 Bloomsbury Square**
**London WC1A 2RA (GB)**

(54) **PYRROLOPYRIMIDINETHIONE DERIVATIVE**

(57) The present invention relates to a compound having GSK-3 inhibiting function.

EP 1 661 896 A1

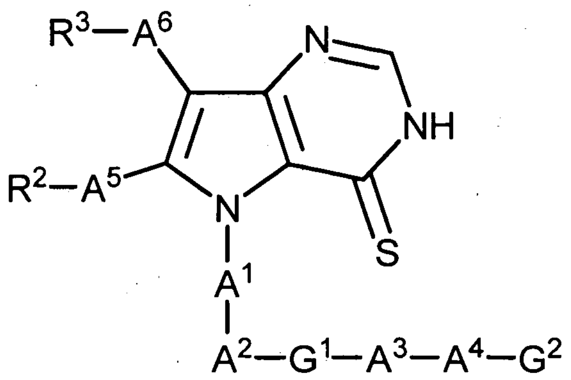

(I)

$A^1$ and $A^3$ are a single bond, an aliphatic hydrocarbon group; $A^2$ and $A^4$ are a single bond, CO, COO, etc.; $G^1$ is a single bond, an alicyclic hydrocarbon, an aromatic hydrocarbon, etc.; $G^2$ is a hydrogen atom, an aliphatic hydrocarbon, an alicyclic hydrocarbon, an aromatic hydrocarbon, etc.; $A^5$ is a single bond, NR; $R^2$ is H, halogen, an aliphatic hydrocarbon, etc.; $A^6$ is a single bond, NR, CO, etc.; $R^3$ is H, halogen, nitro, saturated aliphatic hydrocarbon, etc.; and when R is a hydrogen atom or an aliphatic hydrocarbon group.

## Description

TECHNICAL FIELD

The present invention relates to novel pyrrolopyrimidine-thione derivatives that have an action inhibiting glycogen synthase kinase-3 (GSK-3). More particularly, the invention relates to novel pyrrolo[3,2-d] pyrimidine-thione derivatives useful as pharmaceutical agents for treating and/or preventing disorders mediated by GSK-3 activity, particularly, impaired glucose tolerance, type I diabetes, type II diabetes, diabetic complications (retinopathy, nephropathy, neuropathy or great vessel hindrance), Alzheimer's disease, neurodegenerative diseases (AIDS encephalophy, Huntington's disease, Parkinson's disease, amyotrophic lateral sclerosis or multiple sclerosis), bipolar affective disorder (manic depressive psychosis), traumatic cerebrospinal injury, epilepsy, obesity, atherosclerosis, hypertension, polycystic ovary syndrome, syndrome X, alopecia, inflammatory diseases (arthrosis deformans, rheumatism, atopic dermatitis, psoriasis, ulcerative colitis, Crohn's disease, sepsis or systemic inflammatory response syndrome), cancer and immunodeficiency.

BACKGROUND ART

Glycogen synthase kinase 3 (GSK-3) is a serine/threonine protein kinase. Two isoforms, i.e., α and β, which are encoded by distinct genes, have been identified (see Trends Biochem. Sci., 1991, Vol. 16, p.177). Both GSK-3 isoforms have a monomeric structure and are constitutively active in resting cells. GSK-3 was originally identified as a kinase that inhibits glycogen synthase by direct phosphorylation (see Eur. J. Biochem., 1980, Vol. 107, p. 519). Upon insulin' activation, GSK-3 is inactivated, thereby allowing the activation of glycogen synthase and possibly other insulin-dependent events, such as glucose transport. Also, it has been known that GSK-3 activity is inactivated by other growth factors, such as IGF-1 or FGF, through signaling from receptor tyrosine kinases (see Biochem. J., UK, 1993, Vol. 294, p.625; Biochem. J., UK, 1994, Vol. 303, p. 21; Biochem. J., UK, 1994, Vol. 303, p.27).

GSK-3 inhibitors are useful in the treatment of disorders that are caused by GSK-3 activity. In addition, inhibition of GSK-3 mimics the activation of growth factor signaling pathways and consequently GSK-3 inhibitors are useful in the treatment of diseases caused by inactivation of signaling pathways. The various types of diseases for which GSK-3 inhibitors are considered effective are described below.

Type I diabetes is induced due to autoimmune destruction of β cells as pancreatic insulin production cells, resulting in deficiency of insulin. Due to this, it is necessary for a type I diabetic patient to routinely be administered insulin for maintaining life. Unfortunately, currently available insulin therapy is unable to control blood glucose levels as accurately as normal β cells. Thus, type I diabetes is liable to induce diabetic complications such as retinopathy, nephropathy, neuropathy, great vessels hindrance or the like.

Type II diabetes is a multifactorial disease. Hyperglycemia is due to insulin resistance in the liver, skeletal muscle and lipid tissues coupled with inadequate or defective secretion of insulin from pancreatic islets. As a result, diabetic complications such as retinopathy, nephropathy, neuropathy, or great vessels hindrance are induced. Skeletal muscle is the major site for insulin-stimulated glucose uptake. Glucose removed from the circulation is either metabolized through glycolysis and the TCA cycle or stored as glycogen. Muscle glycogen deposition plays a very important role in glucose homeostasis. Type II diabetic subjects have defective muscle glycogen storage. GSK-3, which is known to phosphorylate glycogen synthase, inhibits the accumulation of glycogen in peripheral tissues and lowers the reactivity of insulin, leading to an increase in blood level of glucose.

Recently, it has been reported that the expression of GSK-3 is stimulated in skeletal muscles of type II diabetic patients, and the GSK-3α activity and insulin in skeletal muscles are inversely correlated (see Diabetes, USA, 2000, Vol. 49, p. 263). Where GSK-3β and active GSK-3β variants (S9A, S9E) are overexpressed in HEK-293 cells, the GSK activity is inhibited (see Proc. Natl. Acad. Sci., USA, 1996, Vol. 93, p. 10228). In CHO cells in which insulin receptor and insulin receptor substrate 1 (IRS-1) are expressed, overexpression of GSK-3β brings about a decrease in the insulin activity (see 8: Proc. Natl. Acad. Sci., USA, 1997, Vol. 94, 9660). Recent research carried out using C57BL/6J mice with pyknic type diabetes has clearly shown that GSK-3 activity stimulation and insuling resistance are correlated to the progress of type II diabetes (see Diabetes, USA, 1999, Vol. 48, p.1662).

Conventionally, lithium salts have been known to have inhibitory effects of GSK-3 activity (see Proc. Natl. Acad. Sci., USA, 1996, Vol. 93, p. 8455). It has been reported that the therapy using the lithium salts lowers glucose levels in both type I and II diabetic patients, thereby alleviating the severity of the disease (see Biol. Trace Elements Res., 1997, Vol. 60, p. 131). However, lithium salts have also been found to exhibit various side effects on molecular targets other than GSK-3.

From the findings described above, it can be concluded that GSK-3 inhibitors are effective therapeutics for the treatment of impaired glucose tolerance, type I diabetes, type II diabetes and complications thereof.

It is also suggested that GSK-3 is associated with progress of Alzheimer's disease. Alzheimer's disease is characterized by formation of senile plaques due to agglomeration of amyloid beta (Aβ) peptide and the formation of

intracellular neurofibrillary tangles. This leads to a large quantity of neuronal cell death, resulting in dementia. It is believed that GSK-3 involves abnormal phosphorylation of tau protein, which causes a neurofibrillary change in the course of progress of Alzheimer's disease (see Acta Neuropathol., 2002, Vol. 103, p. 91). Also, it has been reported that GSK-3 inhibitors can prevent neuronal cell death (see J. Neurochem., 2001, Vol. 77, p. 94). Therefore, it is believed that GSK-3 inhibitors delay the progress of Alzheimer's disease. To date, therapeutic agents for Alzheimer's disease have mainly been used in conjunction with allopathy (see Expert Opin. Pharmacother., 1999, Vol. 1, p. 121). However, there is no known a pharmaceutical agent that is effective in preventing neuronal cell death and delaying the onset or progress of the disease. These findings imply that GSK-3 inhibitors are effective pharmaceutical agents in alleviating the severity of Alzheimer's dementia.

There is a report that GSK-3 inhibitors suppress neuronal cell death, specifically, neuronal cell death due to overexcitement through glutamic acid (see Proc. Natl. Acad. Sci., USA, 1998, Vol. 95, p.2642; J. Neurochem., 2001, Vol. 77, p. 94). This suggests that GSK-3 inhibitors are possibly useful in the treatment of bipolar affective disorder such as manic depressive psychosis, epilepsy or other degenerative brain injury or neurodegenerative diseases. Examples of the neurodegenerative disease include in addition to the Alzheimer's disease, AIDS encephalopathy, Huntington's disease, Parkinson's disease, amyotrophic lateral sclerosis, multiple sclerosis, Pick's disease, progressive supranuclear palsy and so on. Also, overexcitement through glutamic acid is presumably a principal cause of brain dysfunction in stroke (cerebral infarction, intracerebral hemorrhage and subarachnoid hemorrhage), traumatic cerebrospinal injury, bacteria/virus infectious disease. GSK-3 inhibitors are expected to be effectively used in the treatment of these diseases. All of such diseases accompany neuronal death. Currently, no therapeutic agents for effectively suppressing the neuronal death are available. Therefore, GSK-3 inhibitors are believed to become potentially effective pharmaceutical agents for the treatment of various kinds of neurodegenerative diseases, dipolar affective disorders (manic-depressive psychosis), epilepsy, stroke, traumatic cerebrospinal injury, and the like.

Several in vitro research results have led to a report that Wint10B potently suppresses the differentiation of preadipocytes to mature fat cells (see Science, 2000, Vol. 289, p. 950). GSK-3 specific inhibitors mimic Wint10B signaling in preadipocytes, that is, GSK-3 specific inhibitors stabilize free β-catenin in cytoplasm and suppress the induction of C/EBPα and PPARγ, thereby suppressing the formation of fat (see J. Biol. Chem, 2002, Vol. 277, p. 30998). GSK-3 inhibitors are therefore potentially useful as effective pharmaceutical compositions for treating obesity.

Also, β-catenin has been known to be a GSK-3 substrate in vivo. After phosphorylation by GSK-3, β-catenin is subjected to proteosome-dependent degradation (see EMBO J., 1998, Vol. 17, p. 1371). Meanwhile, transient β-catenin stabilization may lead to increase hair development (see Cell, 1998, Vol. 95, p. 605). Consequently, GSK-3 inhibitors are believed to be a useful medicament for the treatment of alopecia.

Further, research into GSK-3β knock out mouse-derived fibroblasts implies that GSK-3β regulates the activity of transcription factor NFκB to be at a positive level (see Nature, 2000, Vol. 406, p. 86). NFκB is in charge of cell responsiveness to numerous inflammatory stimuli. Thus, GSK-3 inhibitors may have beneficial effects in the treatment of inflammatory diseases such as arthrosis deformans, rheumatism, atopic dermatitis, psoriasis, ulcerative colitis, Crohn's Disease, sepsis, or systemic inflammatory response syndrome, by adjusting the NFκB activity to be at a negative level.

A transcription factor NF-AT is dephosphorylated by calcineurine and increases immunosuppressive response (see Science, 1997, Vol. 275, p. 1930). Conversely, GSK-3 phosphorylates NF-AT and transports the same from nuclei, thereby suppressing the expression of initial immune response gene. Thus, GSK-3 inhibitors could be useful to immunity activation for cancer immunotherapy.

Examples of materials that have conventionally been known to have GSK-3 inhibiting activity include hymenialdisine derivatives (see Chemistry & Biology, 2000, Vol. 7, p. 51, and WO01/41768 pamphlet), maleiimide derivatives (see Chemistry & Biology, 2000, Vol. 7, p. 793), paullone derivatives (see EuR. J. Biochem., 2000, Vol. 267, p. 5983 and WO01/60374 Pamphlet), purine derivatives (see WO98/16528 Pamphlet), pyrimidine and pyridine derivatives (see WO99/65897 Pamphlet), hydroxyflavone derivatives (see WO00/17184 Pamphlet), pyrimidone derivatives (see WO00/18758, WO01/70683, WO01/70729, WO01/70728, WO01/70727, WO01/70726, and WO01/70725 Pamphlets), pyrrole-2,5-dione derivatives (see WO00/21927 and WO01/74771 Pamphlets), diamino-1,2,4-triazolecarboxylic acid derivatives (see WO01/09106 Pamphlet), pyrazine derivatives (see WO01/44206 Pamphlet), bicyclic inhibitor (see WO01/44246 Pamphlet), indirubine derivatives (see WO01/37819 Pamphlet), carboxamide derivatives (see WO01/42224 Pamphlet), peptide inhibitors (see WO01/49709 Pamphlet), 2,4-diaminothiazole derivatives (see WO01/56567 Pamphlet), thiadiazolidindione derivatives (see WO01/85685 Pamphlet), aromatic amide derivatives (see WO01/81345 Pamphlet), and so on.

Also, the claims of WO02/085909 Pamphlet contains chemical formulas encompassing a wide variety of compounds including pyrrolopyrimidine derivatives. However, the bicyclic pyrrolopyrimidine derivatives actually synthesized are only those having cyano group at the 7-position of pyrrolopyrimidine ring and limited variety of substituents at other substitutable positions. In addition, while it discloses a method for assaying inhibitory activity of GSK-3, it does not disclose anything specifically about which compounds have such activities.

DISCLOSURE OF THE INVENTION

An object of the present invention is to provide novel compounds which are specific to and capable of strongly inhibiting the activity of GSK-3 while being clinically applicable and pharmaceutical compositions as GSK-3 inhibitors using them as valid components.

Also, another object of the present invention is to provide an agent for treating or preventing a GSK-3-mediated disease.

Further, still another object of the present invention is to provide a method for treating a GSK-3-mediated disease.

The present inventors studied for the above objects and consequently reached the following inventions.

Namely, the present invention provides a compound represented by the formula (I) or a pharmaceutically acceptable salt thereof.

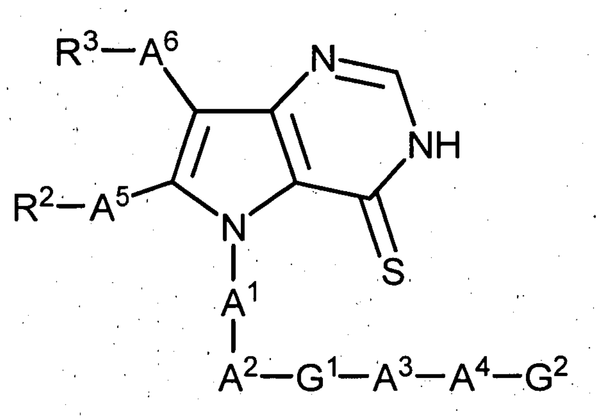

(I)

wherein, in the formula (I),

$A^1$ represents a single bond or a divalent acyclic aliphatic hydrocarbon group having 1 to 6 carbon atoms that links a nitrogen atom bonded to $A^1$ with $A^2$ on the same or different carbon atom;

$A^2$ represents a bond representing a single bond or represents a group that links $A^1$ with $G^1$ in the form of

$A^1$ -C(=O)-$G^1$,

$A^1$-C(=O)-O-$G^1$,

$A^1$-C(=O)-$NR^{101}$-$G^1$,

$A^1$-C(=S)-$NR^{102}$-$G^1$,

$A^1$-C(=$NR^{103}$)-$G^1$,

$A^1$-O-$G^1$,

$A^1$-OC(=O)-$G^1$,

$A^1$-$NR^{104}$-$G^1$,

$A^1$-$NR^{105}$-C(=O)-$G^1$,

$A^1$-$NR^{106}$-$S(=O)_2$-$G^1$,

$A^1$-$NR^{107}$-C(=O)-O-$G^1$,

$A^1$-$NR^{108}$-C(=O)-$NR^{109}$-$G^1$,

$A^1$-$NR^{110}$C(=S)-$G^1$,

$A^1$-$NR^{111}$-C(=S)-$NR^{112}$-$G^1$,

$A^1$-S-$G^1$,

$A^1$-S(=O)-$G^1$,

$A^1$-$S(=O)_2$-$G^1$,

$A^1$-$S(=O)_2$-$NR^{113}$-$G^1$,

$A^1$-$CR^{114}$=CH-$G^1$,

$A^1$-$CR^{115}$=CF-$G^1$,

$A^1$-CH=$CR^{116}$-$G^1$, or

$A^1$-CF=$CR^{117}$-$G^1$;

$G^1$ represents a single bond or represents a divalent group which is obtained by removing two hydrogen atoms from any one of an optionally substituted alicyclic hydrocarbon group having 3 to 10 carbon atoms, an optionally substituted aromatic hydrocarbon having 6 to 14 carbon atoms, and an optionally substituted heterocyclic compound having 1 to 4 atoms selected from the group consisting of an oxygen atom, a nitrogen atom and a sulfur atom, in the ring.
$A^3$ represents a single bond or represents an optionally substituted divalent acyclic aliphatic hydrocarbon group having 1 to 10 carbon atoms that links $G^1$ with $A^4$ on the same or different carbon atom;
$A^4$ represents a single bond or represents a group that links $A^3$ with $G^2$ in the form of

$A^3$-C(=O)-$G^2$,

$A^3$-C(=O)-O-$G^2$,

$A^3$-C(=O)-$NR^{121}$-$G^2$,

$A^3$-C(=S)-$NR^{122}$-$G^2$,

$A^3$-C(=$NR^{123}$)-$G^2$,

$A^3$-O-$G^2$,

$A^3$-O-C(=O)-$G^2$,

$A^3$-$NR^{124}$-$G^2$,

$A^3$-$NR^{125}$-C(=O)-$G^2$,

$A^3$-$NR^{126}$-$S(=O)_2$-$G^2$,

$A^3$-$NR^{127}$-C(=O)-O-$G^2$,

$A^3$-$NR^{128}$-C(=O)-$NR^{129}$-$G^2$,

$A^3$-$NR^{130}$-C(=S)-$G^2$,

$A^3$-$NR^{131}$-C(=S)-$NR^{132}$-$G^2$,

$A^3$-S-$G^2$,

$A^3$-S(=O)-$G^2$,

$A^3$-S(=O)$_2$-$G^2$,

$A^3$-S(=O)$_2$-NR$^{133}$-$G^2$ or

$A^3$-S(=O)$_2$-O-$G^2$;

$G^2$ is a hydrogen atom, an optionally substituted acyclic aliphatic hydrocarbon group having 1 to 10 carbon atoms, an optionally substituted alicyclic hydrocarbon group having 3 to 10 carbon atoms, an optionally substituted aromatic hydrocarbon group having 6 to 14 carbon atoms, or an optionally substituted heterocyclic group having 1 to 4 atoms selected from the group consisting of an oxygen atom, a nitrogen atom and a sulfur atom, in the ring;
$A^5$ is a bond representing a single bond or -NR$^{201}$-;
$R^2$ is a hydrogen atom, a fluorine atom, a chlorine atom, a bromine atom, an iodine atom, an optionally substituted acyclic aliphatic hydrocarbon group having 1 to 10 carbon atoms, an optionally substituted alicyclic hydrocarbon group having 3 to 8 carbon atoms, an optionally substituted aromatic hydrocarbon group having 6 to 14 carbon atoms, or an optionally substituted heterocyclic group having 1 to 4 atoms selected from the group consisting of an oxygen atom, a nitrogen atom and a sulfur atom, in the ring;
$A^6$ represents a single bond or represents a group that links $R^3$ with a carbon atom of a pyrrole ring to which $A^6$ is bonded, in the form of
$R^3$-NR$^{301}$-pyrrole ring,
$R^3$-C(=O)-pyrrole ring,
$R^3$-NR$^{302}$-C(=O)-pyrrole ring,
$R^3$-NR$^{303}$-C(=S)-pyrrole ring,
$R^3$-NR$^{304}$-C(=O)-NR$^{305}$-pyrrole ring,
$R^3$-C(=O)-NR$^{306}$-pyrrole ring,
$R^3$-NR$^{307}$-CH=N-pyrrole ring,
$R^3$-C(=O)-O-pyrrole ring,
$R^3$-O-C(=O)-pyrrole ring,
$R^3$-O-pyrrole ring,
$R^3$-S-pyrrole ring,
$R^3$-S(=O)-pyrrole ring,
$R^3$-S(=O)$_2$-pyrrole ring,
$R^3$-CR$^{308}$=CR$^{309}$-pyrrole ring,
$R^3$-C=C-pyrrole ring, or
$R^3$-S(=O)$_2$-C≡C-pyrrole ring;
$R^3$ is a hydrogen atom, a fluorine atom, a chlorine atom, a bromine atom, an iodine atom, a nitro group, an optionally substituted acyclic saturated aliphatic hydrocarbon group having 1 to 10 carbon atoms, an optionally substituted alicyclic hydrocarbon group having 3 to 8 carbon atoms, an optionally substituted aromatic hydrocarbon group having 6 to 14 carbon atoms, or an optionally substituted heterocyclic group having 1 to 4 atoms selected from the group consisting of an oxygen atom, a nitrogen atom and a sulfur atom, in the ring;
$A^6$-$R^3$ may be a combination wherein $A^6$ represents a group that links a carbon atom of a pyrrole ring to which $A^6$ is bonded, with $R^3$ in the form of $R^3$-CR$^{308}$=CR$^{309}$-pyrrole ring or $R^3$-C≡C-pyrrole ring, and $R^3$ represents a trimethylsilyl group, a formyl group, an optionally substituted $C_2$-$C_7$ acyl group, a carboxyl group, a $C_2$-$C_7$ alkoxycarbonyl group, a carbamoyl group, an optionally substituted $C_2$-$C_7$ alkylcarbamoyl group, or a cyano group;

$R^{101}$-$R^{117}$, $R^{121}$-$R^{133}$, $R^{201}$ and $R^{301}$-$R^{309}$ are each independently a hydrogen atom or an aliphatic hydrocarbon group having 1 to 4 carbon atoms.

However, when both $A^1$ and $A^3$ represent acyclic alphatic hydrocarbon groups, at least one of $A^2$ or $G^1$ is not a single bond.

In addition, the present invention provides a pharmaceutical composition comprising the compound or a pharmaceutically acceptable salt thereof represented by the formula (I) and a pharmaceutically acceptable carrier.

Further, the present invention provides a GSK-3 inhibitor comprising the compound or a pharmaceutically acceptable salt thereof represented by the formula (I).

Furthermore, the present invention provides an agent for treating or preventing a GSK-3-mediated disease, comprising the compound or a pharmaceutically acceptable salt thereof represented by the formula (I).

Furthermore, the present invention provides a method for treating a GSK-3-mediated disease, comprising a step of injecting the compound or a pharmaceutically acceptable salt thereof represented by the formula (I) in treatment valid amount to a patient.

Note that, in $A^1$-$G^2$ portion in the formula.(I), there also exists a case where different combinations consequently represent the same substituent according to the combination of $A^1$, $A^2$, $G^1$, $A^3$, $A^4$, and $G^2$, and combinations containing

also substituents of them where they may have substituents. However, the scope of the present invention will not become clear due to this.

Further, the present invention is a pyrrolopyrimidine derivative represented by the following formula (II) which can be used as the combined intermediate of the pyrimidine-thione derivative represented by the formula (I).

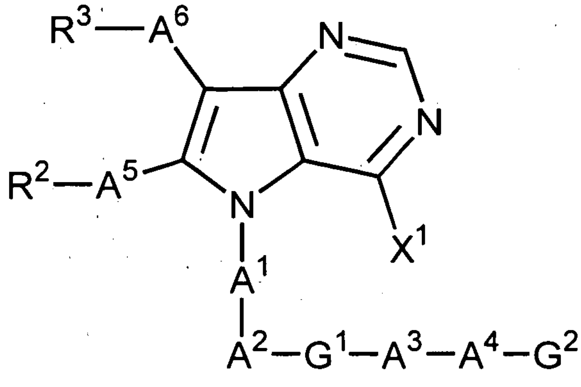

(II)

in formula (II), $A^1$, $A^2$, $A^3$, $A^4$, $A^5$, $A^6$, $G^1$, $G^2$, $R^2$ and $R^3$ are as defined in the formula (I); and $X^1$ is a chlorine atom, a bromine atom, an iodine atom, a $C_2$-$C_{10}$ acylthio group, a $C_2$-$C_8$ alkoxymethylthio group, a $C_1$-$C_8$ alkyl group, or a $C_1$-$C_8$ arylsulfonyloxy group).

Still further, the present invention is a compound represented by the following formula (Ic) which can be used as the manufacture intermediate of the pyrrolopyrimidinone derivative represented by the formula (I).

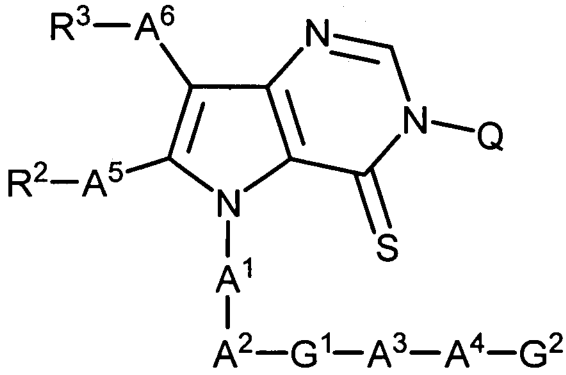

(Ic)

in formula (Ic), $A^1$, $A^2$, $A^3$, $A^4$, $A^5$, $A^6$, $G^1$, $G^2$, $R^2$, and $R^3$ are as defined in the formula (I); and Q represents an optionally substituted a $C_2$-$C_{10}$ acyl group, an optionally substituted $C_2$-$C_{10}$ alkoxymethyl group or an optionally substituted benzyl group.

BEST MODE FOR WORKING THE INVENTION

The "acyclic aliphatic hydrocarbon group" in the present description contains a straight or branched acyclic aliphatic hydrocarbon group. It may be saturated so far as it is the acyclic aliphatic hydrocarbon group as well and may have one or more double bonds or triple bonds in a chemically possible range.

The "alkyl group" in the present description represents a straight or branched saturated acyclic aliphatic hydrocarbon group, for example methyl, ethyl, propyl, butyl, pentyl, hexyl, heptyl, octyl, isopropyl, isobutyl, s-butyl, t-butyl, isopentyl, neopentyl, t-pentyl, or isohexyl.

The "pyridyl group" in the present description contains N-oxyde thereof as well.

The term "cycloalkyl group" in the present description means a saturated alicyclic hydrocarbon group, for example cyclopropyl, cyclobutyl, or cyclohexyl.

The term "heterocyclic" in the present description is not particularly limited so far as it can chemically stably

exist if it is monocyclic to tricyclic having 1 to 4 atoms selected from among a group consisting of an oxygen atom, a nitrogen atom, and a sulfur atom, in the ring, but preferably monocyclic or bicyclic having carbon atoms not more than 9 containing 1 to 3, preferably 1 or 2 atoms selected from among a group consisting of an oxygen atom, a nitrogen atom, and a sulfur atom, in the ring.

In the formula (I), $A^1$ represents a single bond or represents a divalent acyclic aliphatic hydrocarbon group having 1 to 6 carbon atoms that links a nitrogen atom bonded to $A^1$ with $A^2$ on the same or different carbon atoms.

Examples of the acyclic aliphatic hydrocarbon group having 1 to 6 carbon atoms in $A^1$ include divalent groups obtained by removing two hydrogen atoms from methane, ethane, propane, butane, 2-methylpropane, pentane, 2-methylbutane, 2,2-dimethylpropane,' hexane, 2-methylpentane, 3-methylpentane, 2,2-dimethylbutane, 2,3-dimethylbutane and 2,2,3-trimethylpropane.'

Examples of suitable $A^1$ include $-CH_2-$, $-(CH_2)_2-$, $(CH_2)_3-$, $-(CH_2)_4-$, $-(CH_2)_5-$, $-(CH_2)_6-$, $-CH(CH_3)-$, $-CH (CH_3) CH_2-$, $-CH (CH_3)CH (CH_3)-$, $-C(CH_3)_2CH_2-$, $-CH (CH_3)(CH_2)_2-$, $-CH_2CH(CH_3)CH_2-$, $-CH (CH_3)CH (CH_3)CH_2-$, $-CH (CH_3) CH_2CH(CH_3)-$, $-CH_2C(CH_3)_2CH_2-$, $-CH (CH_3)C (CH_3)_2CH_2-$, $-CH (CH_2CH_3)(CH_2)_2-$, $-CH_2CH(CH_2CH_3)CH_2-$, $-CH (CH_2CH_3)CH (CH_3)CH_2-$, $-CH(CH_3)CH(CH_2CH_3)CH_2-$, $-CH(CH_2CH_3)CH_2CH(CH_3)-$, $-CH(CH_3)(CH_2)_3-$, $-CH_2CH (CH_3) (CH_2)_2-$, $-CH (CH_3)CH (CH_3) (CH_2)_2-$, $-CH(CH_3)CH_2CH(CH_3)CH_2-$, $-CH_2CH(CH_3)CH(CH_3)CH_2-$, $-CH_2C(CH_3)_2(CH_2)_2-$, $-CH (CH_3) C(CH_3)_2CH_2-$, $-CH (CH_2CH_3)(CH_2)_3-$, $-CH_2CH(CH_2CH_3)(CH_2)_2-$,$-CH(CH_3) (CH_2)_4-$, $-CH_2CH(CH_3) (CH_2)_3-$, and $-(CH_2)_2CH(CH_3)(CH_2)_2-$. Examples of preferred $A^1$ include $-CH_2-$, $-(CH_2)_2-$, $-(CH_2)_3-$, $-(CH_2)_4-$, $-CH(CH_3)CH_2-$, $-CH (CH_3)CH(CH_3)-$, $-CH(CH_3)(CH_2)_2-$, $-CH_2CH (CH_3) CH_2-$, and $-CH(CH_3)CH(CH_3)CH_2-$. More preferred examples of $A^1$ include $-CH_2-$, $-(CH_2)_2-$, and $-(CH_2)_3-$. As more preferable examples of $A^1$, $-(CH_2)_2-$ may be mentioned.

In the formula (I), $A^2$ represents a single bond or represents a group that links $A^1$ and $G^1$ in the form of $A^1$-C (=O)-$G^1$, $A^1$-C(=O)-O-$G^1$, $A^1$-C(=O)-NR$^{101}$-$G^1$, $A^1$-C(=S)-NR$^{102}$-$G^1$, $A^1$-C(=NR$^{103}$)-$G^1$, $A^1$-O-$G^1$, $A^1$-Q-C(=O)-$G^1$, $A^1$-NR$^{104}$-$G^1$, $A^1$-NR$^{105}$-C(=O)-$G^1$, $A^1$-NR$^{106}$-S(=O)$_2$-$G^1$, $A^1$-NR$^{107}$-C(=O)-O-$G^1$, $A^1$-NR$^{108}$-C(=O)-NR$^{109}$-$G^1$, $A^1$-NR$^{110}$-C(=S)-$G^1$, $A^1$-NR$^{111}$-C(=S)-NR$^{112}$-$G^1$, $A^1$-S-$G^1$, $A^1$-S(=O)-$G^1$, $A^1$-S(=O)$_2$-$G^1$, $A^1$-S(=O)$_2$-NR$^{113}$-$G^1$, $A^1$-CR$^{114}$=CH-$G^1$, $A^1$-CR$^{115}$=CF-$G^1$, $A^1$-CH=CR$^{116}$-$G^1$ or $A^1$-CF=CR$^{117}$-$G^1$ ($R^{101}$-$R^{117}$ are independently a hydrogen atom or a acyclic aliphatic hydrocarbon group having 1 to 4 carbon atoms).

When $A^1$ and $G^1$ are linked to each other in the form of $A^1$-C(=O)-NR$^{101}$-$G^1$, examples of the $C_1$-$C_4$ acyclic aliphatic hydrocarbon group of $R^{101}$ include methyl, ethyl, propyl, isopropyl, butyl, isobutyl, s-butyl, t-butyl, cyclopropylmethyl, 2-propenyl, 2-butenyl, 3-butenyl, 2-propynyl, 2-butynyl and 3-butynyl group. The $C_1$-$C_4$ acyclic aliphatic hydrocarbon group may also be substituted with one or more substituents selected from the group consisting of a fluorine atom, a chlorine atom, a bromine atom, an iodine atom, a hydroxy group, a methoxy group, an ethoxy group, an oxo group, a cyano group, a carboxyl group, a carbamoyl group, an amino group, a sulfo group, and'a phenyl group. Examples of preferred $R^{101}$ include a hydrogen atom, methyl, ethyl, and propyl group. Particularly, a hydrogen atom is preferred.

When $A^1$ and $G^1$ are linked to each other in the form of $A^1$-C(=S)-NR$^{102}$-$G^1$, examples of the $C_1$-$C_4$ aliphatic hydrocarbon group of $R^{102}$ include the same as those selected as the examples of $R^{101}$. Examples of preferred $R^{102}$ include a hydrogen atom, methyl, ethyl, and propyl group. Particularly, a hydrogen atom is preferred.

When $A^1$ and $G^1$ are linked to each other in the form of $A^1$-C(=NR$^{103}$)-$G^1$, examples of the $C_1$-$C_4$ acyclic aliphatic hydrocarbon group of $R^{103}$ include the same as those selected as the examples of $R^{101}$. Examples of preferred $R^{103}$ include a hydrogen atom; methyl, ethyl, and propyl group. Particularly, a hydrogen atom is preferred.

When $A^1$ and $G^1$ are linked to each other in the form of $A^1$-NR$^{104}$-$G^1$, examples of the $C_1$-$C_4$ acyclic aliphatic hydrocarbon group of $R^{104}$ include the same as those selected as the examples of $R^{101}$. Examples of preferred $R^{104}$ include a hydrogen atom, methyl, ethyl, and propyl group. Particularly, a hydrogen atom is preferred.

When $A^1$ and $G^1$ are linked to each other in the form of $A^1$-NR$^{105}$-C(=O)-$G^1$, examples of the $C_1$-$C_4$ acyclic aliphatic hydrocarbon group of $R^{105}$ include the same as those selected as the examples of $R^{101}$. Examples of preferred $R^{105}$ include a hydrogen atom, methyl, ethyl, and propyl group. Particularly, a hydrogen atom is preferred.

When $A^1$ and $G^1$ are linked to each other in the form of $A^1$-NR$^{106}$-S(=O)$_2$-$G^1$, examples of the $C_1$-$C_4$ acyclic aliphatic hydrocarbon group of $R^{106}$ include the same as those selected as the examples of $R^{101}$. Examples of preferred $R^{106}$ include a hydrogen atom, methyl, ethyl, and propyl group. Particularly, a hydrogen atom is preferred.

When $A^1$ and $G^1$ are linked to each other in the form of $A^1$-NR$^{107}$-C(=O)-O-$G^1$, examples of the $C_1$-$C_4$ acyclic aliphatic hydrocarbon group of $R^{107}$ include the same as those selected as the examples of $R^{101}$. Examples of preferred $R^{107}$ include a hydrogen atom, methyl, ethyl, and propyl group. Particularly, a hydrogen atom is preferred.

When $A^1$ and $G^1$ are linked to each other in the form of $A^1$-NR$^{108}$-C(=O)NR$^{109}$-$G^1$, examples of such preferred $C_1$-$C_4$ acyclic aliphatic hydrocarbon group of $R^{108}$ and $R^{109}$ include the same as those selected as the examples of $R^{101}$. Examples of preferred $R^{108}$ and $R^{109}$ include a hydrogen atom, methyl, ethyl, and propyl group. Particularly, a hydrogen atom is preferred.

When $A^1$ and $G^1$ are linked to each other in the form of $A^1$-NR$^{110}$-C(=S)-$G^1$, examples of such preferred $C_1$-$C_4$ acyclic aliphatic hydrocarbon group of $R^{110}$ include the same as those selected as the examples of $R^{101}$. Examples of preferred $R^{110}$ include a hydrogen atom, methyl, ethyl, and propyl group. Particularly, a hydrogen atom is preferred.

When $A^1$ and $G^1$ are linked to each other in the form of $A^1$-NR$^{111}$-C(=S)-NR$^{112}$-$G^1$, examples of the $C_1$-$C_4$

acyclic aliphatic hydrocarbon group of $R^{111}$ and $R^{112}$ include the same as those selected as the examples of $R^{101}$. Examples of preferred $R^{111}$ and $R^{112}$ include a hydrogen atom, methyl, ethyl, and propyl group. Particularly, a hydrogen atom is preferred.

When $A^1$ and $G^1$ are linked to each other in the form of $A^1$-S(=O)$_2$-NR$^{113}$-$G^1$, examples of the $C_1$-$C_4$ acyclic aliphatic hydrocarbon group of $R^{113}$ include the same as those selected as the examples of $R^{101}$. Examples of preferred $R^{113}$ include a hydrogen atom, methyl, ethyl, and propyl group. Particularly, a hydrogen atom.is preferred.

When $A^1$ and $G^1$ are linked to each other in the form of $A^1$-CR$^{114}$=CR$^{115}$-$G^1$, examples of the $C_1$-$C_4$ acyclic aliphatic hydrocarbon group of $R^{114}$ and $R^{115}$ include the same as those selected as the examples of $R^{101}$. Examples of preferred $R^{114}$ and $R^{115}$ include a hydrogen atom, methyl, ethyl, and propyl group. Particularly, a hydrogen atom is preferred.

When $A^1$ and $G^1$ are linked to each other in the form of $A^1$-CF=CR$^{117}$-$G^1$, examples of the $C_1$-$C_4$ acyclic aliphatic hydrocarbon group of $R^{117}$ include the same as those selected as the examples of $R^{101}$. Examples of preferred $R^{117}$ include a hydrogen atom, methyl, ethyl, and propyl group. Particularly, a hydrogen atom is preferred.

When $A^1$ and $G^1$ are linked to each other in the form of $A^1$-CF=CR$^{117}$-$G^1$, examples of the $C_1$-$C_4$ acyclic aliphatic hydrocarbon group of $R^{117}$ include the same as those selected as the examples of $R^{101}$. Examples of preferred $R^{117}$ include a hydrogen atom, methyl, ethyl, and propyl group. Particularly, a hydrogen atom is preferred.

Examples of preferred $A^2$ include groups that link $A^1$ and $G^1$ in the form of $A^1$-C(=O)-$G^1$, $A^1$-C(=O)-NR$^{101}$-$G^1$, $A^1$-O-$G^1$, $A^1$-NR$^{104}$-$G^1$, $A^1$-NR$^{105}$-C(=O)-$G^1$, $A^1$-NR$^{108}$-C(=O)-NR$^{109}$-$G^1$, $A^1$-NR$^{110}$-C(=S)-$G^1$ and $A^1$-NR$^{111}$-C(=S) NR$^{112}$-$G^1$, especially preferably in the form of $A^1$-C(=O)-$G^1$, $A^1$-C(=O)-NR$^{101}$-$G^1$, $A^1$-NR$^{104}$-$G^1$, $A^1$-NR$^{105}$-C(=O)-$G^1$, $A^1$-NR$^{108}$-C(=O)-NR$^{109}$-$G^1$, and $A^1$-NR$^{110}$-C(=S)-$G^1$. Among them, examples of more preferred $A^2$ include groups that link $A^1$ and $G^1$ in the form of $A^1$-C(=O)-NR$^{101}$-$G^1$, $A^1$-NR$^{105}$-C(=O)-$G^1$, and $A^1$-NR$^{108}$-C(=O)-NR$^{109}$-$G^1$. Here, forms of linkage exemplified as preferred and more preferred $A^2$ are preferably combined with structures in which $A^1$ exists in the form of -$(CH_2)_2$- or -$(CH_2)_3$- in the formula (I).

In the formula (I), $A^3$ represents a single bond or represents an optionally substituted divalent acyclic aliphatic hydrocarbon group having 1 to 10 carbon atoms that links $G^1$ and $A^4$ on the same or different carbon atoms.

Examples of the acyclic aliphatic hydrocarbon group having 1 to 10 carbon atoms of $A^3$ include, in addition to the same as those selected as the examples of $A^1$, -CH=CH-, -C($CH_3$)=CH-, -C($CH_3$)=C($CH_3$)-, -C($CH_2CH_3$)=CH-, -C ($CH_2CH_3$)=C ($CH_3$)-, -C($CH_2CH_3$)=C ($CH_2CH_3$)-, -C ($CH_2CH_2CH_3$)=CH-, -C($CH_2CH_2CH_3$)=C ($CH_3$)-, -CH=CHCH$_2$-, -C ($CH_3$)=CHCH$_2$-, -CH=C($CH_3$)CH$_2$-, -CH=CHCH($CH_3$)-, -C($CH_3$)=C($CH_3$)CH$_2$-, -C($CH_3$)=CHCH($CH_3$)-, -C($CH_3$)=C($CH_3$) CH($CH_3$)-, -C($CH_3$)=CHC($CH_3$)$_2$-, -C($CH_2CH_3$)=CHCH$_2$-, -CH=C($CH_2CH_3$)CH$_2$-, -CH=CHCH ($CH_2CH_3$)-, -C($CH_2CH_3$) =C($CH_3$)CH$_2$-, -C($CH_2CH_3$)=CHCH($CH_3$)-, -C($CH_3$)=C($CH_2CH_3$)GH$_2$-, -CH=C($CH_2CH_3$)CH($CH_3$)-, -CH=CHCH ($CH_2CH_3$)-, -C($CH_3$)=CHCH($CH_2CH_3$)-, -CH=C($CH_3$)CH($CH_2CH_3$)-, -CH=CH($CH_2$)$_2$-, -C($CH_3$)=CH($CH_2$)$_2$-, -CH=C ($CH_3$)($CH_2$)$_2$-, -CH=CHC($CH_3$)CH$_2$-, -C H=CHCH$_2$CH($CH_3$)-, -C($CH_3$)=C($CH_3$)($CH_2$)$_2$-, -C ($CH_3$)=CHCH($CH_3$)CH$_2$-, -C ($CH_3$)=CHCH$_2$CH($CH_3$)-, -CH$_2$CH=CHCH$_2$-, -CH ($CH_3$)CH=CHCH$_2$-, -CH$_2$C($CH_3$)=CHCH$_2$-, -CH ($CH_3$)C ($CH_3$) =CHCH$_2$-, -CH($CH_3$)CH=CHCH($CH_3$)-, -CH($CH_3$)CH=C($CH_3$)CH$_2$-, -CH$_2$C($CH_3$)=C ($CH_3$)CH$_2$-, -CH($CH_2CH_3$) CH=CHCH$_2$-, and -CH$_2$C($CH_2CH_3$)=CHCH$_2$-.

Substituents of divalent acyclic aliphatic hydrocarbon group having 1 to 10 carbon atoms of $A^3$ include a hydrocarbon group having 1 to 6 carbon atoms, an alicyclic hydrocarbon group having 3 to 6 carbon atoms, a halogen atom, an alkoxy group having 1 to 6 carbon atoms, a phenoxy group, an amino group, or an alkyl amino group having 1 to 6 carbon atoms.

Examples of such preferred $A^3$ include a single bond, -CH$_2$-, -$(CH_2)_2$-, -$(CH_2)_3$-, -$(CH_2)_4$-, -CH($CH_3$)CH$_2$-, -CH ($CH_3$)CH($CH_3$)-, -CH($CH_3$)($CH_2$)$_2$-, -CH=CH- and -CH=CHCH$_2$-. Further, examples of more preferred $A^3$ include a single bond, -CH$_2$-, -$(CH_2)_2$- and -$(CH_2)_3$-. The same applies when $A^3$ is substituted, but a single bond is excluded.

In the formula (I), $A^4$ represents a single bond or represents a group that links $A^3$ and $G^2$ in the form of $A^3$-C (=O)-$G^2$, $A^3$-C(=O)-O-$G^2$, $A^3$-C(=O)-NR$^{121}$-$G^2$, $A^3$-C(=S)-NR$^{122}$-$G^2$, $A^3$-C(=NR$^{123}$)-$G^2$, $A^3$-O-$G^2$, $A^3$-O-C(=O)-$G^2$, $A^3$-NR$^{124}$-$G^2$, $A^3$-NR$^{125}$-C(=O)-$G^2$, $A^3$-NR$^{126}$-S(=O)$_2$-$G^2$, $A^3$-NR$^{127}$-C(=O)-O-$G^2$, $A^3$-NR$^{128}$-C(=O)-NR$^{129}$-$G^2$, $A^3$-NR$^{130}$-C(=S)-$G^2$, $A^3$-NR$^{131}$-C(=S)-NR$^{132}$-$G^2$, $A^3$-S-$G^2$, $A^3$-S(=O)-$G^2$, $A^3$-S(=O)$_2$-$G^2$, $A^3$-S(=O)$_2$-NR$^{133}$-$G^2$ or $A^3$-S (=O)$_2$-O-$G^2$ (in which $R^{121}$ through $R^{133}$ are each independently a hydrogen atom or a acyclic aliphatic hydrocarbon group having 1 to 4 carbon atoms).

When $A^3$ and $G^2$ are linked to each other in the form of $A^3$-C(=O)-NR$^{121}$-$G^2$' examples the $C_1$-$C_4$ acyclic aliphatic hydrocarbon group of $R^{121}$ include the same as those selected as the examples of $R^{101}$ in $A^2$. Examples of preferred $R^{121}$ include a hydrogen atom, methyl, ethyl, and propyl group. Particularly, a hydrogen atom is preferred.

When $A^3$ and $G^2$ are linked to each other in the form of $A^3$-C(=S)-NR$^{122}$-$G^2$, examples of the $C_1$-$C_4$ acyclic aliphatic hydrocarbon group of $R^{122}$ include the same as those selected as the examples of $R^{101}$ in $A^2$. Examples of preferred $R^{122}$ include a hydrogen atom, methyl, ethyl, and propyl group. Particularly, a hydrogen atom is preferred.

When $A^3$ and $G^2$ are linked to each other in the form of $A^3$-C(=NR$^{123}$)-$G^2$, examples of the $C_1$-$C_4$ acyclic, aliphatic hydrocarbon group of $R^{123}$ include the same as those selected as the examples of $R^{101}$ in $A^2$. Examples of preferred $R^{123}$ include a hydrogen atom, methyl, ethyl, and propyl group. Particularly, a hydrogen atom is preferred.

When $A^3$ and $G^2$ are linked to each other in the form of $A^3$-$NR^{124}$-$G^2$, examples of the $C_1$-$C_4$ acyclic aliphatic hydrocarbon group of $R^{124}$ include the same as those selected as the examples of $R^{101}$ in $A^2$. Examples of preferred $R^{124}$ include a hydrogen atom, methyl, ethyl, and propyl group. Particularly, a hydrogen atom is preferred.

When $A^3$ and $G^2$ are linked to each other in the form of $A^3$-$NR^{125}$=C(=O)-$G^{2'}$ examples of the $C_1$-$C_4$ acyclic aliphatic hydrocarbon group of $R^{125}$ include the same as those selected as the examples of $R^{101}$ in $A^2$. Examples of preferred $R^{125}$ include a hydrogen atom, methyl, ethyl, and propyl group. Particularly, a hydrogen atom is preferred.

When $A^3$ and $G^2$ are linked to each other in the form of $A^3$-$NR^{126}$-$S(=O)_2$-$G^{2'}$ examples of the $C_1$-$C_4$ acyclic aliphatic hydrocarbon group of $R^{126}$ include the same as those selected as the examples of $R^{101}$ in $A^2$. Examples of preferred $R^{126}$ include a hydrogen atom, methyl, ethyl, and propyl group. Particularly, a hydrogen atom is preferred.

When $A^3$ and $G^2$ are linked to each other in the form of $A^3$-$NR^{127}$-C(=O)-O-$G^2$, examples of the $C_1$-$C_4$ acyclic aliphatic hydrocarbon group of $R^{127}$ include the same as those selected as the examples of $R^{101}$ in $A^2$. Examples of preferred $R^{127}$ include a hydrogen atom, methyl, ethyl, and propyl group. Particularly, a hydrogen atom is preferred.

When $A^3$ and $G^2$ are linked to each other in the form of $A^3$-$NR^{128}$-C(=O)-$NR^{129}$-$G^2$, examples of the $C_1$-$C_4$ acyclic aliphatic hydrocarbon group of $R^{128}$ and $R^{129}$ include the same as those selected as the examples of $R^{101}$ in $A^2$. Examples of preferred $R^{128}$ and $R^{129}$ include a hydrogen atom, methyl, ethyl, and propyl group. Particularly, a hydrogen atom is preferred.

When $A^3$ and $G^2$ are linked to each other in the form of $A^3$-$NR^{130}$-C(=S)-$G^2$, examples of the $C_1$-$C_4$ acyclic aliphatic hydrocarbon group of $R^{130}$ include the same as those selected as the examples of $R^{101}$ in $A^2$. Examples of preferred $R^{130}$ include a hydrogen atom, methyl, ethyl, and propyl group. Particularly, a hydrogen atom is preferred.

When $A^3$ and $G^2$ are linked to each other in the form of $A^3$-$NR^{131}$-C(=S)-$NR^{132}$-$G^2$, examples of the $C_1$-$C_4$ acyclic aliphatic hydrocarbon group of $R^{131}$ and $R^{132}$ include the same as those selected as the examples of $R^{101}$ in $A^2$. Examples of preferred $R^{131}$ and $R^{132}$ include a hydrogen atom, methyl, ethyl, and propyl group. Particularly, a hydrogen atom is preferred.

When $A^3$ and $G^2$ are linked to each other in the form of $A^3$-$S(=O)_2$-$NR^{133}$-$G^2$, examples of the $C_1$-$C_4$ acyclic aliphatic hydrocarbon group of $R^{133}$ include the same as those selected as the examples of $R^{101}$ in $A^2$. Examples of preferred $R^{133}$ include a hydrogen atom, methyl, ethyl, and' propyl group. Particularly, a hydrogen atom is preferred.

Examples of such $A^4$ include a single bond and a group that links $A^3$ and $G^2$ in the form of $A^3$-C(=O)-$G^2$, $A^3$-C(=O)-O-$G^2$, $A^3$-C(=O)-$NR^{121}$-$G^2$, $A^3$-O-$G^2$, $A^3$-$NR^{124}$-$G^2$, $A^3$-$NR^{125}$-C(=O)-$G^2$, $A^3$-$S(=O)_2$-$G^2$ or $A^3$-$S(=O)_2$-O-$G^2$.

In the formula (I), $G^1$ represents a single bond or a divalent group obtained by removing two hydrogen atoms from any of groups consisting of a substituted or unsubstituted alicyclic hydrocarbon group having 3 to 10 carbon atoms, a substituted or unsubstituted aromatic hydrocarbon group having 6 to 14 carbon atoms, and a heterocyclic compound having 1 to 4 atoms selected from the group consisting of an oxygen atom, a nitrogen atom and a sulfur atom on its substituted or unsubstituted ring.

In the formula (I), when $G^1$ represents a substituted or unsubstituted divalent alicyclic hydrocarbon group having 3 to 10 carbon atoms, examples of the alicyclic hydrocarbon group having 3 to 10 carbon atoms include cyclopropane, cyclobutane, cyclopentane, cyclopentene, cyclohexane, cyclohexene, cycloheptane, cycloheptene, cyclooctane, bicyclo [2.2.1] heptane, bicyclo[2. 2. 1]heptene, bicyclo[3. 1. 1]heptane and bicyclo[2. 2. 2]octane. Examples of such preferred $C_3$-$C_{10}$ alicyclic hydrocarbon of $G^1$ include monocyclic alicyclic hydrocarbon group having 3 to 6 carbon atoms such as cyclopropane, cyclopentane, cyclohexane, cyclohexane and the like.

Examples of the substituent for the substituted alicyclic hydrocarbon group having 3 to 10 carbon atoms of $G^1$ include: a fluorine atom, a chlorine atom, a bromine atom, an iodine atom, a hydroxy group, methoxy, ethoxy, propoxy, isopropoxy, butoxy, isobutoxy, s-butoxy, t-butoxy, pentyloxy, isopentyloxy, neopentyloxy, t-pentyloxy, hexyloxy, isohexyloxy, 2-methyl- pentyloxy, 1-ethylbutoxy, cyclopropyloxy, cyclobutyloxy, cyclopentyloxy, cyclohexyloxy, cyclopropylmethyloxy, cyclopropylethyloxy, cyclopentylmethyloxy and cyclohexylmethyloxy or another $C_1$-$C_7$ alkoxy group consisting of a straight or branched alkyl, cycloalkyl and oxy group, ethylene dioxy or another $C_1$-$C_4$ alkylenedioxy group, phenoxy, 1-naphthoxy and 2-naphthoxy or another $C_6$-$C_{10}$ aryloxy group, benzyloxy, α-phenethyloxy, β-phenethyloxy and phenylpropyloxy or another $C_7$-$C_9$ aralkoxy group, acetoxy, propionyloxy, butyryloxy, isobutyryloxy, valeryloxy, isovaleryloxy, pivaloyloxy and hexanoyloxy or another $C_2$-$C_7$ acyloxy group, oxo, methylsulfonyloxy, ethylsulfonyloxy, propylsulfonyloxy, butylsulfonyloxy and t-butylsulfonyloxy or another $C_1$-$C_6$ alkylsulfonyloxy group consisting of a straight or branched alkyl and sulfonyloxy, acetyl, propionyl, butyryl, isobutyryl, valeryl, isovaleryl, pivaroyl and hexanoyl or another $C_2$-$C_7$ acyl group, carboxy, methoxycarbonyl, ethoxycarbonyl, propoxycarbonyl, isopropoxycarbonyl, butoxycarbonyl, isobutoxycarbonyl, s-butoxycarbonyl and t-butoxycarbonyl or another $C_2$-$C_7$ alkoxycarbonyl group consisting of a straight or branched alkyl and oxycarbonyl group, carbamoyl, N-methylcarbamoyl, N-ethylcarbamoyl, N-propylcarbamoyl, N-isopropylcarbamoyl, N-butylcarbamoyl, N-isobutylcarbamoyl, N-s-butylcarbamoyl, N-t-butylcarbamoyl, N-pentylcarbamoyl, N-cyclopropylcarbamoyl, N-cyclobutylcarbamoyl, N-cyclopentylcarbamoyl, N-cyclohexylcarbamoyl, N-cycloheptylcarbamoyl, N-cyclopropylmethylcarbamoyl, N,N-dimethylcarbamoyl, N-ethyl-N-methylcarbamoyl, N,N-diethylcarbamoyl and N,N-dipropylcarbamoyl or another $C_2$-$C_7$ alkylcarbamoyl group consisting of a straight or branched alkyl, cycloalkyl and carbamoyl group, amino, methylamino, ethylamino, propylamino, isopropylamino, butylamino, isobutylamino, s-butylamino, t-butylamino, pentylamino, hexylamino, cyclopropylamino, cyclobutylamino, cyclopentylamino, cyclohexylamino, cyclopropylmethylamino, dimethylamino, N-ethylmethylamino, diethylamino, N-methylpropylamino, N-methylisopropylamino, N-methylbutyl- amino, N-methyl-t-butylamino, N-ethylisopropylamino, dipropylamino, diisopropylamino and ethylbutylamino or another $C_1$-$C_6$ alkylamino group consisting of a straight or branched alkyl, cycloalkyl and amino group, acetylamino, propionylamino, butyrylamino, isobutyrylamino, valerylamino and hexanoylamino or another $C_2$-$C_7$ acylamino group, methoxycarbonylamino, ethoxycarbonylamino and t-butoxycarbonylamino or another $C_2$-$C_8$ alkoxycarbonylamino group, methylsulfonylamino, ethylsulfonylamino, butylsulfonylamino and t-butylsulfonylamino or another $C_{1-}C_6$ alkylsulfonylamino group, a cyano group, a nitro group, methylthio, ethylthio, propylthio, isopropylthio, butylthio, isobutylthio, s-butylthio, t-butylthio, pentylthio and hexylthio or another $C_1$-$C_6$ alkylthio group, methylsulfynyl, ethylsulfynyl, propylsulfynyl, isopropylsulfynyl, butylsulfynyl, isobutylsulfynyl, s-butylsulfynyl, t-butylsulfynyl, pentylsulfynyl and cyclopentylsulfynyl or another $C_1$-$C_6$ alkylsulfynyl group consisting of a straight or branched alkyl, cycloalkyl and sulfynyl group, methylsulfonyl, ethylsulfonyl, propylsulfonyl, isopropylsulfonyl, butyl- sulfonyl, isobutylsulfonyl, s-butylsulfonyl, t-butylsulfonyl, pentylsulfonyl, hexylsulfonyl, cyclopentylsulfonyl and cyclohexylsulfonyl or another $C_1$-$C_6$ alkylsulfonyl group consisting of a straight or branched alkyl, cycloalkyl and sulfonyl group, a sulfo group, a sulfamoyl group, methylaminosulfonyl, ethylaminosulfonyl, propylaminosulfonyl, isopropylaminosulfonyl, butylaminosulfonyl, isobutylamino- sulfonyl, s-butylaminosulfonyl, pentylaminosulfonyl, dimethylaminosulfonyl, N-ethyl-N-methylaminosulfonyl, diethylaminosulfonyl, dipropylaminosulfonyl, cyclopropylaminosulfonyl, cyclopentylaminosulfonyl, cyclohexylaminosulfonyl and cyclopropylmethylaminosulfonyl or another $C_{1-}C_6$ aminosulfonyl group consisting of a straight or branched alkyl, a cycloalkyl and aminosulfonyl group, cyclopropyl, cyclobutyl, cyclopentyl and cyclohexyl or another alicyclic hydrocarbon group having 3 to 6 carbon atoms, methyl, ethyl, vinyl, ethynyl, propyl, 1-propenyl, 2-propenyl, isopropyl, isopropenyl, 1-propynyl, 2-propynyl, butyl, isobutyl, s-butyl, t-butyl, 1-butenyl, 2-butenyl, 3-butenyl, 1-methyl-1-propenyl, 2-methyl-1-propenyl, 1-methyl-2-propenyl, 2-methyl-2- propenyl, 1-butynyl, 2-butynyl, pentyl, isopentyl, neopentyl, t-pentyl, 1-pentenyl, 2-pentenyl, 3-pentenyl, 4-pentenyl, hexyl, 5-hexenyl, 4-methyl-3-pentenyl, isohexyl, 2-methylpentyl and 1-ethylbutyl or another acyclic aliphatic hydrocarbon group having 1 to 6 carbon atoms which may contain a straight or branched unsaturated bond.

As the substituent of the substituted alicyclic hydrocarbon group having 3 to 10 carbon atoms as $G^1$, a $C_{1-}C_7$ alkoxy group, a $C_2$-$C_7$ acyl group, a $C_2$-$C_7$ alkylcarbamoyl group, a $C_1$-$C_6$ alkylamino group, a $C_2$-$C_7$ acylamino group, an alicyclic hydrocarbon group having 3 to 6 carbon atoms or an acyclic aliphatic hydrocarbon group having 1 to 6 carbon atoms, may further be substituted with (one or more substituents selected from the group consisting of a fluorine atom; a chlorine atom; a bromine atom; an iodine atom; a hydroxy group; a $C_1$-$C_6$ alkoxy group such as methoxy, ethoxy, propoxy, isopropoxy, butoxy, isobutoxy, s-butoxy, t-butoxy, pentyloxy or cyclopropyloxy; a $C_2$-$C_7$ acyl group such as methoxymethyloxy group, 2-methoxyethoxy group, formyl group, trifluoroacetyl group, acetyl, propionyl, butyryl, isobutyryl, valeryl or isovaleryl; an oxo group; a carboxyl group; a $C_2$-$C_7$ alkoxycarbonyl group such as methoxycarbonyl, ethoxycarbonyl, propoxycarbonyl, isopropoxycarbonyl, butoxycarbonyl, isobutoxycarbonyl or t-butoxycarbonyl; a carbamoyl group; a $C_2$-$C_7$ alkylcarbamoyl group such as N-methylcarbamoyl, N,N-dimethylcarbamoyl, N-ethylcarbamoyl, N-ethyl-N-methylcarbamoyl, N,N-diethyl- carbamoyl, N-propylcarbamoyl, N-isopropylcarbamoyl, N-butylcarbamoyl, N-cyclopropylcarbamoyl or N-cyclopropyl-methylcarbamoyl; an amino group; a $C_1$-$C_6$ alkylamino group such as methylamino, ethylamino, propylamino, isopropylamino, dimethylamino, N-ethylmethylamino, diethylamino, N-methyl- propylamino, N-methylisopropylamino, cyclopropylamino or cyclopropylmethylamino; a $C_4$-$C_6$ cyclic amino group having 1 or 2 atoms selected from the group consisting of an oxygen atom, a nitrogen atom and a sulfur atom, in the ring, such as 1-pyrrolidinyl, piperazinyl, 4-methylpiperazinyl, piperidino or morpholino; a $C_1$-$C_7$ acylamino group such as trifluoroacetylamino group, formylamino, acetylamino, propionylamino, butyrylamino, isobutyrylamino or valerylamino; a $C_1$-$C_6$ alkylsulfonylamino group such as methylsulfonylamino, ethylsulfonylamino, propylsulfonylamino or butylsulfonylamino; a nitro group; and a cyano group).

In the formula (I), when $G^1$ represents a substituted or unsubstituted divalent aromatic hydrocarbon group having 6 to 14 carbon atoms, examples of the aromatic hydrocarbon group having 6 to 14 carbon atoms include a compound having at least one aromatic ring on its molecule, such as benzene, indene, indane, naphthalene, 1,2-dihydronaphthalene, 1,2,3,4-tetrahydronaphthalene, azulene, acenaphthylene, acenaphthene, fluorene, phenanthrene or anthracene.

Examples of such preferred aromatic hydrocarbon group having 6 to 14 carbon atoms of $G^1$ include benzene, naphthalene and indane. Examples of more preferred aromatic hydrocarbon group having 6 to 14 carbon atoms of $G^1$ include benzene.

Exemplary substituents of the substituted aromatic hydrocarbon group having 6 to 14 carbon atoms of $G^1$ include a fluorine atom, a chlorine atom, a bromine atom, an iodine atom, a hydroxy group, an optionally substituted $C_1$-$C_7$ alkoxy group, a $C_6$-$C_{10}$ aryloxy group, a $C_7$-$C_9$ aralkoxy group, a $C_2$-$C_7$ acyloxy group, an oxo group, a $C_1$-$C_6$ alkylsulfonyloxy group, an optionally substituted $C_2$-$C_7$ acyl group, a carboxyl group, a $C_2$-$C_7$ alkoxycarbonyl group, a carbamoyl group, an optionally substituted $C_2$-$C_7$ alkylcarbamoyl group, an amino group, an optionally substituted $C_1$-$C_6$ alkylamino group, an optionally substituted $C_2$-$C_7$ acylamino group, a $C_2$-$C_8$ alkoxycarbonylamino group, a $C_1$-$C_6$ alkyl-

sulfonylamino group, a cyano group, a nitro group, a $C_1$-$C_6$ alkylthio group, a $C_1$-$C_6$ alkylsulfynyl group, a $C_1$-$C_6$ alkylsulfonyl group, a sulfamoyl group, a $C_1$-$C_6$ alkylaminosulfonyl group, a sulfo group, an optionally substituted alicyclic hydrocarbon group having 3 to 6 carbon atoms and an optionally substituted acyclic aliphatic hydrocarbon group having 1 to 6 carbon atoms.

Specific examples of the substituent of the substituted aromatic hydrocarbon group having 6 to 14 carbon atoms of $G^1$ include the same as those specifically exemplified as the substituents of the substituted alicyclic hydrocarbon group having 3 to 10 carbon atoms of $G^1$.

As the substituent of the substituted aromatic hydrocarbon group having 6 to 14 carbon atoms, the $C_1$-$C_7$ alkoxy group, the $C_2$-$C_7$ acyl group, the $C_2$-$C_7$ alkylcarbamoyl group, the $C_1$-$C_6$ alkylamino group, the $C_2$-$C_7$ acylamino group, the alicyclic hydrocarbon group having 3 to 6 carbon atoms or the aliphatic acyclic hydrocarbon group having 1 to 6 carbon atoms, may further be substituted with (one or more substituents selected from the group consisting of a fluorine atom; a chlorine atom; a bromine atom; an iodine atom; a hydroxy group; a $C_1$-$C_6$ alkoxy group such as methoxy, ethoxy, propoxy, isopropoxy, butoxy, isobutoxy, s-butoxy, t-butoxy, pentyloxy or cyclopropyloxy; a methoxymethyloxy group; a 2-methoxyethoxy group; a formyl group; a trifluoroacetyl group; a $C_2$-$C_7$ acyl group such as acetyl, propionyl, butyryl, isobutyryl, valeryl or isovaleryl; an oxo group; a carboxyl group; a $C_2$-$C_7$ alkoxycarbonyl group such as methoxycarbonyl, ethoxycarbonyl, propoxycarbonyl, isopropoxycarbonyl, butoxycarbonyl, isobutoxycarbonyl or t-butoxycarbonyl; a carbamoyl group; a $C_2$-$C_7$ alkylcarbamoyl group such as N-methylcarbamoyl, N,N-dimethylcarbamoyl, N-ethylcarbamoyl, N-ethyl-N-methylcarbamoyl, N,N-diethyl- carbamoyl, N-propylcarbamoyl, N-isopropylcarbamoyl, N-butylcarbamoyl, N-cyclopropylcarbamoyl or N-cyclopropylmethylcarbamoyl; an amino group; a $C_1$-$C_6$ alkylamino group such as methylamino, ethylamino, propylamino, isopropylamino, dimethylamino, N-ethylmethylamino, diethylamino, N-methylpropylamino, N-methylisopropylamino, cyclopropylamino or cyclopropylmethylamino; a $C_4$-$C_6$ cyclic amino group having 1 or 2 atoms selected from the group consisting of an oxygen atom, a nitrogen atom and a sulfur atom, in the ring, such as 1-pyrrolidinyl, piperazinyl, 4-methylpiperazinyl, piperidino or morpholino; a trifluoroacetylamino group; a $C_1$-$C_7$ acylamino group such as formylamino, acetylamino, propionylamino, butyrylamino, isobutyrylamino or valerylamino; a $C_1$-$C_6$ alkylsulfonylamino group such as methylsulfonylamino, ethylsulfonylamino, propylsulfonylamino or butylsulfonylamino; a nitro group; and a cyano group).

Preferred examples of the substituents of the substituted aromatic hydrocarbon group having 6 to 14 carbon atoms of $G_1$ include a fluorine atom; a chlorine atom; a bromine atom; a $C_1$-$C_6$ alkoxy group consisting of a straight or branched alkyl and oxy group, including methoxy, ethoxy, propoxy, isopropoxy, butoxy, isobutoxy, s-butoxy, t-butoxy, pentyloxy, isopentyloxy, neopentyloxy, t-pentyloxy and hexyloxy; a cyano group; a nitro group; a carboxyl group; a hydroxy group; an amino group; a $C_1$-$C_6$ mono or dialkylamino group consisting of a straight or branched alkyl and amino group, including methylamino, ethylamino, propylamino, isopropylamino, butylamino, isobutylamino, s-butylamino, t-butylamino, pentylamino, hexylamino, dimethylamino, N-ethylmethylamino, diethylamino, N-methylpropylamino, N-methylisopropylamino, N-methylbutyl- amino, N-methyl-t-butylamino, N-ethylisopropylamino, dipropylamino, diisopropylamino and ethylbutylamino; a carbamoyl group; an aminosulfonyl group; an alicyclic hydrocarbon group having 3 to 6 carbon atoms, including cyclopropyl, cyclobutyl, cyclopentyl and cyclohexyl; a $C_2$-$C_7$ acyl group including acetyl, propionyl, butyryl, isobutyryl, pivaroyl and hexanoyl; a $C_1$-$C_6$ alkylthio group including methylthio, ethylthio, propylthio, isopropylthio, butylthio, isobutylthio, s-butylthio, t-butylthio, pentylthio and hexylthio; a $C_1$-$C_6$ alkylsulfonyl group including methylsulfonyl, ethylsulfonyl, propylsulfonyl, isopropylsulfonyl, butylsulfonyl, isobutylsulfonyl, s-butylsulfonyl, t-butylsulfonyl, pentylsulfonyl and hexylsulfonyl; a $C_2$-$C_7$ alkoxycarbonyl group including acetoxy, propionyloxy, butyryloxy, isobutyryloxy, valeryloxy, isovaleryloxy, pivaloyloxy and hexanoyloxy; a $C_2$-$C_7$ acylamino group including acetylamino, propionylamino, butyrylamino, isobutyrylamino, valerylamino and hexanoylamino; trifluoromethyl group; trifluoromethoxy group;and an acyclic aliphatic hydrocarbon group having 1 to 6 carbon atoms which may contain a.straight or branched unsaturated bond including methyl, ethyl, vinyl, ethynyl, propyl, 1-propenyl, 2-propenyl, isopropyl, isopropenyl, 1-propynyl, 2-propynyl, butyl, isobutyl, s-butyl, t-butyl, 1-butenyl, 2-butenyl, 3-butenyl, 1-methyl-1-propenyl, 2-methyl-1- propenyl, 1-methyl-2-propenyl, 2-methyl-2-propenyl, 1-butynyl, 2-butynyl, pentyl, isopentyl, neopentyl, t-pentyl, 1-pentenyl, 2-pentenyl, 3-pentenyl, 4-pentenyl, hexyl, 5-hexenyl, 4-methyl-3-pentenyl, isohekyl, 2-methylpentyl and 1-ethylbutyl.

Specifically, examples of more preferred substituents of the substituted aromatic hydrocarbon group having 6 to 14 carbon atoms include a fluorine atom, a chlorine atom, a bromine atom, $C_1$-$C_6$ alkoxy group, cyano group, a nitro group, a carboxyl group, a hydroxy group, an amino group, a $C_1$-$C_6$ mono or dialkylamino group, a carbamoyl group, an alicyclic hydrocarbon group having 3 to 6 carbon atoms, a $C_2$-$C_7$ acyl group, a $C_1$-$C_6$ alkylsulfonyl group, a $C_2$-$C_7$ alkoxycarboxyl group, trifluoromethyl group, trifluoromethoxy group, and a $C_1$-$C_6$ alkyl group including methyl, ethyl, propyl, isopropyl, butyl, isobutyl, s-butyl, t-butyl, pentyl, isopentyl, neopentyl, t-pentyl, hexyl, isohexyl, 2-methylpentyl and 1-ethylbutyl. Examples of particularly preferred substituents include a fluorine atom, a chlorine atom, a $C_1$-$C_6$ alkoxy group, a cyano group, a nitro group, a carboxyl group, a hydroxy group, an amino group, a $C_1$-$C_6$ mono or dialkylamino group, an alicyclic hydrocarbon group having 3 to 6 carbon atoms, a $C_2$-$C_7$ acyl group, a trifluoromethyl group, a trifluoromethoxy group and a $C_{1}$-$C_6$ alkyl group.

In the formula (I), when $G^1$ represents a divalent group derived from heterocyclic compounds having 1 to 4

atoms selected from the group consisting of an oxygen atom, a nitrogen atom and a sulfur atom on its substituted or unsubstituted ring, examples of such heterocyclic compounds include monocyclic, bicyclic or tricyclic heterocyclic compounds, such as furan, thiophene, pyrrole, pyrroline, pyrrolidine, oxazole, oxazolidine, isooxazole, isooxazolidine, thiazole, thiazolidine, isothiazole, isothiazolidine, furazan, imidazole, imidazoline, imidazolidine, pyrazole, pyrazoline, pyrazolidine, triazole, thiadiazole, oxadiazole, tetrazole, pyran, tetrahydropyran, thiopyran, tetrahydrothiopyran, tetrahydrofuran, 1,3-dioxolane, 1,4-dioxane, pyridine, pyrazine, pyrimidine, pyridazine, benzofuran, dibenzofuran, 1, 4-dioxacycloheptane, benzothiophene, indole, 1,2-methylene- dioxybenzene, benzimidazole, benzothiazole, benzooxazole, chroman, isochroman, quinoline, decahydroquinoline, isoquinoline, phthalazine, cinnoline, 1,8-naphthylidine, 1,2,3,4-tetrahydroisoquinoline, quinazoline, quinoxaline, purine, pteridine, azetidine, morpholine, thiomorpholine, piperidine, homopiperidine, piperazine, homopiperazine, indoline, isoindoline, phenoxazine, phenazine, phenothiazine, pyrrolopyrimidine, pyrazolpyrimidine or quinuclidine.

Preferred examples of the heterocyclic compound having 1 to 4 atoms selected from the group consisting of an oxygen atom, a nitrogen atom and a sulfur atom, in the ring of $G^1$ include monocyclic or bicyclic $C_2$-$C_9$ aromatic heterocyclic compounds having 1 to 3 atoms selected from the group consisting of an oxygen atom, a nitrogen atom and a sulfur atom, in the ring, such as furan, pyrrole, thiophene, pyrazole, oxazole, thiazole, isooxazole, isothiazole, pyrazole, imidazole, pyridine, pyrimidine, pyrazine, pyridazine, benzothiophene, benzofuran, 1,2-methylenedioxybenzene, benzimidazole, indole, quinoline, isoquinoline, quinazoline, phthalazine, cinnoline or 1,8-naphthylidin; or monocyclic $C_2$-$C_9$ heterocyclic compounds having 1 or 2 atoms selected from the group consisting of an oxygen atom, a nitrogen atom and a sulfur atom, in the ring, such as pyrrolidine, piperidine, morpholine, thiomorpholine, homopiperidine, homopiperazine, 1,2,3,6-tetrahydropyridine or piperazine.

The heterocyclic group having 1 to 4 atoms selected from the group consisting of an oxygen atom, a nitrogen atom and'a sulfur atom, in the ring of $G^1$ links to $A^2$ on a carbon atom or a nitrogen atom.

More preferred examples of the heterocyclic group linking to $A^2$ on a carbon atom include divalent groups derived from monocyclic or bicyclic $C_3$-$C_9$ aromatic heterocyclic compounds having 1 or 2 atoms selected from the group consisting of an oxygen atom, a nitrogen atom and a sulfur atom, in the ring, such as furan, pyrrole, thiophene, pyrazole, oxazole, thiazole, isooxazole, isothiazole, pyrazole, imidazole, pyridine, pyrimidine, pyrazine, pyridazine, benzothiophene, benzofuran, 1,2-methylenedioxybenzene, benzimidazole, indole, quinoline, isoquinoline or quinazoline.

Meanwhile, preferred examples of the heterocyclic group having 1 to 4 atoms selected from the group consisting of an oxygen atom, a nitrogen atom and a sulfur atom, in the ring, the heterocyclic group linking to $A^2$ on a nitrogen atom, include divalent groups derived from monocyclic or bicyclic $C_2$-$C_9$ heterocyclic compounds having 1 or 2 atoms selected from the group consisting of an oxygen atom, a nitrogen atom and a sulfur atom, in the ring, such as pyrrolidine, piperidine, morpholine, thiomorpholine, homopiperidine, homopiperazine, 1,2,3,6-tetrahydropyridine or piperazine. More preferred examples of the monocyclic $C_2$-$C_9$ heterocyclic compounds having 1 or 2 atoms selected from the group consisting of an oxygen atom, a nitrogen atom and a sulfur atom, in the ring, include piperidine, homopiperidine, morpholine, homopiperazine and piperazine.

Exemplary substituent of the heterocyclic group having 1 to 4 atoms selected from the group consisting of an oxygen atom, a nitrogen atom and a sulfur atom on its substituted ring of $G^1$, include a fluorine atom, a chlorine atom, a bromine atom, an iodine atom, a hydroxy group, an optionally substituted $C_1$-$C_7$ alkoxy group, a $C_6$-$C_{10}$ aryloxy group, a $C_7$-$C_9$ aralkoxy group, a $C_2$-$C_7$ acyloxy group, an oxo group, a $C_1$-$C_6$ alkylsulfonyloxy group, an optionally substituted $C_2$-$C_7$ acyl group, a carboxyl group, a $C_2$-$C_7$ alkoxycarbonyl group, a carbamoyl group, an optionally substituted $C_2$-$C_7$ alkylcarbamoyl group, an amino group, an optionally substituted $C_1$-$C_6$ alkylamino group, an optionally substituted $C_2$-$C_7$ acylamino group, a $C_2$-$C_8$ alkoxycarbonylamino group, a $C_1$-$C_6$ alkylsulfonylamino group, a cyano group, a nitro group, a $C_1$-$C_6$ alkylthio group, a $C_1$-$C_6$ alkylsulfynyl group, a $C_1$-$C_6$ alkylsulfonyl group, a sulfamoyl group, a $C_1$-$C_6$ alkylaminosulfonyl group, a sulfo group, an optionally substituted alicyclic hydrocarbon group having 3 to 6 carbon atoms, and an optionally substituted acyclic aliphatic hydrocarbon group having 1 to 6 carbon atoms.

Specific examples of the substituent of the heterocyclic group having 1 to 4 atoms selected from the group consisting of an oxygen atom, a nitrogen atom and a sulfur atom on its substituted ring of $G^1$ include the same as those exemplified in the substituted alicyclic hydrocarbon group having 3 to 10 carbon atoms of $G^1$.

As the substituent of the heterocyclic group having 1 to 4 atoms selected from the group consisting'of an oxygen atom, a nitrogen atom and a sulfur atom on its substituted ring of $G^1$, a $C_1$-$C_7$ alkoxy group, a $C_2$-$C_7$ acyl group, a $C_2$-$C_7$ alkylcarbamoyl group, a $C_1$-$C_6$ alkylamino group, a $C_2$-$C_7$ acylamino group, an alicyclic hydrocarbon group having 3 to 6 carbon atoms, and an acyclic aliphatic hydrocarbon group having 1 to 6 carbon atoms may further be substituted with (one or more substituents selected from the group consisting of a fluorine atom; a chlorine atom; a bromine atom; an iodine atom; a hydroxy group; a $C_1$-$C_6$ alkoxy group such as methoxy, ethoxy, propoxy, isopropoxy, butoxy, isobutoxy, s-butoxy, t-butoxy, pentyloxy or cyclopropyloxy; a methoxymethyloxy group; a 2-methoxyethoxy group; a formyl group; a trifluoroacetyl group; a $C_2$-$C_7$ acyl group such as acetyl, propionyl, butyryl, isobutyryl, valeryl or isovaleryl; an oxo group; a carboxyl group; a $C_2$-$C_7$ alkoxycarbonyl group such as methoxycarbonyl, ethoxycarbonyl, propoxycarbonyl, isopropoxycarbonyl, butoxycarbonyl, isobutoxycarbonyl or t-butoxycarbonyl; a carbamoyl group; a $C_2$-$C_7$ alkylcarbamoyl

group such as N-methylcarbamoyl, N,N-dimethylcarbamoyl, N-ethylcarbamoyl, N-ethyl-N-methylcarbamoyl, N,N-diethylcarbamoyl, N-propylcarbamoyl, N-isopropyl- carbamoyl, N-butylcarbamoyl, N-cyclopropylcarbamoyl or N-cyclopropylmethylcarbamoyl; an amino group; a $C_1$-$C_6$ alkylamino group such as methylamino, ethylamino, propylamino, isopropylamino, dimethylamino, N-ethylmethylamino, diethylamino, N-methylpropylamino, N-methylisopropylamino, cyclopropylamino or cyclopropylmethylamino; a $C_4$-$C_6$ cyclic amino group having 1 or 2 atoms selected from the group consisting of an oxygen atom, a nitrogen atom and a sulfur atom, in the ring, such as 1-pyrrolidinyl, piperazinyl, 4-methylpiperazinyl, piperidino or morpholino; a trifluoroacetylamino group; a $C_1$-$C_7$ acylamino group such as formylamino, acetylamino, propionylamino, butyrylamino, isobutyrylamino or valerylamino; a $C_1$-$C_6$ alkylsulfonylamino group such as methylsulfonylamino, ethylsulfonylamino, propylsulfonylamino or butylsulfonylamino; a nitro group; and a cyano group).

Preferred examples of the substituent of the heterocyclic group having 1 to 4 atoms selected from the group consisting of an oxygen atom, a nitrogen atom and a sulfur atom on its substituted ring of $G^1$, include a fluorine atom; a chlorine atom; a bromine atom; a $C_1$-$C_6$ alkoxy group consisting of a straight or branched alkyl and oxy group, including methoxy, ethoxy, propoxy, isopropoxy, butoxy, isobutoxy, s-butoxy, t-butoxy, pentyloxy, isopentyloxy, neopentyloxy, t-pentyloxy and hexyloxy; a cyano group; a nitro group; a carboxyl group; a hydroxy group; an amino group; a $C_1$-$C_6$ mono or dialkylamino group consisting of a straight or branched alkyl and amino group, including methylamino, ethylamino, propylamino, isopropylamino, butylamino, isobutylamino, s-butylamino, t-butylamino, pentylamino, hexylamino, dimethylamino, N-ethylmethylamino, diethylamino, N-methylpropylamino, N-methylisopropylamino, N-methylbutylamino, N-methyl-t- butylamino, N-ethylisopropylamino, dipropylamino, diisopropylamino and ethylbutylamino; a carbamoyl group; an aminosulfonyl group; an alicyclic hydrocarbon group having 3 to 6 carbon atoms, including cyclopropyl, cyclobutyl, cyclopentyl and cyclohexyl; a $C_2$-$C_7$ acyl group including acetyl, propionyl, butyryl, isobutyryl, pivaroyl and hexanoyl; a $C_1$-$C_6$ alkylthio group including methylthio, ethylthio, propylthio, isopropylthio, butylthio, isobutylthio, s-butylthio, t-butylthio, pentylthio'and hexylthio; a $C_1$-$C_6$ alkylsulfonyl group, including methylsulfonyl, ethylsulfonyl, propylsulfonyl, isopropylsulfonyl, butylsulfonyl, isobutylsulfonyl, s-butylsulfonyl, t-butylsulfonyl, pentylsulfonyl and hexylsulfonyl; a $C_2$-$C_7$ alkoxycarbonyl group including acetoxy, propionyloxy, butyryloxy, isobutyryloxy, valeryloxy, isovaleryloxy, pivaloyloxy and hexanoyloxy; a $C_2$-$C_7$ acylamino group including acetylamino, propionylamino, butyrylamino, isobutyrylamino, valerylamino and hexanoylamino; trifluoromethyl group; trifluoromethoxy group; and an acyclic aliphatic hydrocarbon group having 1 to 6 carbon atoms which may contain a straight or branched unsaturated bond including methyl, ethyl, vinyl, ethynyl, propyl, 1-propenyl, 2-propenyl, isopropyl, isopropenyl, 1-propynyl, 2-propynyl, butyl, isobutyl, s-butyl, t-butyl, 1-butenyl, 2-butenyl, 3-butenyl, 1-methyl-1-propenyl, 2-methyl-1-propenyl, 1-methyl-2- propenyl, 2-methyl-2-propenyl, 1-butynyl, 2-butynyl, pentyl, isopentyl, neopentyl, t-pentyl, 1-pentenyl, 2-pentenyl, 3-pentenyl, 4-pentenyl, hexyl, 5-hexenyl, 4-methyl-3-pentenyl, isohexyl, 2-methylpentyl and 1-ethylbutyl.

Specifically, more preferred examples of the substituent of the heterocyclic group having 1 to 4 atoms selected from the group consisting of an oxygen atom, a nitrogen atom and a sulfur atom on its substituted ring of $G^1$, include a fluorine atom, a chlorine atom, a bromine atom, $C_1$-$C_6$ alkoxy group, cyano group, a nitro group, a carboxyl group, a hydroxy group, an amino group, a $C_1$-$C_6$ mono or dialkylamino group, a carbamoyl group, an alicyclic hydrocarbon group having 3 to 6 carbon atoms, a $C_2$-$C_7$ acyl group, a $C_1$-$C_6$ alkylsulfonyl group, a $C_2$-$C_7$ alkoxycarboxyl group, a trifluoromethyl group, a trifluoromethoxy group, and a $C_1$-$C_6$ alkyl group including methyl, ethyl, propyl, isopropyl, butyl, isobutyl, s-butyl, t-butyl, pentyl, isopentyl, neopentyl, t-pentyl, hexyl, isohexyl, 2-methylpentyl and 1-ethylbutyl. Examples of particularly preferred substituents include a fluorine atom, a chlorine atom, a $C_1$-$C_6$ alkoxy group, a cyano group, a nitro group, a carboxyl group, a hydroxy group, an amino group, a $C_1$-$C_6$ mono or dialkylamino group, an alicyclic hydrocarbon group having 3 to 6 carbon atoms, a $C_2$-$C_7$ acyl group, a trifluoromethyl group, a trifluoromethoxy group and a $C_1$-$C_6$ alkyl group.

In the present invention, $G^1$ in the formula (I) is preferably a single bond, a monocyclic aliphatic hydrocarbon group having 3 to 6 carbon atoms, a phenylene group, a monocyclic or bicyclic aromatic hydrocarbon group having 3 to 9 carbon atoms having 1 or 2 atoms selected from among a group consisting of an oxygen atom, a nitrogen atom, and a sulfur atom, in the ring, or a monocyclic heterocyclic group having 2 to 9 carbon atoms having 1 or 2 atoms selected from among a group consisting of an oxygen atom, a nitrogen atom, and a sulfur atom, in the ring.

In the formula (I), $G^2$ represents a hydrogen atom, a substituted or unsubstituted acyclic aliphatic hydrocarbon group having 1 to 10 carbon atoms, a substituted or unsubstituted alicyclic hydrocarbon group having 3 to 10 carbon atoms, a substituted or unsubstituted aromatic hydrocarbon group having 6 to 14 carbon atoms, or a substituted or unsubstituted heterocyclic group having 1 to 4 atoms selected from among a group consisting of an oxygen atom, a nitrogen atom, and a sulfur atom, in the ring.

In formula (I), when $G^2$ represents a substituted or unsubstituted acyclic aliphatic hydrocarbon group having 1 to 10 carbon atoms, examples of such a acyclic aliphatic hydrocarbon group having 1 to 10 carbon atoms of $G^2$ include an alkyl group such as methyl, ethyl, propyl, isopropyl, butyl, isobutyl, s-butyl, t-butyl, pentyl, isopentyl, neopentyl, t-pentyl, 2-methylpentyl, 4-methylpentyl, 1-ethylbutyl, hexyl, heptyl, 2-methylhexyl, 5-methylhexyl, 1,1-dimethylpentyl, 6-methylheptyl, octyl, nonyl or decyl, an alkenyl group such as vinyl, 1-methylvinyl, 1-ethylvinyl, 1-propenyl, 2-propenyl, 2-methyl-1-propenyl, 1-butenyl, 2-butenyl, 2-methyl-1-butenyl, 1,3-butadienyl, 1-pentenyl, 2-pentenyl, 4-methyl-1-pentenyl, 1-hexenyl, 2-hexenyl, 3-hexenyl, 4-hexenyl, 5-hexenyl, 1, 5-hexadienyl, 2-heptenyl, 2-octenyl, 2-nonenyl or 2-decenyl, or an alkynyl group such as ethynyl, 1-propynyl, 2-propynyl, 1-butynyl, 3-methyl-1-butynyl, 3, 3-dimethyl-1-butynyl, 1-pentynyl, 2-pentynyl, 3-pentynyl, 1-hexynyl, 2-hexynyl, 3-hexynyl, 4-hexynyl, 1-methyl-3-pentynyl, 1-methyl-3-hexynyl, 2-heptynyl, 2-octynyl, 2-nonynyl or 2-decynyl.

Specifically, more preferred examples of such a acyclic aliphatic hydrocarbon group having 1 to 10 carbon atoms include a straight or branched $C_1$-$C_6$ alkyl group which may contain a unsaturated bond such as methyl, ethyl, propyl, isopropyl, butyl, pentyl, hexyl, vinyl, 1-prophenyl, 1-butenyl, ethynyl or 1-propynyl. Particularly preferred examples of such a acyclic aliphatic hydrocarbon group having 1 to 10 carbon atoms include a straight or branched $C_1$-$C_6$ alkyl group such as methyl, ethyl, propyl, isopropyl, butyl or hexyl.

Exemplary substituents of the substituted acyclic aliphatic hydrocarbon group having 1 to 10 carbon atoms of $G^2$ include: a fluorine atom; a chlorine atom; a bromine atom; an iodine atom; a hydroxy group; a $C_1$-$C_7$ alkoxy group consisting of a straight or branched alkyl group, cycloalkyl group and oxy group, including methoxy, ethoxy, propoxy, isopropoxy, butoxy, isobutoxy, s-butoxy, t-butoxy, pentyloxy, isopentyloxy, neopentyloxy, t-pentyloxy, hexyloxy, isohexyloxy, 2-methylpentyloxy, 1-ethylbutoxy, cyclopropyloxy, cyclobutyloxy, cyclopentyloxy, cyclohexyloxy, cyclopropylmethyloxy, cyclopropylethyloxy, cyclopentyl methyloxy and cyclohexylmethyloxy; an alkyldioxy group having 1 to 4 carbon atoms such as ethylene dioxy; a $C_6$-$C_{10}$ aryloxy group, including phenoxy, 1-naphthoxy and 2-naphthoxy; a $C_7$-$C_9$ aralkoxy group, including benzyloxy, $\alpha$-phenethyloxy, $\beta$-phenethyloxy and phenylpropyloxy; a $C_2$-$C_7$ acyloxy group including acetoxy, propionyloxy, butyryloxy, isobutyryloxy, valeryloxy, isovaleryloxy, pivaloyloxy and hexanoyloxy; an oxo group; a $C_1$-$C_6$ alkylsulfonyloxy group consisting of a straight or branched alkyl and sulfonyloxy, including oxo, methylsulfonyloxy, ethylsulfonyloxy, propylsulfonyloxy, butylsulfonyloxy and t-butylsulfonyloxy; a $C_2$-$C_7$ acyl group, including acetyl, propionyl, butyryl, isobutyryl, valeryl, isovaleryl, pivaroyl and hexanoyl; a carboxyl group; a $C_2$-$C_7$ alkoxycarbonyl group consisting of a straight or branched alkyl and oxycarbonyl group, including methoxycarbonyl, ethoxycarbonyl, propoxycarbonyl, isopropoxycarbonyl, butoxycarbonyl, isobutoxycarbonyl, s-butoxycarbonyl and t-butoxycarbonyl; a carbamoyl group; a $C_2$-$C_7$ alkylcarbamoyl group consisting of a straight or branched alkyl, cycloalkyl and carbamoyl group, including N-methylcarbamoyl, N-ethylcarbamoyl, N-propylcarbamoyl, N-isopropylcarbamoyl, N-butylcarbamoyl, N-isobutylcarbamoyl, N-s-butylcarbamoyl, N-t-butylcarbamoyl, N-pentylcarbamoyl, N-cyclopropylcarbamoyl, N-cyclobutylcarbamoyl, N-cyclopentylcarbamoyl, N-cyclohexylcarbamoyl, N-cycloheptylcarbamoyl, N-cyclopropylmethylcarbamoyl, N,N-dimethylcarbamoyl, N-ethyl-N-methylcarbamoyl, N,N-diethylcarbamoyl and N,N-dipropylcarbamoyl; an amino group; a $C_1$-$C_6$ alkylamino group consisting of a straight or branched alkyl, cycloalkyl and amino group, including methylamino, ethylamino, propylamino, isopropylamino, butylamino, isobutylamino,'s-butylamino, t-butylamino, pentylamino, hexylamino, cyclopropylamino, cyclobutylamino, cyclopentylamino, cyclohexylamino, cyclopropylmethylamino, dimethylamino, N-ethylmethylamino, diethylamino, N-methylpropylamino, N-methylisopropylamino, N-methylbutylamino, N-methyl-t-butylamino, N-ethylisopropyl- amino, dipropylamino, diisopropylamino and ethylbutylamino; a $C_2$-$C_7$ acylamino group including acetylamino, propionylamino, butyrylamino, isobutyrylamino, valerylamino and hexanoylamino; a $C_2$-$C_8$ alkoxycarbonylamino group, including methoxycarbonylamino, ethoxycarbonylamino and t-butoxycarbonylamino; a $C_1$-$C_6$ alkylsulfonylamino group including methylsulfonylamino, ethylsulfonylamino, butylsulfonylamino and t-butylsulfonylamino; a cyano group; a nitro group; a $C_1$-$C_6$ alkylthio group including methylthio, ethylthio, propylthio, isopropylthio, butylthio, isobutylthio, s-butylthio, t-butylthio, pentylthio and hexylthio; a $C_1$-$C_6$ alkylsulfynyl group consisting of a straight or branched alkyl, cycloalkyl and sulfynyl group, including methylsulfynyl, ethylsulfynyl, propylsulfynyl, isopropylsulfynyl, butylsulfynyl, isobutylsulfynyl, s-butylsulfynyl, t-butylsulfynyl, pentylsulfynyl and cyclopentylsulfynyl; a $C_1$-$C_6$ alkylsulfonyl group consisting of a straight or branched alkyl, cycloalkyl and sulfonyl group, including methylsulfonyl, ethylsulfonyl, propylsulfonyl, isopropylsulfonyl, butylsulfonyl, isobutylsulfonyl, s-butylsulfonyl, t-butylsulfonyl, pentylsulfonyl, hexylsulfonyl, cyclopentylsulfonyl and cyclohexylsulfonyl; a sulfo group; a sulfamoyl group; a $C_1$-$C_6$ aminosulfonyl group consisting of a straight or branched alkyl, cycloalkyl and aminosulfonyl group, including methylaminosulfonyl, ethylaminosulfonyl, propylaminosulfonyl, isopropylaminosulfonyl, butylaminosulfonyl, isobutylaminosulfonyl, s-butylaminosulfonyl, pentylaminosulfonyl, dimethylaminosulfonyl, N-ethyl-N-methylaminosulfonyl, diethylaminosulfonyl, dipropylaminosulfonyl, cyclopropylaminosulfonyl, cyclopentylaminosulfonyl, cyclohexylaminosulfonyl and cyclopropylmethylaminosulfonyl; an alicyclic hydrocarbon group having 3 to 6 carbon atoms, including cyclopropyl, cyclobutyl, cyclopentyl and cyclohexyl; and an acyclic aliphatic hydrocarbon group having 1 to 6 carbon atoms which may contain a straight or branched unsaturated bond, including methyl, ethyl, vinyl, ethynyl, propyl, 1-propenyl, 2-propenyl, isopropyl, isopropenyl, 1-propynyl, 2-propynyl, butyl, isobutyl, s-butyl, t-butyl, 1-butenyl, 2-butenyl, 3-butenyl, 1-methyl-1-propenyl, 2-methyl-1-propenyl, 1-methyl-2-propenyl, 2-methyl-2- propenyl, 1-butynyl, 2-butynyl, pentyl, isopentyl, neopentyl, t-pentyl, 1-pentenyl, 2-pentenyl, 3-pentenyl, 4-pentenyl, hexyl, 5-hexenyl, 4-methyl-3-pentenyl, isohexyl, 2-methylpentyl and 1-ethylbutyl; an aromatic hydrocarbon group having 6 to 14 carbon atoms which is a monovalent group derived from monocyclic, bicyclic or tricyclic aromatic hydrocarbon group, including benzene, naphthalene, indene, indane, 1,2,3,4-tetrahydronaphthalene, and fluorene; and a monovalent group derived from monocyclic, bicyclic or tricyclic heterocyclic compound, including furan, thiophene, pyrrole, pyrroline, pyrrolidine, oxazole, oxazolidine, isooxazole, isooxazolidine, thiazole, thiazolidine, isothiazole, isothiazolidine, imidazole, imidazoline, imidazolidine, pyrazole, pyrazoline, pyrazolidine, triazole,

thiadiazole, oxadiazole, tetrazole, pyran, tetrahydropyran, thiopyran, tetrahydrothiopyran, pyridine, pyrazine, pyrimidine, pyridazine, benzofuran, dibenzofuran, benzothiophene, indole, benzimidazole, benzothiazole, benzooxazole, chroman, isochroman, quinoline, decahydroquinoline, isoquinoline, quinazolin, quinoxaline, purine, pteridine, azetidine, morpholine, thiomorpholine, piperidine, homopiperidine, piperazine, homopiperazine, indoline, isoindoline, phenoxazine, phenazine, phenothiazine and quinuclidine, the heterocyclic compound (having 1 to 4 atoms selected from the group consisting of an oxygen atom, a nitrogen atom and a sulfur atom, in the ring).

Preferred examples of the substituent of the substituted acyclic aliphatic hydrocarbon group having 1 to 10 carbon atoms as $G^2$ include a fluorine atom, a hydroxy group, an optionally substituted $C_1$-$C_7$ alkoxy group, an oxo group, an optionally substituted $C_2$-$C_7$ acyl group, a carboxyl group, a $C_2$-$C_7$ alkoxycarbonyl group, a carbamoyl group, an optionally substituted $C_2$-$C_7$ alkylcarbamoyl group, an amino group, an optionally substituted, $C_1$-$C_6$ alkylamino group, an optionally substituted $C_2$-$C_7$ acylamino group, a $C_1$-$C_6$ alkylsulfonylamino group, a cyano group, a $C_1$-$C_6$ alkylsulfonyl group, a sulfamoyl group, an optionally substituted aromatic hydrocarbon group having 6 to 14 carbon atoms and an optionally substituted heterocyclic group (having 1 to 4 atoms selected from the group consisting of an oxygen atom, a nitrogen atom and a sulfur atom, in the ring).

More preferred exemplary substituents of the substituted acyclic aliphatic hydrocarbon group having 1 to 10 carbon atoms as $G^2$ include a fluorine atom, a hydroxy group, an optionally substituted $C_1$-$C_7$ alkoxy group, a carboxyl group, an amino group, an optionally substituted $C_1$-$C_6$ alkylamino group, a cyano group, a benzyl group, and an optionally substituted heterocyclic group (having 1 to 4 atoms selected from the group consisting of an oxygen atom, a nitrogen atom and a sulfur atom, in the ring).

As the substituent of the substituted acyclic aliphatic hydrocarbon group having 1 to 10 carbon atoms as $G^2$, the heterocyclic group (having 1 to 4 atoms selected from the group consisting of an oxygen atom, a nitrogen atom and a sulfur atom, in the ring), links to the acyclic aliphatic hydrocarbon group having 1 to 10 carbon atoms as $G^2$ on a carbon atom or a nitrogen atom.

Preferred examples of the heterocyclic group (having 1 to 4 atoms selected from the group consisting of an oxygen atom, a nitrogen atom and a sulfur atom, in the ring), links to the acyclic aliphatic hydrocarbon group having 1 to 10 carbon atoms as $G^2$ on a carbon atom, include a monovalent group derived from a monocyclic or bicyclic $C_3$-$C_9$ aromatic heterocyclic compound, including furan, pyrrole, thiophene, pyrazole, oxazole, thiazole, isooxazole, isothiazole, pyrazole, imidazole, pyridine, pyrimidine, pyrazine, pyridazine, benzothiophene, benzofuran, 1,2-methylenedioxybenzene, benzimidazole, indole, quinoline, isoquinoline and quinazolin, the monovalent group having 1 or 2 atoms selected from the group consisting of an oxygen atom, a nitrogen atom and a sulfur atom, in the ring.

Meanwhile, preferred examples of the heterocyclic group (having 1 to 4 atoms selected from the group consisting of an oxygen atom, a nitrogen atom and a sulfur atom, in the ring), links to the acyclic aliphatic hydrocarbon group having 1 to 10 carbon atoms as $G^2$ on a nitrogen atom, include a monovalent group derived from a monocyclic $C_2$-$C_9$ heterocyclic compound, including pyrrolidine, piperidine, morpholine, thiomorpholine, homopiperidine, homopiperazine, 1,2,3,6-tetrahydropyridine and piperazine, the monovalent group having 1 or 2 atoms selected from the group consisting of an oxygen atom, a nitrogen atom and a sulfur atom, in the ring.

As the substituent of the substituted acyclic aliphatic hydrocarbon group having 1 to 10 carbon atoms as $G^2$, a $C_1$-$C_7$ alkoxy group, a $C_2$-$C_7$ acyl group, $C_2$-$C_7$ alkylcarbamoyl, a $C_1$-$C_6$ alkylamino group, a $C_2$-$C_7$ acylamino group, an alicyclic hydrocarbon group having 3 to 6 carbon atoms, an acyclic aliphatic hydrocarbon group having 1 to 6 carbon atoms, aromatic hydrocarbon group having 6 to 14 carbon atoms, and heterocyclic group having 1 to 4 atoms selected from the group consisting of an oxygen atom, a nitrogen atom and a sulfur atom, in the ring, may further be substituted with (one or more substituents selected from the group consisting of: a fluorine atom; a chlorine atom; a.bromine atom; an iodine atom; a hydroxy group; a $C_1$-$C_6$ alkoxy group such as methoxy, ethoxy, propoxy, isopropoxy, butoxy, isobutoxy, s-butoxy, t-butoxy, pentyloxy or cyclopropyloxy; a methoxymethyloxy group; a 2-methoxyethoxy group; a formyl group; a trifluoroacetyl group; a $C_2$-$C_7$ acyl group such as acetyl, propionyl, butyryl, isobutyryl, valeryl or isovaleryl; an oxo group; a carboxyl group; a $C_2$-$C_7$ alkoxycarbonyl group such as methoxycarbonyl, ethoxycarbonyl, propoxycarbonyl, isopropoxycarbonyl, butoxycarbonyl, isobutoxycarbonyl or t-butoxycarbonyl; a carbamoyl group; a $C_2$-$C_7$ alkylcarbamoyl group such as N-methylcarbamoyl, N,N-dimethylcarbamoyl, N-ethylcarbamoyl, N-ethyl-N- methylcarbamoyl, N,N-diethylcarbamoyl, N-propylcarbamoyl, N-isopropylcarbamoyl, N-butylcarbamoyl, N-cyclopropyl- carbamoyl or N-cyclopropylmethylcarbamoyl; an amino group; a $C_1$-$C_6$ alkylamino group such as methylamino, ethylamino, propylamino, isopropylamino, dimethylamino, N-ethylmethylamino, diethylamino, N-methylpropylamino, N-methylisopropylamino, cyclopropylamino or cyclopropylmethylamino; a $C_4$-$C_6$ cyclic amino group having 1 or 2 atoms selected from the group consisting of an oxygen atom, a nitrogen atom and a sulfur atom, in the ring, such as 1-pyrrolidinyl, piperazinyl, 4-methylpiperazinyl, piperidino or morpholino; a trifluoroacetylamino group; a $C_1$-$C_7$ acylamino group such as formylamino, acetylamino, propionylamino, butyrylamino, isobutyrylamino or valerylamino; a $C_1$-$C_6$ alkylsulfonylamino group such as methylsulfonylamino, ethylsulfonylamino, propylsulfonylamino or butylsulfonylamino; a nitro group; a cyano group; a $C_1$-$C_6$ alkyl group including methyl, ethyl, propyl, isopropyl, butyl, isobutyl, s-butyl, and t-butyl; a trifluoromethyl group; and a trifluoromethoxy group).

In the formula (I), when $G^2$ represents a substituted or unsubstituted alicyclic hydrocarbon group having 3 to 10 carbon atoms, examples of the alicyclic hydrocarbon group having 3 to 10 carbon atoms of $G^2$ include cyclopropyl, cyclobutyl, cyclopentyl, cyclopentenyl, cyclohexyl, cyclohexenyl, cycloheptyl, cycloheptenyl, and cyclooctyl group. Preferred examples of the alicyclic hydrocarbon group having 3 to 10 carbon atoms of $G^2$ include cyclopropyl, cyclobutyl, cyclopentyl, cyclohexyl, 3-cyclopentenyl, 4-cyclopentenyl, 1-cyclohexenyl, 3-cyclohexenyl, 4-cyclohexenyl, and 1-cycloheptenyl.

Exemplary substituents of the substituted alicyclic hydrocarbon group having 3 to 10 carbon atoms of $G^2$ include a fluorine atom, a chlorine atom, a bromine atom, an iodine atom, a hydroxy group, an optionally substituted $C_1$-$C_7$ alkoxy group, $C_1$-$C_4$ alkylenedioxy group, a $C_6$-$C_{10}$ aryloxy group, a $C_7$-$C_9$ aralkoxy group, a $C_2$-$C_7$ acyloxy group, an oxo group, a $C_1$-$C_6$ alkylsulfonyloxy group, an optionally substituted $C_2$-$C_7$ acyl group, a carboxyl group, a $C_2$-$C_7$ alkoxycarbonyl group, a carbamoyl group, an optionally substituted $C_2$-$C_7$ alkylcarbamoyl group, an amino group, an optionally substituted $C_1$-$C_6$ alkylamino group, an optionally substituted $C_2$-$C_7$ acylamino group, a $C_2$-$C_8$ alkoxycarbonylamino group, a $C_1$-$C_6$ alkylsulfonylamino group, a cyano group, a nitro group, a $C_1$-$C_6$ alkylthio group, a $C_1$-$C_6$ alkylsulfynyl group, a $C_1$-$C_6$ alkylsulfonyl group, a sulfamoyl group, a $C_1$-$C_6$ alkylaminosulfonyl group, a sulfo group, an optionally substituted alicyclic hydrocarbon group having 3 to 6 carbon atoms, and an optionally substituted acyclic aliphatic hydrocarbon group having 1 to 6 carbon atoms,' an optionally substituted aromatic hydrocarbon group having 6 to 14 carbon atoms.

Specific examples of the substituent of the substituted alicyclic hydrocarbon group having 3 to 10 carbon atoms of $G^2$ include the same as those exemplified in the substituted acyclic aliphatic hydrocarbon group having 1 to 10 carbon atoms of $G^2$.

As the substituent of the substituted alicyclic hydrocarbon group having 3 to 10 carbon atoms of $G^2$, a $C_{1\text{-}}C_7$ alkoxy group, a $C_2$-$C_7$ acyl group, a $C_2$-$C_7$ alkylcarbamoyl group, a $C_1$-$C_6$ alkylamino group, a $C_2$-$C_7$ acylamino group, an alicyclic hydrocarbon group having 3 to 6 carbon atoms, an acyclic aliphatic hydrocarbon group having 1 to 6 carbon atoms, and an aromatic hydrocarbon group having 6 to 14 carbon atoms, may further be substituted with (one or more substituents selected from the group consisting of: a fluorine atom; a chlorine atom; a bromine atom; an iodine atom; a hydroxy group; a $C_1$-$C_6$ alkoxy group such as methoxy, ethoxy, propoxy, isopropoxy, butoxy, isobutoxy, s-butoxy, t-butoxy, pentyloxy or cyclopropyloxy; a methoxymethyloxy group; a 2-methoxyethoxy group; a formyl group; a trifluoroacetyl group; a $C_2$-$C_7$ acyl group such as acetyl, propionyl, butyryl, isobutyryl, valeryl or isovaleryl; an oxo group; a carboxyl group; a $C_2$-$C_7$ alkoxycarbonyl group such as methoxycarbonyl, ethoxycarbonyl, propoxycarbonyl, isopropoxycarbonyl, butoxycarbonyl, isobutoxycarbonyl or t-butoxycarbonyl; a carbamoyl group; a $C_2$-$C_7$ alkylcarbamoyl group such as N-methylcarbamoyl, N,N-dimethylcarbamoyl, N-ethylcarbamoyl, N-ethyl-N-methylcarbamoyl, N,N-diethyl- carbamoyl, N-propylcarbamoyl, N-isopropylcarbamoyl, N-butylcarbamoyl, N-cyclopropylcarbamoyl or N-cyclopropylmethylcarbamoyl; an amino group; a $C_1$-$C_6$ alkylamino group such as methylamino, ethylamino, propylamino, isopropylamino, dimethylamino, N-ethylmethylamino, diethylamino, N-methylpropylamino, N-methylisopropylamino, cyclopropylamino or cyclopropylmethylamino; a $C_4$-$C_6$ cyclic amino group having 1 or 2 atoms selected from the group consisting of an oxygen atom, a nitrogen atom and a sulfur atom, in the ring, such as 1-pyrrolidinyl, piperazinyl, 4-methylpiperazinyl, piperidino or morpholino; a trifluoroacetylamino group; a $C_1$-$C_7$ acylamino group such as formylamino, acetylamino, propionylamino, butyrylamino, isobutyrylamino or valerylamino, a $C_1$-$C_6$ alkylsulfonylamino group such as methylsulfonylamino, ethylsulfonylamino, propylsulfonylamino or butylsulfonylamino; a nitro group; a cyano group; a $C_1$-$C_6$ alkyl group including methyl, ethyl, propyl, isopropyl, butyl, isobutyl, s-butyl, and t-butyl; a trifluoromethyl group; and a trifluoromethoxy group).

In the formula (I), when $G^2$ represents a substituted or unsubstituted aromatic hydrocarbon group having 6 to 14 carbon atoms, examples of the aromatic hydrocarbon group having 6 to 14 carbon atoms include a monovalent group having at least one aromatic ring on its molecule, such as benzene, indene, indane, naphthalene, 1,2-dihydronaphthalene, 1,2,3,4- tetrahydronaphthalene, azulene, acenaphthylene, acenaphthene, fluorene, phenanthrene or anthracene. Examples of such preferred aromatic hydrocarbon group having 6 to 14 carbon atoms of $G^2$ include a phenyl group.

Exemplary substituents of the aromatic hydrocarbon group having 6 to 14 carbon atoms of $G^2$ include at least one substituent selected from the group consisting of a fluorine atom, a chlorine atom, a bromine atom, an iodine atom, a hydroxy group, an optionally substituted $C_1$-$C_7$ alkoxy group, a $C_1$-$C_4$ alkylenedioxy group, a $C_6$-$C_{10}$ aryloxy group, a $C_7$-$C_9$ aralkoxy group, a $C_2$-$C_7$ acyloxy group, an oxo group, a $C_1$-$C_6$ alkylsulfonyloxy group, an optionally substituted $C_2$-$C_7$ acyl group, a carboxyl group, a $C_2$-$C_7$ alkoxycarbonyl group, a carbamoyl group, an optionally substituted $C_2$-$C_7$ alkylcarbamoyl group, an amino group, an optionally substituted $C_1$-$C_6$ alkylamino group, an optionally, substituted $C_2$-$C_7$ acylamino group, a $C_2$-$C_8$ alkoxycarbonylamino group, a $C_1$-$C_6$ alkylsulfonylamino group, a cyano group, a nitro group, a $C_1$-$C_6$ alkylthio group, a $C_1$-$C_6$ alkylsulfynyl group, a $C_1$-$C_6$ alkylsulfonyl group, a sulfamoyl group, a $C_1$-$C_6$ alkylaminosulfonyl group, a sulfo group, an optionally substituted alicyclic hydrocarbon group having' 3 to 6 carbon atoms, an optionally substituted acyclic aliphatic hydrocarbon group having 1 to 6 carbon atoms, and an optionally substituted aromatic hydrocarbon group having 6 to 14 carbon atoms.

Specific examples of the substituent of the substituted $C_6$-$C_{14}$ aromatic hydrocarbon group of $G^2$ include the

same as those exemplified in the substituent of the substituted acyclic aliphatic hydrocarbon group having 1 to 10 carbon atoms of $G^2$.

As the substituents of the aromatic hydrocarbon group having 6 to 14 carbon atoms of $G^2$, a $C_1$-$C_7$ alkoxy group, a $C_2$-$C_7$ acyl group, a $C_2$-$C_7$ alkylcarbamoyl group, a $C_1$-$C_6$ alkylamino group, a $C_2$-$C_7$ acylamino group, an alicyclic hydrocarbon group having 3 to 6 carbon atoms, an acyclic aliphatic hydrocarbon group having 1 to 6 carbon atoms, and an aromatic hydrocarbon group having 6 to 14 carbon atoms, may further be substituted with (one or more substituents selected from the group consisting of: a fluorine atom; a chlorine atom; a bromine atom; an iodine atom; a hydroxy group; a $C_1$-$C_6$ alkoxy group such as methoxy, ethoxy, propoxy, isopropoxy, butoxy, isobutoxy, s-butoxy, t-butoxy, pentyloxy or cyclopropyloxy; a methoxymethyloxy group; a 2-methoxyethoxy group; a formyl group; a trifluoroacetyl group; a $C_2$-$C_7$ acyl group such as acetyl, propionyl, butyryl, isobutyryl, valeryl or isovaleryl; an oxo group; a carboxyl group; a $C_2$-$C_7$ alkoxycarbonyl group such as methoxycarbonyl, ethoxycarbonyl, propoxycarbonyl, isopropoxycarbonyl, butoxycarbonyl, isobutoxycarbonyl or t-butoxycarbonyl; a carbamoyl group; a $C_2$-$C_7$ alkylcarbamoyl group such as N-methylcarbamoyl, N,N-dimethylcarbamoyl, N-ethylcarbamoyl, N-ethyl-N-methylcarbamoyl, N,N-diethyl- carbamoyl, N-propylcarbamoyl, N-isopropylcarbamoyl, N-butylcarbamoyl, N-cyclopropylcarbamoyl or N-cyclopropylmethylcarbamoyl; an amino group; a $C_1$-$C_6$ alkylamino group such as methylamino, ethylamino, propylamino, isopropylamino, dimethylamino, N-ethylmethylamino, diethylamino, N-methylpropylamino, N-methylisopropylamino, cyclopropylamino or cyclopropylmethylamino; a $C_4$-$C_6$ cyclic amino group having 1 or 2 atoms selected from the group consisting of an oxygen atom, a nitrogen atom and a sulfur atom, in the ring, such as 1-pyrrolidinyl, piperazinyl, 4-methylpiperazinyl, piperidino or morpholino; a trifluoroacetylamino group; a $C_1$-$C_7$ acylamino group such as formylamino, acetylamino, propionylamino, butyrylamino, isobutyrylamino or valerylamino; a $C_1$-$C_6$ alkylsulfonylamino group such as methylsulfonylamino, ethylsulfonylamino, propylsulfonylamino or butylsulfonylamino; a nitro group; a cyano group; a $C_1$-$C_6$ alkyl group including methyl, ethyl, propyl, isopropyl, butyl, isobutyl, s-butyl, and t-butyl; a trifluoromethyl group; and a trifluoromethoxy group).

In the formula (I), when $G^2$ represents a heterocyclic group having 1 to 4 atoms selected from the group consisting of an oxygen atom, a nitrogen atom and a sulfur atom on its substituted or unsubstituted ring, examples of such heterocyclic group include a monovalent group derived from monocyclic, bicyclic or tricyclic compounds, including furan, thiophene, pyrrole, pyrroline, pyrrolidine, oxazole, oxazolidine, isooxazole, isooxazolidine, thiazole, thiazolidine, isothiazole, isothiazolidine, imidazole, imidazoline, imidazolidine, pyrazole, pyrazoline, pyrazolidine, triazole, thiadiazole, oxadiazole, tetrazole, pyran, tetrahydropyran, thiopyran, tetrahydrothiopyran, pyridine, pyrazine, pyrimidine, pyridazine, benzofuran, dibenzofuran,'benzothiophene, indole, 1,2-methylenedioxybenzene, benzimidazole, benzothiazole, benzooxazole, chroman, isochroman, quinoline, decahydroquinoline, isoquinoline, quinazolin, quinoxaline, purine, pteridine, azetidine, morpholine, thiomorpholine, piperidine, homopiperidine, piperazine, homopiperazine, indoline, isoindoline, phenoxazine, phenazine, phenothiazine and quinuclidine.

Preferred examples of the heterocyclic group having 1 to 4 atoms selected from the group consisting of an oxygen atom, a nitrogen atom and a sulfur atom, in the ring of $G^2$ include 2-pyridyl, 3-pyridyl, 4-pyridyl, piperidino, 2-piperizyl, 3-piperizyl, 4-piperizyl, morpholino, 1-homopiperidinyl, 1-pyrrolidinyl, 2-thiazolyl, 4-thiazolyl, 5-thiazolyl, 2-oxazolyl, 4-oxazolyl, 5-oxazolyl, 1-imidazolyl, 2-imidazolyl, 4-imidazolyl, 5-imidazolyl, 3-pyrazolyl, 2-furyl, 3-furyl, 2-thienyl, 3-thienyl, 2-pyrrolyl, 3-pyrrolyl, 4-isooxazolyl, 2-pyrimidinyl, 4-pyrimidinyl, 2-pyrazinyl, 4-triazolyl, 5-tetrazolyl, 1-piperazinyl, 4-tetrahydropyranyl, 2=1,3,4-oxadiazolyl, 4-1,2,3-thiadiazolyl, 2-benzofuranyl, 2-benzothiazolyl, 2-indolyl, 3-indolyl, 5-benzoimidazolyl and 2-1,2,3,4-tetrahydroisoquinolinyl group.

The heterocyclic group having 1 to 4 atoms selected from the group consisting of an oxygen atom, a nitrogen atom and a sulfur atom, in the ring of $G^2$, links to $A^4$ on a carbon atom or a nitrogen atom.

More preferred examples of the heterocyclic group having 1 to 4 atoms selected from the group consisting of an oxygen atom, a nitrogen atom and a sulfur atom, in the ring of $G^2$, the heterocyclic group linking to $A^4$ on a carbon atom, include a monovalent group derived from a monocyclic or bicyclic $C_3$-$C_9$ aromatic heterocyclic compound having 1 or 2 atoms selected from the group consisting of an oxygen atom, a nitrogen atom and a sulfur atom, in the ring, such as furan, pyrrole, thiophene, pyrazole, oxazole, thiazole, isooxazole, isothiazole, pyrazole, imidazole, pyridine, pyrimidine, pyrazine, pyridazine, benzothiophene, benzofuran, 1,2-methylenedioxy- benzene, benzimidazole, indole, quinoline, isoquinoline or quinazolin.

Meanwhile, preferred examples of the heterocyclic group having 1 to 4 atoms selected from the group consisting of an oxygen atom, a nitrogen atom and a sulfur atom, in the ring of $G^2$, the heterocyclic group linking to $A^4$ on a nitrogen'atom, include a monovalent group derived from a monocyclic $C_2$-$C_9$ heterocyclic compound having 1 or 2 atoms selected from the group consisting of an oxygen atom, a nitrogen atom and a sulfur atom, in the ring, such as pyrrolidine, piperidine, morpholine, thiomorpholine, homopiperidine, homopiperazine, 1,2,3,6-tetrahydropyridine or piperazine.

More preferred examples of the heterocyclic group as $G^2$ include a monovalent group derived from a monocyclic $C_4$-$C_6$ heterocyclic compound having 1 or 2 atoms selected from the group consisting of an oxygen atom, a nitrogen atom and a sulfur atom, in the ring, such as piperidine, homopiperidine, morpholine, homopiperazine, or piperazine.

Exemplary substituents of the heterocyclic group having 1 to 4 atoms selected from the group consisting of an

oxygen atom, a nitrogen atom and a sulfur atom on its substituted ring of $G^2$ include at least one substituent selected from the group consisting of a fluorine atom, a chlorine atom, a bromine atom, an iodine atom, a hydroxy group, an optionally substituted $C_1$-$C_7$ alkoxy group, a $C_1$-$C_4$ alkylenedioxy group, a $C_6$-$C_{10}$ aryloxy group, a $C_7$-$C_9$ aralkoxy group, a $C_2$-$C_7$ acyloxy group, an oxo group, a $C_1$-$C_6$ alkylsulfonyloxy group, an optionally substituted $C_2$-$C_7$ acyl group, a carboxyl group, a $C_2$-$C_7$ alkoxycarbonyl group, a carbamoyl group, an optionally substituted $C_2$-$C_7$ alkylcarbamoyl group, an amino group, an optionally substituted $C_1$-$C_6$ alkylamino group, an optionally substituted $C_2$-$C_7$ acylamino group, a $C_2$-$C_8$ alkoxycarbonylamino group, a $C_1$-$C_6$ alkylsulfonylamino group, a cyano group, a nitro group, a $C_1$-$C_6$ alkylthio group, a $C_1$-$C_6$ alkylsulfynyl group, a $C_1$-$C_6$ alkylsulfonyl group, a sulfamoyl group, a $C_1$-$C_6$ alkylaminosulfonyl group, a sulfo group, an optionally substituted alicyclic hydrocarbon group having 3 to 6 carbon atoms, an optionally substituted acyclic aliphatic hydrocarbon group having 1 to 6 carbon atoms, and an optionally substituted aromatic hydrocarbon group having 6 to 14 carbon atoms and.

The substituents of the heterocyclic group having 1 to 4 atoms selected from the group consisting of an oxygen atom, a nitrogen atom and a sulfur atom, in the ring of $G^2$ are as defined above for the substituent of the substituted acyclic aliphatic hydrocarbon group having 1 to 10 carbon atoms of $G^2$.

As the substituent of the heterocyclic group having 1 to 4 atoms selected from the group consisting of an oxygen atom, a nitrogen atom and a sulfur atom on its substituted ring of $G^2$, a $C_1$-$C_7$ alkoxy group, a $C_2$-$C_7$ acyl group, a $C_2$-$C_7$ alkylcarbamoyl group, a $C_1$-$C_6$ alkylamino group, a $C_2$-$C_7$ acylamino group, an alicyclic hydrocarbon group having 3 to 6 carbon atoms, an acyclic aliphatic hydrocarbon group having 1 to 6 carbon atoms, and an aromatic hydrocarbon group having 6 to 14 carbon atoms, may further be substituted with (one or more substituents selected from the group consisting of: a fluorine atom; a chlorine atom; a bromine atom; an iodine atom; a hydroxy group; a $C_1$-$C_6$ alkoxy group such as methoxy, ethoxy, propoxy, isopropoxy, butoxy, isobutoxy, s-butoxy, t-butoxy, pentyloxy or cyclopropyloxy; a methoxymethyloxy group; a 2-methoxyethoxy group; a formyl group; a trifluoroacetyl group; a $C_2$-$C_7$ acyl group such as acetyl, propionyl, butyryl, isobutyryl, valeryl or isovaleryl; an oxo group; a carboxyl group; a $C_2$-$C_7$ alkoxycarbonyl group such as methoxycarbonyl, ethoxycarbonyl, propoxycarbonyl, isopropoxycarbonyl, butoxycarbonyl, isobutoxycarbonyl or t-butoxycarbonyl; a carbamoyl group; a $C_2$-$C_7$ alkylcarbamoyl group.such as N-methylcarbamoyl, N,N-dimethylcarbamoyl, N-ethylcarbamoyl, N-ethyl-N-methylcarbamoyl, N,N-diethylcarbamoyl, N-propylcarbamoyl, N-isopropyl- carbamoyl, N-butylcarbamoyl, N-cyclopropylcarbamoyl or N-cyclopropylmethylcarbamoyl; an amino group; a $C_1$-$C_6$ alkylamino group such as methylamino, ethylamino, propylamino, isbpropylamino, dimethylamino, N-ethylmethylamino, diethylamino, N-methylpropylamino, N-methylisopropylamino, cyclopropylamino or cyclopropylmethylamino; a $C_4$-$C_6$ cyclic amino group having 1 or 2 atoms selected from the group consisting of an oxygen atom, a nitrogen atom and a sulfur atom, in the ring, such as 1-pyrrolidinyl, piperazinyl, 4-methylpiperazinyl, piperidino or morpholino; a trifluoroacetylamino group; a $C_1$-$C_7$ acylamino group such as formylamino, acetylamino, propionylamino, butyrylamino, isobutyrylamino or valerylamino; a $C_1$-$C_6$ alkylsulfonylamino group such as methylsulfonylamino, ethylsulfonylamino, propylsulfonylamino or butylsulfonylamino; a nitro group; a cyano group; a $C_1$-$C_6$ alkyl group including methyl, ethyl, propyl, isopropyl, butyl, isobutyl, s-butyl, and t-butyl; a trifluoromethyl group; and a trifluoromethoxy group).

In the present description, when $G^1$, $G^2$, or the substituent of $G^2$ represents a substituted or unsubstituted aromatic hydrocarbon group, a substituted or unsubstituted alicyclic hydrocarbon group, or a substituted or unsubstituted heterocyclic group, the aromatic hydrocarbon group, alicyclic hydrocarbon group, or heterocyclic group is preferably selected from a group consisting of cyclopropane, cyclopentane, cyclohexane, cyclohexene, cycloheptane, *nolvolnane, adamantine, benzene, naphthalene, indane, indoles, 1,3-benzodioxol, benzoimidazol, benzotriazol, pyrazol, imidazol, pyrazoron, thiazol, tetrazol, 1,2,4-oxadiazol, isooxazol, furan, thiophene, pyridine, pyradine, pyrrole, morpholine, benzofuran, benzothiophene, piperazine, pyrrolidine, homopiperizine, tetrahydroisoquinoline, pyrimidine, and quinazoline.

Next, an explanation will be given of preferred combinations of $A^1$, $A^2$, $G^1$, $A^3$, $A^4$ and $G^2$ in the formula (I).

When both of $A^1$ and $A^3$ represent acyclic aliphatic hydrocarbon group, at least one of $A^2$ and $G^1$ is not a single bond.

The preferred combinations of $A^1$, $A^2$, $G^1$, $A^3$, $A^4$ and $G^2$, and preferred combinations including also substituents of them if they have substituents are basically preferably combinations of those preferably selected from among $A^1$, $A^2$, $G^1$, $A^3$, $A^4$ and $G^2$, and substituents of them. Then, more preferred combinations are combinations of more preferred elements.

In the formula (I), $A^1$ represents a divalent acyclic aliphatic hydrocarbon group having 1 to 6 carbon atoms, particularly preferably represents $-(CH_2)_2-$ or $-(CH_2)_3-$.

More preferably, $A^2$ simultaneously represents those other than the single bond, and especially preferably $A^2$ represents -C(=O)-, -C(=O)-O-, -C(=O)-NH-, -C(=O)-NMe-, -NH-, -NH-C(=O)-, -NH-C(=O)-O-, -NH-C(=O)-NH-, -NH-C (=O)- NMe-, or -NH-C(=S)-. Specifically preferably $A^2$ represents -C(=O)-NH-, -NH-, -NH-C(=O)-, -NH-C(=O)-O-, or -NH-C(=O)-NH-.

Meanwhile, where $A^1$ represents a single bond, preferably also $A^2$ represents a single bond.

Preferred combinations of $G^1$, $A^3$, $A^4$ and $G^2$ of $G^1$-$G^2$ portion include combinations of 1 to 10 of the following table.

| Combination | $G^1$ | $A^3$ | $A^4$ | $G^2$ |
|---|---|---|---|---|
| 1 | Group other than single bond | Single bond | Single bond | Hydrogen atom |
| 2 | Single bond | Group other than single bond | Single bond | Hydrogen atom |
| 3 | Group other than single bond ’ | Single bond | Single bond | Group other than hydrogen atom |
| 4 | Single bond | Group other than single bond | Single bond | Group other than hydrogen atom |
| 5 | Group other than single bond | Single bond | Group other than single bond | Group other than hydrogen atom |
| 6 | Single bond | Group other than single bond | Group other than single bond | Group other than hydrogen atom |
| 7 | Group other than single bond | Group other than single bond | Single bond | Group other than hydrogen atom |
| 8 | Group other than single bond | Group other than single bond | Group other than single bond | Group other than hydrogen atom |
| 9 | Group other than single bond | Group other than single bond | Group other than single bond | Hydrogen atom |
| 10 | Single bond | Single bond | Single bond | Hydrogen atom |

In the table, in combinations of numbers 4 to 7, $A^3$ represents an alkylene group having 1 to 3 carbon atoms.

Also, in the combination of number 5, $A^4$ preferably represents -C(=O)-, -C(=O)-NH-, -O-, or -NH-C(=O)-.

Also, in the combination of number 8, $A^4$ preferably represents -O-.

Further, combinations of the following a) to f) are preferable.

a) $A^1$ represents $-(CH_2)_2-$ or $-(CH_2)_3-$, $A^2$ represents -NH-(C=O)- or -NH-(C=O)-NH-, $G^1$ represents a single bond, and $A^3$ represents a divalent acyclic aliphatic hydrocarbon group having 1 to 6 carbon atoms.

b) $A^1$ represents $-(CH_2)_2-$ or $-(CH_2)_3-$, $A^2$ represents -NH-(C=O)-, -NH-(C=O)-NH-, -NH-, or -C-(=O)-NH-, and $G^1$ represents a group other than the single bond.

c) $A^1$ represents a divalent acyclic aliphatic hydrocarbon group having 1 to 10 carbon atoms, specifically $-(CH_2)_2-$ or $-(CH_2)_3-$, $A^2$ represents a single bond, and $G^1$ represents an optionally substituted heterocyclic group (note, where a heterocyclic group of $G^1$ is 5-6 membered monocyclic, the 5-6 membered monocyclic heterocyclic group of $G^1$ is’ substituted, or $A^3$-$G^2$ portion represents those other than the hydrogen atom).

d) $A^1$ represents a divalent acyclic aliphatic hydrocarbon group having 1 to 6 carbon atoms, specifically $-(CH_2)_2-$ or $-(CH_2)_3-$, $A^2$ represents those other than a single bond, and $G^1$ represents an optionally substituted aromatic hydrocarbon group, an optionally substituted alicyclic hydrocarbon group having 7 to 10 carbon atoms, or an optionally substituted heterocyclic group (note, where the aromatic hydrocarbon group of $G^1$ is a phenyl group, or where the heterocyclic group of $G^1$ is 5-6 membered monocyclic, the phenyl group of $G^1$ or 5-6 membered monocyclic heterocyclic group 6 is substituted, or $A^3$-$G^2$ portion represents those other than the hydrogen atom).

e) $A^1$ represents a divalent acyclic aliphatic hydrocarbon group having 1 to 6 carbon atoms, specifically $-(CH_2)_2-$ or $-(CH_2)_3-$, $A^2$ represents those other than a single bond, $G^1$ and $A^4$ represent the single bond, $A^3$ represents an optionally substituted acyclic aliphatic hydrocarbon group having 1 to 10 carbon atoms, $G^2$ represents an optionally substituted alicyclic hydrocarbon group having 5 to 10 carbon atoms, an optionally substituted aromatic hydrocarbon group, or optionally substituted heterocyclic group.

f) $A^1$ represents a divalent acyclic aliphatic hydrocarbon group having 1 to 6 carbon atoms, specifically $-(CH_2)_2-$ or $-(CH_2)_3-$, $A^2$ represents those other than a single bond, $G^1$ represents the single bond, $A^3$ represents an optionally substituted acyclic aliphatic hydrocarbon group having 1 to 10 carbon atoms, and $A^4$ represents -C(=O)-, -C(=O)-$NR^{121}$-, -C(=S)-$NR^{122}$-, -C(=$NR^{123}$)-, -O-C(=O)-, -$NR^{125}$-C(=O)-, -$NR^{126}$-$S(=O)_2$-, -$NR^{127}$-C(=O)-O-, -$NR^{128}$-C(=O)-$NR^{129}$-, -$NR^{130}$-C(=S)-, -$NR^{131}$-C(=S) -$NR^{132}$-, -S-, -S(=O)-, -$S(=O)_2$-, -$S(=O)_2$-$NR^{133}$- or -$S(=O)_2$-O-.

In the cases of d) to f), $A^2$ preferably represents -C(=O)-, -C(=O)-O-, -C(=O)-NH-, -C(=O)-NMe-, -NH-, -NH-C(=O)-, -NH-C(=O)-O-, -NH-C(-O)-NH-, -NH-C(=O)-NMe-, or -NH-C(=S)-, especially preferably represents -C(=O)-NH-, -NH-, -NH-C(=6)-, -NH-C(=O)-O-, or -NH-C(=O)-NH-.

In the formula (I), $A^5$ represents a single bond or represents a group that links $R^2$ with a carbon atom of a pyrrole ring to which $A^5$ is bonded, in the form of $R^{2-}NR^{201}$-pyrrole ring ($R^{201}$ represents a hydrogen atom or a acyclic aliphatic hydrocarbon group having 1 to 4 carbon atoms), when $A^5$ bonds $R^2$ and a carbon atom of a pyrrole ring to which $A^5$ is bonded, in the form of $R^2$-$NR^{201}$-pyrrole ring, examples of the acyclic aliphatic hydrocarbon group having 1 to 4 carbon atoms of $R^{201}$ are the same as those exemplified as $R^{101}$ of $A^2$ described above. Preferred examples of $R^{102}$ include a hydrogen atom, methyl, ethyl or propyl group, and specifically preferably hydrogen atom and methyl group.

Preferred examples of $A^5$ include a single bond, -NH-, and -N($CH_3$)-, and specifically preferably single bond.

In the formula (I), $R^2$ represents a hydrogen atom, a fluorine atom, a chlorine atom, a bromine atom, an iodine atom, an optionally substituted acyclic aliphatic hydrocarbon group having 1 to 10 carbon atoms, an optionally substituted alicyclic hydrocarbon group having 3 to 8 carbon atoms, an optionally substituted aromatic hydrocarbon group having 6 to 14 carbon atoms, or an optionally substituted heterocyclic group having 1 to 4 atoms selected from the group consisting of an oxygen atom, a nitrogen atom and a sulfur atom, in the ring.

$R^2$ in the formula (I) is preferably a chlorine atom and a bromine atom among a fluorine atom, a chlorine atom, a bromine atom, and an iodine atom.

In the formula (I), when $R^2$ represents an optionally substituted acyclic aliphatic hydrocarbon group having 1 to 10 carbon atoms, examples of acyclic aliphatic hydrocarbon group having 1 to 10 carbon atoms of $R^2$ are the same as those exemplified of the acyclic aliphatic hydrocarbon group having 1 to 10 carbon atoms of $G^2$. Preferred examples of the acyclic aliphatic hydrocarbon group having 1 to 10 carbon atoms of $R^2$ include methyl, ethyl, isopropyl, butyl, isobutyl, t-butyl, t-pentyl, vinyl, 2-propenyl, 2-methyl-1-propenyl, and 2-propenyl.

Substituents for the substituted acyclic aliphatic hydrocarbon group having 1 to 10 carbon atoms of $R^2$ include at least one substituent selected from the group consisting of a fluorine atom, a chlorine atom, a bromine atom, an iodine atom, a hydroxy group, an optionally substituted $C_1$-$C_7$ alkoxy group, a $C_6$-$C_{10}$ aryloxy group, a $C_7$-$C_9$ aralkoxy group, a $C_2$-$C_7$ acyloxy group, an oxo group, a $C_1$-$C_6$ alkylsulfonyloxy group, an optionally substituted $C_2$-$C_7$ acyl group, a carboxyl group, a $C_2$-$C_7$ alkoxycarbonyl group, a carbamoyl group, an optionally substituted $C_2$-$C_7$ alkylcarbamoyl group, an amino group, an optionally substituted $C_1$-$C_6$ alkylamino group, an optionally substituted $C_2$-$C_7$ acylamino group, a $C_2$-$C_8$ alkoxycarbonylamino group, a $C_1$-$C_6$ alkylsulfonylamino group, a cyano group, a nitro group, a $C_1$-$C_6$ alkylthio group, a $C_1$-$C_6$ alkylsulfynyl group, a $C_1$-$C_6$ alkylsulfonyl group, a sulfamoyl group, a $C_1$-$C_6$ alkylaminosulfonyl group, a sulfo group, an optionally substituted alicyclic hydrocarbon group having 1 to 6 carbon atoms, an optionally substituted acyclic aliphatic hydrocarbon group having 1 to 6 carbon atoms, an optionally substituted aromatic hydrocarbon group having 6 to 14 carbon atoms, and an optionally substituted heterocyclic group (having 1 to 4 atoms selected from the group consisting of an oxygen atom, a nitrogen atom and a sulfur atom, in the ring).

Specific examples of the substituent of the substituted acyclic aliphatic hydrocarbon group having 1 to 10 carbon atoms of $R^2$ include the same as those exemplified as the substituents of the substituted acyclic aliphatic hydrocarbon group having 1 to 10 carbon atoms of $G^2$.

As the substituent of substituted acyclic aliphatic hydrocarbon group having 1 to 10 carbon atoms as $R^2$, a $C_1$-$C_7$ alkoxy group, a $C_2$-$C_7$ acyl group, $C_2$-$C_7$ alkylcarbamoyl group, a $C_1$-$C_6$ alkylamino group, a $C_2$-$C_7$ acylamino group, a acyclic alicyclic hydrocarbon group having 3 to 6 carbon atoms, a acyclic aliphatic hydrocarbon group having 1 to 6 carbon atoms, aromatic hydrocarbon group having 6 to 14 carbon atoms, and heterocyclic group (having 1 to 4 atoms selected from the group consisting of an oxygen atom, a nitrogen atom and a sulfur atom, in the ring), may further be substituted with (one or more substituents selected from the group consisting of: a fluorine atom; a chlorine atom; a bromine atom; an iodine atom; a hydroxy group; a $C_1$-$C_6$ alkoxy group such as methoxy, ethoxy, propoxy, isopropoxy, butoxy, isobutoxy, s-butoxy, t-butoxy, pentyloxy or cyclopropyloxy; a methoxymethyloxy group; a 2-methoxyethoxy group; a formyl group; a trifluoroacetyl group; a $C_2$-$C_7$ acyl group such as acetyl, propionyl, butyryl, isobutyryl, valeryl or isovaleryl; an oxo group; a carboxyl group; a $C_2$-$C_7$ alkoxycarbonyl group such as methoxycarbonyl, ethoxycarbonyl, propoxycarbonyl, isopropoxycarbonyl, butoxycarbonyl, isobutoxycarbonyl or t-butoxycarbonyl; a carbamoyl group; a $C_2$-$C_7$ alkylcarbamoyl group such as N-methylcarbamoyl, N,N-dimethylcarbamoyl, N-ethylcarbamoyl, N-ethyl-N-methylcarbamoyl, N,N-diethyl- carbamoyl, N-propylcarbamoyl, N-isopropylcarbamoyl, N-butylcarbamoyl, N-cyclopropylcarbamoyl or N-cyclopropyl- methylcarbamoyl; an amino group; a $C_1$-$C_6$ alkylamino group such as methylamino, ethylamino, propylamino, isopropylamino, dimethylamino, N-ethylmethylamino, diethylamino, N-methylpropylamino, N-methylisopropylamino, cyclopropylamino or cyclopropylmethylamino; a $C_4$-$C_6$ cyclic amino group having 1 or 2 atoms selected from the group consisting of an oxygen atom, a nitrogen atom and a sulfur atoni, in the ring, such as 1-pyrrolidinyl, piperazinyl, 4-methylpiperazinyl, piperidino or morpholino; a trifluoroacetylamino group; a $C_1$-$C_7$ acylamino group such as formylamino, acetylamino, propionylamino, butyrylamino, isobutyrylamino or valerylamino; a $C_1$-$C_6$ alkylsulfonylamino group such as methylsulfonylamino, ethylsulfonylamino, propylsulfonylamino or butylsulfonylamino; a nitro group; a cyano group; a $C_1$-$C_6$ alkyl group including methyl, ethyl, propyl, isopropyl, butyl, isobutyl, s-butyl, and t-butyl; a trifluoromethyl group;

and a trifluoromethoxy group).

In the formula (I), when $R^2$ represents a substituted or unsubstituted alicyclic hydrocarbon group having 3 to 8 carbon atoms, examples of the alicyclic hydrocarbon group having 3 to 8 carbon atoms of $R^2$ the same as defined above for the substituents of the substituted alicyclic hydrocarbon group having 3 to 8 carbon atoms of $G^2$. Preferred examples of the alicyclic hydrocarbon group having 3 to 8 carbon atoms of $R^2$ include cyclopropyl, cyclobutyl, cyclopentyl and cyclohexyl. Among them, the cyclopropyl group is preferred.

Substituents of the substituted alicyclic hydrocarbon group having 3 to 8 carbon atoms of $R^2$ may be selected from the group consisting of a fluorine atom, a chlorine atom, a bromine atom, an iodine atom, a hydroxy group, an optionally substituted $C_1$-$C_7$ alkoxy group, a $C_6$-$C_{10}$ aryloxy group, a $C_7$-$C_9$ aralkoxy group, a $C_2$-$C_7$ acyloxy group, an oxo group, a $C_1$-$C_6$ alkylsulfonyloxy group, an optionally substituted $C_2$-$C_7$ acyl group, a carboxyl group, a $C_2$-$C_7$ alkoxycarbonyl group, a carbamoyl group, an optionally substituted $C_2$-$C_7$ alkylcarbamoyl group, an amino group, an optionally substituted $C_1$-$C_6$ alkylamino group, an optionally substituted $C_2$-$C_7$ acylamino group, a $C_2$-$C_8$ alkoxycarbonylamino group, a $C_1$-$C_6$ alkylsulfonylamino group, a cyano group, a nitro group, a $C_1$-$C_6$ alkylthio group, a $C_1$-$C_6$ alkylsulfynyl group, a $C_1$-$C_6$ alkylsulfonyl group, a sulfamoyl group, a $C_1$-$C_6$ alkylaminosulfonyl group, a sulfo group, an optionally substituted alicyclic hydrocarbon group having 3 to 6 carbon atoms, an optionally substituted aliphatic hydrocarbon group having 1 to 6 carbon atoms, an optionally substituted aromatic hydrocarbon group having 6 to 14 carbon atoms, and an optionally substituted heterocyclic group (having 1 to 4 atoms selected from the group consisting of an oxygen atom, a nitrogen atom and a sulfur atom, in the ring).

Specific examples of the substituent of the substituted alicyclic hydrocarbon group having 3 to 8 carbon atoms of $R^2$ include the same as those exemplified in the substituted acyclic alicyclic hydrocarbon group having 1 to 10 carbon atoms of $G^2$.

As the substituent of the substituted alicyclic hydrocarbon group having 3 to 8 carbon atoms of $R^2$, a $C_1$-$C_7$ alkoxy group, a $C_2$-$C_7$ acyl group, a $C_2$-$C_7$ alkylcarbamoyl group, a $C_1$-$C_6$ alkylamino group, a $C_2$-$C_7$ acylamino group, an alicyclic hydrocarbon group having 3 to 6 carbon atoms, an acyclic aliphatic hydrocarbon group having 1 to 6 carbon atoms, an aromatic hydrocarbon group having 6 to 14 carbon atoms and a heterocyclic group (having 1 to 4 atoms selected from the group consisting of an oxygen atom, a nitrogen atom and a sulfur atom, in the ring), may further be substituted with (one or more substituents selected from the group consisting of: a fluorine atom; a chlorine atom; a bromine atom; an iodine atom; a hydroxy group; a $C_1$-$C_6$ alkoxy group such as methoxy, ethoxy, propoxy, isopropoxy, butoxy, isobutoxy, s-butoxy, t-butoxy, pentyloxy or cyclopropyloxy; a methoxymethyloxy group; a 2-methoxyethoxy group; a formyl group; a trifluoroacetyl group; a $C_2$-$C_7$ acyl group such as acetyl, propionyl, butyryl, isobutyryl, valeryl or isovaleryl; an oxo'group; a carboxyl group; a $C_2$-$C_7$ alkoxycarbonyl group such as methoxycarbonyl, ethoxycarbonyl, propoxycarbonyl, isopropoxycarbonyl, butoxycarbonyl, isobutoxycarbonyl or t-butoxycarbonyl; a carbamoyl group; a $C_2$-$C_7$ alkylcarbamoyl group such as N-methylcarbamoyl, N,N-dimethylcarbamoyl, N-ethylcarbamoyl, N-ethyl-N-methylcarbamoyl, N,N-diethylcarbamoyl, N-propylcarbamoyl, N-isopropylcarbamoyl, N-butylcarbamoyl, N-cyclopropylcarbamoyl or N-cyclopropylmethylcarbamoyl; an amino group; a $C_1$-$C_6$ alkylamino group such as methylamino, ethylamino, propylamino, isopropylamino, dimethylamino, N-ethylmethyl- amino, diethylamino, N-methylpropylamino, N-methylisopropyl- amino, cyclopropylamino or cyclopropylmethylamino; a $C_4$-$C_6$ cyclic amino group having 1 or 2 atoms selected from the group consisting of an oxygen atom, a nitrogen atom and a sulfur atom, in the ring, such as 1-pyrrolidinyl, piperazinyl, 4-methylpiperazinyl, piperidino or morpholino; a trifluoroacetylamino group; a $C_1$-$C_7$ acylamino group such as formylamino, acetylamino, propionylamino, butyrylamino, isobutyrylamino or valerylamino; a $C_1$-$C_6$ alkylsulfonylamino group such as methylsulfonylamino, ethylsulfonylamino, propylsulfonylamino or butylsulfonylamino; a nitro group; a cyano group; a $C_1$-$C_6$ alkyl group including methyl, ethyl, propyl, isopropyl, butyl, isobutyl, s-butyl, and t-butyl; a trifluoromethyl group, and a trifluoromethoxy group).

In the formula (I), when $R^2$ is a substituted or unsubstituted aromatic hydrocarbon group having 6 to 14 carbon atoms, examples of the aromatic hydrocarbon group having 6 to 14 carbon atoms of $R^2$ include the same as those'exemplified in the aromatic hydrocarbon group having 6 to 14 carbon atoms of $G^2$. Examples of such preferred aromatic hydrocarbon group having 6 to 14 carbon atoms of $R^2$ include a phenyl group.

Exemplary substituents of the substituted aromatic hydrocarbon group having 6 to 14 carbon atoms include at least one substituent selected from the group consisting of a fluorine atom, a chlorine atom, a bromine atom, an iodine atom, a hydroxy group, a $C_1$-$C_6$ alkyl group, an optionally substituted $C_1$-$C_7$ alkoxy group, a $C_6$-$C_{10}$ aryloxy group, a $C_7$-$C_9$ aralkoxy group, a $C_2$-$C_7$ acyloxy group, an oxo group, a $C_1$-$C_6$ alkylsulfonyloxy group, an optionally substituted $C_2$-$C_7$ acyl group, a carboxyl group, a $C_2$-$C_7$ alkoxycarbonyl group, a.carbamoyl group, an optionally substituted $C_2$-$C_7$ alkylcarbamoyl group, an amino group, an optionally substituted $C_1$-$C_6$ alkylamino group, an optionally substituted $C_2$-$C_7$ acylamino group, a $C_2$-$C_8$ alkoxycarbonylamino group, a $C_1$-$C_6$ alkylsulfonylamino group, a cyano group, a nitro group, a $C_1$-$C_6$ alkylthio group, a $C_1$-$C_6$ alkylsulfynyl group, a $C_1$-$C_6$ alkylsulfonyl group, a sulfamoyl group, a $C_1$-$C_6$ alkylaminosulfonyl group, a sulfo group, an optionally substituted alicyclic hydrocarbon group having 3 to 6 carbon atoms, an optionally substituted acyclic aliphatic hydrocarbon group having 1 to 6 carbon atoms, an optionally substituted aromatic hydrocarbon group having 6 to 14 carbon atoms, and an optionally substituted heterocyclic group (having 1 to 4 atoms

selected from the group consisting of an oxygen atom, a nitrogen atom and a sulfur atom, in the ring).

Specific examples of the substituent of the substituted aromatic hydrocarbon group having 6 to 14 carbon atoms $R^2$ include the same as those exemplified for the substituted acyclic aliphatic hydrocarbon group having 1 to 10 carbon atoms of $G^2$.

As the substituent of the aromatic hydrocarbon group having 6 to 14 carbon atoms of $R^2$, a $C_1$-$C_7$ alkoxy group, a $C_2$-$C_7$ acyl group, a $C_2$-$C_7$ alkylcarbamoyl group, a $C_1$-$C_6$ alkylamino group, a $C_2$-$C_7$ acylamino group, an alicyclic hydrocarbon group having 3 to 6 carbon atoms, an acyclic aliphatic hydrocarbon group having 1 to 6 carbon atoms, an aromatic hydrocarbon group having 6 to 14 carbon atoms and heterocyclic group (having 1 to 4 atoms selected from the group.consisting of an oxygen atom, a nitrogen atom and a sulfur atom, in the ring), may further be substituted with (one or more substituents selected from the group consisting of: a fluorine atom; a chlorine atom; a bromine atom; an iodine atom; a hydroxy group; a $C_1$-$C_6$ alkoxy group such as methoxy, ethoxy, propoxy, isopropoxy, butoxy, isobutoxy, s-butoxy, t-butoxy, pentyloxy or cyclopropyloxy; a methoxymethyloxy group; a 2-methoxyethoxy group; a formyl group; a trifluoroacetyl group; a $C_2$-$C_7$ acyl group such as acetyl, propionyl, butyryl, isobutyryl, valeryl or isovaleryl; an oxo group; a carboxyl group; a $C_2$-$C_7$ alkoxycarbonyl group such as methoxycarbonyl, ethoxycarbonyl, propoxycarbonyl, isopropoxycarbonyl, butoxycarbonyl, isobutoxycarbonyl or t-butoxycarbonyl; a carbamoyl group; a $C_2$-$C_7$ alkylcarbamoyl group such as N-methylcarbamoyl, N,N-dimethylcarbamoyl, N-ethylcarbamoyl, N-ethyl-N-methylcarbamoyl, N,N-diethylcarbamoyl, N-propylcarbamoyl, N-isopropylcarbamoyl, N-butylcarbamoyl, N-cyclopropylcarbamoyl or N-cyclopropylmethylcarbamoyl; an amino group; a $C_1$-$C_6$ alkylamino group such as methylamino, ethylamino, propylamino, isopropylamino, dimethylamino, N-ethylmethylamino, diethylamino, N-methylpropylamino, N-methylisopropylamino, cyclopropylamino or cyclopropylmethylamino; a $C_4$-$C_6$ cyclic amino group having 1 or 2 atoms selected from the group consisting of an oxygen atom, a nitrogen atom and a sulfur atom, in the ring, such as 1-pyrrolidinyl, piperazinyl, 4-methylpiperazinyl, piperidino or morpholino; a trifluoroacetylamino group; a $C_1$-$C_7$ acylamino group such as formylamino, acetylamino, propionylamino, butyrylamino, isobutyrylamino or valerylamino; a $C_1$-$C_6$ alkylsulfonylamino group such as methylsulfonylamino, ethylsulfonylamino, propylsulfonylamino or butylsulfonylamino; a nitro group; a cyano group; a $C_1$-$C_6$ alkyl group including methyl, ethyl, propyl, isopropyl, butyl, isobutyl, s-butyl, and t-butyl; a trifluoromethyl group, and a trifluoromethoxy group).

In the formula (I), when $R^2$ represents a heterocyclic group having 1 to 4 atoms selected from the group consisting of an oxygen atom, a nitrogen atom and a sulfur atom on its substituted or unsubstituted ring of $R^2$, examples of heterocyclic group of $R^2$ include the same as those exemplified for the heterocyclic group of $G^2$. The heterocyclic group of $R^2$ links to $A^5$ on a carbon atom or a nitrogen atom.

Examples of preferred heterocyclic group linking to $A^5$ on a carbon atom include a monocyclic or cyclic $C_3$-$C_9$ aromatic heterocyclic group having 1 to 3 atoms selected from the group consisting of an oxygen atom, a nitrogen atom and a sulfur atom, in the ring, such as furyl, thienyl, pyrrolyl, pyrazolyl, oxazolyl, isooxazolyl, thiazolyl, isothiazolyl, imidazolyl, pyridyl, pyrazinyl, pyrimidinyl, pyridazinyl, benzofuranyl, indolyl, benzothienyl, quinolyl, isoquinolyl, quinazolyl, benzoimidazolyl or benzooxazolyl. More preferred example of the heterocyclic group include a monocyclic or bicyclic $C_3$-$C_9$ aromatic heterocyclic compound having 1 or 2 atoms selected from the group consisting of an oxygen atom, a nitrogen atom and a sulfur atom, in the ring, such as 2-furyl, 2-thienyl, 2-pyrrolyl, 2-imidazolyl, 5-imidazolyl, 4-pyrazolyl, 2-oxazolyl, 5-oxazolyl, 5-isooxazolyl, 2-thiazolyl, 5-thiazolyl, 5-isothiazolyl, 3-isothiazolyl, 2-pyridyl, 2-pyrimidinyl, 2-benzofuranyl or 2-benzothiophenyl group. Further, particularly preferable examples of the heterocyclic group include a monocyclic $C_3$-$C_5$ aromatic heterocyclic group having 1 or 2 atoms selected from the group consisting of an oxygen atom, a nitrogen atom and a sulfur atom, in the ring, and most preferably, 2-furyl, 2-thienyl, 2-pyrrolyl, 2-pyridyl or 4-pyrazolyl.

Meanwhile, preferred examples of the heterocyclic group having 1 to 4 atoms selected from the group consisting of an oxygen atom, a nitrogen atom and a sulfur atom, in the ring, links to $A^5$ on a nitrogen atom, include 1-pyrazolyl, 1-imidazolyl, 1-pyrrolidinyl, piperidino, morpholino, 1-homopiperidinyl and 1-piperazinyl. When the heterocyclic group having 1 to 4 atoms selected from the group consisting of an oxygen atom, a nitrogen atom and a sulfur atom on a ring of $R^2$, links to $A^5$ on a nitrogen atom, $A^5$ represents a single bond.

Substituents of the heterocyclic group having 1 to 4 atoms selected from the group consisting of an oxygen atom, a nitrogen atom and a sulfur atom on the substituted ring of $R^2$ may be selected from the group consisting of a fluorine atom, a chlorine atom, a bromine atom, an iodine atom, a hydroxy group, an optionally substituted $C_1$-$C_7$ alkoxy group, a $C_6$-$C_{10}$ aryloxy group, a $C_7$-$C_9$ aralkoxy group, a $C_2$-$C_7$ acyloxy group, an oxo group, a $C_1$-$C_6$ alkylsulfonyloxy group, an optionally substituted $C_2$-$C_7$ acyl group, a carboxyl group, a $C_2$-$C_7$ alkoxycarbonyl group, a carbamoyl group, an optionally substituted $C_2$-$C_7$ alkylcarbamoyl group, an amino group, an optionally substituted $C_1$-$C_6$ alkylamino group, an optionally substituted $C_2$-$C_7$ acylamino group, a $C_2$-$C_8$ alkoxycarbonylamino group, a $C_1$-$C_6$ alkylsulfonylamino group, a cyano group, a nitro group, a $C_1$-$C_6$ alkylthio group, a $C_1$-$C_6$ alkylsulfynyl group, a $C_1$-$C_6$ alkylsulfonyl group, a sulfamoyl group, a $C_1$-$C_6$ alkylaminosulfonyl group, a sulfo group, an optionally substituted alicyclic hydrocarbon group having 3 to 6 carbon atoms, an optionally substituted acyclic aliphatic hydrocarbon group having 1 to 6 carbon atoms, an optionally substituted aromatic hydrocarbon group having 6 to 14 carbon atoms, and an optionally substituted heterocyclic group

(having 1 to 4 atoms selected from the group consisting of an oxygen atom, a nitrogen atom and a sulfur atom, in the ring).

Specific examples of the substituent of the heterocyclic group having 1 to 4 atoms selected from the group consisting of an oxygen atom, a nitrogen atom and a sulfur atom on its substituted ring of $R^2$ include the same as those exemplified as the substituents of the acyclic substituted aliphatic hydrocarbon group having 1 to 10 carbon atoms of $G^2$.

As the substituent of the heterocyclic group having 1 to 4 atoms selected from the group consisting of an oxygen atom, a nitrogen atom and a sulfur atom on its substituted ring of $R^2$, a $C_1$-$C_7$ alkoxy group, a $C_2$-$C_7$ acyl group, a $C_2$-$C_7$ alkylcarbamoyl group, a $C_1$-$C_6$ alkylamino group, a $C_2$-$C_7$ acylamino group, an alicyclic hydrocarbon group having 3 to 6 carbon atoms, an acyclic aliphatic hydrocarbon group having 1 to 6 carbon atoms, an aromatic hydrocarbon group having 6 to 14 carbon atoms, and a heterocyclic group (having 1 to 4 atoms selected from the group consisting of an oxygen atom, a nitrogen atom and a sulfur atom, in the ring) may further be substituted with (one or more substituents selected from the group consisting of: a fluorine atom; chlorine atom; a bromine atom; an iodine atom; a hydroxy group; a $C_1$-$C_6$ alkoxy group such as methoxy, ethoxy, propoxy, isopropoxy, butoxy, isobutoxy, s-butoxy, t-butoxy, pentyloxy or cyclopropyloxy; a methoxymethyloxy group; a 2-methoxyethoxy group; a formyl group; a trifluoroacetyl group; a $C_2$-$C_7$ acyl group such as acetyl, propionyl, butyryl, isobutyryl, valeryl or isovaleryl; an oxo group; a carboxyl group; a $C_2$-$C_7$ alkoxycarbonyl group such as methoxycarbonyl, ethoxycarbonyl, propoxycarbonyl, isopropoxycarbonyl, butoxycarbonyl, isobutoxycarbonyl or t-butoxycarbonyl; a carbamoyl group; a $C_2$-$C_7$ alkylcarbamoyl group such as N-methylcarbamoyl, N,N-dimethylcarbamoyl, N-ethylcarbamoyl, N-ethyl-N-methylcarbamoyl, N,N-diethylcarbamoyl, N-propylcarbamoyl, N-isopropylcarbamoyl, N-butylcarbamoyl, N-cyclopropylcarbamoyl or N-cyclopropylmethylcarbamoyl; an amino group; a $C_1$-$C_6$ alkylamino group such as methylamino, ethylamino, propylamino, isopropylamino, dimethylamino, N-ethylmethylamino, diethylamino, N-methylpropylamino, N-methylisopropylamino, cyclopropylamino or cyclopropylmethylamino; a $C_4$-$C_6$ cyclic amino group having 1 or 2 atoms selected from the group consisting of an oxygen atom, a nitrogen atom and a sulfur atom, in the ring, such as 1-pyrrolidinyl, piperazinyl, 4-methylpiperazinyl, piperidino or morpholino; a trifluoroacetylamino group; a $C_1$-$C_7$ acylamino group such as formylamino, acetylamino, propionylamino, butyrylamino, isobutyrylamino or valerylamino; a $C_1$-$C_6$ alkylsulfonylamino group such as methylsulfonylamino, ethylsulfonylamino, propylsulfonylamino or butylsulfonylamino; a nitro group; a cyano group; a $C_1$-$C_6$ alkyl group including methyl, ethyl, propyl, isopropyl, butyl, isobutyl, s-butyl, and t-butyl; a trifluoromethyl group; and a trifluoromethoxy group).

Among exemplary substituents of the heterocyclic group having 1 to 4 atoms selected from the group consisting of an oxygen atom, a nitrogen atom and a sulfur atom on its substituted ring of $R^2$, preferred examples of the substituent include a fluorine atom, a chlorine atom, a bromine atom, an iodine atom, a hydroxy group, a cyano group, a nitro group, an amino group, a $C_{1-}C_6$ mono or dialkylamino group consisting of a straight or branched alkyl group and an amino group, such as substituted or unsubstituted methylamino, ethylamino, propylamino, isopropylamino, butylamino, isobutylamino, s-butylamino, t-butylamino, pentylamino, hexylamino, dimethylamino, N-ethylmethylamino, diethylamino, N-methylpropylamino, N-methylisopropylamino, N-methylbutylamino, N-methyl-t- butylamino, N-ethylisopropylamino, dipropylamino, diisopropylamino and ethylbutylamino, a'carboxyl group, an optionally substituted saturated a $C_1$-$C_6$ alkyl group including a substituted or unsubstituted methyl, ethyl, propyl, isopropyl, butyl, isobutyl, s-butyl, t-butyl, pentyl, isopentyl, neopentyl, t-pentyl, hexyl, isohexyl, 2-methylpentyl and 1-ethylbutyl, an alicyclic hydrocarbon group having 3 to 6 carbon atoms including cyclopropyl, cyclobutyl, cycle pentyl and cyclohexyl, an optionally substituted $C_1$-$C_6$ alkoxy group consisting of a straight or branched alkyl group and an oxy group, including a substituted or unsubstituted methoxy, ethoxy, propoxy, isopropoxy, butoxy, isobutoxy, s-butoxy, t-butoxy, pentyloxy, isopentyloxy, neopentyloxy, t-pentyloxy and hexyloxy, a $C_2$-$C_7$ acyl group, including a substituted or unsubstituted acetyl, propionyl, butyryl, isobutyryl, pivaroyl and hexanoyl, a $C_1$-$C_6$ alkylthio group, including methylthio, ethylthio, propylthio, isopropylthio, butylthio, isobutylthio, s-butylthio, t-butylthio, pentylthio and hexylthio, trifluoromethyl group, trifluoromethoxy group, a $C_2$-$C_7$ acylamino group, including substituted or unsubstituted acetylamino, propionylamino, butyrylamino, isobutyrylamino, valerylamino and hexanoylamino, and a $C_2$-$C_7$ alkylcarbamoyl group consisting of a straight or branched alkyl group and a carbamoyl group, including a substituted or unsubstituted N-methylcarbamoyl, N-ethylcarbamoyl, N-propylcarbamoyl, N-isopropylcarbamoyl, N-butylcarbamoyl, N-isobutylcarbamoyl, N-s-butylcarbamoyl, N-t-butylcarbamoyl, N-pentylcarbamoyl, N,N-dimethylcarbamoyl, N-ethyl-N-methylcarbamoyl and N,N-diethylcarbamoyl.

More preferred examples of the substituent of the heterocyclic group having 1 to 4 atoms selected from the group consisting of an oxygen atom, a nitrogen atom and a sulfur atom on its substituted ring as $R^2$, include any or more of a fluorine atom, a chlorine atom, a bromine atom, an acyl group having 2 to 4 carbon atoms, a hydroxy group, a carboxyl group, an alkoxycarbonyl group, a substituted or unsubstituted $C_1$-$C_6$ alkyl group, a hydroxy group, and a substituted or unsubstituted $C_1$-$C_6$ alkoxy group.

Here, an explanation will be given of preferred combinations of $R^2$ and $A^5$ of the formula (I).

In combinations of $R^2$ and $A^5$ of the formula (I) in the present invention, when $R^2$ is a fluorine atom, a chlorine atom, a bromine atom, or an iodine atom, $A^5$ represents a single bond.

Preferred examples of the combinations of $R^2$ and $A^5$ of the formula (I) in the present invention include combinations wherein $A^5$ represents a single bond and $R^2$ represents an optionally substituted acyclic aliphatic hydrocarbon group having 1 to 10 carbon atoms, an optionally substituted alicyclic hydrocarbon group having 3 to 8 carbon atoms,

an optionally substituted aromatic hydrocarbon group having 6 to 14 carbon atoms, or an optionally substituted heterocyclic group. Specific preferred combinations are combinations wherein $A^5$ represents a single bond and $R^2$ represents an acylic aliphatic hydrocarbon group having 1 to 10 carbon atoms, an alicyclic hydrocarbon group having 3 to 8 carbon atoms, an optionally substituted phenyl group, or an optionally substituted heterocyclic group having 1 or 2 atoms selected from the group consisting of an oxygen atom, a nitrogen atom and a sulfur atom, in the ring. Among them, cases where $R^2$ represents a cyclopropyl group, a cyclobutyl group, a cyclopropylmethyl group, a methyl group, an ethyl group, a vinyl group, an isopropyl group, an isobutyl group or 2-methyl-1-propenyl group are preferred.

Other preferable examples of combinations of $A^5$ and $R^2$ are combinations wherein $A^5$ represents a single bond and $R^2$ represents a thienyl group, a pyridyl group, a furyl group, a pyrrolyl group, a pyrazolyl group or phenyl group, any of which may be further substituted by one or more of a $C_1$-$C_4$ alkyl group, a $C_1$-$C_4$ alkoxy group, a $C_2$-$C_4$ acyl group, a hydroxy group, a carboxyl group, an alkoxycarbonyl group, a fluorine atom or a chlorine atom.

Also a combination wherein $A^5$ is $NR^{201}$-, and $R^2$ represents a hydrogen atom or an optionally substituted acyclic aliphatic hydrocarbon group having 1 to 10 carbon atoms is preferred.

In the formula (I), $A^6$ is a bond representing a single bond, a group that links a carbon atom of a pyrrole ring in which $R^3$ and $A^6$ are linked to each other in the form of $R^3$-$NR^{301}$-pyrrole ring, $R^3$-C(=O)-pyrrole ring, $R^3$-$NR^{302}$-C (=O)-pyrrole ring, $R^3$-$NR^{303}$-C(=S)-pyrrole ring, $R^3$-$NR^{304}$-C(=O)-$NR^{305}$-pyrrole ring, $R^3$-C(=O)-$NR^{306}$-pyrrole ring, $R^3$-$NR^{307}$-CH=N-pyrrole ring, $R^3$-O-C(=O)-pyrrole ring, $R^3$-C(=O)-O-pyrrole ring, $R^3$-O-pyrrole ring, $R^3$-S-pyrrole ring, $R^3$-S(=O)-pyrrole ring, $R^3$-$S(=O)_2$-pyrrole ring, $R^3$-$CR^{308}$=$CR^{309}$-pyrrole ring, $R^3$-C≡C-pyrrole ring, or $R^3$- $S(=O)_2$-C≡C-pyrrole ring($R^{301}$ through $R^{309}$ are each independently a hydrogen atom or a $C_1$-$C_4$ acyclic aliphatic hydrocarbon group.)

When $R^3$ and the carbon atom on the pyrrole ring are connected each other in the form of $R^3$-$NR^{301}$-pyrrole ring, examples of such $C_1$-$C_4$ acyclic aliphatic hydrocarbon group of $R^{301}$ include the same as those selected as the examples of $R^{101:}$ in $A^2$. Examples of such preferred $R^{301}$ include a hydrogen atom, methyl, and ethyl group. Particularly, a hydrogen atom is preferred.

When linking a carbon atom of a pyrrole ring in which $R^3$ and $A^6$ are linked to each other in the form of $R^3$-$NR^{302}$-C (=O)-pyrrole ring, examples of such $C_1$-$C_4$ acyclic aliphatic hydrocarbon group of $R^{302}$ include the same as those selected as the examples of $R^{101}$ in $A^2$. Examples of such preferred $R^{302}$ include a hydrogen atom, methyl, and ethyl group. Particularly, a hydrogen atom is preferred.

When linking a carbon atom of a pyrrole ring in which $R^3$ and $A^6$ are linked to each other in the form of $R^3$-$NR^{303}$-C (=S)-pyrrole ring, examples of such $C_1$-$C_4$ acyclic aliphatic hydrocarbon group of $R^{303}$ include the same as those selected as the examples of $R^{101}$ in $A^2$. Examples of such preferred $R^{303}$ include a hydrogen atom, methyl, and ethyl group. Particularly, a hydrogen atom is preferred.

When linking a carbon atom of a pyrrole ring in which $R^3$ and $A^6$ are linked to each other in the form of $R^3$-$NR^{304}$-C (=O)-$NR^{305}$-pyrrole ring, examples of such $C_1$-$C_4$ acyclic' aliphatic hydrocarbon group of $R^{304}$ and $R^{305}$ include the same as those selected as the examples of $R^{101}$ in $A^2$. Examples of such preferred $R^{304}$ and $R^{305}$ include a hydrogen atom, methyl, and ethyl group. Particularly, a hydrogen atom is preferred.

When linking a carbon atom of a pyrrole ring in which $R^3$ and $A^6$ are linked to each other in the form of $R^3$-C (=O)-$NR^{306}$-pyrrole ring, examples of such $C_1$-$C_4$ acyclic aliphatic hydrocarbon group of $R^{306}$ include the same as those selected as the examples of $R^{101}$ in $A^2$. Examples of such preferred $R^{306}$ include a hydrogen atom, methyl, and ethyl group. Particularly, a hydrogen atom'is preferred.

When linking a carbon atom of a pyrrole ring in which $R^3$ and $A^6$ are linked to each other in the form of $R^3$-$NR^{307}$-CH=N-pyrrole ring, examples of such $C_1$-$C_4$ acyclic aliphatic hydrocarbon group of $R^{307}$ include the same as those selected as the examples of $R^{101}$ in $A^2$. Examples of such preferred $R^{307}$ include a hydrogen atom, methyl, and ethyl group. Particularly, methyl group is preferred.

When linking a carbon atom of a pyrrole ring in which $R^3$ and $A^6$ are linked to each other in the form of $R^3$-$CR^{308}$=$CR^{309}$-pyrrole ring, examples of such $C_1$-$C_4$ acyclic aliphatic hydrocarbon group of $R^{308}$ and $R^{309}$ include the same as those selected as the examples of $R^{101}$ in $A^2$.

In the formula (I), $R^3$ represents a hydrogen atom, a fluorine atom, a chlorine atom, a bromine atom, an iodine atom, a nitro group, a substituted or unsubstituted saturated acyclic aliphatic hydrocarbon group having 1 to 10 carbon atoms, a substituted or unsubstituted alicyclic hydrocarbon group having 3 to 8 carbon atoms, a substituted or unsubstituted aromatic hydrocarbon group having 6 to 14 carbon atoms, or a heterocyclic group having 1 to 4 atoms selected from the group consisting of an oxygen atom, a nitrogen atom and a sulfur atom.

As $R^3$ in the formula (I), among a fluorine atom, a chlorine atom, a bromine atom, and an iodine atom, a chlorine atom, a bromine atom, and an iodine atom are preferred.

In the formula (I), when $R^3$ represents a substituted or unsubstituted acyclic saturated aliphatic hydrocarbon group having 1 to 10 carbon atoms, examples of the saturated acyclic aliphatic hydrocarbon group having 1 to 10 carbon atoms of $R^3$ include an alkyl group, including methyl, ethyl, propyl, isopropyl, butyl, isobutyl, s-butyl, t-butyl, pentyl, isopentyl, neopentyl, t-pentyl, 2-methylpentyl, 4-methylpentyl, 1-ethylbutyl, hexyl, heptyl, 2-methylhexyl, 5-methylhexyl, 1,1-dimethylpentyl, 6-methylheptyl, octyl, nonyl, and decyl. Preferred examples of the acyclic saturated aliphatic hydrocarbon group having 1 to 10 carbon atoms of $R^3$ include methyl, ethyl, isopropyl, butyl, t-butyl, and t-pentyl group.

As the substituent of the substituted acyclic saturated aliphatic hydrocarbon group having 1 to 10 carbon atoms of $R^3$ include at least one substituent selected from the group consisting of a fluorine atom, a chlorine atom, a bromine atom, an iodine atom, a hydroxy group, an optionally substituted $C_1$-$C_7$ alkoxy group, a $C_6$-$C_{10}$ aryloxy group, a $C_7$-$C_9$ aralkoxy group, a $C_2$-$C_7$ acyloxy group, an oxo group, an optionally substituted $C_2$-$C_7$ acyl group, a carboxyl group, a $C_2$-$C_7$ alkoxycarbonyl group, a carbamoyl group, an optionally substituted $C_2$-$C_7$ alkylcarbamoyl group, an amino group, an optionally substituted $C_1$-$C_6$ alkylamino group, an optionally substituted $C_2$-$C_7$ acylamino group, a $C_2$-$C_8$ alkoxycarbonylamino group, a $C_1$-$C_6$ alkylsulfonylamino group, a cyano group, a nitro group, a $C_1$-$C_6$ alkylthio group, a $C_1$-$C_6$ alkylsulfynyl group, an optionally substituted alicyclic hydrocarbon group having 3 to 6 carbon atoms, an optionally substituted acyclic aliphatic hydrocarbon group having 1 to 6 carbon atoms, an optionally substituted aromatic hydrocarbon group having 6 to 14 carbon atoms and an optionally substituted heterocyclic group (having 1 to 4 atoms selected from the group consisting of an oxygen atom, a nitrogen atom and a sulfur atom, in the ring).

Specific examples of the substituent of the substituted acyclic saturated aliphatic hydrocarbon group having 1 to 10 carbon atoms of $R^3$ include the same as those exemplified in the substituted acyclic aliphatic hydrocarbon group having 1 to 10 carbon atoms of $G^2$.

As the substituent of the substituted acyclic saturated aliphatic hydrocarbon group having 1 to 10 carbon atoms of $R^3$, a $C_1$-$C_7$ alkoxy group, a $C_2$-$C_7$ acyl group, a $C_2$-$C_7$ alkylcarbamoyl group, a $C_1$-$C_6$ alkylamino group, a $C_2$-$C_7$ acylamino group, an alicyclic hydrocarbon group having 3 to 6 carbon atoms, an aliphatic acyclic hydrocarbon group having 1 to 6 carbon atoms, an aromatic hydrocarbon group having 6 to 14 carbon atoms and a heterocyclic group (having 1 to 4 atoms selected from the group consisting of an oxygen atom, a nitrogen atom and a sulfur atom, in the ring), may further be substituted with (one or more substituents selected from the group consisting of: a fluorine atom; a chlorine atom; a bromine atom; an iodine atom; a hydroxy group; a $C_1$-$C_6$ alkoxy group such as methoxy, ethoxy, propoxy, isopropoxy, butoxy, isobutoxy, s-butoxy, t-butoxy, pentyloxy or cyclopropyloxy; a methoxymethyloxy group; a 2-methoxyethoxy group; a formyl group; a trifluoroacetyl group; a $C_2$-$C_7$ acyl group such as acetyl, propionyl, butyryl, isobutyryl, valeryl or isovaleryl; an oxo group; a carboxyl group; a $C_2$-$C_7$ alkoxycarbonyl group such as methoxycarbonyl, ethoxycarbonyl, propoxycarbonyl, isopropoxycarbonyl, butoxycarbonyl, isobutoxycarbonyl or t-butoxycarbonyl; a carbamoyl group; a $C_2$-$C_7$ alkylcarbamoyl group such as N-methylcarbamoyl, N,N-dimethylcarbamoyl, N-ethylcarbamoyl, N-ethyl-N-methylcarbamoyl, N,N-diethylcarbamoyl, N-propylcarbamoyl, N-isopropylcarbamoyl, N-butylcarbamoyl, N-cyclopropylcarbamoyl or N-cyclopropylmethylcarbamoyl; an amino group; a $C_1$-$C_6$ alkylamino group such as methylamino, ethylamino, propylamino, isopropylamino, dimethylamino, N-ethylmethyl- amino, diethylamino, N-methylpropylamino, N-methylisopropyl- amino, cyclopropylamino or cyclopropylmethylamino; a $C_4$-$C_6$ cyclic amino group having 1 or 2 atoms selected from the group consisting of an oxygen atom, a nitrogen atom and a sulfur atom, in the ring, such as 1-pyrrolidinyl, piperazinyl, 4-methylpiperazinyl, piperidino or morpholino; a trifluoroacetylamino group; a $C_1$-$C_7$ acylamino group such as formylamino, acetylamino, propionylamino, butyrylamino, isobutyrylamino or valerylamino; a $C_1$-$C_6$ alkylsulfonylamino group such as methylsulfonylamino, ethylsulfonylamino, propylsulfonylamino or butylsulfonylamino; a nitro group; a cyano group; a $C_1$-$C_6$ alkyl group including methyl, ethyl, propyl, isopropyl, butyl, isobutyl, s-butyl, and t-butyl; a trifluoromethyl group; and a trifluoromethoxy group).

In the formula (I), when $R^3$ represents a substituted or unsubstituted alicyclic hydrocarbon group having 3 to 8 carbon atoms, examples of the alicyclic hydrocarbon group having 3 to 8 carbon atoms of $R^2$ include the same as those exemplified in the alicyclic hydrocarbon group having 3 to 10 carbon atoms of $G^2$. Preferred examples of the alicyclic hydrocarbon group having 3 to 8 carbon atoms of $R^2$ include cyclopropyl, cyclobutyl and cyclopentyl, cyclohexyl.

Exemplary substituents of the substituted alicyclic hydrocarbon group having 3 to 8 carbon atoms of $R^3$ include at least one substituent selected from the group consisting of a fluorine atom, a chlorine atom, a bromine atom, an iodine atom, a hydroxy group, an optionally substituted $C_1$-$C_7$ alkoxy group, a $C_6$-$C_{10}$ aryloxy group, a $C_7$-$C_9$ aralkoxy group, a $C_2$-$C_7$ acyloxy group, an optionally substituted $C_2$-$C_7$ acyl group, a carboxyl group, a $C_2$-$C_7$ alkoxycarbonyl group, a carbamoyl group, an optionally substituted $C_2$-$C_7$ alkylcarbamoyl group, an amino group, an optionally substituted $C_1$-$C_6$ alkylamino group, an optionally substituted $C_2$-$C_7$ acylamino group, a $C_2$-$C_8$ alkoxycarbonylamino group, a cyano group, a nitro group, an optionally substituted alicyclic hydrocarbon group having 3 to 6 carbon atoms, an optionally substituted acyclic aliphatic hydrocarbon group having 1 to 6 carbon atoms, an optionally substituted aromatic hydrocarbon group having 6 to 14 carbon atoms and an optionally substituted heterocyclic group (having 1 to 4 atoms selected from the group consisting of an oxygen atom, a nitrogen atom and a sulfur atom, in the ring).

Specific examples'of the substituent of the substituted alicyclic hydrocarbon group having 3 to 8 carbon atoms of $R^3$ include the same as those exemplified as the substituents of the substituted acyclic aliphatic hydrocarbon group having 1 to 10 carbon atoms of $G^2$.

As the substituent of the substituted alicyclic hydrocarbon group having 3 to 8 carbon atoms of $R^3$, a $C_1$-$C_7$ alkoxy group, a $C_2$-$C_7$ acyl group, a $C_2$-$C_7$ alkylcarbamoyl group, a $C_1$-$C_6$ alkylamino group, a $C_2$-$C_7$ acylamino, an alicyclic hydrocarbon group having 3 to 6 carbon atoms, an acyclic aliphatic hydrocarbon group having 1 to 6 carbon atoms, an aromatic hydrocarbon group having 6 to 14 carbon atoms, and a heterocyclic group (having 1 to 4 atoms

selected from the group consisting of an oxygen atom, a nitrogen atom and a sulfur atom, in the ring), may further be substituted with (one or more substituents selected from the group consisting of: a fluorine atom; a chlorine atom; a bromine atom; an iodine atom; a hydroxy group; a $C_1$-$C_6$ alkoxy group such as methoxy, ethoxy, propoxy, isopropoxy, butoxy, isobutoxy, s-butoxy, t-butoxy, pentyloxy or cyclopropyloxy; a methoxymethyloxy group; a 2-methoxyethoxy group; a formyl group; a trifluoroacetyl group; a $C_2$-$C_7$ acyl group such as acetyl, propionyl, butyryl, isobutyryl, valeryl or isovaleryl; an oxo group; a carboxyl group; a $C_2$-$C_7$ alkoxycarbonyl group such as methoxycarbonyl, ethoxycarbonyl, propoxycarbonyl, isopropoxycarbonyl, butoxycarbonyl, isobutoxycarbonyl or t-butoxycarbonyl; a carbamoyl group; a $C_2$-$C_7$ alkylcarbamoyl group such as N-methylcarbamoyl, N,N-dimethylcarbamoyl, N-ethylcarbamoyl, N-ethyl-N-methylcarbamoyl, N,N-diethyl- carbamoyl, N-propylcarbamoyl, N-isopropylcarbamoyl, N-butylcarbamoyl, N-cyclopropylcarbamoyl or N-cyclopropyl- methylcarbamoyl; an amino group; a $C_1$-$C_6$ alkylamino group such as methylamino, ethylamino, propylamino, isopropylamino, dimethylamino, N-ethylmethylamino, diethylamino, N-methylpropylamino, N-methylisopropylamino, cyclopropylamino or cyclopropylmethylamino; a $C_4$-$C_6$ cyclic amino group having 1 or 2 atoms selected from the group consisting of an oxygen atom, a nitrogen atom and a sulfur atom, in the ring, such as 1-pyrrolidinyl, piperazinyl, 4-methylpiperazinyl, piperidino or morpholino; a trifluoroacetylamino group; a $C_1$-$C_7$ acylamino group such as formylamino, acetylamino, propionylamino, butyrylamino, isobutyrylamino or valerylamino; a $C_1$-$C_6$ alkylsulfonylamino group such as methylsulfonylamino, ethylsulfonylamino, propylsulfonylamino or butylsulfonylamino; a nitro group; a cyano group; a $C_1$-$C_6$ alkyl group including methyl, ethyl, propyl, isopropyl, butyl, isobutyl, s-butyl, and t-butyl; a trifluoromethyl group; and a trifluoromethoxy group).

In the formula (I), when $R^3$ represents a substituted or unsubstituted aromatic hydrocarbon group having 6 to 14 carbon atoms, examples of the aromatic hydrocarbon group having 6 to 14 carbon atoms of $R^3$ include the same as those exemplified in the aromatic hydrocarbon group having 6 to 14 carbon atoms of $G^2$. Preferred examples of the aromatic hydrocarbon group having 6 to 14 carbon atoms of $R^3$ include a phenyl group.

Exemplary substituents of the substituted aromatic hydrocarbon group having 6 to 14 carbon atoms of $R^3$ include at least one substituent selected from the group consisting of a fluorine atom, a chlorine atom, a bromine atom, an iodine atom, a hydroxy group, an optionally substituted $C_1$-$C_7$ alkoxy group, a $C_6$-$C_{10}$ aryloxy group, a $C_7$-$C_9$ aralkoxy group, a $C_2$-$C_7$ acyloxy group, an oxo group, a $C_1$-$C_6$ alkylsulfonyloxy group, an optionally substituted $C_2$-$C_7$ acyl group, a carboxyl group, a $C_2$-$C_7$ alkoxycarbonyl group, a carbamoyl group, an optionally substituted $C_2$-$C_7$ alkylcarbamoyl group, an amino group, an optionally substituted $C_1$-$C_6$ alkylamino group, an optionally substituted $C_2$-$C_7$ acylamino group, a $C_2$-$C_8$ alkoxycarbonylamino group, a $C_1$-$C_6$ alkylsulfonylamino group, a cyano group, a nitro group, a $C_1$-$C_6$ alkylthio group, a $C_1$-$C_6$ alkylsulfynyl group, a $C_1$-$C_6$ alkylsulfonyl group, a sulfamoyl group, a $C_1$-$C_6$ alkylaminosulfonyl group, a sulfo group, an optionally substituted alicyclic hydrocarbon group having 3 to 6 carbon atoms, an optionally substituted acyclic aliphatic hydrocarbon group having 1 to 6 carbon atoms, an optionally substituted aromatic hydrocarbon group having 6 to 14 carbon atoms and an optionally substituted heterocyclic group (having 1 to 4 atoms selected from the group consisting of an oxygen atom, a nitrogen atom and a sulfur atom, in the ring).

Specific examples of the substituent of the substituted aromatic hydrocarbon group having 6 to 14 carbon atoms of $R^3$ include the same as those exemplified in the substituent of the substituted acyclic aliphatic hydrocarbon group having 1 to 10 carbon atoms of $G^2$.

As the substituent of the substituted aromatic hydrocarbon group having 6 to 14 carbon atoms of $R^3$, a $C_1$-$C_7$ alkoxy group, a $C_2$-$C_7$ acyl group, a $C_2$-$C_7$ alkylcarbamoyl group, a $C_1$-$C_6$ alkylamino group, a $C_2$-$C_7$ acylamino group, an alicyclic hydrocarbon group having 3 to 6 carbon atoms, an acyclic aliphatic hydrocarbon group having 1 to 6 carbon atoms, an aromatic hydrocarbon group having 6 to 14 carbon atoms and a heterocyclic group (having 1 to 4 atoms selected from the group consisting of an oxygen atom, a nitrogen atom and a sulfur atom, in the ring), may further be substituted with (one or more substituents selected from the group consisting of: a fluorine atom; a chlorine atom; a bromine atom; an iodine atom; a hydroxy group; a $C_1$-$C_6$ alkoxy group such as methoxy, ethoxy, propoxy, isopropoxy, butoxy, isobutoxy, s-butoxy, t-butoxy, pentyloxy or cyclopropyloxy; a methoxymethyloxy group; a 2-methoxyethoxy group; a formyl group; a trifluoroacetyl group; a $C_2$-$C_7$ acyl group such as acetyl, propionyl, butyryl, isobutyryl, valeryl or isovaleryl; an oxo group; a carboxyl group; a $C_2$-$C_7$ alkoxycarbonyl group such as methoxycarbonyl, ethoxycarbonyl, propoxycarbonyl, isopropoxycarbonyl, butoxycarbonyl, isobutoxycarbonyl or t-butoxycarbonyl; a carbamoyl group; a $C_2$-$C_7$ alkylcarbamoyl group such as N-methylcarbamoyl, N,N-dimethylcarbamoyl, N-ethylcarbamoyl, N-ethyl-N-methylcarbamoyl, N,N-diethylcarbamoyl, N-propylcarbamoyl, N-isopropylcarbamoyl, N-butylcarbamoyl, N-cyclopropylcarbamoyl or N-cyclopropylmethylcarbamoyl; an amino group; a $C_1$-$C_6$ alkylamino group such as methylamino, ethylamino, propylamino, isopropylamino, dimethylamino, N-ethylmethyl- amino, diethylamino, N-methylpropylamino, N-methylisopropyl- amino, cyclopropylamino or cyclopropylmethylamino; a $C_4$-$C_6$ cyclic amino group having 1 or 2 atoms selected from the group consisting of an oxygen atom, a nitrogen atom and a sulfur atom, in the ring, such as 1-pyrrolidinyl, piperazinyl, 4-methylpiperazinyl, piperidino or morpholino; a trifluoroacetylamino group; a $C_1$-$C_7$ acylamino group such as formylamino, acetylamino, propionylamino, butyrylamino, isobutyrylamino or valerylamino; a $C_1$-$C_6$ alkylsulfonylamino group such as methylsulfonylamino, ethylsulfonylamino, propylsulfonylamino or butylsulfonylamino; a nitro group; a cyano group; a $C_1$-$C_6$ alkyl group including methyl, ethyl, propyl, isopropyl, butyl, isobutyl, s-butyl, and t-butyl; a trifluoromethyl group;

and a trifluoromethoxy group).

In the formula (I), when $R^3$ represents a heterocyclic group having 1 to 4 atoms selected from the group consisting of an oxygen atom, a nitrogen atom and a sulfur atom on its substituted or unsubstituted ring, examples of such heterocyclic group having 1 to 4 atoms selected from the group consisting of an oxygen atom, a nitrogen atom and a sulfur atom on the ring of $R^3$ include the same as those exemplified in the heterocyclic group having 1 to 4 atoms selected from the group consisting of an oxygen atom, a nitrogen atom and a sulfur atom on the ring of $G^2$.

The heterocyclic group having 1 to 4 atoms selected from the group consisting of an oxygen atom, a nitrogen atom and a sulfur atom on the ring of $R^3$ links to $A^6$ on a carbon atom or a nitrogen atom.

Preferred examples of the heterocyclic group having 1 to 4 atoms selected from the group consisting of an oxygen atom, a nitrogen atom and a sulfur atom on the ring of $R^3$ and linking to $A^6$ on a carbon atom, include a monocyclic or bicyclic $C_3$-$C_9$ aromatic heterocyclic group having 1 to 3 atoms selected from the group consisting of an oxygen atom, a nitrogen atom and a sulfur atom, in the ring, including furyl, thienyl, pyrrolyl, pyrazolyl, oxazolyl, isooxazolyl, thiazolyl, isothiazolyl, imidazolyl, pyridyl, N-oxopyridyl, pyrazinyl, pyrimidinyl, pyridazinyl, benzofuranyl, indolyl, benzothienyl, quinolyl, isoquinolyl, quinazolyl, benzoimidazolyl and benzooxazolyl, preferably 2-furyl, 3-furyl, 2-thienyl, 3-thienyl, 2-pyrrolyl, 2-oxazolyl, 2-thiazolyl, 2-pyridyl, 3-pyridyl, 4-pyridyl, 2-N-oxopyridyl, 3-N-oxopyridyl, 4-N-oxopyridyl, 3-pyrazolyl, 4-pyrazolyl, 4-imidazolyl, 2-pyrimidinyl, or 5-pyrimidinyl.

Meanwhile, preferred examples of the heterocyclic group having 1 to 4 atoms selected from the group consisting of an oxygen atom, a nitrogen atom and a sulfur atom on the ring of $R^3$ and linking to $A^6$ on a nitrogen atom, include 1-imidazolyl, 1-pyrazolyl, 1-pyrrolyl, 1-pyrrolidinyl, piperidino, morpholino, 1-homopiperidinyl and 1-piperazinyl, preferably 1-imidazolyl.

When the heterocyclic group having 1 to 4 atoms selected from the group consisting of an oxygen atom, a nitrogen atom and a sulfur atom on the ring of $R^3$ links to $A^6$ on a nitrogen atom, $A^6$ is a single bond, or a group that links a carbon atom of a pyrrole ring in which $R^3$ and $A^6$ are linked to each other in the form of $R^3$-C(=O)-pyrrole ring.

Exemplary substituents of the heterocyclic group having 1 to 4 atoms selected from the group consisting of an oxygen atom, a nitrogen atom and a sulfur atom on the ring of $R^3$ include at least one substituent selected from the group consisting of a fluorine atom, a chlorine atom, a bromine atom, an iodine atom, a hydroxy group, an optionally substituted $C_1$-$C_7$ alkoxy group, a $C_6$-$C_{10}$ aryloxy group, a $C_7$-$C_9$ aralkoxy group, a $C_2$-$C_7$ acyloxy group, an oxo group, a $C_1$-$C_6$ alkylsulfonyloxy group, an optionally substituted $C_2$-$C_7$ acyl group, a carboxyl group, a $C_2$-$C_7$ alkoxycarbonyl group, a carbamoyl group, an optionally substituted $C_2$-$C_7$ alkylcarbamoyl group, an amino group, an optionally substituted $C_1$-$C_6$ alkylamino group, an optionally substituted $C_2$-$C_7$ acylamino group, a $C_2$-$C_8$ alkoxycarbonylamino group, a $C_1$-$C_6$ alkylsulfonylamino group, a cyano group, a nitro group, a $C_1$-$C_6$ alkylthio group, a $C_1$-$C_6$ alkylsulfynyl group, a $C_1$-$C_6$ alkylsulfonyl group, a sulfamoyl group, a $C_1$-$C_6$ alkylaminosulfonyl group, a sulfo group, an optionally substituted alicyclic hydrocarbon group having 3 to 6 carbon atoms, an optionally substituted acyclic aliphatic hydrocarbon group having 1 to 6 carbon atoms, an optionally substituted aromatic hydrocarbon group having 6 to 14 carbon atoms and an optionally substituted heterocyclic group (having 1 to 4 atoms selected from the group consisting of an oxygen atom, a nitrogen atom and a sulfur atom, in the ring).

Specific examples of the substituent of the heterocyclic group having 1 to 4 atoms selected from the group consisting of an oxygen atom, a nitrogen atom and a sulfur atom on its substituted ring of $R^3$ include the same as those exemplified in the substituted acyclic aliphatic hydrocarbon group having 1 to 10 carbon atoms of $G^2$.

As the substituent of the heterocyclic group having 1 to 4 atoms selected from the group consisting of an oxygen atom, a nitrogen atom and a sulfur atom on its substituted ring of $R^3$, a $C_1$-$C_7$ alkoxy group, a $C_2$-$C_7$ acyl group, a $C_2$-$C_7$ alkylcarbamoyl group, a $C_1$-$C_6$ alkylamino group, a $C_2$-$C_7$ acylamino group, an alicyclic hydrocarbon group having 3 to 6 carbon atoms and an acyclic aliphatic hydrocarbon group having 1 to 6 carbon atoms, an aromatic hydrocarbon group having 6 to 14 carbon atoms, and a heterocyclic group (having 1 to 4 atoms selected from the group consisting of oxygen atom, a nitrogen atom and a sulfur atom in the ring) may further be substituted with (one or more substituents selected from the group consisting of: a fluorine atom; a chlorine atom; a bromine atom; an iodine atom; a hydroxy group; a $C_1$-$C_6$ alkoxy group such as methoxy, ethoxy, propoxy, isopropoxy, butoxy, isobutoxy, s-butoxy, t-butoxy, pentyloxy or cyclopropyloxy; a methoxymethyloxy group; a 2-methoxyethoxy group; a formyl group; a trifluoroacetyl group; a $C_2$-$C_7$ acyl group such as acetyl, propionyl, butyryl, isobutyryl, valeryl or isovaleryl; an oxo group; a carboxyl group; a $C_2$-$C_7$ alkoxycarbonyl group such as methoxycarbonyl, ethoxycarbonyl, propoxycarbonyl, isopropoxycarbonyl, butoxycarbonyl, isobutoxycarbonyl or t-butoxycarbonyl; a carbamoyl group; a $C_2$-$C_7$ alkylcarbamoyl group such as N-methylcarbamoyl, N, N-dimethylcarbamoyl, N-ethylcarbamoyl, N-ethyl-N-methylcarbamoyl, N,N-diethylcarbamoyl, N-propylcarbamoyl, N-isopropylcarbamoyl, N-butylcarbamoyl, N-cyclopropylcarbamoyl or N-cyclopropylmethylcarbamoyl; an amino group; a $C_1$-$C_6$ alkylamino group such as methylamino, ethylamino, propylamino, isopropylamino, dimethylamino, N-ethylmethylamino, diethylamino, N-methylpropylamino, N-methylisopropylamino, cyclopropylamino or cyclopropylmethylamino; a $C_4$-$C_6$ cyclic amino group having 1 or 2 atoms selected from the group consisting of an oxygen atom, a nitrogen atom and a sulfur atom, in the ring, such as 1-pyrrolidinyl, piperazinyl, 4-methylpiperazinyl, piperidino or morpholino; a trifluoroacetylamino group; a $C_1$-$C_7$ acylamino group such as formylamino, acetylamino, propionylamino, butyrylamino,

isobutyrylamino or valerylamino; a $C_1$-$C_6$ alkylsulfonylamino group such as methylsulfonylamino, ethylsulfonylamino, propylsulfonylamino or butylsulfonylamino; a nitro group; a cyano group; a $C_1$-$C_6$ alkyl group including methyl, ethyl, propyl, isopropyl, butyl, isobutyl, s-butyl, and t-butyl; a trifluoromethyl group; and a trifluoromethoxy group).

Among those exemplified as substituents of the heterocyclic group having 1 to 4 atoms selected from the group consisting of an oxygen atom, a nitrogen atom and a sulfur atom on the ring of $R^3$, preferred examples thereof include: a fluorine atom; a chlorine atom; a bromine atom; an iodine atom; a hydroxy group; a cyano group; a nitro group; an amino group; a $C_1$-$C_6$ mono or dialkylamino group consisting of a straight or branched alkyl group and an amino group, including a substituted or unsubstituted methylamino, ethylamino, propylamino, isopropylamino, butylamino, isobutylamino, s-butylamino, t-butylamino, pentylamino, hexylamino, dimethylamino, N-ethylmethylamino, diethylamino, N-methylpropylamino, N-methylisopropylamino, N-methylbutylamino, N-methyl-t-butylamino, N-ethylisopropylamino, dipropylamino, diisopropylamino and ethylbutylamino; a carboxyl group; a saturated a $C_1$-$C_6$ alkyl group including a substituted or unsubstituted methyl, ethyl, propyl, isopropyl, butyl, isobutyl, s-butyl, t-butyl, pentyl, isopentyl, neopentyl, t-pentyl, hexyl, isohexyl, 2-methylpentyl and 1-ethylbutyl; an alicyclic hydrocarbon group having 3 to 6 carbon atoms including cyclopropyl, cyclobutyl, cyclopentyl and cyclohexyl; a $C_1$-$C_6$ alkoxy group consisting of a straight or branched alkyl group and an oxy group, including a substituted or unsubstituted methoxy, ethoxy, propoxy, isopropoxy, butoxy, isobutoxy, s-butoxy, t-butoxy, pentyloxy, isopentyloxy, neopentyloxy, t-pentyloxy and hexyloxy; a $C_2$-$C_7$ acyl group including a substituted or unsubstituted acetyl, propionyl, butyryl, isobutyryl, pivaroyl and hexanoyl; a $C_1$-$C_6$ alkylthio group, including methylthio, ethylthio, propylthio, isopropylthio, butylthio, isobutylthio, s-butylthio, t-butylthio, pentylthio and hexylthio; a trifluoromethyl group; a trifluoromethoxy group; a $C_2$-$C_7$ acylamino group including a substituted or unsubstituted acetylamino, propionylamino, butyrylamino, isobutyrylamino, valerylamino and hexanoylamino; and a $C_{2}$-$C_7$ alkylcarbamoyl group consisting of a straight or branched alkyl group and a carbamoyl group including a substituted or unsubstituted N-methylcarbamoyl, N-ethylcarbamoyl, N-propylcarbamoyl, N-isopropylcarbamoyl, N-butylcarbamoyl, N-isobutylcarbamoyl, N-s-butylcarbamoyl, N-t-butylcarbamoyl, N-pentylcarbamoyl, N,N-dimethylcarbamoyl, N-ethyl-N-methylcarbamoyl and N,N-diethylcarbamoyl.

More preferred substituents of the heterocyclic group having 1 to 4 atoms selected from the group consisting of an oxygen atom, a nitrogen atom and a sulfur atom on the ring of $R^3$, include a fluorine atom, a chlorine atom, a bromine atom, a substituted or unsubstituted $C_1$-$C_6$ alkyl group, a hydroxy group, and a substituted or unsubstituted $C_1$-$C_6$ alkoxy group. Specifically, a methyl group and an ethyl group are preferred.

In the formula (I), $A^6$ is a group that links $R^3$ and a carbon atom of a pyrrole ring in the form of $R^3$-$CR^{308}$=$CR^{309}$-pyrrole ring or $R^3$-C≡C-pyrrole ring. $R^3$ represents a trimethylsilyl group, a formyl group, an optionally substituted $C_2$-$C_7$ acyl group, a carboxyl group, a $C_2$-$C_7$ alkoxycarbonyl group, a carbamoyl group, an optionally substituted $C_2$-$C_7$ alkylcarbamoyl group or a cyano group, preferred examples thereof include a formyl group, an acetyl group, a carboxyl group, a methoxycarbonyl group, an ethoxycarbonyl group and a cyano group.

Here, an explanation will be given of preferred combinations of $R^3$ and $A^6$ in the formula (I).

As combinations of $R^3$ and $A^6$ of the formula (I) in the present invention, when $R^3$ represents a fluorine atom, a chlorine atom, a bromine atom, or an iodine atom, $A^3$ represents a single bond.

Also, when $R^3$ represents a trimethylsilyl group, a formyl group, an optionally substituted a $C_2$-$C_7$ acyl group, a carboxyl group, a $C_2$-$C_7$ alkoxycarbonyl group, a carbamoyl group, an optionally substituted $C_2$-$C_7$ alkylcarbamoyl'group, or a cyano group, $A^6$ is a group that links a carbon atom of a pyrrole ring in which $R^3$ and $A^6$ are linked to each other in the form of a carbon atom of $R^3$-$CR^{308}$=$CR^{309}$-pyrrole ring or $R^3$-C≡C-pyrrole carbon atom.

Preferred combinations of $R^3$ and $A^6$ of the formula (I) in the present invention include cases where $A^6$ represents a single bond and $R^3$ represents an optionally substituted aromatic hydrocarbon group having 6 to 14 carbon atoms or an optionally substituted heterocyclic group. Among them, a case where $R^3$ represents a thienyl group, a furyl group, a pyrrolyl group, a pyrazolyl group or a phenyl group optionally substituted with one or more alkyl group having 1 to 4 carbon atoms is preferred.

Also a case where $A^6$ represents a single bond, and $R^3$ represents a pyridyl group or 1-oxypyridyl group or pyrazolyl group or N-methylpyrazolyl group optionally substituted by an alkyl group having 1 to 4 carbon atoms or one halogen atom.

In addition, the following combinations can be mentioned: a combination in which $A^6$ represents a single bond, and $R^3$ is a fluorine atom, chlorine atom, bromine atom, or iodine atom, a combination in which $A^6$ represents a single bond, and $R^3$ is a substituted or unsubstituted saturated acyclic saturated aliphatic hydrocarbon group having 1 to 10 carbon atoms; a combination in which $A^6$ represents a single bond, and $R^3$ is a substituted or unsubstituted alicyclic hydrocarbon group having 3 to 8 carbon atoms; a combination in which $A^6$ represents a single bond, and $R^3$ is a substituted or unsubstituted aromatic hydrocarbon group having 6 to 14 carbon atoms; a combination in which $A^6$ represents a single bond, and $R^3$ is a substituted or unsubstituted monocyclic $C_3$-$C_5$ aromatic heterocyclic group having 1 or 2 atoms selected from the group consisting of an oxygen atom, a nitrogen atom and a sulfur atom, in the ring; a combination in which $A^6$ is a group that links $R^3$ and a carbon atom of a pyrrole ring in which $A^6$ is linked in the form of the carbon atom of a $R^3$-NH-C(=O)-pyrrole ring, and $R^3$ is a hydrogen atom; a combination in which $A^6$ is a group that

links $R^3$ and a carbon atom of a pyrrole ring in which $A^6$ is linked in the form of the carbon atom of a $R^3$-C(=O)-NH-pyrrole ring, and $R^3$ is a substituted or unsubstituted acyclic saturated aliphatic hydrocarbon group having 1 to 10 carbon atoms; a combination in which $A^6$ is a group that links $R^3$ and a carbon atom of a pyrrole ring in which $A^6$ is linked in the form of the carbon atom of the carbon atom of a $R^3$-C(=O)-NH-pyrrole ring, and $R^3$ is a substituted or unsubstituted alicyclic hydrocarbon group having 3 to 8 carbon atoms; a combination in which $A^6$ is a group that links $R^3$ and a carbon atom of a pyrrole ring in which $A^6$ is linked in the form of the carbon atom of a $R^3$-C(=O)-NH-pyrrole ring, and $R^3$ is a substituted or unsubstituted aromatic hydrocarbon group having 6 to 14 carbon atoms; a combination in which $A^6$ is a group that links $R^3$ and a carbon atom of a pyrrole ring in which $A^6$ is linked in the form of the carbon atom of the $R^3$-C (=O)-NH-pyrrole ring, and $R^3$ is a monocyclic $C_3$-$C_5$ aromatic heterocyclic group having 1 or 2 atoms selected from the group consisting of an oxygen atom, a nitrogen atom and a sulfur atom on the substituted or unsubstituted ring; a combination in which $A^6$ is a group that links $R^3$ and a carbon atom of a pyrrole ring in which $A^6$ is linked in the form of the carbon atom of a $R^3$-NH-pyrrole ring, and $R^3$ is a hydrogen atom; a combination in which $A^6$ is a group that links $R^3$ and a carbon atom of a pyrrole ring in which $A^6$ is linked in the form of the carbon atom of a $R^3$-NH-pyrrole ring, and $R^3$ is a substituted or unsubstituted acyclic saturated aliphatic hydrocarbon group having 1 to 10 carbon atoms; a combination in which $A^6$ is a group that links $R^3$ and a carbon atom of a pyrrole ring in which $A^6$ is linked in the form of the carbon atom of a $R^3$-NH-pyrrole ring, and $R^3$ is a substituted or unsubstituted alicyclic hydrocarbon group having 3 to 8 carbon atoms; a combination in which $A^6$ is a group that links $R^3$ and a carbon atom of a pyrrole ring in which $A^6$ is linked in the form of the carbon atom of a $R^3$-NH-pyrrole ring, and $R^3$ is a substituted or unsubstituted aromatic hydrocarbon group having 6 to 14 carbon atoms; a combination in which $A^6$ is a group that links $R^3$ and a carbon atom of a pyrrole ring in which $A^6$ is linked in the form of the carbon atom of $R^3$-NH-pyrrole ring, and $R^3$ is a monocyclic $C_3$-$C_5$ aromatic heterocyclic group having 1 or 2 atoms selected from the group consisting of an oxygen atom, a nitrogen atom and a sulfur atom on the substituted or unsubstituted ring; a combination in which $A^6$ is a group that links $R^3$ and a carbon atom of a pyrrole ring in which $A^6$ is linked in the form of the carbon atom of a $R^3$-HC=CH-pyrrole ring, and $R^3$ is a hydrogen atom; a combination in which $A^6$ is a group that links $R^3$ and a carbon atom of a pyrrole ring in which $A^6$ is linked to each other in the form of the carbon atom of a $R^3$-HC=CH-pyrrole ring, and $R^3$ is a substituted or unsubstituted acyclic saturated aliphatic hydrocarbon group having 1 to 10 carbon atoms; a combination in which $A^6$ is a group that links a carbon atom of a pyrrole ring in which $R^3$ and $A^6$ are linked to each other in the form of the carbon atom of a $R^3$-HC=CH-pyrrole ring, and $R^3$ is a substituted or unsubstituted alicyclic hydrocarbon group having 3 to 8 carbon atoms; a combination in which $A^6$ is a group that links $R^3$ and a carbon atom of a pyrrole ring in which $A^6$ is linked in the form of the carbon atom of a $R^3$-HC=CH-pyrrole ring, and $R^3$ is a substituted or unsubstituted aromatic hydrocarbon group having 6 to 14 carbon atoms; a combination in which $A^6$ is a group that links $R^3$ and a carbon atom of a pyrrole ring in which $A^6$ is linked in the form of the carbon atom of a $R^3$-HC=CH-pyrrole ring, and $R^3$ is a monocyclic $C_3$-$C_5$ aromatic heterocyclic group having 1 or 2 atoms selected from the group consisting of an oxygen atom, a nitrogen atom and a sulfur atom on the substituted or unsubstituted ring; a combination in which $A^6$ is a group that links $R^3$ and a carbon atom of a pyrrole ring in which $A^6$ is linked in the form of the carbon atom of a $R^3$-C≡C-pyrrole ring, and $R^3$ is a hydrogen atom; a combination in which $A^6$ is a group that links $R^3$ and a carbon atom of a pyrrole ring in which $A^6$ is linked in the form of the carbon atom of a $R^3$-C≡C-pyrrole ring, and $R^3$ is a substituted or unsubstituted acyclic saturated aliphatic hydrocarbon group having 1 to 10 carbon atoms; a combination in which $A^6$ is a group that links $R^3$ and a carbon atom of a pyrrole ring in which $A^6$ is linked in the form of the carbon atom of a $R^3$-C≡C-pyrrole ring, and $R^3$ is a substituted or unsubstituted alicyclic hydrocarbon group having 3 to 8 carbon atoms; a combination in which $A^6$ is a group that links $R^3$ and a carbon atom of a pyrrole ring in which $A^6$ is linked in the form of the carbon atom of a $R^3$-C≡C-pyrrole ring, and $R^3$ is a substituted or unsubstituted aromatic hydrocarbon group having 6 to 14 carbon atoms; a combination in which $A^6$ is a group that links $R^3$ and a carbon atom of a pyrrole ring in which $A^6$ is linked in the form of the carbon atom of a $R^3$-C≡C-pyrrole ring, and $R^3$ is a monocyclic $C_3$-$C_5$ aromatic heterocyclic group having 1 or 2 atoms selected from the group consisting of an oxygen atom, a nitrogen atom and a sulfur atom on the substituted or unsubstituted ring; and a combination in which $A^6$ is a group that links $R^3$ and a carbon atom of a pyrrole ring in which $A^6$ is linked in the form of the carbon atom of a $R^3$-C≡C-pyrrole ring, and $R^3$ is a trimethylsilyl group or cyano group.

Here, an explanation will be given of preferred combinations of $R^2$-$A^5$ portion and $R^3$- $A^6$ portion in the formula (I).

Preferred.combinations of $R^2$-$A^5$ portion and $R^3$- $A^6$ portion include cases where both of $A^5$ and $A^6$ represent a single bond. In this case, more preferred combinations include cases where $R^2$ represents a cyclopropyl group, a cyclobutyl group, a cyclopropylmethyl group, a methyl group, an ethyl group, a vinyl group, an isopropyl group, an isobutyl group or 2-methyl-1-propenyl group, and $R^3$ represents a pyridyl group or 1-oxypyridyl group or pyrazolyl group or N-methylpyrazolyl group optionally substituted with one alkyl group having 1 to 4 carbon atoms or one halogen atom.

Also combinations wherein both of $A^5$ and $A^6$ represent a single bond, and $R^2$ represents a $C_1$-$C_4$ alkyl group, a $C_1$-$C_4$ alkoxy group, a $C_2$-$C_4$ acyl group, a hydroxy group, a carboxyl group, an alkoxycarbonyl group, a thienyl group, a pyridyl group, a furyl group, a pyrrolyl group, a pyrazolyl group or phenyl group which can be substituted by any or more of a fluorine atom or a chlorine atom, and $R^3$ represents a pyridyl group or 1-oxypyridyl group or pyrazolyl group or N-methylpyrazolyl group which can be substituted by one alkyl group having 1 to 4 carbon atoms or one halogen

atom can be mentioned as preferred examples.

Further, an explanation will be given of preferred combinations of $A^1$-$G^2$ portion, $R^2$- $A^5$ portion and $R^3$- $A^6$ portion in the formula (I). Basically, preferably those mentioned as preferred examples for $A^1$-$G^2$ portion, $R^2$-$A^5$ portion and $R^3$- $A^6$ portion are combined, and more preferably more preferred examples are combined.

More specifically, in the combinations of the following a) to f) mentioned as preferred combinations of the $A^1$-$G^2$ portion, further a case where both of $A^5$ and $A^6$ represent a single bond is preferred.

a) $A^1$ represents $-(CH_2)_2-$ or $-(CH_2)_3-$, $A^2$ represents -NH-(C=O)- or -NH-(C=O)-NH-, $G^1$ represents a single bond, and $A^3$ represents a divalent acyclic aliphatic hydrocarbon group having 1 to 10 carbon atoms.

b) $A^1$ represents $-(CH_2)_2-$ or $-(CH_2)_3-$, $A^2$ represents -NH-(C=O)-, -NH-(C=O)-NH-, -NH-, or -C-(=O)-NH-, and $G^1$ represents a group other than the single bond.

c) $A^1$ represents a divalent acyclic aliphatic hydrocarbon group having 1 to 6 carbon atoms, specifically $-(CH_2)_2-$ or $-(CH_2)_3-$, $A^2$ represents a single bond, and $G^1$ represents an optionally substituted heterocyclic group (note, where a heterocyclic group of $G^1$ is 5-6membered monocyclic ring, the 5-6 membered monocyclic heterocyclic group of $G^1$ is substituted, or $A^3$-$G^2$ portion represents those other than the hydrogen atom).

d) $A^1$ represents a divalent acyclic aliphatic hydrocarbon group having 1 to 6 carbon atoms, specifically $-(CH_2)_2-$ or $-(CH_2)_3-$, $A^2$ represents those other than a single bond, and $G^1$ represents an optionally substituted aromatic hydrocarbon group, an optionally substituted alicyclic hydrocarbon group having 7 to 10 carbon atoms, or an optionally substituted heterocyclic group (note, where the aromatic hydrocarbon group of $G^1$ is a phenyl group, or where the heterocyclic group of $G^1$ is 5-6 membered monocyclic ring, the phenyl group of $G^1$ or 5-6 membered monocyclic heterocyclic group is substituted, or $A^3$-$G^2$ portion represents those other than the hydrogen atom).

e) $A^1$ represents a divalent acyclic aliphatic hydrocarbon group having 1 to 6 carbon atoms, specifically $-(CH_2)_2-$ or $-(CH_2)_3-$, $A^2$ represents those other than a single bond, $G^1$ and $A^4$ represent the single bond, $A^3$ represents an optionally substituted acyclic aliphatic hydrocarbon group having 1 to 10 carbon atoms, $G^2$ represents an optionally substituted alicyclic hydrocarbon group having 5 to 10 carbon atoms, an optionally substituted aromatic hydrocarbon group, or optionally substituted heterocyclic group.

f) $A^1$ represents a divalent acyclic aliphatic hydrocarbon group having 1 to 6 carbon atoms, specifically $-(CH_2)_2-$ or $-(CH_2)_3-$, $A^2$ represents those other than a single bond, $G^1$ represents the single bond, $A^3$ represents an optionally substituted acyclic aliphatic hydrocarbon group having 1 to 10 carbon atoms, and $A^4$ represents -C(=O)-, -C(=O)-$NR^{121}$-, -C(=S)-$NR^{122}$-, -C(=$NR^{123}$)-, -O-C(=O)-, -$NR^{125}$-C(=O)-, -$NR^{126}$-$S(=O)_2$-, -$NR^{127}$-C(=O)-O-, -$NR^{128}$-C(=O)-$NR^{129}$-, -$NR^{130}$-C(=S)-, -$NR^{131}$-C(=S) -$NR^{132}$-, -S-, -S(=O)-, -$S(=O)_2$-, -$S(=O)_2$-$NR^{133}$- or -$S(=O)_2$-O-.

In the cases of d) to f), $A^2$ preferably represents -C(=O)-, -C(=O)-O-, -C(=O)-NH-, -C(=O)-NMe-, -NH-, -NH-C(=O)-, -NH-C(=O)-O-, -NH-C(=O)-NH-, -NH-C(=O)-NMe-, or -NH-C(=S)-, especially preferably represents -C(=O)-NH-, -NH-, -NH-C(=O)-, -NH-C(=O)-O-, or -NH-C(=O)-NH-.

In these cases of combinations, further preferably $R^2$ represents an optionally substituted acyclic aliphatic hydrocarbon group having 1 to 10 carbon atoms, an optionally substituted alicyclic hydrocarbon group having 3 to 8 carbon atoms, an optionally substituted aromatic hydrocarbon group having 6 to 14 carbon atoms or an optionally substituted heterocyclic group, and $R^3$ represents an optionally substituted aromatic hydrocarbon group having 6 to 14 carbon atoms or an optionally substituted heterocyclic group.

In further detail, in these cases, combinations wherein $R^2$ represents an acyclic aliphatic hydrocarbon group having 1 to 10 carbon atoms, an alicyclic hydrocarbon group having 3 to 8 carbon atoms, an optionally substituted phenyl group, or an optionally substituted heterocyclic group having 1 or 2 atoms selected from the group consisting of an oxygen atom, a nitrogen atom and a sulfur atom, in the ring, and $R^3$ represents a thienyl group; a pyridyl group, a furyl group, a pyrrolyl group, a pyrazolyl group or a phenyl group optionally substituted with one or more alkyl group having 1 to 4 carbon atoms are specifically preferred. Especially, preferred combinations can include cases where $R^2$ represents a cyclopropyl group, a methyl group, an ethyl group, a vinyl group, an isopropyl group, an isobutyl group or 2-methyl-1-propenyl group, and $R^3$ represents a pyridyl group or 1-oxypyridyl group or pyrazolyl group or N-methylpyrazolyl group which can be substituted with an alkyl group having 1 to 4 carbon atoms or one halogen atom, and cases where $R^2$ represents a thienyl group, a pyridyl group, a furyl group, a pyrrolyl group, a pyrazolyl group or phenyl group which can be substituted by any or more of a $C_1$-$C_4$ alkyl group, a $C_1$-$C_4$ alkoxy group, and a chlorine group, and $R^3$ represents a pyridyl group or 1-oxypyridyl group or pyrazolyl group or N-methylpyrazolyl group which can be substituted by an alkyl group having 1 to 4 carbon atoms or one halogen atom.

In the pyrrolo-pyrimidine-thione derivatives of the formula (I), specific preferred combinations of -$G^1$-$A^3$-$A^4$-$G^2$ portion include groups represented by the following formulae, K1-K431. In the respective chemical formula, symbol "---" is used to denote a binding site between $A^2$ and the group -$G^1$-$A^3$-$A^4$-$G^2$.

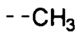
K001

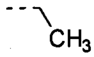
K002

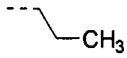
K003

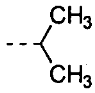
K004

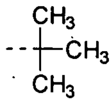
K005

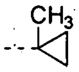
K006

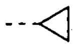
K007

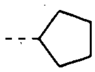
K008

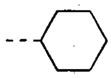
K009

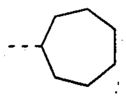
K010

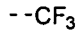
K011

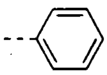
K012

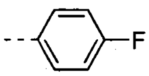
K013

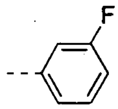
K014

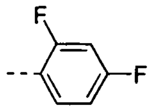
K015

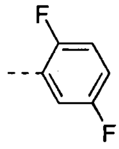
K016

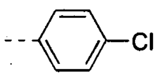
K017

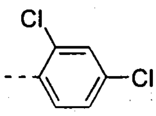
K018

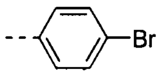
K019

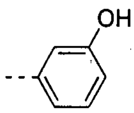
K020

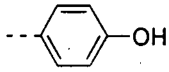
K021

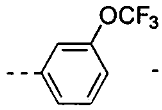
K022

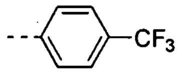
K023

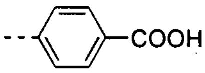
K024

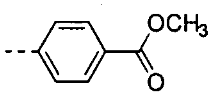
K025

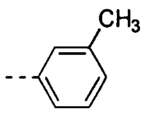
K026

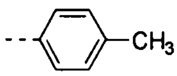
K027

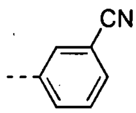
K028

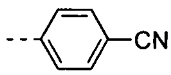
K029

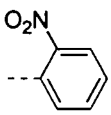
K030

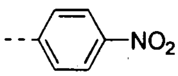
K031

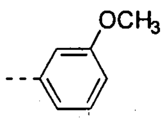
K032

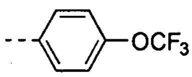
K033

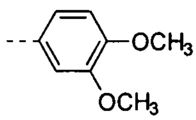
K034

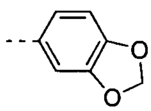
K035

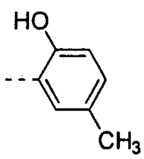
K036

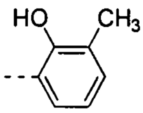
K037

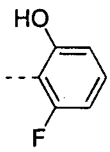
K038

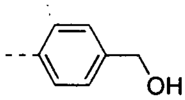
K039

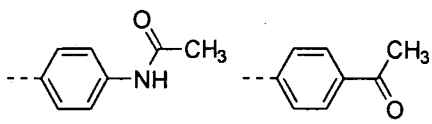
K040

K041

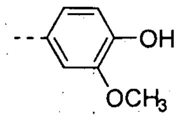
K042

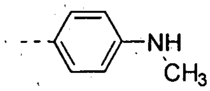
K043

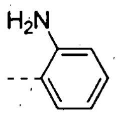
K044

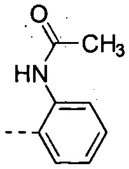
K045

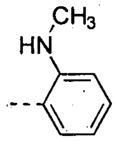
K046

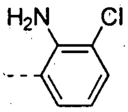
K047

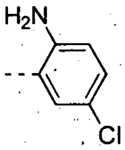
K048

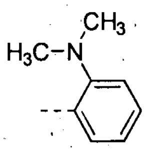
K049

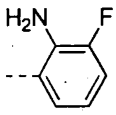
K050

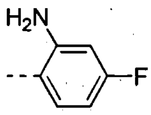
K051

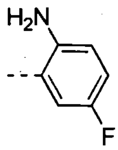
K052

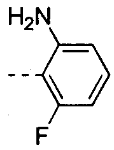
K053

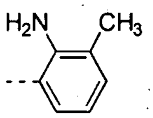
K054

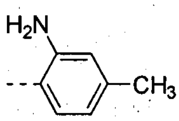
K055

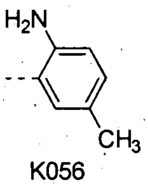
K056

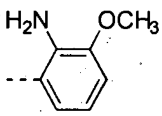
K057

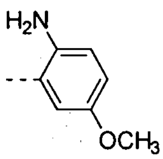
K058

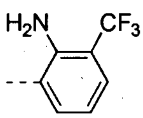
K059

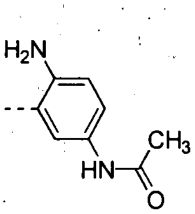
K060

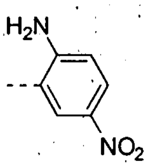
K061

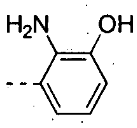
K062

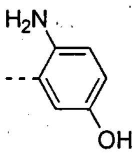
K063

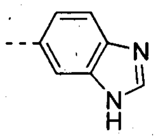
K064

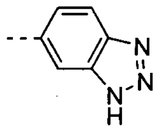
K065

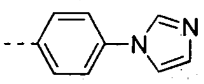
K066

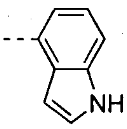
K067

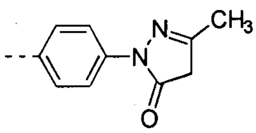
K068

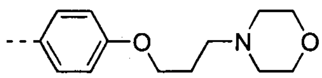
K069

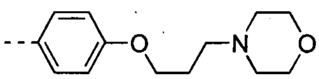
K070

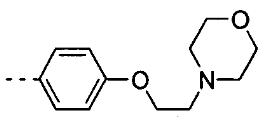
K071

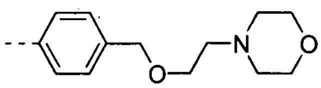
K072

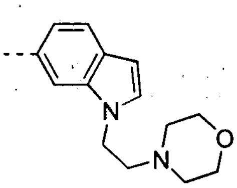
K073

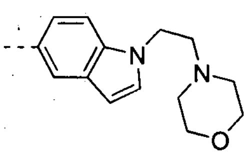
K074

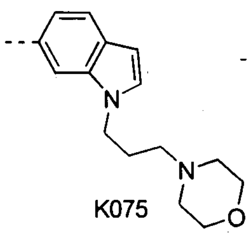
K075

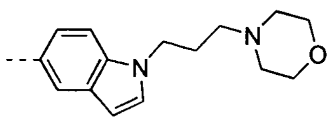
K076

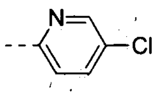
K077

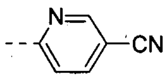
K078

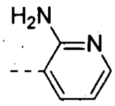
K079

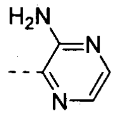
K080

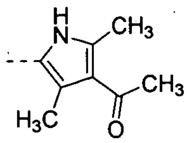
K081

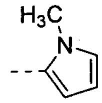
K082

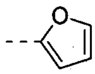
K083

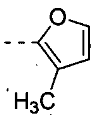
K084

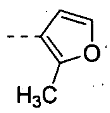
K085

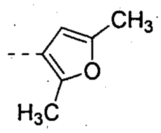
K086

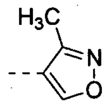
K087

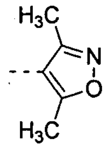
K088

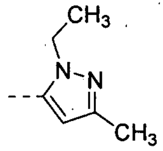
K089

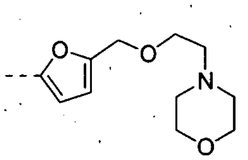
K090

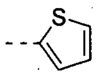
K091

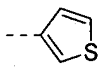
K092

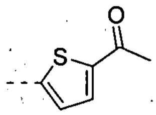
K093

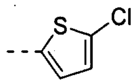
K094

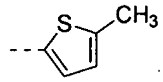
K095

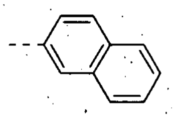
K096

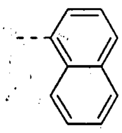
K097

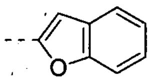
K098

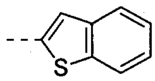
K099

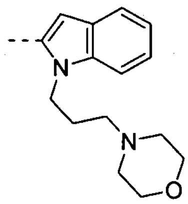
K100

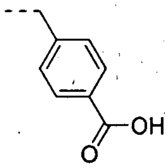
K101

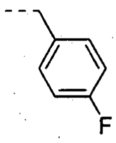
K102

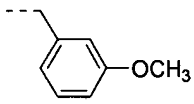
K103

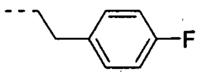
K104

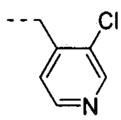
K105

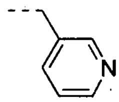
K106

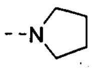 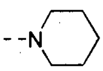 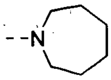 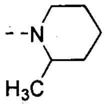 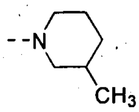 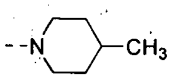

K107 K108 K109 K110 K111 K112

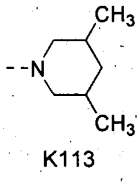 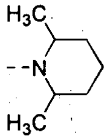 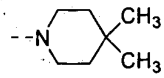 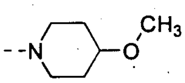 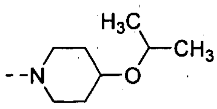 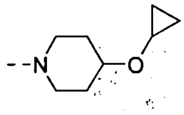

K113 K114 K115 K116 K117 K118

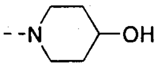 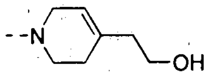 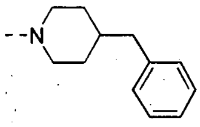 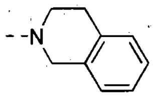 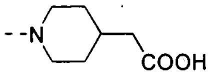

K119 K120 K121 K122 K123

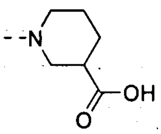 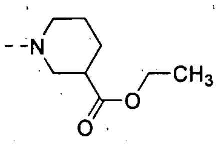 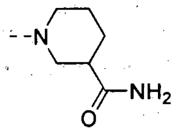 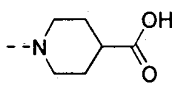 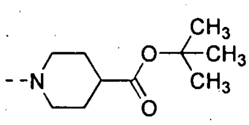

K124 K125 K126 K127 K128

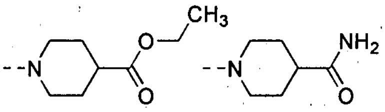 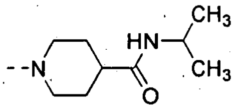 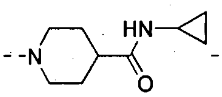 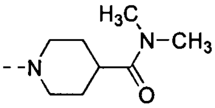

K129 K130 K131 K132 K133

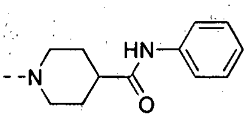 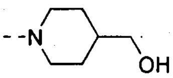 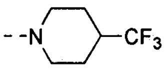 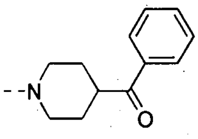 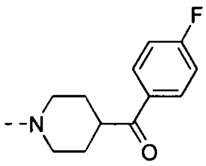

K134 K135 K136 K137 K138

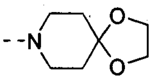 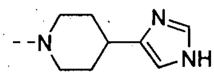 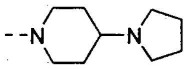 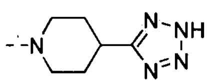

K139 K140 K141 K142

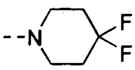 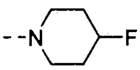 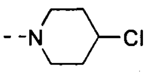 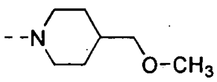 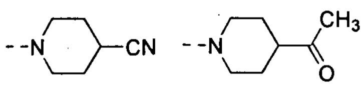

K143 K144 K145 K146 K147 K148

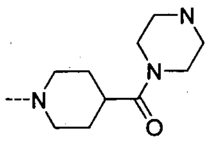
K149

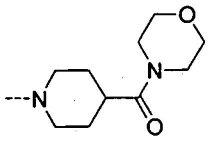
K150

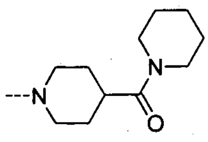
K151

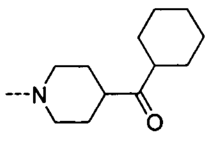
K152

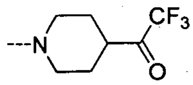
K153

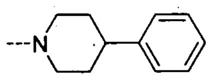
K154

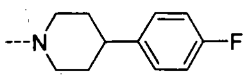
K155

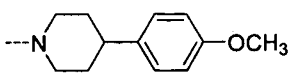
K156

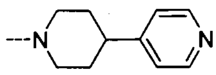
K157

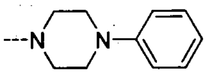
K158

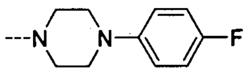
K159

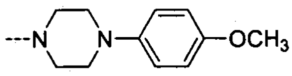
K160

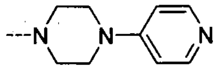
K161

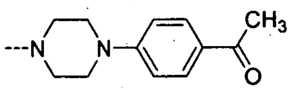
K162

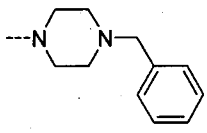
K163

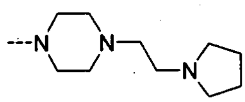
K164

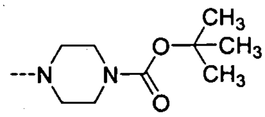
K165

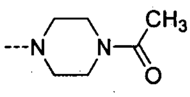
K166

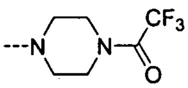
K167

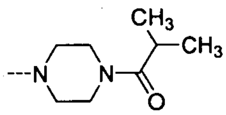
K168

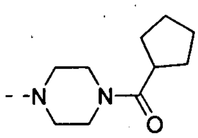
K169

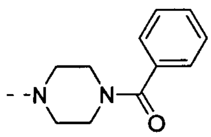
K170

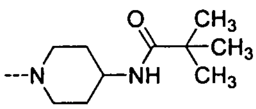
K171

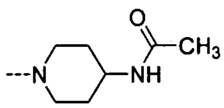
K172

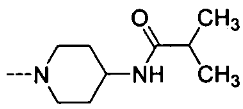
K173

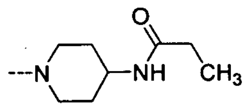
K174

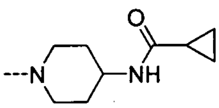
K175

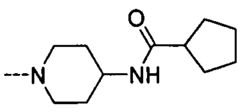
K176

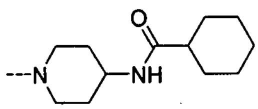
K177

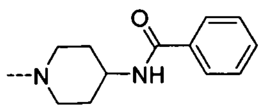
K178

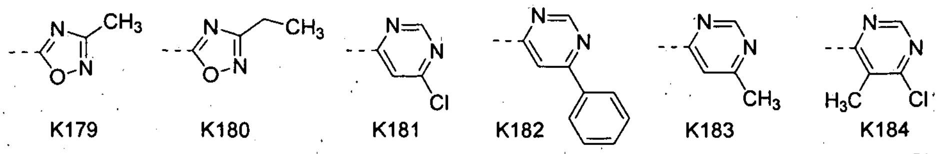

K179 K180 K181 K182 K183 K184

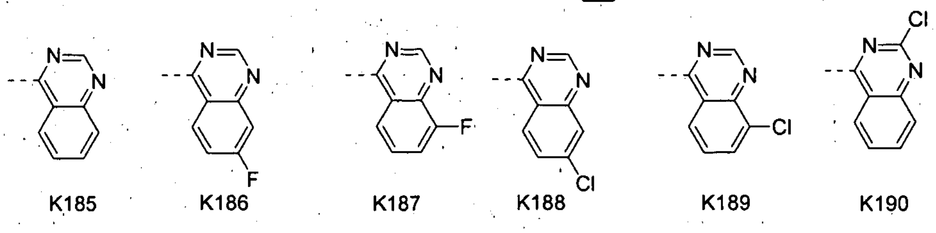

K185 K186 K187 K188 K189 K190

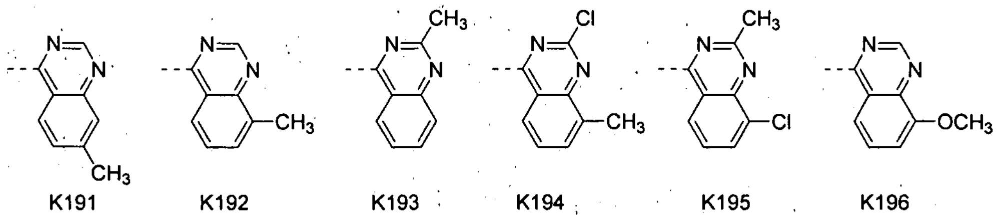

---H

K191 K192 K193 K194 K195 K196 K197

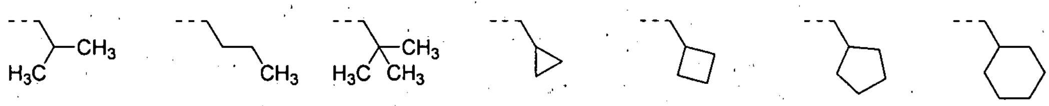

K198 K199 K200 K201 K202 K203 K204

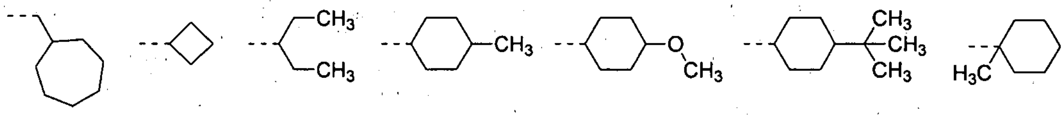

K205 K206 K207 K208 K209 K210 K211

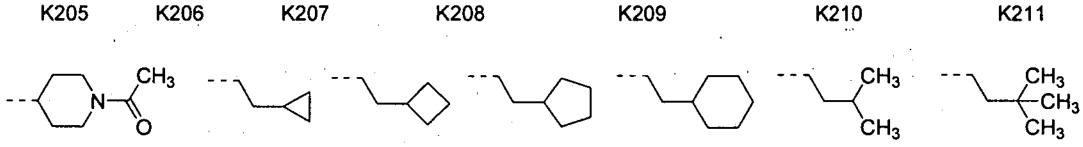

K212 K213 K214 K215 K216 K217 K218

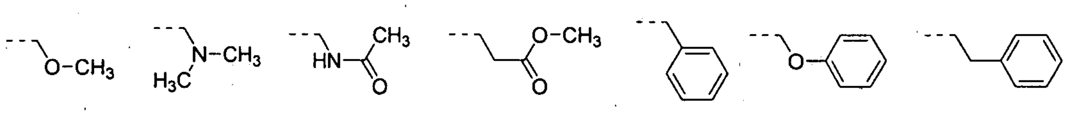

K219 K220 K221 K222 K223 K224 K225

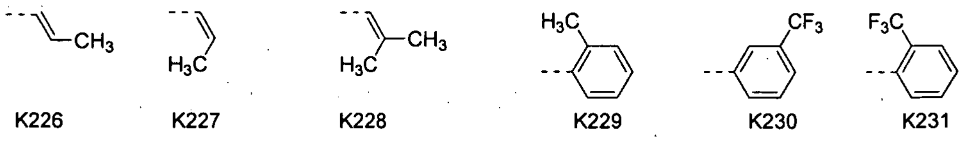

K226 K227 K228 K229 K230 K231

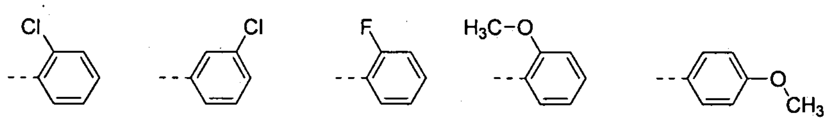

K232 K233 K234 K235 K236

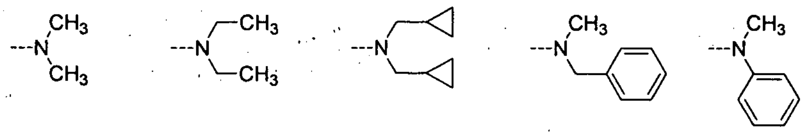

K237 K238 K239 K240 K241

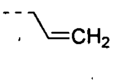 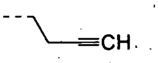 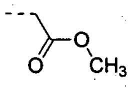 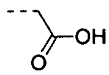 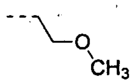 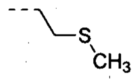

K242 K243 K244 K245 K246 K247

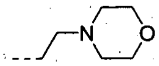 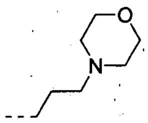 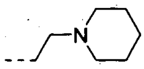 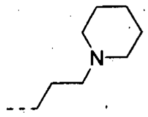 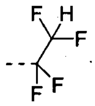 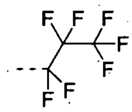

K248 K249 K250 K251 K252 K253

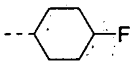 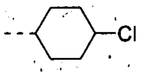 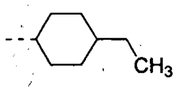 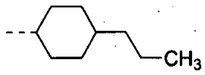 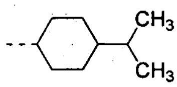

K254 K255 K256 K257 K258

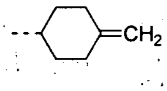 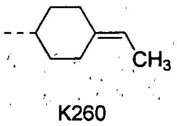 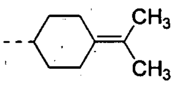 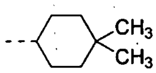 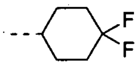 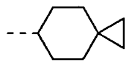

K259 K260 K261 K262 K263 K264

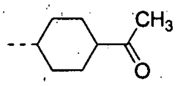 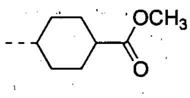 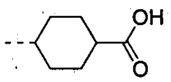 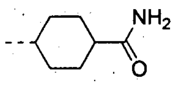 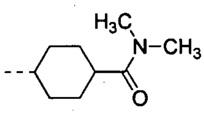 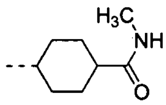

K265 K266 K267 K268 K269 K270

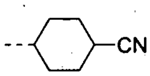 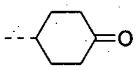 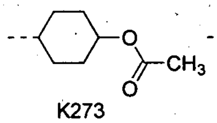 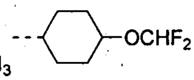 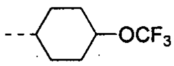 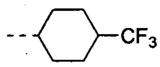

K271 K272 K273 K274 K275 K276

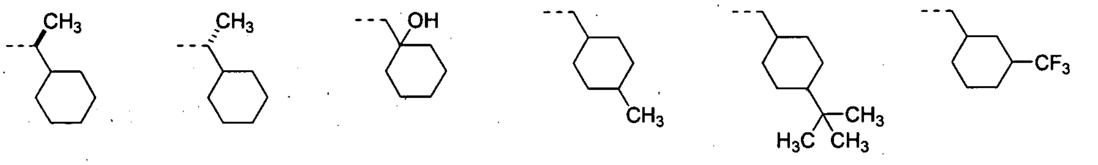

K277 K278 K279 K280 K281 K282

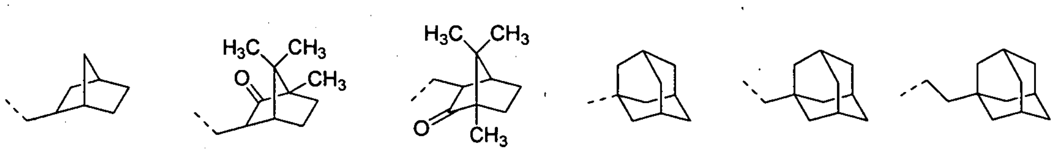

K283 K284 K285 K286 K287 K288

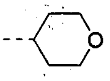
K289

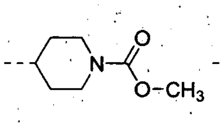
K290

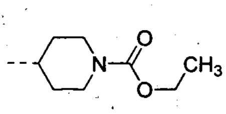
K291

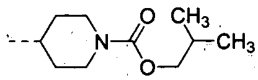
K292

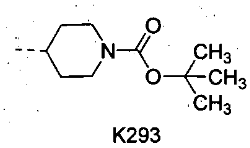
K293

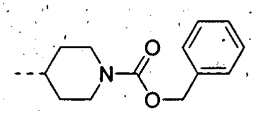
K294

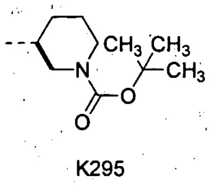
K295

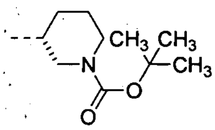
K296

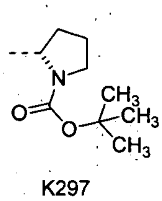
K297

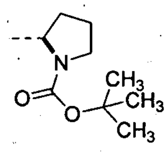
K298

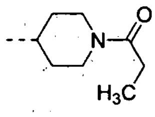
K299

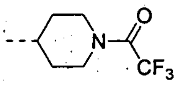
K300

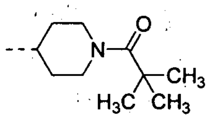
K301

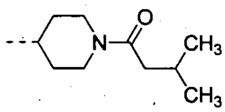
K302

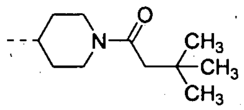
K303

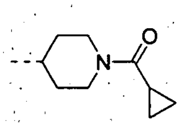
K304

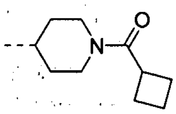
K305

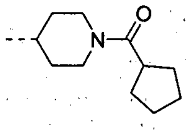
K306

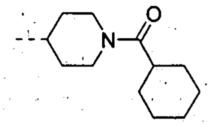
K307

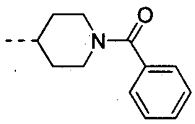
K308

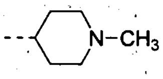
K309

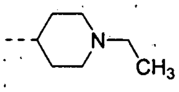
K310

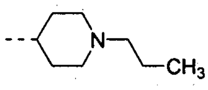
K311

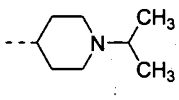
K312

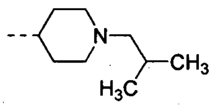
K313

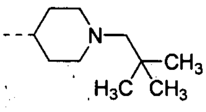
K314

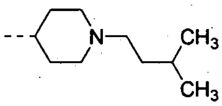
K315

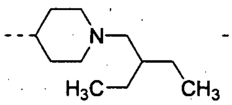
K316

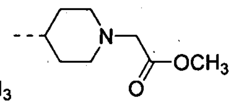
K317

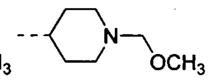
K318

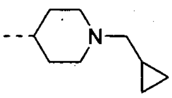
K319

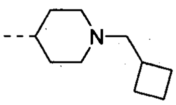
K320

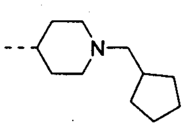
K321

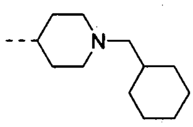
K322

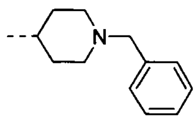
K323

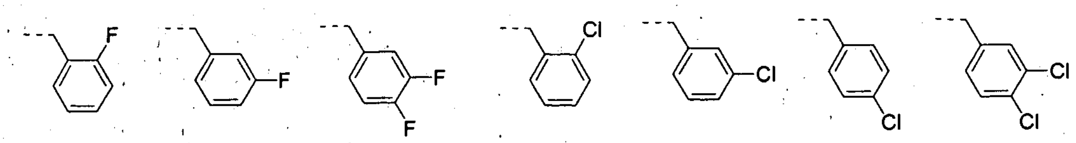

K324 K325 K326 K327 K328 K329 K330

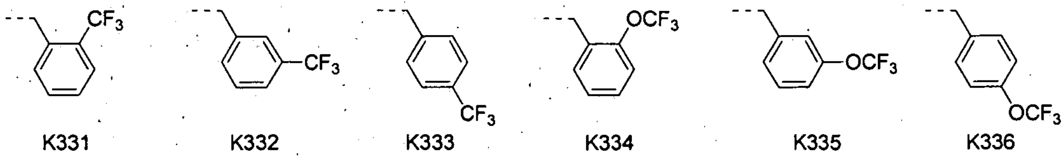

K331 K332 K333 K334 K335 K336

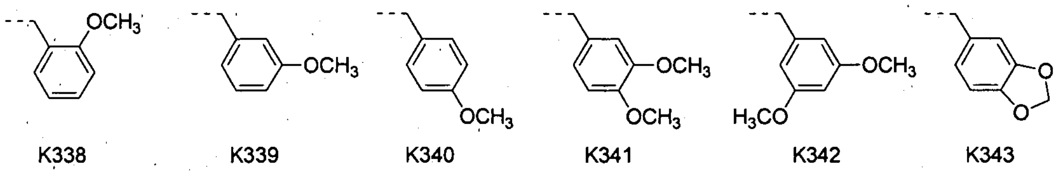

K338 K339 K340 K341 K342 K343

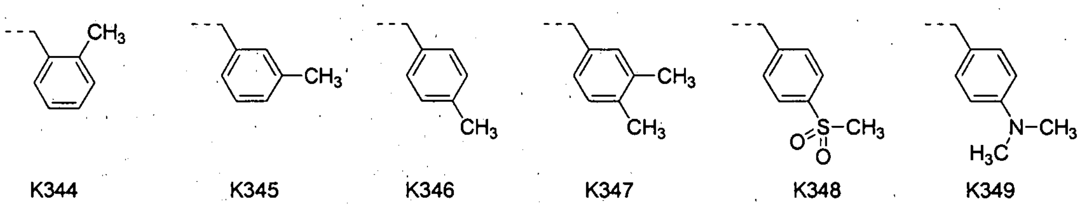

K344 K345 K346 K347 K348 K349

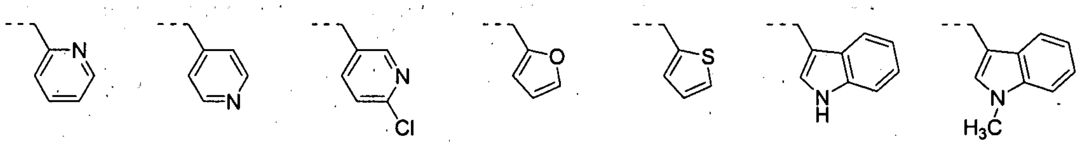

K350 K351 K352 K353 K354 K355 K356

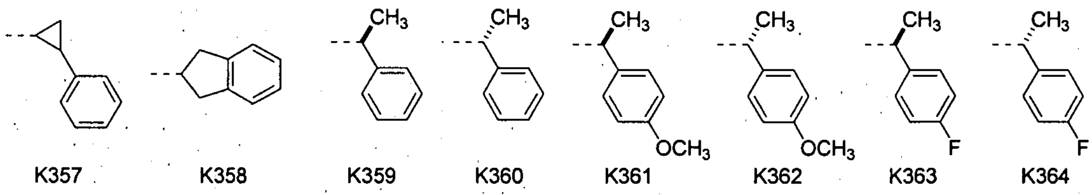

K357 K358 K359 K360 K361 K362 K363 K364

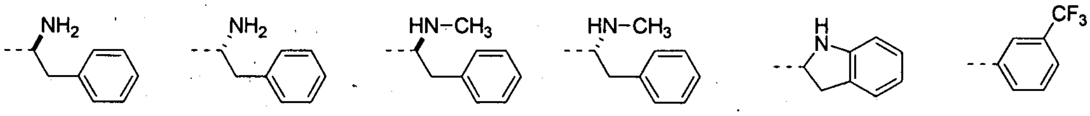

K365 K366 K367 K368 K369 K370

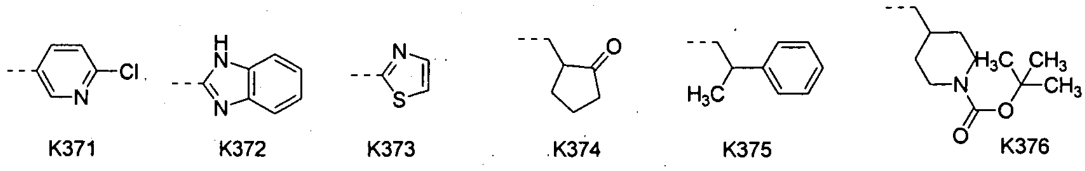

K371 K372 K373 K374 K375 K376

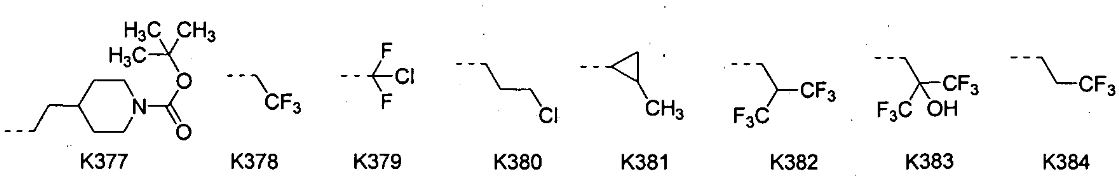

K377 K378 K379 K380 K381 K382 K383 K384

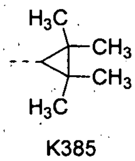
K385

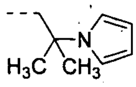
K386

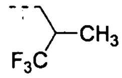
K387

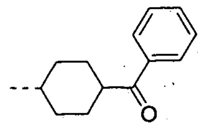
K388

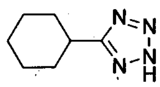
K389

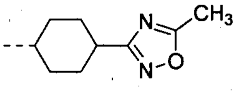
K390

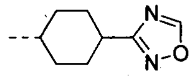
K391

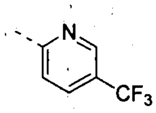
K392

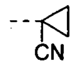
K393

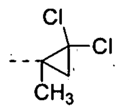
K394

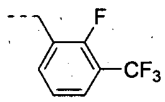
K395

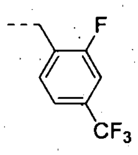
K396

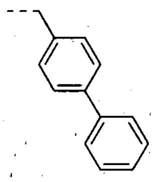
K397

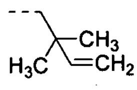
K398

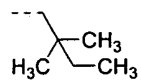
K399

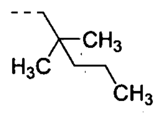
K400

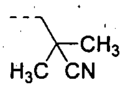
K401

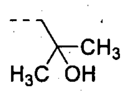
K402

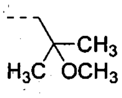
K403

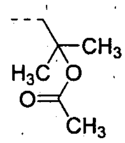
K404

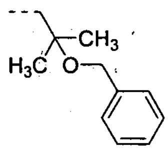
K405

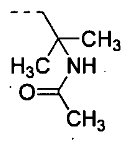
K406

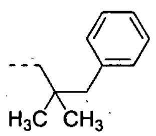
K407

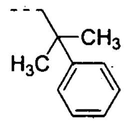
K408

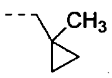
K409

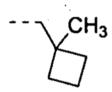
K410

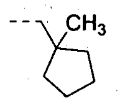
K411

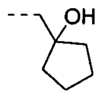
K412

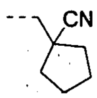
K413

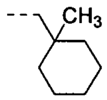
K414

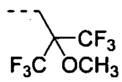
K415

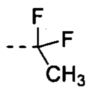
K416

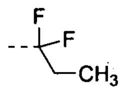
K417

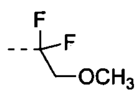
K418

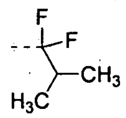
K419

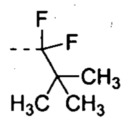
K420

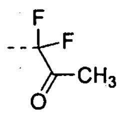
K421

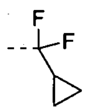
K422

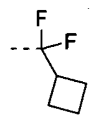
K423

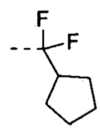
K424

K425

K426

K427

K428

K429

K430

K431

In the pyrrolo-pyrimidine-thione derivatives of the formula (I), as specific examples of preferred combinations of the -$A^5$-$R^2$ portion, groups represented by the following formulae, J01-J166 may be mentioned. In the respective chemical formulae, symbol "---" indicates a binding site between a carbon atom of a pyrrole ring and -$A^5$-$R^2$.

| | | | | | |
|---|---|---|---|---|---|
| ---H | --Cl | ---Br | ---I | --$CH_3$ | --$CF_3$ |
| J001 | J002 | J003 | J004 | J005 | J006 |
| 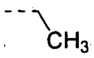 | 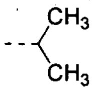 | 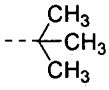 | 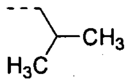 | 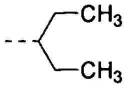 | 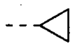 |
| J007 | J008 | J009 | J010 | J011 | J012 |
| 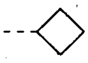 | 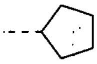 | 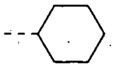 | 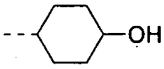 | 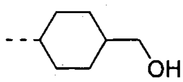 | 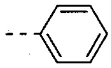 |
| J013 | J014 | J015 | J016 | J017 | J018 |
| 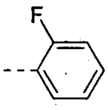 | 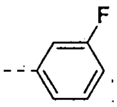 | 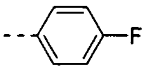 | 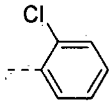 | 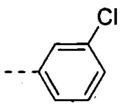 | 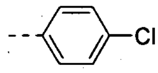 |
| J019 | J020 | J021 | J022 | J023 | J024 |
| 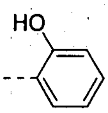 | 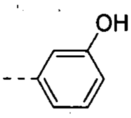 | 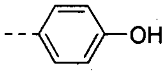 | 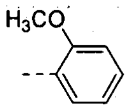 | 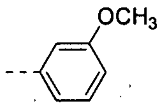 | 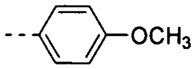 |
| J025 | J026 | J027 | J028 | J029 | J030 |
| 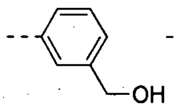 | 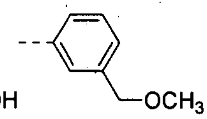 | 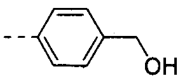 | 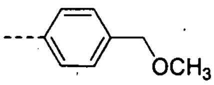 | 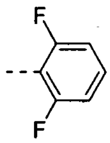 | 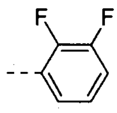 |
| J031 | J032 | J033 | J034 | J035 | J036 |
| 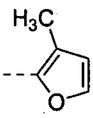 | 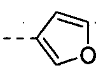 | 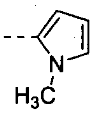 | 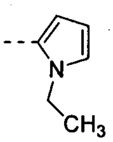 | 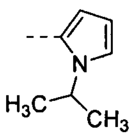 | 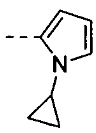 |
| J037 | J038 | J039 | J040 | J041 | J042 |

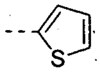
J043

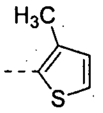
J044

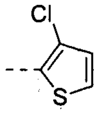
J045

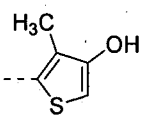
J046

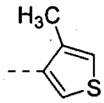
J047

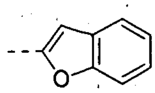
J048

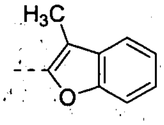
J049

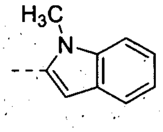
J050

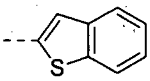
J051

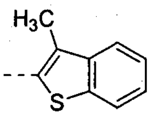
J052

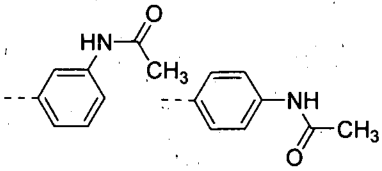
J053

J054

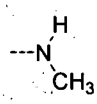
J055

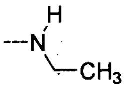
J056

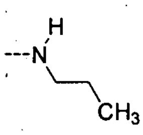
J057

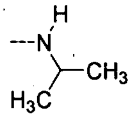
J058

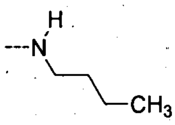
J059

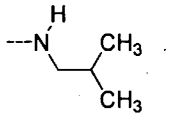
J060

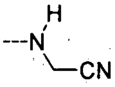
J061

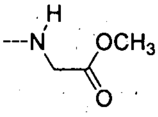
J062

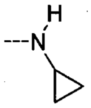
J063

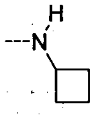
J064

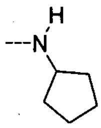
J065

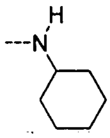
J066

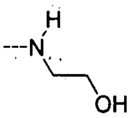
J067

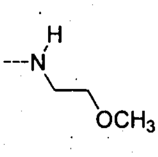
J068

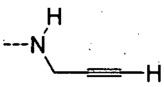
J069

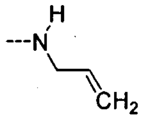
J070

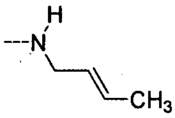
J071

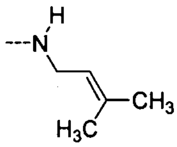
J072

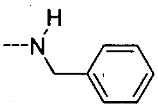
J073

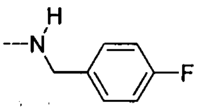
J074

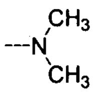
J075

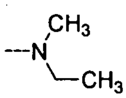
J076

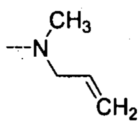
J077

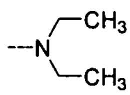
J078

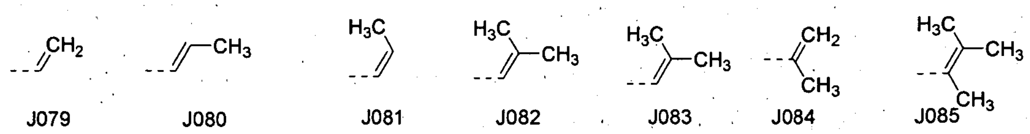

J079 J080 J081 J082 J083 J084 J085

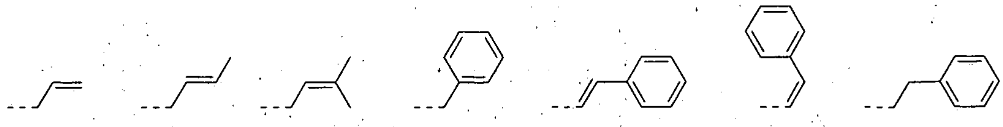

J086 J087 J088 J089 J090 J091 J092

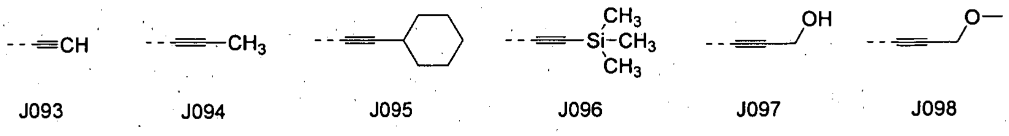

J093 J094 J095 J096 J097 J098

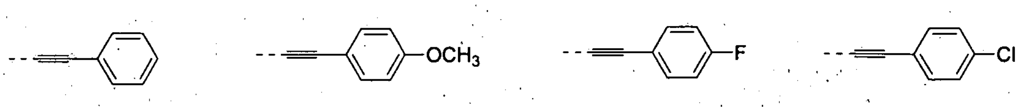

J099 J100 J101 J102

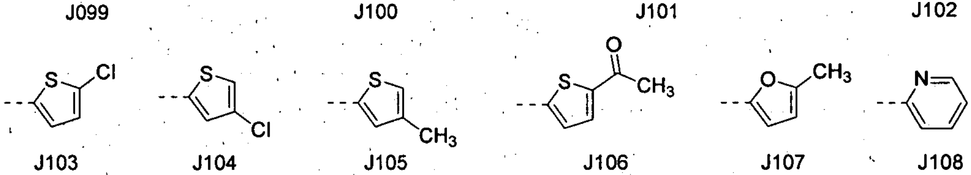

J103 J104 J105 J106 J107 J108

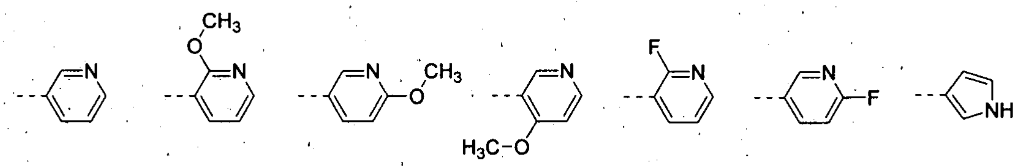

J109 J110 J111 J112 J113 J114 J115

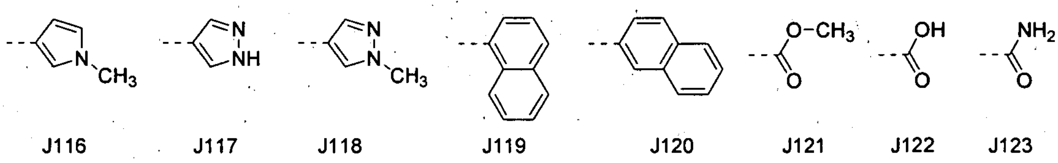

J116 J117 J118 J119 J120 J121 J122 J123

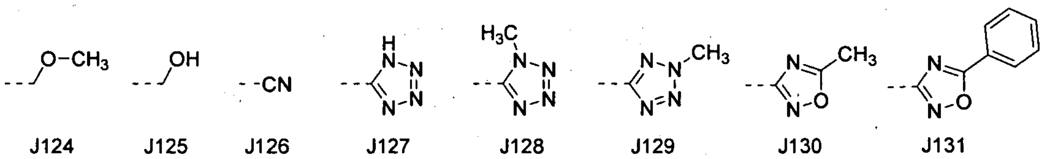

J124 J125 J126 J127 J128 J129 J130 J131

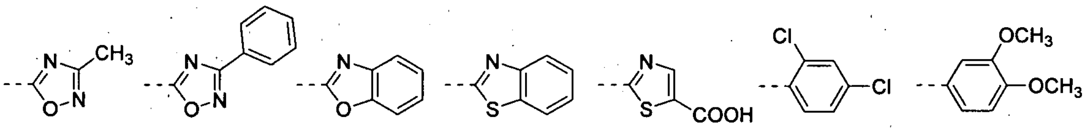

J132 J133 J134 J135 J136 J137 J138

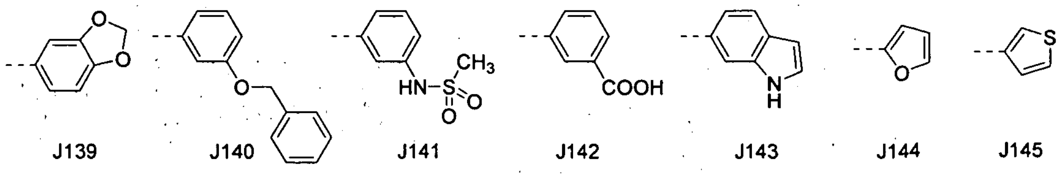

J139 J140 J141 J142 J143 J144 J145

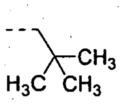
J146

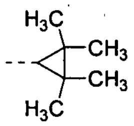
J147

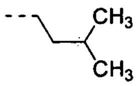
J148

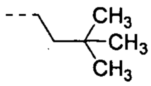
J149

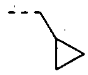
J150

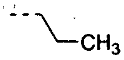
J151

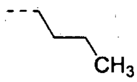
J152

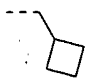
J153

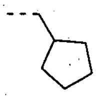
J154

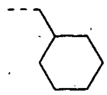
J155

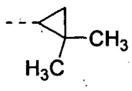
J156

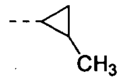
J157

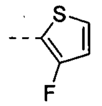
J158

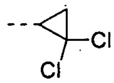
J159

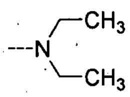
J160

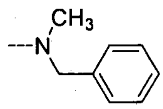
J161

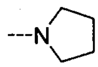
J162

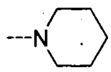
J163

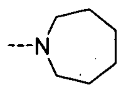
J164

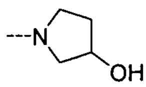
J165

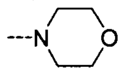
J166

In the pyrrolo-pyrimidine-thione derivatives of the formula (I), as specific examples of preferred combinations of the -$A^6$-$R^3$ portion, groups represented by the following formulae, T001-T181 may be mentioned. In the respective chemical formulae, symbol "---" indicates a binding site between a carbon atom of a pyrrole ring and -$A^6$-$R^3$.

| | | | | | | |
|---|---|---|---|---|---|---|
| --H | --F | --Cl | --Br | ---I | --$NO_2$ | --$CF_3$ |
| T001 | T002 | T003 | T004 | T005 | T006 | T007 |
| --$CH_3$ | 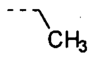 | 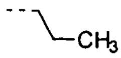 | 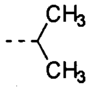 | 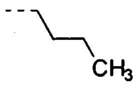 | 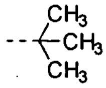 | 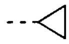 |
| T008 | T009 | T010 | T011 | T012 | T013 | T014 |
| 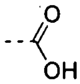 | 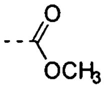 | 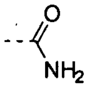 | 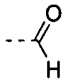 | 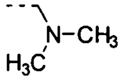 | 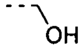 | 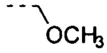 |
| T015 | T016 | T017 | T018 | T019 | T020 | T021 |
| 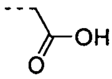 | 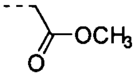 | 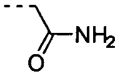 | 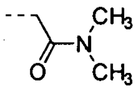 | 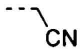 | 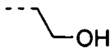 | 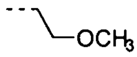 |
| T022 | T023 | T024 | T025 | T026 | T027 | T028 |
| 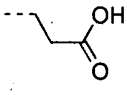 | 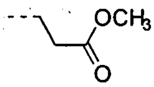 | 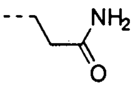 | 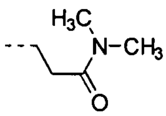 | 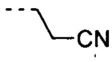 | 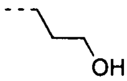 | 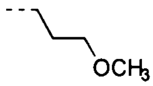 |
| T029 | T030 | T031 | T032 | T033 | T034 | T035 |
| 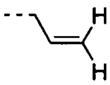 | 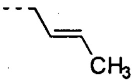 | 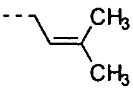 | 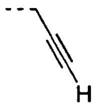 | 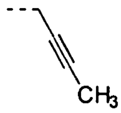 | 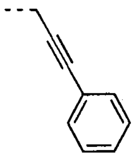 | 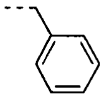 |
| T036 | T037 | T038 | T039 | T040 | T041 | T042 |
| 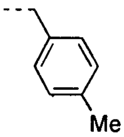 | 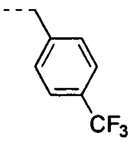 | 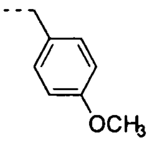 | 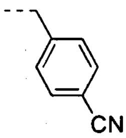 | 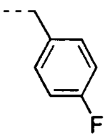 | 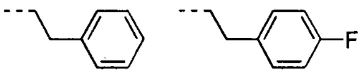 | |
| T043 | T044 | T045 | T046 | T047 | T048 | T049 |

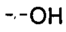
T050
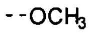
T051
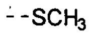
T052
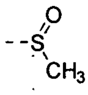
T053
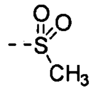
T054

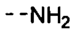
T055
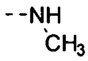
T056
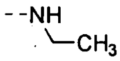
T057
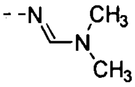
T058
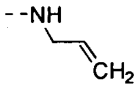
T059

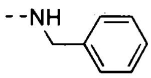
T060
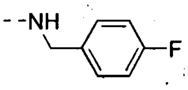
T061
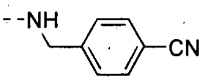
T062
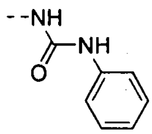
T063
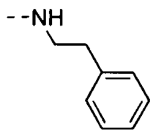
T064

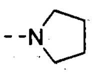
T065
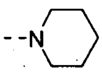
T066
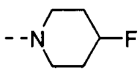
T067
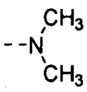
T068
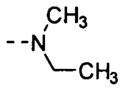
T069

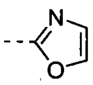
T070
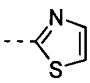
T071
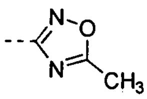
T072
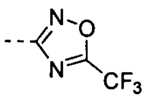
T073
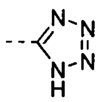
T074
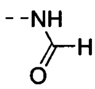
T075
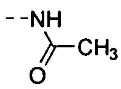
T076

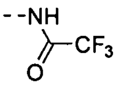
T077
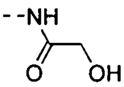
T078
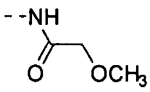
T079
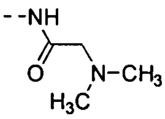
T080
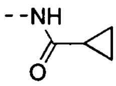
T081
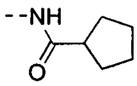
T082
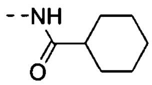
T083

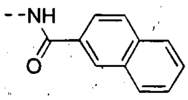
T084

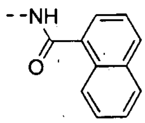
T085

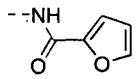
T086

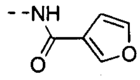
T087

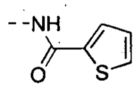
T088

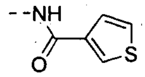
T089

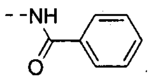
T090

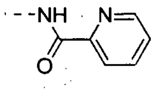
T091

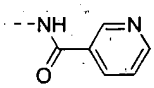
T092

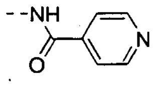
T093

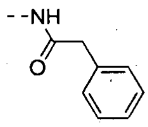
T094

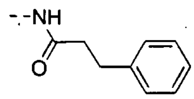
T095

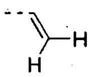
T096

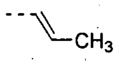
T097

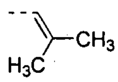
T098

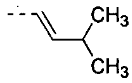
T099

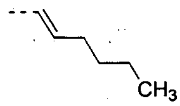
T100

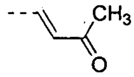
T101

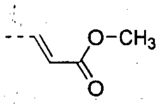
T102

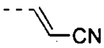
T103

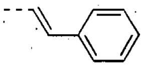
T104

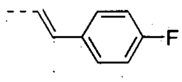
T105

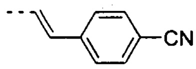
T106

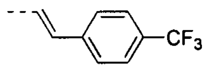
T107

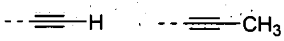
T108

T109

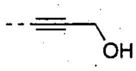
T110

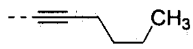
T111

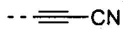
T112

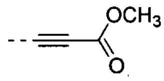
T113

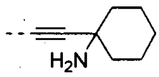
T114

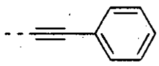
T115

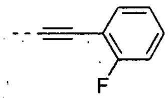
T116

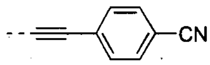
T117

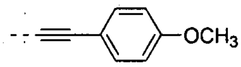
T118

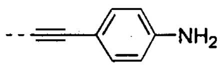
T119

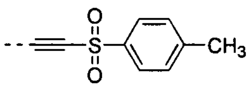
T120

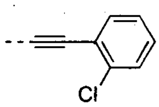
T121

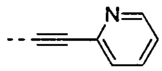
T122

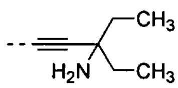
T123

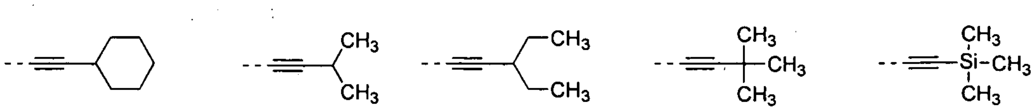
T124

T125

T126

T127

T128

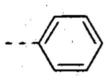
T129
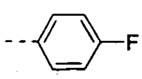
T130
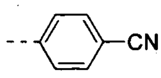
T131
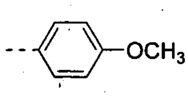
T132
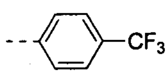
T133
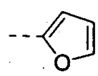
T134
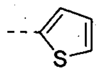
T135

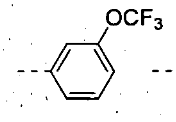
T136
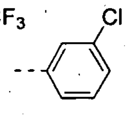
T137
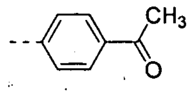
T138
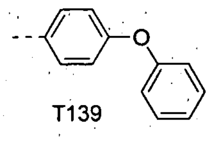
T139
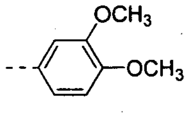
T140
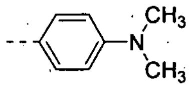
T141

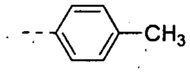
T142
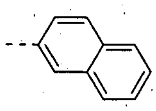
T143
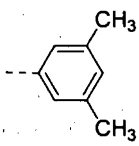
T144
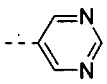
T145
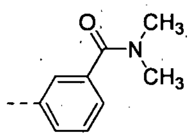
T146
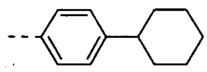
T147

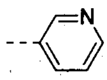
T148
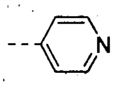
T149
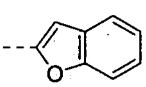
T150
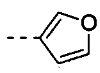
T151
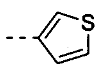
T152
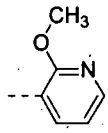
T153
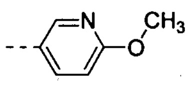
T154

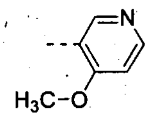
T155
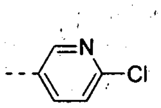
T156
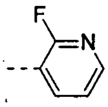
T157
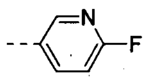
T158
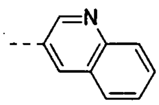
T159
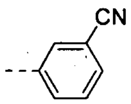
T160

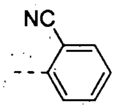
T161
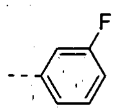
T162
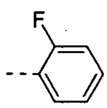
T163
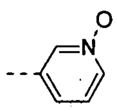
T164
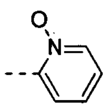
T165
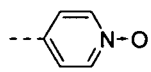
T166

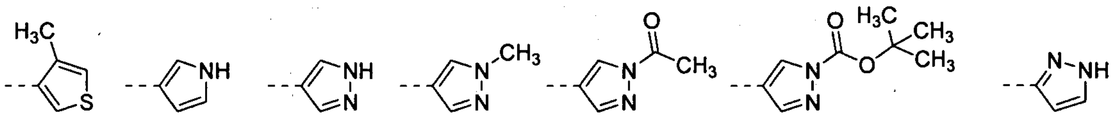
T167 T168 T169 T170 T171 T172 T173

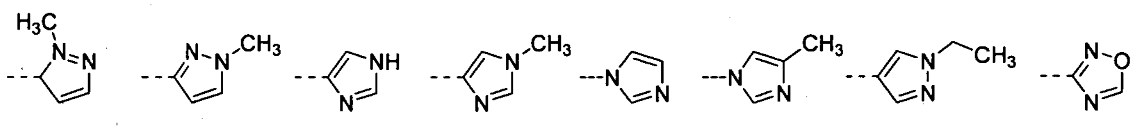
T174 T175 T176 T177 T178 T179 T180 T181

Specific examples of the pyrrolo-pyrimidine-thione derivatives of formula (I) include the compounds having groups described in the following Table 1 as $A^1$, the compounds having groups described in the following Table 1 as $A^2$, the compounds having groups represented by K001-K431 indicated in the formula as $-G^1-A^3-A^4-G^2$, the compounds having groups represented by J01-J166 indicated in the formula as $-A^5-R^2$, the compounds having groups represented by T001-T181 indicated in the formula as $-A^6-R^3$, and the compounds consisting of any combination of groups mentioned above with regard to each moiety. Preferable examples among such compounds are listed in Tables below.

Table 1

| Compound no. | $-A^1-$ | $-A^2-$ | $-G^1-A^3-A^4-G^2$ | $-A^5-R^2$ | $-A^6-R^3$ |
|---|---|---|---|---|---|
| 1 | $-(CH_2)_2-$ | -NH- | K013 | J045 | T005 |
| 2 | $-(CH_2)_2-$ | -NH- | K185 | J007 | T169 |
| 3 | $-(CH_2)_2-$ | -NH- | K185 | J008 | T152 |
| 4 | $-(CH_2)_2-$ | -NH- | K185 | J009 | T151 |
| 5 | $-(CH_2)_2-$ | -NH- | K185 | J010 | T148 |
| 6 | $-(CH_2)_2-$ | -NH- | K185 | J011 | T005 |
| 7 | $-(CH_2)_2-$ | -NH- | K185 | J012 | T003 |
| 8 | $-(CH_2)_2-$ | -NH- | K185 | J012 | T004 |
| 9 | $-(CH_2)_2-$ | -NH- | K185 | J012 | T077 |
| 10 | $-(CH_2)_2-$ | -NH- | K185 | J012 | T089 |
| 11 | $-(CH_2)_2-$ | -NH- | K185 | J012 | T108 |
| 12 | $-(CH_2)_2-$ | -NH- | K185 | J012 | T148 |
| 13 | $-(CH_2)_2-$ | -NH- | K185 | J012 | T151 |
| 14 | $-(CH_2)_2-$ | -NH- | K185 | J012 | T152 |
| 15 | $-(CH_2)_2-$ | -NH- | K185 | J012 | T169 |
| 16 | $-(CH_2)_2-$ | -NH- | K185 | J012 | T170 |
| 17 | $-(CH_2)_2-$ | -NH- | K185 | J121 | T169 |
| 18 | $-(CH_2)_2-$ | -NH- | K185 | J126 | T152 |
| 19 | $-(CH_2)_2-$ | -NH- | K185 | J138 | T152 |
| 20 | $-(CH_2)_2-$ | -NH- | K185 | J144 | T170 |
| 21 | $-(CH_2)_2-$ | -NH- | K185 | J037 | T152 |
| 22 | $-(CH_2)_2-$ | -NH- | K185 | J038 | T151 |
| 23 | $-(CH_2)_2-$ | -NH- | K185 | J039 | T148 |
| 24 | $-(CH_2)_2-$ | -NH- | K185 | J043 | T005 |
| 25 | $-(CH_2)_2-$ | -NH- | K185 | J044 | T003 |
| 26 | $-(CH_2)_2-$ | -NH- | K185 | J044 | T004 |
| 27 | $-(CH_2)_2-$ | -NH- | K185 | J044 | T077 |
| 28 | $-(CH_2)_2-$ | -NH- | K185 | J044 | T089 |
| 29 | $-(CH_2)_2-$ | -NH- | K185 | J044 | T108 |
| 30 | $-(CH_2)_2-$ | -NH- | K185 | J044 | T148 |
| 31 | $-(CH_2)_2-$ | -NH- | K185 | J044 | T151 |
| 32 | $-(CH_2)_2-$ | -NH- | K185 | J044 | T152 |
| 33 | $-(CH_2)_2-$ | -NH- | K185 | J044 | T169 |
| 34 | $-(CH_2)_2-$ | -NH- | K185 | J044 | T170 |
| 35 | $-(CH_2)_2-$ | -NH- | K185 | J045 | T003 |
| 36 | $-(CH_2)_2-$ | -NH- | K185 | J045 | T004 |
| 37 | $-(CH_2)_2-$ | -NH- | K185 | J045 | T005 |

Table continued

| Compound no. | $-A^1-$ | $-A^2-$ | $-G^1-A^3-A^4-G^2$ | $-A^5-R^2$ | $-A^6-R^3$ |
|---|---|---|---|---|---|
| 38 | $-(CH_2)_2-$ | -NH- | K185 | J045 | T090 |
| 39 | $-(CH_2)_2-$ | -NH- | K197 | J045 | T003 |
| 40 | $-(CH_2)_2-$ | -NH- | K197 | J045 | T004 |
| 41 | $-(CH_2)_2-$ | -NH- | K197 | J045 | T005 |
| 42 | $-(CH_2)_2-$ | -NH-C(=O)- | K008 | J001 | T001 |
| 43 | $-(CH_2)_2-$ | -NH-C(=O)- | K008 | J001 | T037 |
| 44 | $-(CH_2)_2-$ | -NH-C(=O)- | K008 | J001 | T042 |
| 45 | $-(CH_2)_2-$ | -NH-C(=O)- | K008 | J001 | T049 |
| 46 | $-(CH_2)_2-$ | -NH-C(=O)- | K008 | J001 | T061 |
| 47 | $-(CH_2)_2-$ | -NH-C(=O)- | K008 | J001 | T073 |
| 48 | $-(CH_2)_2-$ | -NH-C(=O)- | K008 | J001 | T085 |
| 49 | $-(CH_2)_2-$ | -NH-C(=O)- | K008 | J001 | T095 |
| 50 | $-(CH_2)_2-$ | -NH-C(=O)- | K008 | J001 | T097 |
| 51 | $-(CH_2)_2-$ | -NH-C(=O)- | K008 | J012 | T005 |
| 52 | $-(CH_2)_2-$ | -NH-C(=O)- | K008 | J012 | T041 |
| 53 | $-(CH_2)_2-$ | -NH-C(=O)- | K008 | J012 | T053 |
| 54 | $-(CH_2)_2-$ | -NH-C(=O)- | K008 | J012 | T065 |
| 55 | $-(CH_2)_2-$ | -NH-C(=O)- | K008 | J012 | T077 |
| 56 | $-(CH_2)_2-$ | -NH-C(=O)- | K008 | J012 | T087 |
| 57 | $-(CH_2)_2-$ | -NH-C(=O)- | K008 | J012 | T089 |
| 58 | $-(CH_2)_2-$ | -NH-C(=O)- | K008 | J012 | T099 |
| 59 | $-(CH_2)_2-$ | -NH-C(=O)- | K008 | J012 | T101 |
| 60 | $-(CH_2)_2-$ | -NH-C(=O)- | K008 | J045 | T014 |
| 61 | $-(CH_2)_2-$ | -NH-C(=O)- | K008 | J045 | T033 |
| 62 | $-(CH_2)_2-$ | -NH-C(=O)- | K008 | J045 | T045 |
| 63 | $-(CH_2)_2-$ | -NH-C(=O)- | K008 | J045 | T057 |
| 64 | $-(CH2)_2-$ | -NH-C(=O)- | K008 | J045 | T069 |
| 65 | $-(CH_2)_2-$ | -NH-C(=O)- | K008 | J045 | T081 |
| 66 | $-(CH_2)_2-$ | -NH-C(=O)- | K008 | J045 | T091 |
| 67 | $-(CH_2)_2-$ | -NH-C(=O)- | K008 | J045 | T093 |
| 68 | $-(CH_2)_2-$ | -NH-C(=O)- | K008 | J045 | T151 |
| 69 | $-(CH_2)_2-$ | -NH-C(=O)- | K009 | J001 | T016 |
| 70 | $-(CH_2)_2-$ | -NH-C(=O)- | K009 | J001 | T034 |
| 71 | $-(CH_2)_2-$ | -NH-C(=O)- | K009 | J001 | T046 |
| 72 | $-(CH_2)_2-$ | -NH-C(=O)- | K009 | J001 | T058 |
| 73 | $-(CH_2)_2-$ | -NH-C(=O)- | K009 | J001 | T070 |
| 74 | $-(CH_2)_2-$ | -NH-C(=O)- | K009 | J001 | T082 |
| 75 | $-(CH_2)_2-$ | -NH-C(=O)- | K009 | J001 | T092 |

Table continued

| Compound no. | -$A^1$- | -$A^2$- | -$G^1$-$A^3$-$A^4$-$G^2$ | -$A^5$-$R^2$ | -$A^6$-$R^3$ |
|---|---|---|---|---|---|
| 76 | $-(CH_2)_2-$ | -NH-C(=O)- | K009 | J001 | T094 |
| 77 | $-(CH_2)_2-$ | -NH-C(=O)- | K009 | J001 | T152 |
| 78 | $-(CH_2)_2-$ | -NH-C(=O)- | K009 | J012 | T002 |
| 79 | $-(CH_2)_2-$ | -NH-C(=O)- | K009 | J012 | T038 |
| 80 | $-(CH_2)_2-$ | -NH-C(=O)- | K009 | J012 | T050 |
| 81 | $-(CH_2)_2-$ | -NH-C(=O)- | K009 | J012 | T062 |
| 82 | $-(CH_2)_2-$ | -NH-C(=O)- | K009 | J012 | T074 |
| 83 | $-(CH_2)_2-$ | -NH-C(=O)- | K009 | J012 | T084 |
| 84 | $-(CH_2)_2-$ | -NH-C(=O)- | K009 | J012 | T086 |
| 85 | $-(CH_2)_2-$ | -NH-C(=O)- | K009 | J012 | T096 |
| 86 | $-(CH_2)_2-$ | -NH-C(=O)- | K009 | J012 | T098 |
| 87 | $-(CH_2)_2-$ | -NH-C(=O)- | K009 | J045 | T007 |
| 88 | $-(CH_2)_2-$ | -NH-C(=O)- | K009 | J045 | T042 |
| 89 | $-(CH_2)_2-$ | -NH-C(=O)- | K009 | J045 | T054 |
| 90 | $-(CH_2)_2-$ | -NH-C(=O)- | K009 | J045 | T066 |
| 91 | $-(CH_2)_2-$ | -NH-C(=O)- | K009 | J045 | T078 |
| 92 | $-(CH_2)_2-$ | -NH-C(=O)- | K009 | J045 | T088 |
| 93 | $-(CH_2)_2-$ | -NH-C(=O)- | K009 | J045 | T090 |
| 94 | $(CH_2)_2-$ | -NH-C(=O)- | K009 | J045 | T108 |
| 95 | $-(CH_2)_2-$ | -NH-C(=O)- | K009 | J045 | T129 |
| 96 | $-(CH_2)_2-$ | -NH-C(=O)- | K011 | J001 | T008 |
| 97 | $-(CH_2)_2-$ | -NH-C(=O)- | K011 | J001 | T043 |
| 98 | $-(CH_2)_2-$ | -NH-C(=O)- | K011 | J001 | T055 |
| 99 | $-(CH_2)_2-$ | -NH-C(=O)- | K011 | J001 | T067 |
| 100 | $-(CH_2)_2-$ | -NH-C(=O)- | K011 | J001 | T079 |
| 101 | $-(CH_2)_2-$ | -NH-C(=O)- | K011 | J001 | T089 |
| 102 | $-(CH_2)_2-$ | -NH-C(=O)- | K011 | J001 | T091 |
| 103 | $-(CH_2)_2-$ | -NH-C(=O)- | K011 | J001 | T109 |
| 104 | $-(CH_2)_2-$ | -NH-C(=O)- | K011 | J001 | T148 |
| 105 | $-(CH_2)_2-$ | -NH-C(=O)- | K011 | J012 | T017 |
| 106 | $-(CH_2)_2-$ | -NH-C(=O)- | K011 | J012 | T035 |
| 107 | $-(CH_2)_2-$ | -NH-C(=O)- | K011 | J012 | T047 |
| 108 | $-(CH_2)_2-$ | -NH-C(=O)- | K011 | J012 | T059 |
| 109 | $-(CH_2)_2-$ | -NH-C(=O)- | K011 | J012 | T071 |
| 110 | $-(CH_2)_2-$ | -NH-C(=O)- | K011 | J012 | T083 |
| 111 | $-(CH_2)_2-$ | -NH-C(=O)- | K011 | J012 | T093 |
| 112 | $-(CH_2)_2-$ | -NH-C(=O)- | K011 | J012 | T095 |
| 113 | $-(CH_2)_2-$ | -NH-C(=O)- | K011 | J012 | T169 |

Table continued

| Compound no. | $-A^1-$ | $-A^2-$ | $-G^1-A^3-A^4-G^2$ | $-A^5-R^2$ | $-A^6-R^3$ |
|---|---|---|---|---|---|
| 114 | $-(CH_2)_2-$ | -NH-C(=O)- | K011 | J045 | T003 |
| 115 | $-(CH_2)_2-$ | -NH-C(=O)- | K011 | J045 | T039 |
| 116 | $-(CH_2)_2-$ | -NH-C(=O)- | K011 | J045 | T051 |
| 117 | $-(CH_2)_2-$ | -NH-C(=O)- | K011 | J045 | T063 |
| 118 | $-(CH_2)_2-$ | -NH-C(=O)- | K011 | J045 | T075 |
| 119 | $-(CH_2)_2-$ | -NH-C(=O)- | K011 | J045 | T085 |
| 120 | $-(CH_2)_2-$ | -NH-C(=O)- | K011 | J045 | T087 |
| 121 | $-(CH_2)_2-$ | -NH-C(=O)- | K011 | J045 | T097 |
| 122 | $-(CH_2)_2-$ | -NH-C(=O)- | K011 | J045 | T099 |
| 123 | $-(CH_2)_2-$ | -NH-C(=O)- | K011 | J045 | T148 |
| 124 | $-(CH_2)_2-$ | -NH-C(=O)- | K013 | J007 | T005 |
| 125 | $-(CH_2)_2-$ | -NH-C(=O)- | K013 | J008 | T170 |
| 126 | $-(CH_2)_2-$ | -NH-C(=O)- | K013 | J009 | T169 |
| 127 | $-(CH_2)_2-$ | -NH-C(=O)- | K013 | J010 | T152 |
| 128 | $-(CH_2)_2-$ | -NH-C(=O)- | K013 | J011 | T151 |
| 129 | $-(CH_2)_2-$ | -NH-C(=O)- | K013 | J012 | T005 |
| 130 | $-(CH_2)_2-$ | -NH-C(=O)- | K013 | J012 | T017 |
| 131 | $-(CH_2)_2-$ | -NH-C(=O)- | K013 | J012 | T080 |
| 132 | $-(CH_2)_2-$ | -NH-C(=O)- | K013 | J012 | T091 |
| 133 | $-(CH_2)_2-$ | -NH-C(=O)- | K013 | J012 | T131 |
| 134 | $-(CH_2)_2-$ | -NH-C(=O)- | K013 | J012 | T148 |
| 135 | $-(CH_2)_2-$ | -NH-C(=O)- | K013 | J012 | T151 |
| 136 | $-(CH_2)_2-$ | -NH-C(=O)- | K013 | J012 | T152 |
| 137 | $-(CH_2)_2-$ | -NH-C(=O)- | K013 | J012 | T169 |
| 138 | $-(CH_2)_2-$ | -NH-C(=O)- | K013 | J012 | T170 |
| 139 | $-(CH_2)_2-$ | -NH-C(=O)- | K013 | J121 | T005 |
| 140 | $-(CH_2)_2-$ | -NH-C(=O)- | K013 | J126 | T170 |
| 141 | $-(CH_2)_2-$ | -NH-C(=O)- | K013 | J138 | T170 |
| 142 | $-(CH_2)_2-$ | -NH-C(=O)- | K013 | J144 | T148 |
| 143 | $-(CH_2)_2-$ | -NH-C(=O)- | K013 | J037 | T170 |
| 144 | $-(CH_2)_2-$ | -NH-C(=O)- | K013 | J038 | T169 |
| 145 | $-(CH_2)_2-$ | -NH-C(=O)- | K013 | J039 | T152 |
| 146 | $-(CH_2)_2-$ | -NH-C(=O)- | K013 | J043 | T151 |
| 147 | $-(CH_2)_2-$ | -NH-C(=O)- | K013 | J044 | T005 |
| 148 | $-(CH_2)_2-$ | -NH-C(=O)- | K013 | J044 | T017 |
| 149 | $-(CH_2)_2-$ | -NH-C(=O)- | K013 | J044 | T080 |
| 150 | $-(CH_2)_2-$ | -NH-C(=O)- | K013 | J044 | T091 |
| 151 | $-(CH_2)_2-$ | -NH-C(=O)- | K013 | J044 | T131 |

Table continued

| Compound no. | $-A^1-$ | $-A^2-$ | $-G^1-A^3-A^4-G^2$ | $-A^5-R^2$ | $-A^6-R^3$ |
|---|---|---|---|---|---|
| 152 | $-(CH_2)_2-$ | -NH-C(=O)- | K013 | J044 | T148 |
| 153 | $-(CH_2)_2-$ | -NH-C(=O)- | K013 | J044 | T151 |
| 154 | $-(CH_2)_2-$ | -NH-C(=O)- | K013 | J044 | T152 |
| 155 | $-(CH_2)_2-$ | -NH-C(=O)- | K013 | J044 | T169 |
| 156 | $-(CH_2)_2-$ | -NH-C(=O)- | K013 | J044 | T170 |
| 157 | $-(CH_2)_2-$ | -NH-C(=O)- | K013 | J045 | T003 |
| 158 | $-(CH_2)_2-$ | -NH-C(=O)- | K013 | J045 | T004 |
| 159 | $-(CH_2)_2-$ | -NH-C(=O)- | K013 | J045 | T005 |
| 160 | $-(CH_2)_2-$ | -NH-C(=O)- | K013 | J045 | T055 |
| 161 | $-(CH_2)_2-$ | -NH-C(=O)- | K013 | J045 | T056 |
| 162 | $-(CH_2)_2-$ | -NH-C(=O)- | K013 | J045 | T057 |
| 163 | $-(CH_2)_2-$ | -NH-C(=O)- | K013 | J045 | T058 |
| 164 | $-(CH_2)_2-$ | -NH-C(=O)- | K013 | J045 | T059 |
| 165 | $-(CH_2)_2-$ | -NH-C(=O)- | K013 | J045 | T060 |
| 166 | $-(CH_2)_2-$ | -NH-C(=O)- | K013 | J045 | T061 |
| 167 | $-(CH_2)_2-$ | -NH-C(=O)- | K013 | J045 | T062 |
| 168 | $-(CH_2)_2-$ | -NH-C(=O)- | K013 | J045 | T063 |
| 169 | $-(CH_2)_2-$ | -NH-C(=O)- | K013 | J045 | T064 |
| 170 | $-(CH_2)_2-$ | -NH-C(=O)- | K013 | J045 | T065 |
| 171 | $-(CH_2)_2-$ | -NH-C(=O)- | K013 | J045 | T066 |
| 172 | $-(CH_2)_2-$ | -NH-C(=O)- | K013 | J045 | T067 |
| 173 | $-(CH_2)_2-$ | -NH-C(=O)- | K013 | J045 | T068 |
| 174 | $-(CH_2)_2-$ | -NH-C(=O)- | K013 | J045 | T069 |
| 175 | $-(CH_2)_2-$ | -NH-C(=O)- | K013 | J045 | T070 |
| 176 | $-(CH_2)_2-$ | -NH-C(=O)- | K013 | J045 | T071 |
| 177 | $-(CH_2)_2-$ | -NH-C(=O)- | K013 | J045 | T072 |
| 178 | $-(CH_2)_2-$ | -NH-C(=O)- | K013 | J045 | T073 |
| 179 | $-(CH_2)_2-$ | -NH-C(=O)- | K013 | J045 | T074 |
| 180 | $-(CH_2)_2-$ | -NH-C(=O)- | K013 | J045 | T075 |
| 181 | $-(CH_2)_2-$ | -NH-C(=O)- | K013 | J045 | T076 |
| 182 | $-(CH_2)_2-$ | -NH-C(=O)- | K013 | J045 | T077 |
| 183 | $-(CH_2)_2-$ | -NH-C(=O)- | K013 | J045 | T078 |
| 184 | $-(CH_2)_2-$ | -NH-C(=O)- | K013 | J045 | T079 |
| 185 | $-(CH_2)_2-$ | -NH-C(=O)- | K013 | J045 | T080 |
| 186 | $-(CH_2)_2-$ | -NH-C(=O)- | K013 | J045 | T081 |
| 187 | $-(CH_2)_2-$ | -NH-C(=O)- | K013 | J045 | T082 |
| 188 | $-(CH_2)_2-$ | -NH-C(=O)- | K013 | J045 | T083 |
| 189 | $-(CH_2)_2-$ | -NH-C(=O)- | K013 | J045 | T084 |

Table continued

| Compound no. | $-A^1-$ | $-A^2-$ | $-G^1-A^3-A^4-G^2$ | $-A^5-R^2$ | $-A^6-R^3$ |
|---|---|---|---|---|---|
| 190 | $-(CH_2)_2-$ | -NH-C(=O)- | K013 | J045 | T085 |
| 191 | $-(CH_2)_2-$ | -NH-C(=O)- | K013 | J045 | T086 |
| 192 | $-(CH_2)_2-$ | -NH-C(=O)- | K013 | J045 | T088 |
| 193 | $-(CH_2)_2-$ | -NH-C(=O)- | K013 | J045 | T090 |
| 194 | $-(CH_2)_2-$ | -NH-C(=O)- | K013 | J045 | T091 |
| 195 | $-(CH_2)_2-$ | -NH-C(=O)- | K013 | J045 | T092 |
| 196 | $-(CH_2)_2-$ | -NH-C(=O)- | K013 | J045 | T093 |
| 197 | $-(CH_2)_2-$ | -NH-C(=O)- | K013 | J045 | T094 |
| 198 | $-(CH_2)_2-$ | -NH-C(=O)- | K013 | J045 | T095 |
| 199 | $-(CH_2)_2-$ | -NH-C(=O)- | K013 | J045 | T148 |
| 200 | $-(CH_2)_2-$ | -NH-C(=O)- | K013 | J045 | T170 |
| 201 | $-(CH_2)_2-$ | -NH-C(=O)- | K051 | J007 | T152 |
| 202 - | $-(CH_2)_2$ | -NH-C(=O)- | K051 | J008 | T151 |
| 203 | $-(CH_2)_2-$ | -NH-C(=O)- | K051 | J009 | T148 |
| 204 | $-(CH_2)_2-$ | -NH-C(=O)- | K051 | J010 | T005 |
| 205 | $-(CH_2)_2-$ | -NH-C(=O)- | K051 | J011 | T170 |
| 206 | $-(CH_2)_2-$ | -NH-C(=O)- | K051 | J012 | T007 |
| 207 | $-(CH_2)_2-$ | -NH-C(=O)- | K051 | J012 | T076 |
| 208 | $-(CH_2)_2-$ | -NH-C(=O)- | K051 | J012 | T087 |
| 209 | $-(CH_2)_2-$ | -NH-C(=O)- | K051 | J012 | T096 |
| 210 | $-(CH_2)_2-$ | -NH-C(=O)- | K051 | J012 | T148 |
| 211 | $-(CH_2)_2-$ | -NH-C(=O)- | K051 | J012 | T149 |
| 212 | $-(CH_2)_2-$ | -NH-C(=O)- | K051 | J012 | T151 |
| 213 | $-(CH_2)_2-$ | -NH-C(=O)- | K051 | J012 | T152 |
| 214 | $-(CH_2)_2-$ | -NH-C(=O)- | K051 | J012 | T169 |
| 215 | $-(CH_2)_2-$ | -NH-C(=O)- | K051 | J012 | T170 |
| 216 | $-(CH_2)_2-$ | -NH-C(=O)- | K051 | J121 | T152 |
| 217 | $-(CH_2)_2-$ | -NH-C(=O)- | K051 | J126 | T151 |
| 218 | $-(CH_2)_2-$ | -NH-C(=O)- | K051 | J138 | T151 |
| 219 | $-(CH_2)_2-$ | -NH-C(=O)- | K051 | J144 | T169 |
| 220 | $-(CH_2)_2-$ | -NH-C(=O)- | K051 | J037 | T151 |
| 221 | $-(CH_2)_2-$ | -NH-C(=O)- | K051 | J038 | T148 |
| 222 | $-(CH_2)_2-$ | -NH-C(=O)- | K051 | J039 | T005 |
| 223 | $-(CH_2)_2-$ | -NH-C(=O)- | K051 | J043 | T170 |
| 224 | $-(CH_2)_2-$ | -NH-C(=O)- | K051 | J045 | T007 |
| 225 | $-(CH_2)_2-$ | -NH-C(=O)- | K051 | J045 | T076 |
| 226 | $-(CH_2)_2-$ | -NH-C(=O)- | K051 | J045 | T087 |
| 227 | $-(CH_2)_2-$ | -NH-C(=O)- | K051 | J045 | T096 |

Table continued

| Compound no. | $-A^1-$ | $-A^2-$ | $-G^1-A^3-A^4-G^2$ | $-A^5-R^2$ | $-A^6-R^3$ |
|---|---|---|---|---|---|
| 228 | $-(CH_2)_2-$ | -NH-C(=O)- | K051 | J045 | T148 |
| 229 | $-(CH_2)_2-$ | -NH-C(=O)- | K051 | J045 | T149 |
| 230 | $-(CH_2)_2-$ | -NH-C(=O)- | K051 | J045 | T151 |
| 231 | $-(CH_2)_2-$ | -NH-C(=O)- | K051 | J045 | T152 |
| 232 | $-(CH_2)_2-$ | -NH-C(=O)- | K051 | J045 | T169 |
| 233 | $-(CH_2)_2-$ | -NH-C(=O)- | K051 | J045 | T170 |
| 234 | $-(CH_2)_2-$ | -NH-C(=O)- | K089 | J045 | T005 |
| 235 | $-(CH_2)_2-$ | -NH-C(=O)- | K144 | J012 | T109 |
| 236 | $-(CH_2)_2-$ | -NH-C(=O)- | K200 | J001 | T004 |
| 237 | $-(CH_2)_2-$ | -NH-C(=O)- | K200 | J001 | T040 |
| 238 | $-(CH_2)_2-$ | -NH-C(=O)- | K200 | J001 | T052 |
| 239 | $-(CH_2)_2-$ | -NH-C(=O)- | K200 | J001 | T064 |
| 240 | $-(CH_2)_2-$ | -NH-C(=O)- | K200 | J001 | T076 |
| 241 | $-(CH_2)_2-$ | -NH-C(=O)- | K200 | J001 | T086 |
| 242 | $-(CH_2)_2-$ | -NH-C(=O)- | K200 | J001 | T088 |
| 243 | $-(CH_2)_2-$ | -NH-C(=O)- | K200 | J001 | T098 |
| 244 | $-(CH_2)_2-$ | -NH-C(=O)- | K200 | J001 | T100 |
| 245 | $-(CH_2)_2-$ | -NH-C(=O)- | K200 | J007 | T148 |
| 246 | $-(CH_2)_2-$ | -NH-C(=O)- | K200 | J008 | T005 |
| 247 | $-(CH_2)_2-$ | -NH-C(=O)- | K200 | J009 | T170 |
| 248 | $-(CH_2)_2-$ | -NH-C(=O)- | K200 | J010 | T169 |
| 249 | $-(CH_2)_2-$ | -NH-C(=O)- | K200 | J011 | T152 |
| 250 | $-(CH_2)_2-$ | -NH-C(=O)- | K200 | J012 | T005 |
| 251 | $-(CH_2)_2-$ | -NH-C(=O)- | K200 | J012 | T009 |
| 252 | $-(CH_2)_2-$ | -NH-C(=O)- | K200 | J012 | T017 |
| 253 | $-(CH_2)_2-$ | -NH-C(=O)- | K200 | J012 | T032 |
| 254 | $-(CH_2)_2-$ | -NH-C(=O)- | K200 | J012 | T044 |
| 255 | $-(CH_2)_2-$ | -NH-C(=O)- | K200 | J012 | T056 |
| 256 | $-(CH_2)_2-$ | -NH-C(=O)- | K200 | J012 | T068 |
| 257 | $-(CH_2)_2-$ | -NH-C(=O)- | K200 | J012 | T080 |
| 258 | $-(CH_2)_2-$ | -NH-C(=O)- | K200 | J012 | T004 |
| 259 | $-(CH_2)_2-$ | -NH-C(=O)- | K200 | J012 | T090 |
| 260 | $-(CH_2)_2-$ | -NH-C(=O)- | K200 | J012 | T092 |
| 261 | $-(CH_2)_2-$ | -NH-C(=O)- | K200 | J012 | T006 |
| 262 | $-(CH_2)_2-$ | -NH-C(=O)- | K200 | J012 | T145 |
| 263 | $-(CH_2)_2-$ | -NH-C(=O)- | K200 | J012 | T148 |
| 264 | $-(CH_2)_2-$ | -NH-C(=O)- | K200 | J012 | T149 |
| 265 | $-(CH_2)_2-$ | -NH-C(=O)- | K200 | J012 | T151 |

Table continued

| Compound no. | $-A^1-$ | $-A^2-$ | $-G^1-A^3-A^4-G^2$ | $-A^5-R^2$ | $-A^6-R^3$ |
|---|---|---|---|---|---|
| 266 | $-(CH_2)_2-$ | -NH-C(=O)- | K200 | J012 | T152 |
| 267 | $-(CH_2)_2-$ | -NH-C(=O)- | K200 | J012 | T169 |
| 268 | $-(CH_2)_2-$ | -NH-C(=O)- | K200 | J012 | T170 |
| 269 | $-(CH_2)_2-$ | -NH-C(=O)- | K200 | J121 | T148 |
| 270 | $-(CH_2)_2-$ | -NH-C(=O)- | K200 | J126 | T005 |
| 271 | $-(CH_2)_2-$ | -NH-C(=O)- | K200 | J138 | T005 |
| 272 | $-(CH_2)_2-$ | -NH-C(=O)- | K200 | J144 | T151 |
| 273 | $-(CH_2)_2-$ | -NH-C(=O)- | K200 | J037 | T005 |
| 274 | $-(CH_2)_2-$ | -NH-C(=O)- | K200 | J038 | T170 |
| 275 | $-(CH_2)_2-$ | -NH-C(=O)- | K200 | J039 | T169 |
| 276 | $-(CH_2)_2-$ | -NH-C(=O)- | K200 | J043 | T152 |
| 277 | $-(CH_2)_2-$ | -NH-C(=O)- | K200 | J045 | T005 |
| 278 | $-(CH_2)_2-$ | -NH-C(=O)- | K200 | J045 | T017 |
| 279 | $-(CH_2)_2-$ | -NH-C(=O)- | K200 | J045 | T018 |
| 280 | $-(CH_2)_2-$ | -NH-C(=O)- | K200 | J045 | T036 |
| 281 | $-(CH_2)_2-$ | -NH-C(=O)- | K200 | J045 | T048 |
| 282 | $-(CH_2)_2-$ | -NH-C(=O)- | K200 | J045 | T060 |
| 283 | $-(CH_2)_2-$ | -NH-C(=O)- | K200 | J045 | T072 |
| 284 | $-(CH_2)_2-$ | -NH-C(=O)- | K200 | J045 | T080 |
| 285 | $-(CH_2)_2-$ | -NH-C(=O)- | K200 | J045 | T084 |
| 286 | $-(CH_2)_2-$ | -NH-C(=O)- | K200 | J045 | T092 |
| 287 | $-(CH_2)_2-$ | -NH-C(=O)- | K200 | J045 | T094 |
| 288 | $-(CH_2)_2-$ | -NH-C(=O)- | K200 | J045 | T096 |
| 289 | $-(CH_2)_2-$ | -NH-C(=O)- | K200 | J045 | T145 |
| 290 | $-(CH_2)_2-$ | -NH-C(=O)- | K200 | J045 | T148 |
| 291 | $-(CH_2)_2-$ | -NH-C(=O)- | K200 | J045 | T151 |
| 292 | $-(CH_2)_2-$ | -NH-C(=O)- | K200 | J045 | T152 |
| 293 | $-(CH_2)_2-$ | -NH-C(=O)- | K200 | J045 | T169 |
| 294 | $-(CH_2)_2-$ | -NH-C(=O)- | K200 | J045 | T170 |
| 295 | $-(CH_2)_2-$ | -NH-C(=O)- | K200 | J045 | T178 |
| 296 | $-(CH_2)_2-$ | -NH-C(=O)- | K204 | J007 | T151 |
| 297 | $-(CH_2)_2-$ | -NH-C(=O)- | K204 | J008 | T148 |
| 298 | $-(CH_2)_2-$ | -NH-C(=O)- | K204 | J009 | T005 |
| 299 | $-(CH_2)_2-$ | -NH-C(=O)- | K204 | J010 | T170 |
| 300 | $-(CH_2)_2-$ | -NH-C(=O)- | K204 | J011 | T169 |
| 301 | $-(CH_2)_2-$ | -NH-C(=O)- | K204 | J012 | T007 |
| 302 | $-(CH_2)_2-$ | -NH-C(=O)- | K204 | J012 | T076 |
| 303 | $-(CH_2)_2-$ | -NH-C(=O)- | K204 | J012 | T086 |

Table continued

| Compound no. | $-A^1-$ | $-A^2-$ | $-G^1-A^3-A^4-G^2$ | $-A^5-R^2$ | $-A^6-R^3$ |
|---|---|---|---|---|---|
| 304 | $-(CH_2)_2-$ | -NH-C(=O)- | K204 | J012 | T093 |
| 305 | $-(CH_2)_2-$ | -NH-C(=O)- | K204 | J012 | T148 |
| 306 | $-(CH_2)_2-$ | -NH-C(=O)- | K204 | J012 | T151 |
| 307 | $-(CH_2)_2-$ | -NH-C(=O)- | K204 | J012 | T152 |
| 308 | $-(CH_2)_2-$ | -NH-C(=O)- | K204 | J012 | T164 |
| 309 | $-(CH_2)_2-$ | -NH-C(=O)- | K204 | J012 | T169 |
| 310 | $-(CH_2)_2-$ | -NH-C(=O)- | K204 | J012 | T170 |
| 311 | $-(CH_2)_2-$ | -NH-C(=O)- | K204 | J121 | T151 |
| 312 | $-(CH_2)_2-$ | -NH-C(=O)- | K204 | J126 | T148 |
| 313 | $-(CH_2)_2-$ | -NH-C(=O)- | K204 | J138 | T148 |
| 314 | $-(CH_2)_2-$ | -NH-C(=O)- | K204 | J144 | T152 |
| 315 | $-(CH_2)_2-$ | -NH-C(=O)- | K204 | J037 | T148 |
| 316 | $-(CH_2)_2-$ | -NH-C(=O)- | K204 | J038 | T005 |
| 317 | $-(CH_2)_2-$ | -NH-C(=O)- | K204 | J039 | T170 |
| 318 | $-(CH_2)_2-$ | -NH-C(=O)- | K204 | J043 | T169 |
| 319 | $-(CH_2)_2-$ | -NH-C(=O)- | K204 | J044 | T007 |
| 320 | $-(CH_2)_2-$ | -NH-C(=O)- | K204 | J044 | T076 |
| 321 | $-(CH_2)_2-$ | -NH-C(=O)- | K204 | J044 | T086 |
| 322 | $-(CH_2)_2-$ | -NH-C(=O)- | K204 | J044 | T093 |
| 323 | $-(CH_2)_2-$ | -NH-C(=O)- | K204 | J044 | T148 |
| 324 | $-(CH_2)_2-$ | -NH-C(=O)- | K204 | J044 | T151 |
| 325 | $-(CH_2)_2-$ | -NH-C(=O)- | K204 | J044 | T152 |
| 326 | $-(CH_2)_2-$ | -NH-C(=O)- | K204 | J044 | T164 |
| 327 | $-(CH_2)_2-$ | -NH-C(=O)- | K204 | J044 | T169 |
| 328 | $-(CH_2)_2-$ | -NH-C(=O)- | K204 | J044 | T170 |
| 329 | $-(CH_2)_2-$ | -NH-C(=O)- | K208 | J007 | T170 |
| 330 | $-(CH_2)_2-$ | -NH-C(=O)- | K208 | J008 | T169 |
| 331 | $-(CH_2)_2-$ | -NH-C(=O)- | K208 | J009 | T152 |
| 332 | $-(CH_2)_2-$ | -NH-C(=O)- | K208 | J010 | T151 |
| 333 | $-(CH_2)_2-$ | -NH-C(=O)- | K208 | J011 | T148 |
| 334 | $-(CH_2)_2-$ | -NH-C(=O)- | K208 | J012 | T003 |
| 335 | $-(CH_2)_2-$ | -NH-C(=O)- | K208 | J012 | T004 |
| 336 | $-(CH_2)_2-$ | -NH-C(=O)- | K208 | J012 | T077 |
| 337 | $-(CH_2)_2-$ | -NH-C(=O)- | K208 | J012 | T090 |
| 338 | $-(CH_2)_2-$ | -NH-C(=O)- | K208 | J012 | T129 |
| 339 | $-(CH_2)_2-$ | -NH-C(=O)- | K208 | J012 | T148 |
| 340 | $-(CH_2)_2-$ | -NH-C(=O)- | K208 | J012 | T151 |
| 341 | $-(CH_2)_2-$ | -NH-C(=O)- | K208 | J012 | T152 |

Table continued

| Compound no. | -$A^1$- | -$A^2$- | -$G^1$-$A^3$-$A^4$-$G^2$ | -$A^5$-$R^2$ | -$A^6$-$R^3$ |
|---|---|---|---|---|---|
| 342 | -$(CH_2)_2$- | -NH-C(=O)- | K208 | J012 | T169 |
| 343 | -$(CH_2)_2$- | -NH-C(=O)- | K208 | J012 | T170 |
| 344 | -$(CH_2)_2$- | -NH-C(=0)- | K208 | J121 | T170 |
| 345 | -$(CH_2)_2$- | -NH-C(=O)- | K208 | J126 | T169 |
| 346 | -$(CH_2)_2$- | -NH-C(=O)- | K208 | J138 | T169 |
| 347 | -$(CH_2)_2$- | -NH-C(=O)- | K208 | J144 | T005 |
| 348 | -$(CH_2)_2$- | -NH-C(=O)- | K208 | J037 | T169 |
| 349 | -$(CH_2)_2$- | -NH-C(=O)- | K208 | J038 | T152 |
| 350 | -$(CH_2)$2- | -NH-C(=O)- | K208 | J039 | T151 |
| 351 | -$(CH_2)_2$- | -NH-C(=O)- | K208 | J043 | T148 |
| 352 | -$(CH_2)_2$- | -NH-C(=O)- | K208 | J045 | T003 |
| 353 | -$(CH_2)_2$- | -NH-C(=O)- | K208 | J045 | T004 |
| 354 | -$(CH_2)_2$- | -NH-C(=O)- | K208 | J045 | T077 |
| 355 | -$(CH_2)_2$- | -NH-C(=O)- | K208 | J045 | T090 |
| 356 | -$(CH_2)_2$- | -NH-C(=O)- | K208 | J045 | T129 |
| 357 | -$(CH_2)_2$- | -NH-C(=O)- | K208 | J045 | T148 |
| 358 | -$(CH_2)_2$- | -NH-C(=O)- | K208 | J045 | T151 |
| 359 | -$(CH_2)_2$- | -NH-C(=O)- | K208 | J045 | T152 |
| 360 | -$(CH_2)_2$- | -NH-C(=O)- | K208 | J045 | T169 |
| 361 | -$(CH_2)_2$- | -NH-C(=O)- | K208 | J045 | T170 |
| 362 | -$(CH_2)_2$- | -NH-C(=O)-O- | K005 | J045 | T004 |
| 363 | -$(CH_2)_2$- | -NH-C(=S) - | K013 | J045 | T060 |
| 364 | -$(CH_2)_2$- | -NH-C(=S)- | K013 | J045 | T063 |
| 365 | -$(CH_2)_2$- | -NH-C(=S)- | K013 | J045 | T077 |
| 366 | -$(CH_2)_2$- | -NH-C (=S) - | K013 | J045 | T078 |
| 367 | -$(CH_2)_2$- | -NH-C(=S)- | K013 | J045 | T079 |
| 368 | -$(CH_2)_2$- | -NH-C(=S)- | K013 | J045 | T080 |
| 369 | -$(CH_2)_2$- | -NH-C (=S)- | K013 | J045 | T081 |
| 370 | -$(CH_2)_2$- | -NH-C(=S)- | K013 | J045 | T082 |
| 371 | -$(CH_2)_2$- | -NH-C(=S)- | K013 | J045 | T083 |
| 372 | -$(CH_2)_2$- | -NH-C(=S)- | K013 | J045 | T084 |
| 373 | -$(CH_2)_2$- | -NH-C(=S)- | K013 | J045 | T085 |
| 374 | -$(CH_2)_2$- | -NH-C (=S) - | K013 | J045 | T086 |
| 375 | -$(CH_2)_2$- | -NH-C (=S) - | K013 | J045 | T088 |
| 376 | -$(CH_2)_2$- | -NH-C (=S) - | K013 | J045 | T090 |
| 377 | -$(CH_2)_2$- | -NH-C (=S) - | K013 | J045 | T091 |
| 378 | -$(CH_2)_2$- | -NH-C (=S) - | K013 | J045 | T092 |
| 379 | -$(CH_2)_2$- | -NH-C(=S)- | K013 | J045 | T093 |

Table continued

| Compound no. | $-A^1-$ | $-A^2-$ | $-G^1-A^3-A^4-G^2$ | $-A^5-R^2$ | $-A^6-R^3$ |
|---|---|---|---|---|---|
| 380 | $-(CH_2)_2-$ | -NH-C (=S) - | K013 | J045 | T094 |
| 381 | $-(CH_2)_2-$ | -NH-C (=S) - | K013 | J045 | T095 |
| 382 | $-(CH_2)_3-$ | -C(=O)-NH- | K102 | J012 | T003 |
| 383 | $-(CH_2)_3-$ | -C (=O) -NH- | K102 | J012 | T055 |
| 384 | $-(CH_2)_3-$ | -C(=O)-NH- | K102 | J012 | T148 |
| 385 | $-(CH_2)_3-$ | -C(=O)-NH- | K102 | J044 | T148 |
| 386 | $-(CH_2)_3-$ | -C(=O)-NH- | K102 | J044 | T170 |
| 387 | $-(CH_2)_3-$ | -C(=O)-NH- | K102 | J045 | T170 |
| 388 | $-(CH_2)_3-$ | -C(=O)-NH- | K102 | J045 | T005 |
| 389 | $-(CH_2)_3-$ | -C(=O)-NH- | K102 | J045 | T006 |
| 390 | $-(CH_2)_3-$ | -C(=O)-NH- | K102 | J045 | T151 |
| 391 | $-(CH_2)_3-$ | -C(=O)-NH- | K102 | J045 | T164 |
| 392 | $-(CH_2)_3-$ | -C(=O)-NH- | K102 | J045 | T169 |
| 393 | $-(CH_2)_3-$ | -C(=O)-NH- | K333 | J012 | T005 |
| 394 | $-(CH_2)_3-$ | -C(=O)-NH- | K333 | J012 | T007 |
| 395 | $-(CH_2)_3-$ | -C(=O)-NH- | K333 | J012 | T152 |
| 396 | $-(CH_2)_3-$ | -C(=O)-NH- | K333 | J012 | T169 |
| 397 | $-(CH_2)_3-$ | -C(=O)-NH- | K333 | J014 | T148 |
| 398 | $-(CH_2)_3-$ | -C(=O)-NH- | K333 | J144 | T148 |
| 399 | $-(CH_2)_3-$ | -C(=O)-NH- | K333 | J039 | T170 |
| 400 | $-(CH_2)_3-$ | -C(=O)-NH- | K333 | J044 | T170 |
| 401 | $-(CH_2)_3-$ | -C(=O)-NH- | K333 | J045 | T004 |
| 402 | $-(CH_2)_3-$ | -C(=O)-NH- | K333 | J045 | T145 |
| 403 | $-(CH_2)_3-$ | -C(=O)-NH- | K346 | J008 | T148 |
| 404 | $-(CH_2)_3-$ | -C(=O)-NH- | K346 | J012 | T005 |
| 405 | $-(CH_2)_3-$ | -C(=O)-NH- | K346 | J012 | T151 |
| 406 | $-(CH_2)_3-$ | -C(=O)-NH- | K346 | J012 | T170 |
| 407 | $-(CH_2)_3-$ | -C(=O)-NH- | K346 | J013 | T148 |
| 408 | $-(CH_2)_3-$ | -C(=O)-NH- | K346 | J045 | T017 |
| 409 | $-(CH_2)_3-$ | -C(=O)-NH- | K346 | J045 | T152 |
| 410 | $-(CH_2)_3-$ | -C(=O)-NH- | K346 | J045 | T170 |
| 411 | $-(CH_2)_3-$ | -NH-C(=O)- | K200 | J045 | T003 |
| 412 | $-(CH_2)_3-$ | -NH-C(=O)- | K200 | J012 | T005 |
| 413 | $-(CH_2)_3-$ | -NH-C(=O)- | K200 | J012 | T006 |
| 414 | $-(CH_2)_3-$ | -NH-C(=O)- | K200 | J045 | T055 |
| 415 | $-(CH_2)_3-$ | -NH-C(=O)- | K200 | J010 | T148 |
| 416 | $-(CH_2)_3-$ | -NH-C(=O)- | K200 | J014 | T148 |
| 417 | $-(CH_2)_3-$ | -NH-C(=O)- | K200 | J012 | T151 |

Table continued

| Compound no. | $-A^1-$ | $-A^2-$ | $-G^1-A^3-A^4-G^2$ | $-A^5-R^2$ | $-A^6-R^3$ |
|---|---|---|---|---|---|
| 418 | $-(CH_2)_3-$ | -NH-C(=O)- | K200 | J012 | T164 |
| 419 | $-(CH_2)_3-$ | -NH-C(=O)- | K200 | J012 | T169 |
| 420 | $-(CH_2)_3-$ | -NH-C(=O)- | K200 | J012 | T170 |
| 421 | $-(CH_2)_3-$ | -NH-C(=O)- | K200 | J045 | T170 |
| 422 | $-(CH_2)_3-$ | -NH-C(=O)- | K204 | J012 | T004 |
| 423 | $-(CH_2)_3-$ | -NH-C(=O)- | K204 | J045 | T005 |
| 424 | $-(CH_2)_3-$ | -NH-C(=O)- | K204 | J045 | T007 |
| 425 | $-(CH_2)_3-$ | -NH-C(=O)- | K204 | J012 | T145 |
| 426 | $-(CH_2)_3-$ | -NH-C(=O)- | K204 | J013 | T148 |
| 427 | $-(CH_2)_3-$ | -NH-C(=O)- | K204 | J045 | T148 |
| 428 | $-(CH_2)_3-$ | -NH-C(=O)- | K204 | J045 | T152 |
| 429 | $-(CH_2)_3-$ | -NH-C(=O)- | K204 | J045 | T169 |
| 430 | $-(CH_2)_3-$ | -NH-C(=O)- | K204 | J037 | T170 |
| 431 | $-(CH_2)_3-$ | -NH-C(=O)- | K204 | J045 | T170 |
| 432 | $-(CH_2)_3-$ | -NH-C(=O)- | K208 | J045 | T005 |
| 433 | $-(CH_2)_3-$ | -NH-C(=O)- | K208 | J012 | T017 |
| 434 | $-(CH_2)_3-$ | -NH-C(=O)- | K208 | J007 | T148 |
| 435 | $-(CH_2)_3-$ | -NH-C(=O)- | K208 | J012 | T148 |
| 436 | $-(CH_2)_3-$ | -NH-C(=O)- | K208 | J045 | T151 |
| 437 | $-(CH_2)_3-$ | -NH-C(=O)- | K208 | J012 | T152 |
| 438 | $-(CH_2)_3-$ | -NH-C(=O)- | K208 | J144 | T170 |
| 439 | $-(CH_2)_3-$ | -NH-C(=O)- | K208 | J044 | T170 |
| 440 | $-CH_2-$ | -C(=O)- | K107 | J018 | T148 |
| 441 | $-CH_2-$ | -C(=O)- | K108 | J022 | T148 |
| 442 | $-CH_2-$ | -C(=O)- | K112 | J012 | T148 |
| 443 | $-CH_2-$ | -C(=O)- | K129 | J014 | T148 |
| 444 | $-CH_2-$ | -C(=O)- | K133 | J008 | T148 |
| 445 | $-CH_2-$ | -C(=O)- | K137 | J009 | T148 |
| 446 | $-CH_2-$ | -C(=O)-NH- | K003 | J065 | T148 |
| 447 | $-CH_2-$ | -C(=O)-NH- | K004 | J070 | T148 |
| 448 | $-CH_2-$ | -C(=O)-NH- | K005 | J075 | T148 |
| 449 | $-CH_2-$ | -C(=O)-NH- | K007 | J081 | T148 |
| 450 | $-CH_2-$ | -C(=O)-NH- | K008 | J085 | T148 |
| 451 | $-CH_2-$ | -C(=O)-NH- | K009 | J043 | T148 |
| 452 | $-CH_2-$ | -C(=O)-NH- | K012 | J045 | T148 |
| 453 | Single bond | Single bond | K001 | J008 | T148 |
| 454 | Single bond | Single bond | K001 | J008 | T170 |
| 455 | Single bond | Single bond | K001 | J012 | T148 |

Table continued

| Compound no. | -$A^1$- | -$A^2$- | -$G^1$-$A^3$-$A^4$-$G^2$ | -$A^5$-$R^2$ | -$A^6$-$R^3$ |
|---|---|---|---|---|---|
| 456 | Single bond | Single bond | K001 | J012 | T170 |
| 457 | Single bond | Single bond | K001 | J138 | T148 |
| 458 | Single bond | Single bond | K001 | J138 | T170 |
| 459 | Single bond | Single bond | K001 | J014 | T148 |
| 460 | Single bond | Single bond | K001 | J014 | T170 |
| 461 | Single bond | Single bond | K001 | J144 | T148 |
| 462 | Single bond | Single bond | K001 | J144 | T170 |
| 463 | Single bond | Single bond | K001 | J019 | T003 |
| 464 | Single bond | Single bond | K001 | J020 | T004 |
| 465 | Single bond | Single bond | K001 | J020 | T148 |
| 466 | Single bond | Single bond | K001 | J022 | T004 |
| 467 | Single bond | Single bond | K001 | J022 | T148 |
| 468 | Single bond | Single bond | K001 | J022 | T170 |
| 469 | Single bond | Single bond | K001 | J026 | T148 |
| 470 | Single bond | Single bond | K001 | J026 | T170 |
| 471 | Single bond | Single bond | K001 | J029 | T005 |
| 472 | Single bond | Single bond | K001 | J029 | T148 |
| 473 | Single bond | Single bond | K001 | J029 | T170 |
| 474 | Single bond | Single bond | K001 | J037 | T007 |
| 475 | Single bond | Single bond | K001 | J044 | T017 |
| 476 | Single bond | Single bond | K001 | J044 | T148 |
| 477 | Single bond | Single bond | K001 | J044 | T170 |
| 478 | Single bond | Single bond | K001 | J045 | T148 |
| 479 | Single bond | Single bond | K001 | J045 | T170 |
| 480 | Single bond | Single bond | K197 | J008 | T148 |
| 481 | Single bond | Single bond | K197 | J008 | T170 |
| 482 | Single bond | Single bond | K197 | J012 | T148 |
| 483 | Single bond | Single bond | K197 | J012 | T170 |
| 484 | Single bond | Single bond | K197 | J0138 | T148 |
| 485 | Single bond | Single bond | K197 | J0138 | T170 |
| 486 | Single bond | Single bond | K197 | J014 | T148 |
| 487 | Single bond | Single bond | K197 | J014 | T170 |
| 488 | Single bond | Single bond | K197 | J144 | T148 |
| 489 | Single bond | Single bond | K197 | J144 | T170 |
| 490 | Single bond | Single bond | K197 | J020 | T148 |
| 491 | Single bond | Single bond | K197 | J020 | T170 |
| 492 | Single bond | Single bond | K197 | J022 | T148 |
| 493 | Single bond | Single bond | K197 | J022 | T170 |

Table continued

| Compound no. | -$A^1$- | -$A^2$- | -$G^1$-$A^3$-$A^4$-$G^2$ | -$A^5$-$R^2$ | -$A^6$-$R^3$ |
|---|---|---|---|---|---|
| 494 | Single bond | Single bond | K197 | J026 | T148 |
| 495 | Single bond | Single bond | K197 | J026 | T170 |
| 496 | Single bond | Single bond | K197 | J029 | T148 |
| 497 | Single bond | Single bond | K197 | J029 | T170 |
| 498 | Single bond | Single bond | K197 | J044 | T148 |
| 499 | Single bond | Single bond | K197 | J044 | T170 |
| 500 | Single bond | Single bond | K197 | J045 | T148 |
| 501 | Single bond | Single bond | K197 | J045 | T170 |
| 502 | $-(CH_2)_2-$ | -C (=O) -NH- | K137 | J012 | T004 |
| 503 | $-(CH_2)_2-$ | -C(=O)-NH- | K137 | J012 | T005 |
| 504 | $-(CH_2)_2-$ | -C(=O)-NH- | K137 | J012 | T148 |
| 505 | $-(CH_2)_2-$ | -C(=O)-NH- | K137 | J012 | T170 |
| 506 | $-(CH_2)_2-$ | -C(=O)-NH- | K147 | J045 | T004 |
| 507 | $-(CH_2)_2-$ | -C(=O)-NH- | K147 | J045 | T005 |
| 508 | $-(CH_2)_2-$ | -C(=O)-NH- | K147 | J045 | T148 |
| 509 | $-(CH_2)_2-$ | -C(=O)-NH- | K147 | J045 | T170 |
| 510 | $-(CH_2)_2-$ | -NH-C(=O)- | K200 | J002 | T004 |
| 511 | $-(CH_2)_2-$ | -NH-C(=O)- | K200 | J002 | T005 |
| 512 | $-(CH_2)_2-$ | -NH-C(=O)- | K200 | J002 | T148 |
| 513 | $-(CH_2)_2-$ | -NH-C(=O)- | K200 | J002 | T170 |
| 514 | $-(CH_2)_2-$ | -NH-C(=O)- | K200 | J005 | T004 |
| 515 | $-(CH_2)_2-$ | -NH-C(=O)- | K200 | J005 | T148 |
| 516 | $-(CH_2)_2-$ | -NH-C(=O)- | K200 | J007 | T005 |
| 517 | $-(CH_2)_2-$ | -NH-C(=O)- | K200 | J007 | T170 |
| 518 | $-(CH_2)_2-$ | -NH-C(=O)- | K200 | J010 | T004 |
| 519 | $-(CH_2)_2-$ | -NH-C(=O)- | K200 | J010 | T005 |
| 520 | $-(CH_2)_2-$ | -NH-C(=O)- | K200 | J010 | T148 |
| 521 | $-(CH_2)_2-$ | -NH-C(=O)- | K200 | J010 | T170 |
| 522 | $-(CH_2)_2-$ | -NH-C(=O)- | K200 | J013 | T004 |
| 523 | $-(CH_2)_2-$ | -NH-C(=O)- | K200 | J013 | T005 |
| 524 | $-(CH_2)_2-$ | -NH-C(=O)- | K200 | J013 | T148 |
| 525 | $-(CH_2)_2-$ | -NH-C(=O)- | K200 | J013 | T170 |
| 526 | $-(CH_2)_2-$ | -NH-C(=O)- | K200 | J014 | T004 |
| 527 | $-(CH_2)_2-$ | -NH-C(=O)- | K200 | J014 | T005 |
| 528 | $-(CH_2)_2-$ | -NH-C(=O)- | K200 | J014 | T148 |
| 529 | $-(CH_2)_2-$ | -NH-C(=O)- | K200 | J014 | T170 |
| 530 | $-(CH_2)_2-$ | -NH-C(=O)- | K200 | J146 | T004 |
| 531 | $-(CH_2)_2-$ | -NH-C(=O)- | K200 | J146 | T005 |

Table continued

| Compound no. | $-A^1-$ | $-A^2-$ | $-G^1-A^3-A^4-G^2$ | $-A^5-R^2$ | $-A^6-R^3$ |
|---|---|---|---|---|---|
| 532 | $-(CH_2)_2-$ | -NH-C(=O)- | K200 | J146 | T148 |
| 533 | $-(CH_2)_2-$ | -NH-C(=O)- | K200 | J146 | T170 |
| 534 | $-(CH_2)_2-$ | -NH-C(=O)- | K200 | J147 | T004 |
| 535 | $-(CH_2)_2-$ | -NH-C(=O)- | K200 | J147 | T005 |
| 536 | $-(CH_2)_2-$ | -NH-C(=O)- | K200 | J147 | T148 |
| 537 | $-(CH_2)_2-$ | -NH-C(=O)- | K200 | J147 | T170 |
| 538 | $-(CH_2)_2-$ | -NH-C(=O)- | K200 | J148 | T004 |
| 539 | $-(CH_2)_2-$ | -NH-C(=O)- | K200 | J148 | T005 |
| 540 | $-(CH_2)_2-$ | -NH-C(=O)- | K200 | J148 | T148 |
| 541 | $-(CH_2)_2-$ | -NH-C(=O)- | K200 | J148 | T170 |
| 542 | $-(CH_2)_2-$ | -NH-C(=O)- | K200 | J149 | T004 |
| 543 | $-(CH_2)_2-$ | -NH-C(=O)- | K200 | J149 | T005 |
| 544 | $-(CH_2)_2-$ | -NH-C(=O)- | K200 | J149 | T148 |
| 545 | $-(CH_2)_2-$ | -NH-C(=O)- | K200 | J149 | T170 |
| 546 | $-(CH_2)_2-$ | -NH-C(=0)- | K200 | J150 | T004 |
| 547 | $-(CH_2)_2-$ | -NH-C(=0)- | K200 | J150 | T005 |
| 548 | $-(CH_2)_2-$ | -NH-C(=O)- | K200 | J150 | T148 |
| 549 | $-(CH_2)_2-$ | -NH-C(=O)- | K200 | J150 | T170 |
| 550 | $-(CH_2)_2-$ | -NH-C(=O)- | K200 | J151 | T004 |
| 551 | $-(CH_2)_2-$ | -NH-C(=O)- | K200 | J151 | T005 |
| 552 | $-(CH_2)_2-$ | -NH-C(=O)- | K200 | J151 | T148 |
| 553 | $-(CH_2)_2-$ | -NH-C(=O)- | K200 | J151 | T170 |
| 554 | $-(CH_2)_2-$ | -NH-C(=O)- | K200 | J152 | T004 |
| 555 | $-(CH_2)_2-$ | -NH-C(=O)- | K200 | J152 | T005 |
| 556 | $-(CH_2)_2-$ | -NH-C(=O)- | K200 | J152 | T148 |
| 557 | $-(CH_2)_2-$ | -NH-C(=O)- | K200 | J152 | T170 |
| 558 | $-(CH_2)_2-$ | -NH-C(=O)- | K200 | J153 | T004 |
| 559 | $-(CH_2)_2-$ | -NH-C(=O)- | K200 | J153 | T005 |
| 560 | $-(CH_2)_2-$ | -NH-C(=O)- | K200 | J153 | T148 |
| 561 | $-(CH_2)_2-$ | -NH-C(=O)- | K200 | J153 | T170 |
| 562 | $-(CH_2)_2-$ | -NH-C(=O)- | K200 | J154 | T004 |
| 563 | $-(CH_2)_2-$ | -NH-C(=O)- | K200 | J154 | T005 |
| 564 | $-(CH_2)_2-$ | -NH-C(=O)- | K200 | J154 | T148 |
| 565 | $-(CH_2)_2-$ | -NH-C(=O)- | K200 | J154 | T170 |
| 566 | $-(CH_2)_2-$ | -NH-C(=O)- | K200 | J155 | T004 |
| 567 | $-(CH_2)_2-$ | -NH-C(=O)- | K200 | J155 | T005 |
| 568 | $-(CH_2)_2-$ | -NH-C(=O)- | K200 | J155 | T148 |
| 569 | $-(CH_2)_2-$ | -NH-C(=O)- | K200 | J155 | T170 |

Table continued

| Compound no. | $-A^1-$ | $-A^2-$ | $-G^1-A^3-A^4-G^2$ | $-A^5-R^2$ | $-A^6-R^3$ |
|---|---|---|---|---|---|
| 570 | $-(CH_2)_2-$ | -NH-C(=O)- | K200 | J156 | T004 |
| 571 | $-(CH_2)_2-$ | -NH-C(=O)- | K200 | J156 | T005 |
| 572 | $-(CH_2)_2-$ | -NH-C(=O)- | K200 | J156 | T148 |
| 573 | $-(CH_2)_2-$ | -NH-C(=O)- | K200 | J156 | T170 |
| 574 | $-(CH_2)_2-$ | -NH-C(=O)- | K200 | J157 | T148 |
| 575 | $-(CH_2)_2-$ | -NH-C(=O)- | K200 | J158 | T170 |
| 576 | $-(CH_2)_2-$ | -NH-C(=O)- | K200 | J037 | T003 |
| 577 | $-(CH_2)_2-$ | -NH-C(=O)- | K200 | J037 | T004 |
| 578 | $-(CH_2)_2-$ | -NH-C(=O)- | K200 | J037 | T148 |
| 579 | $-(CH_2)_2-$ | -NH-C(=O)- | K200 | J037 | T170 |
| 580 | $-(CH_2)_2-$ | -NH-C(=O)- | K200 | J039 | T004 |
| 581 | $-(CH_2)_2-$ | -NH-C(=O)- | K200 | J039 | T005 |
| 582 | $-(CH_2)_2-$ | -NH-C(=O)- | K200 | J039 | T148 |
| 583 | $-(CH_2)_2-$ | -NH-C(=O)- | K200 | J039 | T170 |
| 584 | $-(CH_2)_2-$ | -NH-C(=O)- | K200 | J044 | T004 |
| 585 | $-(CH_2)_2-$ | -NH-C(=O)- | K200 | J044 | T005 |
| 586 | $-(CH_2)_2-$ | -NH-C(=O)- | K200 | J044 | T148 |
| 587 | $-(CH_2)_2-$ | -NH-C(=O)- | K200 | J044 | T170 |
| 588 | $-(CH_2)_2-$ | -NH-C(=O)- | K200 | J079 | T004 |
| 589 | $-(CH_2)_2-$ | -NH-C(=O)- | K200 | J079 | T005 |
| 590 | $-(CH_2)_2-$ | -NH-C(=O)- | K200 | J079 | T148 |
| 591 | $-(CH_2)_2-$ | -NH-C(=O)- | K200 | J079 | T170 |
| 592 | $-(CH_2)_2-$ | -NH-C(=O)- | K399 | J012 | T004 |
| 593 | $-(CH_2)_2-$ | -NH-C(=O)- | K399 | J012 | T005 |
| 594 | $-(CH_2)_2-$ | -NH-C(=O)- | K399 | J012 | T148 |
| 595 | $-(CH_2)_2-$ | -NH-C(=O)- | K399 | J045 | T004 |
| 596 | $-(CH_2)_2-$ | -NH-C(=O)- | K399 | J045 | T005 |
| 597 | $-(CH_2)_2-$ | -NH-C(=O)- | K399 | J045 | T170 |
| 598 | $-(CH_2)_2-$ | -NH-C(=O)- | K400 | J012 | T004 |
| 599 | $-(CH_2)_2-$ | -NH-C(=O)- | K400 | J012 | T005 |
| 600 | $-(CH_2)_2-$ | -NH-C(=O)- | K400 | J012 | T148 |
| 601 | $-(CH_2)_2-$ | -NH-C(=O)- | K400 | J151 | T148 |
| 602 | $-(CH_2)_2-$ | -NH-C(=O)- | K400 | J158 | T148 |
| 603 | $-(CH_2)_2-$ | -NH-C(=O)- | K400 | J045 | T004 |
| 604 | $-(CH_2)_2-$ | -NH-C(=O)- | K400 | J045 | T005 |
| 605 | $-(CH_2)_2-$ | -NH-C(=O)- | K400 | J045 | T170 |
| 606 | $-(CH_2)_2-$ | -NH-C(=O)- | K401 | J007 | T148 |
| 607 | $-(CH_2)_2-$ | -NH-C(=O)- | K401 | J012 | T148 |

Table continued

| Compound no. | -$A^1$- | -$A^2$- | -$G^1$-$A^3$-$A^4$-$G^2$ | -$A^5$-$R^2$ | -$A^6$-$R^3$ |
|---|---|---|---|---|---|
| 608 | $-(CH_2)_2-$ | -NH-C(=O)- | K401 | J180 | T170 |
| 609 | $-(CH_2)_2-$ | -NH-C(=O)- | K401 | J045 | T170 |
| 610 | $-(CH_2)_2-$ | -NH-C(=O)- | K402 | J007 | T148 |
| 611 | $-(CH_2)_2-$ | -NH-C(=O)- | K402 | J012 | T148 |
| 612 | $-(CH_2)_2-$ | -NH-C(=O)- | K402 | J157 | T170 |
| 613 | $-(CH_2)_2-$ | -NH-C(=O)- | K402 | J045 | T170 |
| 614 | $-(CH_2)_2-$ | -NH-C(=O)- | K403 | J007 | T148 |
| 615 | $-(CH_2)_2-$ | -NH-C(=O)- | K403 | J012 | T004 |
| 616 | $-(CH_2)_2-$ | -NH-C(=O)- | K403 | J012 | T005 |
| 617 | $-(CH_2)_2-$ | -NH-C(=O)- | K403 | J012 | T148 |
| 618 | $-(CH_2)_2-$ | -NH-C(=O)- | K403 | J157 | T170 |
| 619 | $-(CH_2)_2-$ | -NH-C(=O)- | K403 | J044 | T170 |
| 620 | $-(CH_2)_2-$ | -NH-C(=O)- | K403 | J045 | T004 |
| 621 | $-(CH_2)_2-$ | -NH-C(=O)- | K403 | J045 | T005 |
| 622 | $-(CH_2)_2-$ | -NH-C(=O)- | K403 | J045 | T170 |
| 623 | $-(CH_2)_2-$ | -NH-C(=O)- | K404 | J007 | T148 |
| 624 | $-(CH_2)_2-$ | -NH-C(=O)- | K404 | J012 | T004 |
| 625 | $-(CH_2)_2-$ | -NH-C(=O)- | K404 | J012 | T005 |
| 626 | $-(CH_2)_2-$ | -NH-C(=O)- | K404 | J012 | T148 |
| 627 | $-(CH_2)_2-$ | -NH-C(=O)- | K404 | J157 | T170 |
| 628 | $-(CH_2)_2-$ | -NH-C(=O)- | K404 | J044 | T170 |
| 629 | $-(CH_2)_2-$ | -NH-C(=O)- | K404 | J045 | T004 |
| 630 | $-(CH_2)_2-$ | -NH-C(=O)- | K404 | J045 | T005 |
| 631 | $-(CH_2)_2-$ | -NH-C(=O)- | K404 | J045 | T170 |
| 632 | $-(CH_2)_2-$ | -NH-C(=O)- | K405 | J007 | T148 |
| 633 | $-(CH_2)_2-$ | -NH-C(=O)- | K405 | J012 | T148 |
| 634 | $-(CH_2)_2-$ | -NH-C(=O)- | K405 | J157 | T170 |
| 635 | $-(CH_2)_2-$ | -NH-C(=O)- | K405 | J045 | T170 |
| 636 | $-(CH_2)_2-$ | -NH-C(=O)- | K406 | J007 | T148 |
| 637 | $-(CH_2)_2-$ | -NH-C(=O)- | K406 | J012 | T148 |
| 638 | $-(CH_2)_2-$ | -NH-C(=O)- | K406 | J157 | T170 |
| 639 | $-(CH_2)_2-$ | -NH-C(=O)- | K406 | J045 | T170 |
| 640 | $-(CH_2)_2-$ | -NH-C(=O)- | K407 | J007 | T148 |
| 641 | $-(CH_2)_2-$ | -NH-C(=O)- | K407 | J012 | T148 |
| 642 | $-(CH_2)_2-$ | -NH-C(=O)- | K407 | J157 | T170 |
| 643 | $-(CH_2)_2-$ | -NH-C(=O)- | K407 | J45 | T170 |
| 644 | $-(CH_2)_2-$ | -NH-C(=O)- | K408 | J007 | T148 |
| 645 | $-(CH_2)_2-$ | -NH-C(=O)- | K408 | J012 | T148 |

Table continued

| Compound no. | -$A^1$- | -$A^2$- | -$G^1$-$A^3$-$A^4$-$G^2$ | -$A^5$-$R^2$ | -$A^6$-$R^3$ |
|---|---|---|---|---|---|
| 646 | $-(CH_2)_2-$ | -NH-C(=O)- | K408 | J157 | T170 |
| 647 | $-(CH_2)_2-$ | -NH-C(=O)- | K408 | J045 | T170 |
| 648 | $-(CH_2)_2-$ | -NH-C(=O)- | K409 | J007 | T148 |
| 649 | $-(CH_2)_2-$ | -NH-C(=O)- | K409 | J012 | T148 |
| 650 | $-(CH_2)_2-$ | -NH-C(=O)- | K409 | J157 | T170 |
| 651 | $-(CH_2)_2-$ | -NH-C(=O)- | K409 | J045 | T170 |
| 652 | $-(CH_2)_2-$ | -NH-C(=O)- | K410 | J007 | T148 |
| 653 | $-(CH_2)_2-$ | -NH-C(=O)- | K410 | J012 | T148 |
| 654 | $-(CH_2)_2-$ | -NH-C(=O)- | K410 | J157 | T170 |
| 655 | $-(CH_2)_2-$ | -NH-C(=O)- | K410 | J045 | T170 |
| 656 | $-(CH_2)_2-$ | -NH-C(=O)- | K411 | J007 | T148 |
| 657 | $-(CH_2)_2-$ | -NH-C(=O)- | K411 | J012 | T148 |
| 658 | $-(CH_2)_2-$ | -NH-C(=O)- | K411 | J157 | T170 |
| 659 | $-(CH_2)_2-$ | -NH-C(=O)- | K411 | J045 | T170 |
| 660 | $-(CH_2)_2-$ | -NH-C(=O)- | K415 | J012 | T004 |
| 661 | $-(CH_2)_2-$ | -NH-C(=O)- | K415 | J012 | T005 |
| 662 | $-(CH_2)_2-$ | -NH-C(=O)- | K415 | J012 | T148 |
| 663 | $-(CH_2)_2-$ | -NH-C(=O)- | K415 | J045 | T004 |
| 664 | $-(CH_2)_2-$ | -NH-C(=O)- | K415 | J045 | T005 |
| 665 | $-(CH_2)_2-$ | -NH-C(=O)- | K415 | J045 | T170 |
| 666 | $-(CH_2)_2-$ | -NH-C(=O)- | K420 | J012 | T004 |
| 667 | $-(CH_2)_2-$ | -NH-C(=O)- | K420 | J012 | T005 |
| 668 | $-(CH_2)_2-$ | -NH-C(=O)- | K420 | J012 | T148 |
| 669 | $-(CH_2)_2-$ | -NH-C(=O)- | K420 | J045 | T004 |
| 670 | $-(CH_2)_2-$ | -NH-C(=O)- | K420 | J045 | T005 |
| 671 | $-(CH_2)_2-$ | -NH-C(=O)- | K420 | J045 | T170 |
| 672 | $-(CH_2)_2-$ | -NH-C(=O)- | K425 | J012 | T004 |
| 673 | $-(CH_2)_2-$ | -NH-C(=O)- | K425 | J012 | T005 |
| 674 | $-(CH_2)_2-$ | -NH-C(=O)- | K425 | J012 | T148 |
| 675 | $-(CH_2)_2-$ | -NH-C(=O)- | K425 | J045 | T004 |
| 676 | $-(CH_2)_2-$ | -NH-C(=O)- | K425 | J045 | T005 |
| 677 | $-(CH_2)_2-$ | -NH-C(=O)- | K425 | J045 | T170 |
| 678 | $-(CH_2)_3-$ | -C(=O)-NH- | K324 | J012 | T148 |
| 679 | $-(CH_2)_3-$ | -C(=O)-NH- | K324 | J045 | T170 |
| 680 | $-(CH_2)_3-$ | -C(=O)-NH- | K327 | J012 | T148 |
| 681 | $-(CH_2)_3-$ | -C(=O)-NH- | K327 | J045 | T170 |
| 682 | $-(CH_2)_3-$ | -C(=O)-NH- | K331 | J012 | T148 |
| 683 | $-(CH_2)_3-$ | -C(=O)-NH- | K331 | J045 | T170 |

Table continued

| Compound no. | $-A^1-$ | $-A^2-$ | $-G^1-A^3-A^4-G^2$ | $-A^5-R^2$ | $-A^6-R^3$ |
|---|---|---|---|---|---|
| 684 | $-(CH_2)_3-$ | -C(=O)-NH- | K333 | J007 | T005 |
| 685 | $-(CH_2)_3-$ | -C(=O)-NH- | K333 | J007 | T148 |
| 686 | $-(CH_2)_3-$ | -C(=O)-NH- | K333 | J012 | T004 |
| 687 | $-(CH_2)_3-$ | -C(=O)-NH- | K333 | J012 | T148 |
| 688 | $-(CH_2)_3-$ | -C(=O)-NH- | K333 | J045 | T005 |
| 689 | $-(CH_2)_3-$ | -C(=O)-NH- | K333 | J045 | T170 |
| 690 | $-(CH_2)_3-$ | -C(=O)-NH- | K334 | J007 | T148 |
| 691 | $-(CH_2)_3-$ | -C(=O)=NH- | K334 | J012 | T148 |
| 692 | $-(CH_2)_3-$ | -C(=O)-NH- | K334 | J045 | T170 |
| 693 | $-(CH_2)_3-$ | -C(=O)-NH- | K336 | J012 | T005 |
| 694 | $-(CH_2)_3-$ | -C(=O)-NH- | K336 | J012 | T148 |
| 695 | $-(CH_2)_3-$ | -C(=O)-NH- | K336 | J045 | T005 |
| 696 | $-(CH_2)_3-$ | -C(=O)-NH- | K336 | J045 | T170 |
| 697 | $-(CH_2)_3-$ | -C(=O)-NH- | K430 | J012 | T005 |
| 698 | $-(CH_2)_3-$ | -C(=O)-NH- | K430 | J012 | T148 |
| 699 | $-(CH_2)_3-$ | -C(=O)-NH- | K430 | J045 | T005 |
| 700 | $-(CH_2)_3-$ | -C(=O)-NH- | K430 | J045 | T170 |

Preferred combinations of $A^1$, $A^2$, $G^1$, $A^3$, $A^9$ and $G^2$ in the formula (I) were explained above. As another method of arrangement, they can be summarized also as the following combinations 1) through 41). Not only do these combinations indicate preferred relationships among $A^1$, $A^2$, $G^1$, $A^3$, $A^4$ and $G^2$, but also the partial structures per se comprised of these as a whole are preferred substituents'in the pyrrolopyrimidine-thione derivatives of the present invention.

1) In the formula (I), when $A^1$ is $-(CH_2)_2-$, $A^1-A^2-G^1$ links in the form of $A^1-NH-C(=O)-G^1$, and $G^1$ is a phenylene group, the phenylene group as $G^1$ is preferably substituted with one or more substituents selected from those exemplified for the preferred substituents of the substituted aromatic hydrocarbon group having 6 to 14 carbon atoms of $G^1$.

2) In the formula (I), when $A^1$ is $-(CH_2)_2-$, $A^1-A^2-G^1$ links in the form of $A^1-NH-C(=O)-G^1$, $G^1$ is a phenylene group, and the phenylene group as $G^1$ is not substituted, it is preferable that $A^3-A^4-G^2$ as a whole is a group other than hydrogen atom.

3) In the formula (I), when $A^1$ is $-(CH_2)_2-$, and $A^1-A^{2-}G^1$ links in the form of $A^1-NH-C(=O)-G^1$, $G^1$ is preferably a divalent group derived from a monocyclic or bicyclic $C_3-C_9$ aromatic heterocyclic compound having 1 to 3, preferably 1 or 2, atoms selected from the group consisting of an oxygen atom, a nitrogen atom and a sulfur atom, in the ring.

4) In the formula (I), when $A^1$ is $-(CH_2)_2-$, and $A^1-A^{2-}G^1$ links in the form of $A^1-NH-C(=O)-G^1$, $G^1$ is preferably a divalent group derived from a monocyclic or bicyclic $C_2-C_9$ aromatic heterocyclic compound having 1 to 3, preferably 1 or 2 atoms selected from the group consisting of an oxygen atom, a nitrogen atom and a sulfur atom, in the ring. However, the divalent group derived from the aromatic heterocyclic compound as $G^1$ is more preferably substituted with one or more substituents selected from the group consisting of substituents defined as preferred examples for the heterocyclic compound having 1 to 4 atoms selected from the group consisting of an oxygen atom, a nitrogen atom and a sulfur atom, in the ring.

5) In the formula (I), when $A^1$ is $-(CH_2)_2-$, and $A^1-A^{2-}G^1$ links in the form of $A^1-NH-C(=O)-G^1$, $G^1$ is preferably a divalent group derived from a monocyclic or bicyclic $C_2-C_9$ aromatic heterocyclic compound having 1 to 3, preferably 1 or 2 atoms selected from the group consisting of an oxygen atom, a nitrogen atom and a sulfur atom, in the ring. However, when the divalent group derived from the aromatic heterocyclic compound as $G^1$ is not substituted, it is more preferable that $A^3-A^4-G^2$ as a whole is a group other than hydrogen atom.

6) In the formula (I), when $A^1$ is $-(CH_2)_2-$, and $A^1-A^{2-}G^1$ links in the form of $A^1-NH-C(=O)-G^1$, and $G^1$ represents a single bond, it is more preferable that $A^3-A^4-G^2$ as a whole is a acyclic aliphatic hydrocarbon group having 1 to 6

carbon atoms or a cycloalkylalkyl group consisting of a acyclic aliphatic hydrocarbon group having 1 to 6 carbon atoms and an alicyclic hydrocarbon group having 3 to 8 carbon atoms.

7) In the formula (I), when $A^1$ is $-(CH_2)_2-$, $A^1$-$A^2$-$G^1$ links in the form of $A^1$-NH-C(=O)-$G^1$, and $G^1$ represents a single bond, $A^3$-$A^4$-$G^2$ is preferably a acyclic aliphatic hydrocarbon group having 1 to 6 carbon atoms or a cycloalkylalkyl group consisting of a acyclic aliphatic hydrocarbon group having 1 to 6 carbon atoms and an alicyclic hydrocarbon group having 3 to 8 carbon atoms as a whole, but a acyclic aliphatic hydrocarbon group having 1 to 6 carbon atoms as $A^3$-$A^4$-$G^2$ is more preferably substituted with one or more substituents selected from those exemplified for the preferred substituents of the substituted acyclic aliphatic hydrocarbon group having 1 to 10 carbon atoms of $A^3$. Also, in a cycloalkylalkyl group consisting of a acyclic aliphatic hydrocarbon group having 1 to 6 carbon atoms and an alicyclic hydrocarbon group having 3 to 8 carbon atoms as $A^3$-$A^4$-$G^2$, a acyclic aliphatic hydrocarbon group portion having 1 to 6 carbon atoms is further preferably substituted with one or more substituents selected from those exemplified for the preferred substituents of the substituted acyclic aliphatic hydrocarbon group having 1 to 10 carbon atoms of $A^3$, or an alicyclic hydrocarbon group portion having 3 to 8 carbon atoms is further preferably substituted with one or more substituents selected from those exemplified for the preferred substituents of the substituted alicyclic hydrocarbon group having 3 to 10 carbon atoms of $G^2$ (including a case where both are substituted).

8) In the formula (I), when $A^1$ is $-(CH_2)_2-$, $A^1$-$A^2$-$G^1$ links in the form of $A^1$-NH-C(=O)-$G^1$, and $G^1$ represents a single bond, $A^3$-$A^4$-$G^2$ is preferably an aralkyl group consisting of a acyclic aliphatic hydrocarbon group having 1 to 6 carbon atoms and an aromatic hydrocarbon group having 6 to 10 carbon atoms as a whole.

9) In the formula (I), when $A^1$ is $-(CH_2)_2-$, $A^1$-$A^2$-$G^1$ links in the form of $A^1$-NH-C(=O)-$G^{1}$' and $G^1$ represents a single bond, $A^3$-$A^4$-$G^2$ is preferably an aralkyl group consisting of a acyclic aliphatic hydrocarbon group having 1 to 6 carbon atoms and an aromatic hydrocarbon group having 6 to 10 carbon atoms as a whole, but in an aralkyl group as $A^3$-$A^4$-$G^2$, a acyclic aliphatic hydrocarbon group portion having 1 to 6 carbon atoms is further preferably substituted with one or more substituents selected from those exemplified for the preferred substituents of the substituted acyclic aliphatic hydrocarbon group having 1 to 10 carbon atoms of $A^3$, or an aromatic hydrocarbon group portion having 6 to 10 carbon atoms is further preferably substituted with one or more substituents selected from those exemplified for the preferred substituents of the substituted aromatic hydrocarbon group having 6 to 14 carbon atoms of $G^2$ (including a case where both are substituted).

10) In the formula (I), when $A^1$ is $-(CH_2)_2-$, $A^1$-$A^2$-$G^1$ links in the form of $A^1$-NH-C(=O)-$G^{1,}$ and $G^1$ represents a single bond, $A^3$-$A^4$-$G^2$ is preferably a heterocyclic substituted alkyl group consisting of a acyclic aliphatic hydrocarbon group having 1 to 6 carbon atoms and a heterocyclic group having 1 to 4 atoms selected from the group consisting of an oxygen atom, a nitrogen atom and a sulfur atom, in the ring as a whole.

11) In the formula (I), when $A^1$ is $-(CH_2)_2-$, $A^1$-$A^2$-$G^1$ links in the form of $A^1$-NH-C(=O)-$G^{1}$' and $G^1$ represents a single bond, $A^3$-$A^4$-$G^2$ is preferably a heterocyclic substituted alkyl group consisting of a acyclic aliphatic hydrocarbon group having 1 to 6 carbon atoms and a heterocyclic group having 1 to 4 atoms selected from the group consisting of an oxygen atom, a nitrogen atom and a sulfur atom, in the ring as a whole. However, in the heterocyclic substituted alkyl group as $A^3$-$A^4$-$G^2$, a acyclic aliphatic hydrocarbon group portion having 1 to 6 carbon atoms is further preferably substituted with one or more substituents selected from those exemplified for the preferred substituents of the substituted acyclic aliphatic hydrocarbon group having 1 to 10 carbon atoms of $A^3$, or a heterocyclic portion is further preferably substituted with one or more substituents selected from those exemplified for the preferred substituents of the heterocyclic group having 1 to 4 atoms selected from the group consisting of an oxygen atom, a nitrogen atom and a sulfur atom, in the ring, of $G^2$ (including a case where both are substituted).

12) In the formula (I), when $A^1$ is $-(CH_2)_2-$, $A^1$-$A^2$-$G^1$ links in the form of $A^1$-NH-C(=O)-$G^{1'}$ and $G^1$ is a phenylene' group, the phenylene group as $G^1$ is preferably substituted with one or more substituents selected from those exemplified for the preferred substituents of the substituted aromatic hydrocarbon group having 6 to 14 carbon atoms of $G^1$.

13) In the formula (I), when $A^1$ is $-(CH_2)_2-$, $A^1$-$A^2$-$G^1$ links in the form of $A^1$-NH-C(=O)-NH-$G^1$, $G^1$ is a phenylene group, and the phenylene group as $G^1$ is not substituted, it is preferable that $A^3$-$A^4$-$G^2$ as a whole is a group other than hydrogen atom.

14) In the formula (I), when $^1$ is $-(CH)_2-$, and $A^1$-$A^{2-}G^1$ links in the form of $A^1$-NH-C(=O)-NH-$G^1$, $G^1$ is preferably a divalent group derived from a monocyclic or bicyclic $C_{3-}C_9$ aromatic heterocyclic compound having 1 to 3, preferably 1 or 2, atoms selected from the group consisting of an oxygen atom, a nitrogen atom and a sulfur atom, in the ring.

15) In the formula (I), when $A^1$ is $-(CH_2)_2-$, and $A^{1-}A^2$-$G^1$ links in the form of $A^1$-NH-C(=O)-NH-$G^1$, $G^1$ is preferably a divalent group derived from a monocyclic or bicyclic $C_2$-$C_9$ aromatic heterocyclic compound having 1 to 3, preferably 1 or 2 atoms selected from the group consisting of an oxygen atom, a nitrogen atom and a sulfur atom, in the ring. However, the divalent group derived from the aromatic heterocyclic compound as $G^1$ is more preferably substituted with one or more substituents selected from the group consisting of substituents defined as preferred examples for the heterocyclic compound having 1 to 4 atoms selected from the group consisting of an oxygen atom, a nitrogen

atom and a sulfur atom, in the ring.

16) In the formula (I), when $A^1$ is $-(CH_2)_2-$, and $A^{1-}A^2-G^1$ links in the form of $A^1$-NH-C(=O)-NH-$G^1$, $G^1$ is preferably a divalent group derived from a monocyclic or bicyclic $C_2$-$C_9$ aromatic heterocyclic compound having 1 to 3, preferably 1 or 2 atoms selected from the group consisting of an oxygen atom, a nitrogen atom and a sulfur atom, in the ring. However, when the divalent group derived from the aromatic heterocyclic compound as $G^1$ is not substituted, it is more preferable that $A^3$-$A^4$-$G^2$ as a'whole is a group other than hydrogen atom.

17) In the formula (I), when $A^1$ is $-(CH_2)_2-$, $A^1$-$A^2$-$G^1$ links in the form of $A^1$-NH-C(=O)-NH-$G^1$, and $G^1$ represents a single bond, it is more preferable that $A^3$-$A^4$-$G^2$ as a whole is'a cycloalkylalkyl group consisting of a acyclic aliphatic hydrocarbon group having 1 to 6 carbon atoms and an alicyclic hydrocarbon group having 3 to 8 carbon atoms.

18) In the formula (I), when, $A^1$ is $-(CH_2)_2-$, $A^1$-$A^2$-$G^1$ links in the form of $A^1$-NH-C(=O)-NH-$G^1$, and $G^1$ represents a single bond, it is more preferable that $A^3$-$A^4$-$G^2$ as a whole is a cycloalkylalkyl group consisting of a acyclic aliphatic hydrocarbon group having 1 to 6 carbon atoms and an alicyclic hydrocarbon group having 3 to 8 carbon atoms. However, in the cycloalkylalkyl group as $A^3$-$A^4$-$G^2$, a acyclic aliphatic hydrocarbon group portion having 1 to 6 carbon atoms is more preferably substituted with one or more substituents selected from those exemplified for the preferred substituents of the substituted acyclic aliphatic hydrocarbon group having 1 to 10 carbon atoms of $A^3$, or an alicyclic hydrocarbon group portion having 3 to 8 carbon atoms is further preferably substituted with one or more substituents selected from those exemplified for the preferred substituents of the substituted alicyclic hydrocarbon group having 3 to 10 carbon atoms of $G^2$ (including a case where both are substituted).

19) In the formula (I), when $A^1$ is $-(CH_2)_2-$, $A^1$-$A^2$-$G^1$ links in the form of $A^1$-NH-C(=O)-NH-$G^1$, and $G^1$ represents a single bond, it is more preferable that $A^3$-$A^4$-$G^2$ as a whole is an aralkyl group consisting of a acyclic aliphatic hydrocarbon group having 1 to 6 carbon atoms and an aromatic hydrocarbon group having 6 to 10 carbon atoms.

20) In the formula (I), when $A^1$ is $-(CH_2)_2-$, $A^1$-$A^2$-$G^1$ links in the form of $A^1$-NH-C(=O)-NH-$G^1$, and $G^1$ represents a single bond, it is more preferable that $A^3$-$A^4$-$G^2$ as a whole is an aralkyl group consisting of a acyclic aliphatic hydrocarbon group having 1 to 6 carbon atoms and an aromatic hydrocarbon group having 6 to 10 carbon atoms. However, in the arakyl group as $A^3$-$A^4$-$G^2$, a acyclic aliphatic hydrocarbon group portion having 1 to 6 carbon atoms is more preferably substituted with one or more substituents selected from those exemplified for the preferred substituents of the substituted acyclic aliphatic hydrocarbon group having 1 to 10 carbon atoms of $A^3$, or an aromatic hydrocarbon group portion having 6 to 10 carbon atoms is further preferably substituted with one or more substituents selected from those exemplified for the preferred substituents of the substituted aromatic hydrocarbon group having 6 to 14 carbon atoms of $G^2$ (including a case where both are substituted).

21) In the formula (I), when $A^1$ is $-(CH_2)_2-$, $A^1$-$A^2$-$G^1$ links in the form of $A^1$-NH-C(=O)-NH-$G^1$, and $G^1$ represents a single bond, it is preferable that $A^3$-$A^4$-$G^2$ as a whole is a heterocyclic substituted alkyl group consisting of a acyclic aliphatic hydrocarbon group having 1 to 6 carbon atoms and a heterocyclic group having 1 to 4 atoms selected from the group consisting of an oxygen atom, a nitrogen atom and a sulfur atom, in the ring.

22) In the formula (I), when $A^1$ is $-(CH_2)_2-$, $A^1$-$A^2$-$G^1$ links in the form of $A^1$-NH-C(=O)-NH-$G^1$, and $G^1$ represents a single bond, it is preferable that $A^3$-$A^4$-$G^2$ as a whole is a heterocyclic substituted alkyl group consisting of a acyclic aliphatic hydrocarbon group having 1 to 6 carbon atoms and a heterocyclic group having 1 to 4 atoms selected from the group consisting of an oxygen atom, a nitrogen atom and a sulfur atom, in the ring. However, in the heterocyclic substituted alkyl group as $A^3$-$A^4$-$G^2$, a acyclic aliphatic hydrocarbon group portion having 1 to 6 carbon atoms is further preferably substituted with one or more substituents selected from those exemplified for the preferred substituents of the substituted acyclic aliphatic hydrocarbon group having 1 to 10 carbon atoms of $A^3$, or a heterocyclic group portion is further preferably substituted with one or more substituents selected from those exemplified for the preferred substituents of the heterocyclic group having 1 to 4 atoms selected from the group consisting of an oxygen atom, a nitrogen atom and a sulfur atom, in the ring, of $G^2$ (including a case where both are substituted).

23) In the formula (I), when $A^1$ is $-(CH_2)_2-$, $A^1$-$A^2$-$G^1$ links in the form of $A^1$-NH-$G^1$' and $G^1$ is a phenylene group, the phenylene group as $G^1$ is preferably substituted with one or more substituents selected from those exemplified for the preferred substituents of the substituted aromatic hydrocarbon group having 6 to 14 carbon atoms of $G^1$.

24) In the formula (I), when $A^1$ is $-(CH_2)_2-$, $A^1$-$A^2$-$G^1$ links in the form of $A^1$-NH-$G^1$' $G^1$ is a phenylene group, and the phenylene group as $G^1$ is not substituted, it is preferable that $A^3$-$A^4$-$G^2$ as a whole is a group other than hydrogen atom.

25) In the formula (I), when $A^1$ is $-(CH_2)_2-$, $A^1$-$A^2$-$G^1$ links in the form of $A^1$-NH-$G^1$, and $G^1$ is a divalent group derived from a monocyclic or bicyclic $C_2$-$C_9$ aromatic heterocyclic compound having 1 to 3 atoms selected from the group consisting of an oxygen atom, a nitrogen atom and a sulfur atom, in the ring, the aromatic heterocyclic group is preferably substituted with one or more substituents selected from the group consisting of substituents defined as preferred examples for the heterocyclic group having 1 to 4 atoms selected from the group consisting of an oxygen atom, a nitrogen atom and a sulfur atom, in the ring.

26) In the formula (I), when $A^1$ is $-(CH_2)_2-$, $A^1$-$A^2$-$G^1$ links in the form of $A^1$-NH-$G^1$, $G^1$ is a divalent group derived from a monocyclic or bicyclic $C_2$-$C_9$ aromatic heterocyclic compound having 1 to 3 atoms selected from the group

consisting of an oxygen atom, a nitrogen atom and a sulfur atom, in the ring, and the aromatic heterocyclic compound is not substituted, it is more preferable that $A^3$-$A^4$-$G^2$ as a whole is a group other than a hydrogen atom.

27) In the formula (I), when $A^1$ is $-(CH_2)_2-$, $A^1$-$A^2$-$G^1$ links in the form of $A^1$-C(=O)-$G^1$, and $G^1$ is preferably a divalent group derived from a monocyclic $C_2$-$C_9$ heterocyclic compound having 1 or 2 atoms selected from the group consisting of an oxygen atom, a nitrogen atom and a sulfur atom, in the ring, such as pyrrolidine, piperidine, morpholine, thiomorpholine, homopiperidine, homopiperazine, 1,2,3,6-tetrahydropyridine or piperazine, and $G^1$ is bonded with $A^1$-C(=O)- through a nitrogen atom.

28) In the formula (I), when $A^1$ is $-(CH_2)_2-$ and $A^1$-$A^2$-$G^1$ links in the form of $A^1$-C(=O)-$G^1$, $G^1$ is a divalent group of a monocyclic $C_2$-$C_9$ heterocyclic compound having 1 or 2 atoms selected from an oxygen atom, a nitrogen atom and a sulfur atom, in the ring, such as pyrrolidine, piperidine, morpholine, thiomorpholine, homopiperidine, homopiperazine, 1,2,3,6-tetrahydropyridine or piperazine, and $G^1$ is preferably bonded with $A^1$-C(=O)- through a nitrogen atom. However, the divalent group derived from the monocyclic $C_2$-$C_9$ heterocyclic compound having 1 or 2 atoms selected from an oxygen atom, a nitrogen atom and a sulfur atom, in the ring as $G^1$ is more preferably substituted with one or more substituents selected from the group consisting of substituents defined as proffered examples for the heterocyclic group having 1 to 4 atoms selected from the group consisting of an oxygen atom, a nitrogen atom and a sulfur atom on the substituted ring of $G^1$.

29) In the formula (I), when $A^1$ is $-(CH_2)_2-$, and $A^1$-$A^2$-$G^1$ links in the form of $A^1$-C(=O)-$G^1$, $G^1$ is a preferably divalent group derived from a monocyclic $C_2$-$C_9$ heterocyclic compound having 1 or 2 atoms selected from an oxygen atom, a nitrogen atom and a sulfur atom, in the ring, such as pyrrolidine, piperidine, morpholine, thiomorpholine, homopiperidine, homopiperazine, 1,2,3,6-tetrahydropyridine or piperazine, and $G^1$ is preferably bonded with $A^1$-C(=O)- through a nitrogen atom. However, when the divalent group derived from the monocyclic $C_2$-$C_9$ heterocyclic compound having 1 or 2 atoms selected from an oxygen atom, a nitrogen atom and a sulfur atom, in the ring as $G^1$ is not substituted, it is more preferable that $A^3$-$A^4$-$G^2$ as a whole is a group other than hydrogen atom.

30) In the formula (I), when $A^1$ is $-(CH_2)_3-$, $A^1$-$A^2$-$G^1$ links in the form of $A^1$-C(=O)-NH-$G^1$, and $G^1$ is a phenylene group, the phenylene group as $G^1$ is preferably substituted with one or more substituents selected from those exemplified for the preferred substituents of the substituted aromatic hydrocarbon group having 6 to 14 carbon atoms of $G^1$.

31) In the formula (I), when $A^1$ is $-(CH_2)_2-$, $A^1$-$A^2$-$G^1$ links in the form of $A^1$-C(=O)-NH-$G^1$, $G^1$ is a phenylene group, and the phenylene group as $G^1$ is not substituted, it is preferable that $A^3$-$A^4$-$G^2$ as a whole is a group other than a hydrogen atom.

32) In the formula (I), when $^1$ is $-(CH_2)_3-$, and $A^1$-$A^2$-$G^1$ links in the form of $A^1$-C (=O) -NH-$G^1$, $G^1$ is preferably a divalent group derived from a monocyclic or bicyclic $C_3$-$C_9$ aromatic heterocyclic compound having 1 to 3, preferably 1 or 2, atoms selected from the group consisting of an oxygen atom, a nitrogen atom and a sulfur atom, in the ring.

33) In the formula (I), when $A^1$ is $-(CH_2)_3-$, and $A^1$-$A^2$-$G^1$ links in the form of $A^1$-C(=O)-NH-$G^1$, $G^1$ is preferably a divalent group derived from a monocyclic or bicyclic $C_2$-$C_9$ aromatic heterocyclic compound having 1 to 3, preferably 1 or 2 atoms selected from the group consisting of an oxygen atom, a nitrogen atom and a sulfur atom, in the ring. However, the divalent group derived from the aromatic heterocyclic compound as $G^1$ is more preferably substituted with one or more substituents selected from the group consisting of substituents defined as preferred examples for the heterocyclic compound having 1 to 4 atoms selected from the group consisting of an oxygen atom, a nitrogen atom and a sulfur atom, in the ring.

34) In the formula (I), when $A^1$ is $-(CH_2)_3-$, and $A^1$-$A^2$-$G^1$ links in the form of $A^1$-C(=O)-NH-$G^1$, $G^1$ is preferably a divalent group derived from a monocyclic or bicyclic $C_2$-$C_9$ aromatic heterocyclic compound having 1 to 3, preferably 1 or 2 atoms selected from the group consisting of an oxygen atom, a nitrogen atom and a sulfur atom, in the ring. However, when the divalent group derived from the aromatic heterocyclic compound as $G^1$ is not substituted, it is more preferable that $A^3$-$A^4$-$G^2$ as a whole is a group other than hydrogen atom.

35) In the formula (I), when $A^1$ is $-(CH_2)_3-$, $A^1$-$A^2$-$G^1$ links in the form of $A^1$-C(=O)-NH-$G^1$, and $G^1$ represents a single bond, it is more preferable that $A^3$-$A^4$-$G^2$ as a whole is a cycloalkylalkyl group consisting of a acyclic aliphatic hydrocarbon group having 1 to 6 carbon atoms and an alicyclic hydrocarbon group having 3 to 8 carbon atoms.

36) In the formula (I), when, $A^1$ is $-(CH_2)_3-$, $A^1$-$A^2$-$G^1$ links in the form of $A^1$-C(=O)-NH-$G^1$, and $G^1$ represents a single bond, it is more preferable that $A^3$-$A^4$-$G^2$ as a whole is a cycloalkylalkyl group consisting of a acyclic aliphatic hydrocarbon group having 1 to 6 carbon atoms and an alicyclic hydrocarbon group having 3 to 8 carbon atoms. However, in the cycloalkylalkyl group as $A^3$-$A^4$-$G^2$, a acyclic aliphatic hydrocarbon group portion having 1 to 6 carbon atoms is substituted with one or more substituents selected from those exemplified for the preferred substituents of the substituted acyclic aliphatic hydrocarbon group having 1 to 10 carbon atoms of $A^3$, or an aliphatic hydrocarbon group portion having 3 to 8 carbon atoms is substituted with one or more substituents selected from those exemplified for the preferred substituents of the substituted alicyclic hydrocarbon group having 3 to 10 carbon atoms of $G^2$ (including a case where both are substituted).

37) In the formula (I), when $A^1$ is $-(CH_2)_3-$, $A^1$-$A^2$-$G^1$ links in the form of $A^1$-C(=O)-NH-$G^1$, and $G^1$ represents a single

bond, it is more preferable that $A^3$-$A^4$-$G^2$ as a whole is an aralkyl group consisting of a acyclic aliphatic hydrocarbon group having 1 to 6 carbon atoms and an aromatic hydrocarbon group having 6 to 10 carbon atoms.

38) In the formula (I), when $A^1$ is $-(CH_2)_3-$, $A^1$-$A^2$-$G^1$ links in the form of $A^1$-C(=O)-NH-$G^1$, and $G^1$ represents a single bond, it is more preferable that $A^3$-$A^4$-$G^2$ as a whole is an aralkyl group consisting of a acyclic aliphatic hydrocarbon group having 1 to 6 carbon atoms and an aromatic hydrocarbon group having 6 to 10 carbon atoms. However, in the aralkyl group as $A^3$-$A^4$-$G^2$, a acyclic aliphatic hydrocarbon group portion having 1 to 6 carbon atoms is more preferably substituted with one or more substituents selected from those exemplified for the preferred substituents of the substituted acyclic aliphatic hydrocarbon group having 1 to 10 carbon atoms of $A^3$, or an aromatic hydrocarbon group portion having 6 to 10 carbon atoms is further preferably substituted with one or more substituents selected from those exemplified for the preferred substituents of the substituted aromatic hydrocarbon group having 6 to 14 carbon atoms of $G^2$ (including a case where both are substituted).

39) In the formula (I), when $A^1$ is $-(CH_2)_3-$, $A^1$-$A^2$-$G^1$ links in the form of $A^1$-C(=O)-NH-$G^1$, and $G^1$ represents a single bond, it is preferable that $A^3$-$A^4$-$G^2$ as a whole is a heterocyclic substituted alkyl group consisting of a acyclic aliphatic hydrocarbon group having 1 to 6 carbon atoms and a heterocyclic group having 1 to 4 atoms selected from the group consisting of an oxygen atom, a nitrogen atom and a sulfur atom, in the ring.

40) In the formula (I), when $A^1$ is $-(CH_2)_3-$, $A^1$-$A^2$-$G^1$ links in the form of $A^1$-C(=O)-NH-$G^1$, and $G^1$ represents a single bond, it is preferable that $A^3$-$A^4$-$G^2$ as a whole is a heterocyclic substituted alkyl group consisting of a acyclic aliphatic hydrocarbon group having 1 to 6 carbon atoms and a heterocyclic group having 1 to 4 atoms selected from the group consisting of an oxygen atom, a nitrogen atom and a sulfur atom, in the ring. However, in the heterocyclic substituted alkyl group as $A^3$-$A^4$-$G^2$, a acyclic aliphatic hydrocarbon group portion having 1 to 6 carbon atoms is further preferably substituted with one or more substituents selected from those exemplified for the preferred substituents of the substituted acyclic aliphatic hydrocarbon group having 1 to 10 carbon atoms of $A^3$, or a heterocyclic group portion is further preferably substituted with one or more substituents selected from those exemplified for the preferred substituents of the heterocyclic group having 1 to 4 atoms selected from the group consisting of an oxygen atom, a nitrogen atom and a sulfur atom, in the ring, of $G^2$ (including a case where both are substituted).

41) In the formula (I), when all of $A^1$, $A^2$, $G^1$, $A^3$, and $A^4$ represent a single bond, $G^2$ is preferably a hydrogen atom or a acyclic aliphatic hydrocarbon group having 1 to 6 carbon atoms.

Also, the preferred combinations of X, $A^1$, $A^2$, $G^1$, $A^3$, $A^4$ and $G^2$ in formula (I) as described in above 1) through 41) are more preferably combined with a preferred group represented by $R^2$-$A^5$-, exemplified as preferred combinations of $R^2$ and $A^5$, that is $R^2$-$A^5$- group in which $A^5$ is a bond representing a single bond and $R^2$ is a substituted or unsubstituted monocyclic $C_3$-$C_5$ aromatic heterocyclic group having 1 or 2 atoms selected from the group consisting of an oxygen atom, a nitrogen atom and a sulfur atom, in the ring, or $R^2$-$A^5$- group in which $R^2$ is a substituted or unsubstituted aliphatic hydrocarbon group, and with a preferred group represented by $R^3$-$A^6$-, exemplified as preferred combinations of $R^3$ and $A^6$.

The pyrrolopyrimidine-thione derivative of the formula (I) has tautomeric forms represented by the following formula (III):

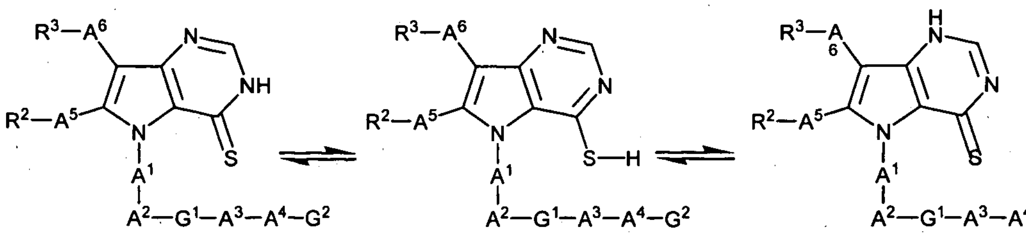
(III)

[wherein $A^1$, $A^2$, $A^3$, $A^4$, $A^5$, $A^6$, $G^1$, $G^2$, $R^2$, and $R^3$ are the same as those defined above in the formula (I).]

However, needless to say all such tautomeric forms are within the scope of the present invention.

When one or more asymmetric structures exist on atoms constituting molecules of the pyrrolopyrimidine-thione derivative formula (I), optically active forms of the respective asymmetric structures and their mixtures combined in any ratio are also within the scope of the present invention.

When there exist stereochemical isomers of molecules of the pyrrolopyrimidine-thione derivative of formula (I), the stereochemical isomers and mixtures of these in any ratio are also within the scope of the present invention.

The pyrrolopyrimidine-thione derivative of the formula (I) may have a basic group in its molecules. In this case,

if necessary, it can be converted into pharmaceutically acceptable acid addition salts. Such acids include inorganic acids such as hydrochloric acid, hydrobromic acid, sulfuric acid, phosphoric acid, and carbonic acid; or organic acids such as acetic acid, citric acid, malic acid, oxalic acid, tartaric acid, lactic acid, maleic acid, fumaric acid, and methanesulfonic acid.

The pyrrolopyrimidine-thione derivative of formula (I) may have an acidic group in its molecules. In this case, when required, the acidic group may be converted into pharmaceutically acceptable salts, including nontoxic cation salts, exemplified by alkali metal ions such as $Na^+$ or $K^+$, alkaline earth metal ions such as $Mg^{2+}$ or $Ca^{2+}$, metal ions such as $Al^{3+}$ or $Zn^{2+}$, ammonia, and salts with an organic base such as triethylamine, ethylenediamine, propanediamine, pyrrolidine, piperidine, piperazine, pyridine, lysine, choline, ethanolamine, N,N-dimethylethanolamine, 4-hydroxypiperidine, glucosamine, or N-methylglucamine.

In the formula (II), $A^1$, $A^2$, $A^3$, $A^4$, $A^5$, $A^6$, $G^1$, $G^2$, $R^2$ and $R^3$ are the same as those defined above in the formula (I), and examples thereof include the same as those exemplified in the formula (I), respectively. Also preferred examples of $A^1$, $A^2$, $A^3$, $A^4$, $A^5$, $A^6$, $G^1$, $G^2$, $R^2$ and $R^3$ and preferred combinations of them are the same as those described for the pyrrolopyrimidine-thione derivative of the present invention represented in the formula (I) except those being obstacle on the chemical reaction in both of the reaction from the pyrrolopyrimidine derivative of the present invention represented by the formula (I) to the pyrrolopyrimidine-thione derivative of the present invention represented by the formula (I), and the reaction from the pyrrolopyrimidine derivative represented by the formula (II) to the pyrrolopyrimidine-thione derivative of the present invention represented by the formula (I).

In the formula (II), $X^1$ represents a chlorine atom, a bromine atom, an iodine atom, or a $C_1$-$C_8$ alkyl or arylsulfonyloxy group. When $X^1$ represents a $C_1$-$C_8$ alkyl or arylsulfonyloxy group, examples of the $C_1$-$C_8$ alkyl or arylsulfonyloxy group include sulfonyloxy group consisting optionally substituted $C_1$-$C_8$ alkyl or aryl group and sulfonyl group, such as methylsulfonyloxy, trifluoromethylsulfonyloxy, ethylsulfonyloxy, propylsulfonyloxy, butylsulfonyloxy, t-butylsulfonyloxy, nonafluorobutylsulfonyloxy, phenylsulfonyloxy, p-bromophenylsulfonyloxy, p-toluylsulfonyloxy, benzylsulfonyloxy, α-phenethylsulfonyloxy and β-phenethylsulfonyloxy. Examples of such preferred $X^1$ include a chlorine atom, a bromine atom, an iodine atom and a trifluoromethylsulfonyloxy group. Particularly, a chlorine atom or a trifluoromethylsulfonyloxy group is more preferred.

From the compounds represented by the formula (Ic), the pyrrolopyrimidine-thione derivative of formula (I) of the present invention can be easily manufactured based on the technical common sense of the person skilled in the art.

In the formula (Ic) , $A^1$, $A^2$, $A^3$, $A^4$, $A^5$, $A^6$, $G^1$, $G^2$, $R^2$, and $R^3$ are the same as those defined above in formula (I), and examples thereof include the same as those exemplified in formula (I), respectively.

In the formula (Ic), Q represents a $C_2$-$C_{10}$ acyl group, a $C_2$-$C_{10}$ alkoxymethyl group, or a substituted or unsubstituted benzyl group. When Q represents a $C_2$-$C_{10}$ acyl group, examples of the $C_2$-$C_{10}$ acyl group include acetyl, trifluoroacetyl, propionyl, butyryl, isobutyryl, valeryl, isovaleryl, pivaloyl, hexanoyl, benzoyl, phenylacetyl, phenylpropionyl, cinnamoyl. When Q represents a $C_2$-$C_{10}$ alkoxymethyl, examples of the $C_2$-$C_{10}$ alkoxymethyl group include methoxymethyl, methoxyethoxymethyl, t-butoxymethyl, 2-(trimethylsilyl)ethoxymethyl, benzyloxymethyl, p-methoxybenzyloxymethyl, p-nitrobenzyloxymethyl, o-nitrobenzyloxymethyl and 4-methoxyphenoxymethyl. When Q represents a substituted or unsubstituted benzyl group, examples of the substituted or unsubstituted benzyl group include benzyl, p-methoxybenzyl, 3,4-dimethoxybenzyl, o-nitrobenzyl, p-nitrobenzyl and p-cyanobenzyl. Examples of such preferred Q include 2-(trimethylsilyl)ethoxymethyl.

The pyrrolopyrimidine-thione derivative of the formula (I) can be prepared from pyrrolo[3, 2-d]pyrimidine derivative of the formula (II) by the following synthesis (A).

Note that, the pyrrolopyrimidine-thione derivative represented by the formula (I) is described as (Ib) in the following synthesis, and sometimes expressed as pyrrolo[3, 2-d]pyrimidine derivative.

[Synthesis (A)]

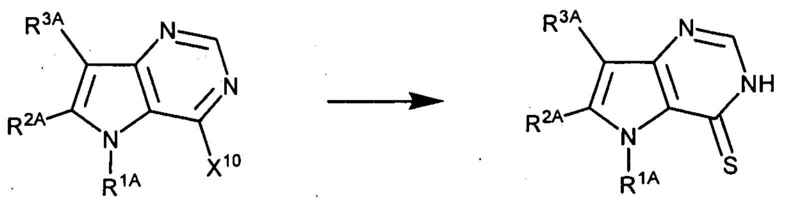

(II-A) (Ib-A)

[wherein $R^{1A}$ represents a group capable of withstanding a conversion reaction among groups defined to be represented by $A^1$-$A^2$-$G^1$-$A^3$-$A^4$-$G^2$ in formula (I). $R^{2A}$ represents a group capable of withstanding a conversion reaction among groups defined to be represented by $R^2$-$A^5$ in formula (I). $R^{3A}$ represents a group capable of withstanding a conversion reaction among groups defined to be represented by $R^3$-$A^6$ in formula (I). $X^{10}$ represents a chlorine atom, a bromine atom, an iodine atom, or an optionally substituted $C_1$-$C_8$ alkyl or arylsulfonyloxy group.]

In other words, the pyrrolo[3, 2-d]pyrimidine derivative Ia-A) of the present invention can be synthesized by reacting the pyrrolo[3, 2-d]pyrimidine derivative (II-A) with a thiourea. The thioxodizing reaction with the thiourea can be carried out using a solvent, for example, dioxane, ethanol, or 2-propanol, at a temperature in a range of 0°C to 150°C.

Among the pyrrolo[3,2-d]pyrimidine derivatives of formula (II), a pyrrolo[3, 2-d]pyrimidine derivative of formula (II-B) can be synthesized from the pyrrolo[3,2-d]pyrimidine derivative of formula (Ib) by the following synthesis.

[Synthesis (B)]

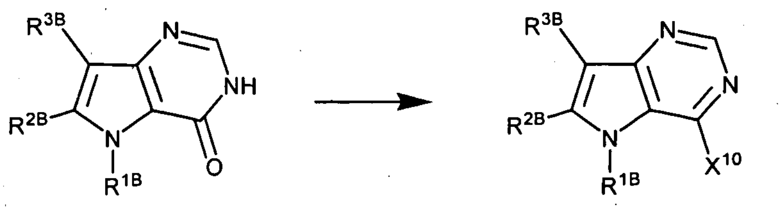

(Ib-B) (II-B)

[wherein $R^{1B}$ represents a group capable of withstanding a conversion reaction among groups defined to be represented by $A^1$-$A^2$-$G^1$-$A^3$-$A^4$-$G^2$ in formula (I). $R^{2B}$ represents a group capable of withstanding a conversion reaction among groups defined to be represented by $R^2$-$A^5$ in formula (I). $R^{3B}$ represents a group capable of withstanding a conversion reaction among groups defined to be represented by $R^3$-$A^6$ in formula (I). $X^{10}$ has the same meaning as defined above.]

In other words, when $X^{10}$ is a chlorine atom, the pyrrolo[3, 2-d]pyrimidine derivative (II-B) of the present invention can be synthesized by reacting the pyrrolo[3, 2-d]pyrimidine derivative (Ib-B) with phosphorus oxychloride. In the chlorination using phosphorus oxychloride, the reaction is carried out in a solvent such as acetonitrile under general chlorination reaction conditions, for example, in the presence or absence of a solvent such as triethylamine, 4-dimethylaminopyridine or dimethyl type aniline, at a temperature in a range of 0°C to 150°C.

Also, when $X^{10}$ is a trifluoromethanesulfonyloxy group, for example, the pyrrolo[3,2-d]pyrimidine derivative (II-B) can be synthesized by reacting pyrrolo[3,2-d]pyrimidine derivative (Ib-B) with trifluoromethanesulfonic anhydride. In trifluoromethane sulfonyloxylation using trifluoromethane sulfonic anhydride, the reaction can be carried out together with pyridine or amines such as triethylamine in the presence or absence of a solvent such as dichloromethane at a temperature in a range of 0°C to 100°C.

Among the pyrrolo[3,2-d]pyrimidine derivatives of formula (Ib-B), the pyrrolo[3,2-d]pyrimidine derivative of formula (Ib-B1) can be synthesized from a 7-cyanopyrrolo[3,2-d] pyrimidine derivative of formula (Ib-CN) by the following synthesis (B1).

[Synthesis (B1)]

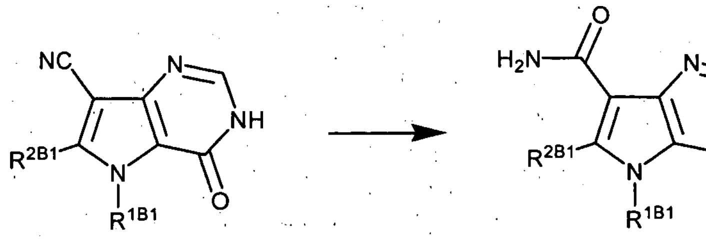

(Ib-CN) (Ib-B1)

[wherein $R^{1B1}$ represents a group capable of withstanding a conversion reaction among groups defined to be represented by $A^1$-$A^2$-$G^1$-$A^3$-$A^4$-$G^2$ in the formula (I). $R^{2B1}$ represents a group capable of withstanding a conversion reaction among groups defined to be represented by $R^2$-$A^5$ in the formula (I).]

In other words, the pyrrolo[3,2-d]pyrimidine derivative (Ib-B1) can be synthesized by hydrolyzing a pyrrolo[3,2-d] pyrimidine derivative (Ib-CN). The hydrolysis reaction is carried out using a base such as sodium hydroxide or lithium hydroxide in a solvent such as ethanol, 2-propanol or dimethylsulfoxide in the presence or absence of hydrogen peroxide at a temperature in a range of 0°C to 100°C.

Among the pyrrolo[3,2-d]pyrimidine derivatives of the formula (Ib-B), a pyrrolo[3, 2-d]pyrimidine derivative of the formula (Ib-B2) can be synthesized from the pyrrolo[3,2-d] pyrimidine derivative of the formula (Ib-B1) by the following synthesis (B2).

[Synthesis (B2)]

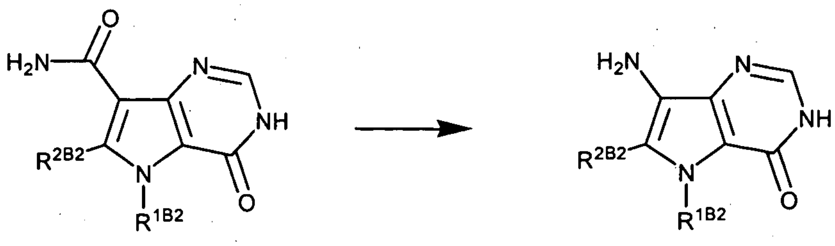

(Ib-B1) (Ib-B2)

[wherein $R^{1B2}$ represents a group capable of withstanding a conversion reaction among groups defined to be represented by $A^1$-$A^2$-$G^1$-$A^3$-$A^4$-$G^2$ in formula (I). $R^{2B2}$ represents a group capable of withstanding a conversion reaction among groups defined to be represented by $R^2$-$A^5$ in formula (I).]

In other words, the pyrrolo[3,2-d]pyrimidine derivative (Ib-B2) can be synthesized by performing Hoffmann rearrangement on the pyrrolo[3,2-d]pyrimidine derivative (Ib-B1). The Hoffmann rearrangement is carried out in a solvent such as ethanol, 2-propanol, acetonitrile or water, using a reagent such as sodium hypochlorite, bromine, or benzyltrimethyl ammonium tribromide in the presence or absence of a base such as sodium hydroxide at a temperature of 0°C to 150°C.

Among the pyrrolo[3,2-d]pyrimidine derivatives of formula (Ib-B), a pyrrolo[3,2-d]pyrimidine derivative of formula (Ib-B3) can be synthesized from the pyrrolo[3,2-d] pyrimidine derivative of formula (Ib-B2) by the following synthesis (B3).

[Synthesis (B3)]

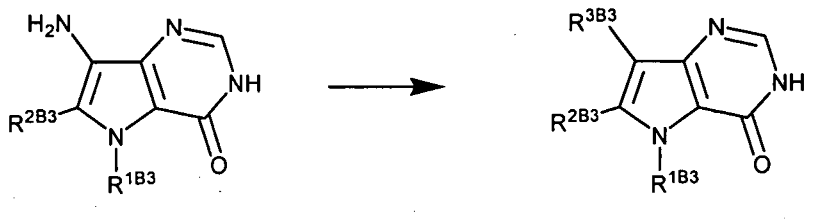

(Ib-B2) (Ib-B3)

[wherein $R^{1B3}$ represents a group capable of withstanding a conversion reaction among groups defined to be represented by $A^1$-$A^2$-$G^1$-$A^3$-$A^4$-$G^2$ in the formula (I). $R^{2B3}$ represents a group capable of withstanding a conversion reaction among groups defined to be represented by $R^2$-$A^5$ in the formula (I). $R^{3B3}$ represents a fluorine atom, a chlorine atom, a bromine atom or an iodine atom.]

In other words, the pyrrolo[3,2-d]pyrimidine derivative (Ib-B3) can be synthesized by reacting the pyrrolo[3,2-d] pyrimidine derivative (Ib-B2) with nitrous acid or nitrite ester, and performing a Sandmayer reaction. In the Sandmayer reaction using nitrous acid or nitrite ester, reagents, for example, nitrous acid, sodium nitrite, isoamyl nitrite, or t-butyl nitrite is used, and the reaction can be performed in the presence of halogenation reagents, for example hydrofluoric acid or fluoroboric acid for fluorination, for example copper chloride or carbon tetrachloride for chlorination, for example carbon tetrabromide or bromoform for bromination, and diiodomethane or iodine for iodination, in the presence or absence of an acid such as sulfuric acid or hydrochloric acid, in the presence or absence of an acid such as sulfuric acid or hydrochloric acid, by using or without using a solvent such as ethanol, acetonitrile or water, at a temperature in a range of 0°C to 150°C

Among the pyrrolo[3,2-d]pyrimidine derivatives of formula (Ib-B), a pyrrolo[3,2-d]pyrimidine derivative of formula (Ib-B4) can be synthesized from the pyrrolo[3,2-d]pyrimidine derivative of formula (Ib-B2) by the following synthesis (B4).

[Synthesis (B4)]

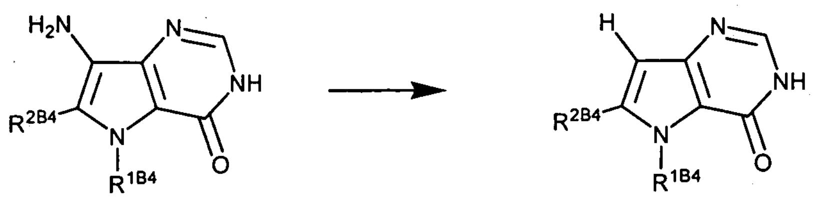

(Ib-B2) (Ib-B4)

[wherein $R^{1B4}$ represents a group capable of withstanding a conversion reaction among groups defined to be represented by $A^1$-$A^2$-$G^1$-$A^3$-$A^4$-$G^2$ in the formula (I). $R^{2B4}$ represents a group capable of withstanding a conversion reaction among groups defined to be represented by $R^2$-$A^5$ in the formula (I).]

In other words, the pyrrolo[3,2-d]pyrimidine derivative (Ib-B4) can be synthesized by reacting the pyrrolo[3,2-d] pyrimidine derivative (Ib-B2) with nitrous acid or nitrite ester. The reaction using nitrous acid or nitrite ester can be performed by using nitrous acid, sodium nitrite, isoamyl nitrite, or t-butyl nitrite as a reagent, in the presence of or in the absence of an acid such as sulfuric acid or hydrochloric acid in the presence of dimethylformamide, tetrahydrofuran, ethanol or water as a solvent, at a temperature in a range of 0°C to 150°C.

Among the pyrrolo[3,2-d]pyrimidine derivatives of formula (Ib-B), a pyrrolo[3,2-d]pyrimidine derivative of formula (Ib-B5) can be synthesized from the pyrrolo[3,2-d] pyrimidine derivative of formula (Ib-B4) by the following synthesis (B5).

[Synthesis (B5)]

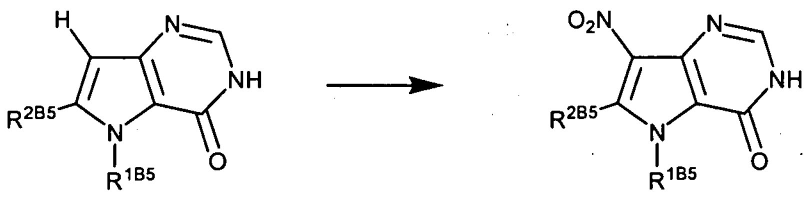

(Ib-B4) (Ib-B5)

[wherein $R^{1B5}$ represents a group capable of withstanding a conversion reaction among groups defined to be represented by $A^1$-$A^2$-$G^1$-$A^3$-$A^4$-$G^2$ in the formula (I). $R^{2B5}$ represents a group capable of withstanding a conversion reaction among groups defined to be represented by $R^2$-$A^5$ in the formula (I).]

In other words, the pyrrolo[3,2-d]pyrimidine derivative (Ib-B5) can be synthesized by reacting nitric acid or nitrogen dioxide with the pyrrolo[3,2-d]pyrimidine derivative (Ib-B4). The reaction using nitric acid or nitrogen dioxide can be performed by using nitric acid, nitrogen dioxide, cerium ammonium nitrate or sodium nitrite as a reagent, in the presence or absence of sulfuric acid, hydrochloric acid, acetic acid or ozone, in the presence of dichloroethane, dichloromethane, acetonitrile or water as a solvent, at a temperature in a range of 0°C to 100°C.

Among the pyrrolo[3,2-d]pyrimidine derivatives of formula (Ib-B), a pyrrolo[3,2-d]pyrimidine derivative of formula (Ib-B6) can be synthesized from the pyrrolo[3,2-d] pyrimidine derivative of formula (Ib-B6a) by the following synthesis (B6).

[Synthesis (B6)]

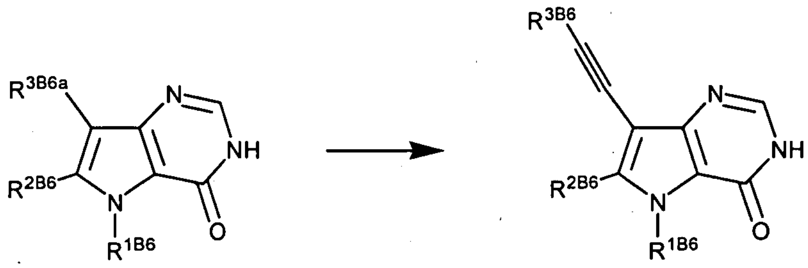

(Ib-B6a) (Ib-B6)

[wherein $R^{1B6}$ represents a group capable of withstanding a conversion reaction among groups defined to be represented by $A^1$-$A^2$-$G^1$-$A^3$-$A^4$-$G^2$ in the formula (I). $R^{2B6}$ represents a group capable of withstanding a conversion reaction among groups defined to be represented by $R^2$-$A^5$ in the formula (I). $R^{3B6a}$ is a bromine atom or iodine atom, and among groups defined as $R^3$ in the formula (I), $R^{3B6}$ is a substituted or unsubstituted saturated aliphatic hydrocarbon group having 1 to 10 carbon atoms, a substituted or unsubstituted alicyclic hydrocarbon group having 3 to 8 carbon atoms, a substituted or unsubstituted aromatic hydrocarbon group having 6 to 14 carbon atoms, a monocyclic $C_3$-$C_5$ aromatic heterocyclic group having 1 or 2 atoms selected from the group consisting of an oxygen atom, a nitrogen atom and a sulfur atom on the substituted or unsubstituted ring, or a trimethylsilyl.]

In other words, the pyrrolo[3,2-d]pyrimidine derivative (Ib-B6) can be synthesized by reacting the pyrrolo[3,2-d] pyrimidine derivative (Ib-B6a) with a terminal alkyne derivative represented by formula $R^{3B6}$-C≡C-H in the presence of a catalytic amount of palladium. The reaction with the terminal alkyne derivative using the catalytic amount of palladium is carried out using the terminal alkyne derivative together with a palladium catalyst, e.g., tetrakis(triphenylphosphine) palladium, chlorobis(triphenylphosphine) palladium, or palladium acetate, in the presence or absence of a ligand, such as triphenylphosphine, tri(o-tolyl)phosphine, or 1,1'-bis(diphenylphosphino)ferrocene, in the presence or absence of a catalytic amount of copper salts, e.g., copper iodide or copper bromide, in the presence of a base such as triethylamine, diethylamine, piperizine or pyrrolidine, using solvents such as tetrahydrofuran, dimethylformamide, and toluene, at a temperature in a range of 0°C to 150°C.

Among the pyrrolo[3,2-d]pyrimidine derivatives of formula (Ib-B), a pyrrolo[3,2-d]pyrimidine derivative of formula (Ib-B7) can be prepared from the pyrrolo[3,2-d] pyrimidine derivative of formula (Ib-B7a) by the following synthesis (B7).

[Synthesis (B7)]

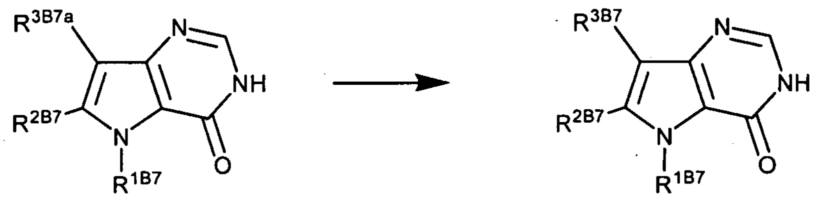

(Ib-B7a) (Ib-B7)

[wherein $R^{1B7}$ represents a group capable of withstanding a conversion reaction among groups defined to be represented by $A^1$-$A^2$-$G^1$-$A^3$-$A^4$-$G^2$ in the formula (I). $R^{2B7}$ represents a group capable of withstanding a conversion reaction among groups defined to be represented by $R^2$-$A^5$ in the formula (I). $R^{3B7a}$ is a bromine atom or an iodine atom. $R^{3B7}$ is a substituted or unsubstituted aromatic hydrocarbon group having 6 to 14 carbon atoms, or an aromatic heterocyclic group having 1 or 2 atoms selected from the group consisting of an oxygen atom, a nitrogen atom and a sulfur atom on the substituted or unsubstituted ring among groups defined as $R^3$ in the formula (I).]

In other words, the pyrrolo[3,2-d]pyrimidine derivative (Ib-B7) can be synthesized, in the presence of a catalytic amount of palladium, by adding a boric acid derivative [$R^{3B7}$-$B(OR)_2$, wherein $R^{3B7}$ is the same as defined above in the synthesis(B7), and R represents a hydrogen atom or an alkyl group] to the pyrrolo[3,2-d]pyrimidine derivative (Ib-B7a). That is, in the reaction with the boric acid derivative using the catalytic amount of palladium, the reaction can be performed by using, together with the boric acid derivative, a palladium catalyst, for example, chlorobis(triphenylphosphine) palladium, palladium acetate, and tris(dibenzylideneacetone) dipalladium-chloroform adduct in the presence or absence of a ligand, such as triphenylphosphine, tri(o-tolyl)phosphine, or 1,1'-bis(diphenylphosphino)ferrocene, in the presence of base such as potassium phosphate, sodium carbonate, potassium hydroxide, or sodium ethoxide, using a solvent such as tetrahydrofuran, dimethylformamide, 2-propanol and water, at a temperature in a range of 0°C to 150°C.

Among the pyrrolo[3,2-d]pyrimidine derivatives of formula (Ib-B), a pyrrolo[3,2-d]pyrimidine derivative of formula (Ib-B8) can be synthesized from the pyrrolo[3,2-d] pyrimidine derivative of formula (Ib-B8a) by the following synthesis (B8).

[Synthesis (B8)]

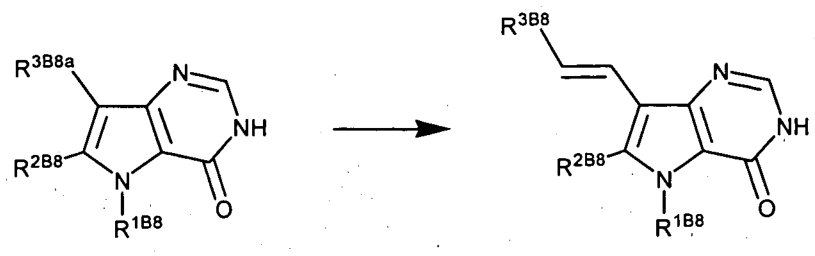

(Ib-B8a) (Ib-B8)

[wherein $R^{1B8}$ represents a group capable of withstanding a conversion reaction among groups defined to be represented by $A^1$-$A^2$-$G^1$-$A^3$-$A^4$-$G^2$ in the formula (I). $R^{2B8}$ represents a group capable of withstanding a conversion reaction among groups defined to be represented by $R^2$-$A^5$ in the formula (I). $R^{3B8a}$ is a bromine atom or an iodine atom, and $R^{3B8}$ is a group defined as $R^3$ in the formula (I).]

In other words, the pyrrolo[3,2-d]pyrimidine derivative (Ib-B8) can be synthesized by reacting a terminal alkene derivative upon the pyrrolo[3,2-d]pyrimidine derivative (Ib-B8a) in the presence of a catalytic amount of palladium. That

is, in the reaction with a terminal alkene derivative using the catalytic amount of palladium, the reaction can be performed by using, together with the terminal alkene derivative, a palladium catalyst, for example, palladium chloride, palladium acetate, or tris(dibenzylideneacetone)dipalladium-chloroform adduct in the presence or absence of a ligand, such as triphenylphosphine, tri(o-tolyl)phosphine, or 1,1'-bis(diphenylphosphino)ferrocene, in the presence of a base such as a potassium phosphate, potassium carbonate or triethylamine, and using a solvent such as tetrahydrofuran, dimethylformamide or water, at a temperature in a range of 0°C to 150°C.

Alternatively, the pyrrolo[3,2-d]pyrimidine derivative (Ib-B8) can also be synthesized by performing a catalytic semi-reduction or hydroboration-protonation on the pyrrolo[3,2-d]pyrimidine derivative (Ib-B6) having an alkynyl group prepared by the Synthesis (B6). For example, the catalytic semi-reduction is performed using a solvent such as methanol, ethanol or tetrahydrofuran, in the presence of a palladium catalyst, e.g., palladium-barium sulfate-quinoline, palladium-activated carbon-quinoline, under a hydrogen atmosphere, at a temperature in a range of 0°C to 100°C. The hydroboration-protonation is performed such that hydroboratino is performed using a hydroborating reagent, e.g., 9-borabicyclo [3.3.1]nonane or dicyclohexylborane, and protonation is then performed using acetic acid. The reaction can be performed using a solvent such as tetrahydrofuran, diethylether, methylenedichloride, or toluene, at a temperature in a range of 0°C to 100°C.

Among the pyrrolo[3,2-d]pyrimidine derivatives of formula (Ib-B), a pyrrolo[3,2-d]pyrimidine derivative of formula (Ib-B9) can be synthesized from the pyrrolo[3,2-d] pyrimidine derivative of formula (Ib-B9a) by the following synthesis (B9).

[Synthesis (B9)]

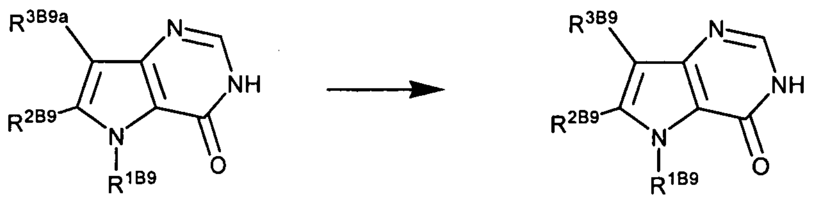

(Ib-B9a) (Ib-B9)

[wherein $R^{1B9}$ represents a group capable of withstanding a conversion reaction among groups defined to be represented by $A^1$-$A^2$-$G^1$-$A^3$-$A^4$-$G^2$ in formula (I). $R^{2B9}$ represents a group capable of withstanding a conversion reaction among groups defined to be represented by $R^2$-$A^5$ in formula (I). $R^{3B9a}$ is a bromine atom or an iodine atom. $R^{3B9}$ is a substituted or unsubstituted saturated aliphatic hydrocarbon group having 1 to 10 carbon atoms, substituted or unsubstituted $C_1$-$C_{10}$ alicyclic hydrocarbon group, or a vinyl group.]

In other words, the pyrrolo[3,2-d]pyrimidine derivative (Ib-B9) can be synthesized by reacting an organometalic reagent to the pyrrolo[3,2-d]pyrimidine derivative (Ib-B9a) using a catalytic amount of palladium or nickel. For example, in the reaction with the organometalic reagent using the catalytic amount of palladium or nickel, an organozinc reagent, e.g., phenylzinc chloride or an organozinc compound prepared from a Grignard reagent and zinc chloride, an organotin reagent, e.g., phenyltrimethyltin or tetramethyltin can be used. As the Grignard reagent, organometallic reagents, such as phenylbromomagnesium or n-butylbromomagnesium, can be used. Useful examples of the palladium catalyst include tetrakis(triphenylphosphine) palladium, tris(dibenzylidene- acetone)dipalladium-chloroform adduct, chloro{1,1'-bis (diphenylphosphino)ferrocene}palladium, and the like. Useful examples of the nickel catalyst include chloro{1,3-bis (diphenylphosphino)propane}nickel or nickel bromide. The reaction can be performed using a solvent such as diethylether, tetrahydrofuran or dimethylformamide, in the presence or absence of a ligand, such as triphenylphosphine, tri(o-tolyl)phosphine, or 1,1'-bis(diphenylphosphino) ferrocene, at a temperature in a range of 0°C to 150°C.

The pyrrolo[3,2-d]pyrimidine derivative (Ib-B9) can also be synthesized through hydrogen reduction of the pyrrolo[3,2-d] pyrimidine derivative (Ib-B6) having an alkynyl group prepared by the synthesis (B6) or the pyrrolo[3,2-d] pyrimidine derivative (Ib-B8) having an alkenyl group prepared by the synthesis (B8). The hydrogen reduction is performed using a solvent such as methanol, ethanol or tetrahydrofuran in the presence of a catalytic amount of palladium-activated carbon under a hydrogen atmosphere at a temperature in a range of 0°C to 100°C.

Among the pyrrolo[3,2-d]pyrimidine derivatives of formula (Ib-B), a pyrrolo[3,2-d]pyrimidine derivative of formula (Ib-B10) can be synthesized from the pyrrolo[3,2-d] pyrimidine derivative of formula (Ib-10a) by the following synthesis (B10).

[Synthesis (B10)]

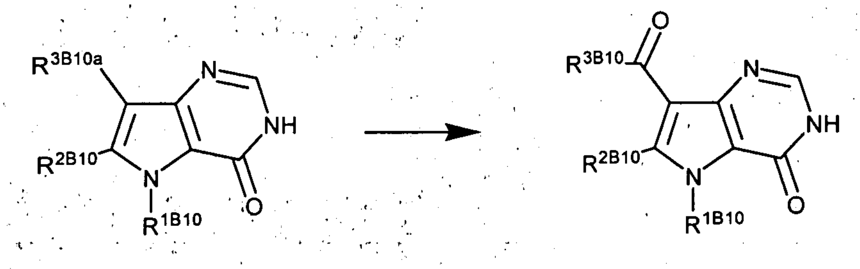

(Ib-B10a) (Ib-B10)

wherein $R^{1B10}$ represents a group capable of withstanding a conversion reaction among groups defined to be represented by $A^1$-$A^2$-$G^1$-$A^3$-$A^4$-$G^2$ in the formula (I). $R^{2B10}$ represents a group capable of withstanding a conversion reaction among groups defined to be represented by $R^2$-$A^5$ in the formula (I). $R^{3B10a}$ is a bromine atom or an iodine atom. $R^{3B10}$ is a $C_2$-$C_{10}$ hydroxyl, alkoxy, N-substituted amino or N,N-disubstituted amino group.

In other words, the pyrrolo[3,2-d]pyrimidine derivative (Ib-B10) can be synthesized by reacting the pyrrolo[3,2-d] pyrimidine derivative (Ib-B10a) with carbon monoxide in the presence of a catalytic amount of palladium. For example, the carbonyl insertion reaction using a catalytic amount of palladium is performed under a carbon monoxide atmosphere, using a palladium catalyst, e.g., tetrakis(triphenyl- phosphine) palladium, palladium acetate, or tris (dibenzylideneacetone)dipalladium-chloroform adduct, in the presence or absence of a ligand, e.g., triphenylphosphine, tri(o-tolyl)phosphine, or 1,1'-bis(diphenylphosphino) ferrocene, in the'presence or absence of a base, e.g., potassium carbonate, or triethylamine. A solvent such as acetonitrile, tetrahydrofuran, or dimethylformamide is used, and the reaction is carried out at a temperature ranging between 0°C and 150°C. In this case, addition of water as a reacting agent gives a compound with a carboxy group, and addition of an alcohol gives a compound with an alkoxycarbonyl group. Addition of a primary or secondary amine gives a compound with N-substituted or N,N-disubstituted aminocarbonyl group.

Among the pyrrolo[3,2-d]pyrimidine derivatives of formula (Ib-B), a pyrrolo[3,2-d]pyrimidine derivative of formula (Ib-B11) can be synthesized from the pyrrolo[3,2-d] pyrimidine derivative of formula (Ib-11a) by the following synthesis (B11).

[Synthesis (B11)]

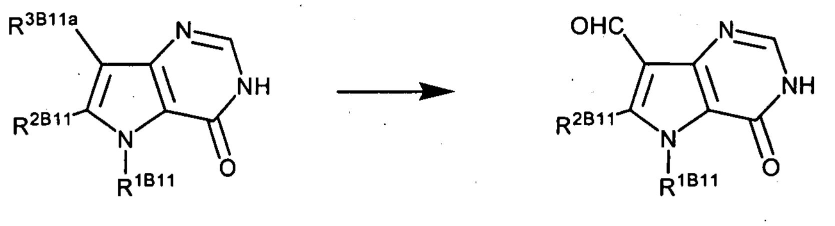

(Ib-B11a) (Ib-B11)

[wherein $R^{1B11}$ represents a group capable of withstanding a conversion reaction among groups defined to be represented by $A^1$-$A^2$-$G^1$-$A^3$-$A^4$-$G^2$ in the formula (I). $R^{2B11}$ represents a group capable of withstanding a conversion reaction among groups defined to be represented by $R^2$-$A^5$ in the formula (I). $R^{2B11a}$ is a bromine atom or an iodine atom.]

In other words, the pyrrolo[3,2-d]pyrimidine derivative (Ib-B11) can be synthesized by reacting the pyrrolo[3,2-d] pyrimidine derivative (Ib-B11a) under a carbon monoxide atmosphere in the presence of a reducing agent and a catalytic amount of palladium. For example, the formylation reaction using a catalytic amount of palladium is performed under the carbon monoxide atmosphere. Useful examples of the palladium catalyst include tetrakis(triphenylphosphine)

palladium, palladium acetate, tris(dibenzylideneacetone)dipalladium-chloroform adduct. The reaction is performed using a solvent such as acetonitrile, tetrahydrofuran, or dimethylformamide in the presence or absence of a ligand such as triphenylphosphine or tri(o-tolyl)phosphine or 1,1'-bis(diphenylphosphino) ferrocene in a temperature range of 0°C to 150°C. The reaction is performed in the presence of or in the absence of a base such as potassium carbonate or triethylamine. Addition of a reducing agent such as tributyltin hydride or triethylsilane gives a compound with a formyl group, and addition of an organometallic agent such as alkyl zinc, alkyl boron or an organotin reagent give a compound with an alkylcarbonyl group.

Among the pyrrolo[3,2-d]pyrimidine derivatives of formula (Ib-B), a pyrrolo[3,2-d]pyrimidine derivative of formula (Ib-B12) can be synthesized from the pyrrolo[3,2-d] pyrimidine derivative of formula (Ib-B12a) by the following synthesis (B12).

[Synthesis (B12)]

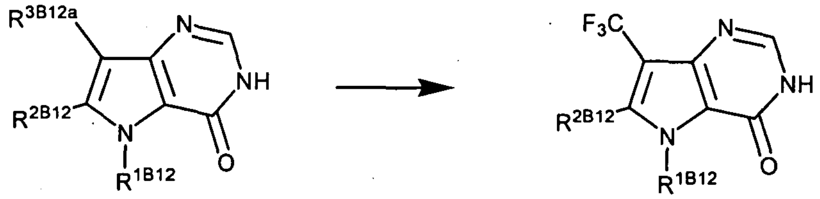

(Ib-B12a) (Ib-B12)

[wherein $R^{1B12}$ represents a group capable of withstanding a conversion reaction among groups defined to be represented by $A^1$-$A^2$-$G^1$-$A^3$-$A^4$-$G^2$ in the formula (I). $R^{2B12}$ represents a group capable of withstanding a conversion reaction among groups defined to be represented by $R^2$-$A^5$ in the formula (I). $R^{3B12a}$ is a bromine atom or an iodine atom.]

In other words, the pyrrolo[3,2-d]pyrimidine derivative (Ib-B12) can be synthesized by reacting the pyrrolo[3,2-d] pyrimidine derivative (Ib-B12a) with a trifluoromethyl donating reagent. That is, in the trifluoromethylation reaction, the reaction can be performed by utilizing various methods, for example, a method using copper (I) iodide or cesium fluoride together with a trifluoromethyl donator such as sodium trifluoroacetate or trifluoromethylacetate, a method for preparing a trifluoromethyl copper compound from a trifluoromethyl zinc compound or a trifluoromethyl cadmium compound and copper (I) bromide, or a method for preparing a trifluoromethyl copper compound from a trifluoromethyl iodide and copper powder, by using a solvent such as dimethylformamide, N-methylpyrrolidinone, hexamethylphosphoramide, acetonitrile, or pyridine, at a temperature in a range of 0°C to 150°C.

Among the pyrrolo[3,2-d]pyrimidine derivatives of formula (Ib-B), a pyrrolo[3,2-d]pyrimidine derivative of Formula (Ib-B13) can be synthesized from the pyrrolo[3,2-d] pyrimidine derivative of Formula (Ib-B13a) by the following synthesis (B13).

[Synthesis (B13)]

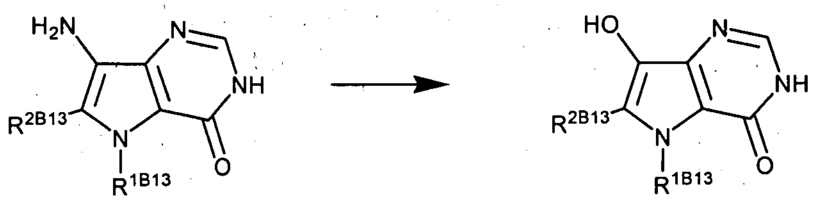

(Ib-B13a) (Ib-B13)

[wherein $R^{1B13}$ represents a group capable of withstanding a conversion reaction among groups defined to be repre-

sented by $A^1$-$A^2$-$G^1$-$A^3$-$A^4$-$G^2$ in the formula (I). $R^{2B13}$ represents a group capable of withstanding a conversion reaction among groups defined to be represented by $R^2$-$A^5$ in the formula (I).]

The pyrrolo[3,2-d]pyrimidine derivative (Ib-B13) can be synthesized by reacting the pyrrolo[3,2-d]pyrimidine derivative (Ib-B13a) with water in the presence of nitrous acid. That is, the hydroxylation reaction in the presence of nitrous acid is performed using sodium nitrite or isoamyl nitrite in the presence of trifluoroacetic acid or sulfuric acid. The reaction can be performed using water as a solvent in the presence or absence of a cosolvent such as acetonitrile or dimethylformamide, at a temperature in a range of 0°C to 150°C.

Among the pyrrolo[3,2-d]pyrimidine derivatives of formula (Ib-B), a pyrrolo[3,2-d]pyrimidine derivative of formula (Ib-B14) can be synthesized from the pyrrolo[3,2-d] pyrimidine derivative of formula (Ib-B14a) by the following synthesis (B14).

[Synthesis (B14)]

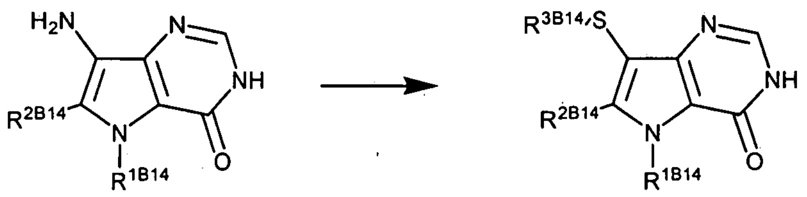

(Ib-B14a) (Ib-B14)

[wherein $R^{1B14}$ represents a group capable of withstanding a conversion reaction among groups defined to be represented by $A^1$-$A^2$-$G^1$-$A^3$-$A^4$-$G^2$ in the formula (I). $R^{2B14}$ represents a group capable of withstanding a conversion reaction among groups defined to be represented by $R^2$-$A^5$ in the formula (I). $R^{3B14}$ is a $C_1$-$C_6$ aliphatic hydrocarbon group.]

In other words, the pyrrolo[3,2-d]pyrimidine derivative (Ib-B14) can be synthesized by reacting the pyrrolo[3,2-d] pyrimidine derivative (Ib-B14a) with water in the presence of nitrous acid. That is, the alkylthioration reaction in the presence of nitrous acid is performed using sodium nitrite or isoamylnitrite in the presence or absence of acids such as hydrochloric acid or sulfuric acid. The reaction is carried out using dialkyldisulfide or alkanethiol as a reagent in a solvent such as acetonitrile or dimethylformamide at a temperature in a range of 0°C to 150°C.

Among the pyrrolo[3, 2-d]pyrimidine derivatives represented by (Ib-B) of the synthesis (B) or (Ib-CN) of the synthesis (B1), a pyrrolo[3,2-d]pyrimidine derivative of formula (Ib-C2) can be synthesized from the pyrrolo[3,2-d] pyrimidine derivative of formula (Ib-C1) by the following synthesis (C).

[Synthesis (C)]

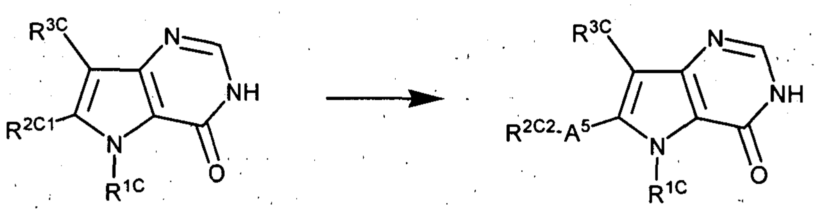

(Ib-C1) (Ib-C2)

[wherein $R^{1C}$ represents a group capable of withstanding a conversion reaction among groups defined to be represented by $A^1$-$A^2$-$G^1$-$A^3$-$A^4$-$G^2$ in the formula (I). $R^{2C1}$ is a chlorine atom or a bromine atom. $R^{3C}$ represents a cyano group or a group capable of withstanding a conversion reaction among groups defined to be represented by $R^2$-$A^5$ in

the formula (I). When $A^5$ is $-NR^{201}-$ ($R^{201}$ is the same as defined above for $R^{201}$ in the formula (1)), $R^{2C2}$ is as defined to exclude a fluorine atom, a chlorine atom, a bromine atom and an iodine atom from groups defined for $R^2$ in formula (I). Also, when $A^5$ is a bond representing a single bond, $R^{2C2}$ is a heterocyclic group having 1 to 4 atoms selected from the group consisting of an oxygen atom, a nitrogen atom and a sulfur atom on the substituted or unsubstituted ring in which $R^{2C2}$ is linked to $A^5$ on a nitrogen atom.

In other words, the pyrrolo[3,2-d]pyrimidine derivatives (Ib-C2) can be synthesized by reacting the pyrrolo[3,2-d] pyrimidine derivatives(Ib-C1) with a primary or secondary amine. Amination using the primary or secondary amine is performed without the use of a solvent or with the use of a solvent such as dimethylsulfoxide, dimethylformamide, dioxane, tetrahydrofuran or toluene in the presence or absence of a base such as pyridine, triethylamine, diisopropylethylamine, 4-dimethylaminopyridine or sodium carbonate. The reaction is performed in the presence or absence of a transition metal complex catalyst prepared by mixing a palladium salt such as palladium acetate with a phosphorus ligand such as triphenylphosphine, at a temperature in a range of 0°C to 150°C.

Among the pyrrolo[3,2-d]pyrimidine derivatives represented by the formula (Ib) or (Ib-CN) of the synthesis (B1), a pyrrolo[3,2-d]pyrimidine derivative of formula (Ib-D2) can be synthesized from the pyrrolo[3,2-d]pyrimidine derivative of formula (Ib-D1) by the following synthesis (D).

[Synthesis (D)]

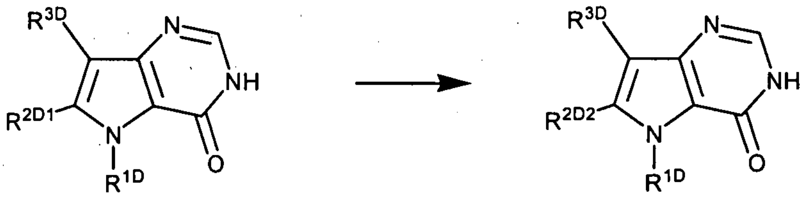

(Ib-D1) (Ib-D2)

[wherein $R^{1D}$ represents a group capable of withstanding a conversion reaction among groups defined to be represented by $A^1$-$A^2$-$G^1$-$A^3$-$A^4$-$G^2$ in the formula (I). $R^{2D1}$ is a chlorine atom or a bromine atom. $R^{2D2}$ is a substituted or unsubstituted aromatic hydrocarbon group having 6 to 14 carbon atoms, or an aromatic heterocyclic group having 1 or 2 atoms selected from the group consisting of an oxygen atom, a nitrogen atom and a sulfur atom on the substituted or unsubstituted ring. $R^{3D}$ is a cyano group or a group capable of withstanding a conversion reaction among groups defined to be represented $A^6$-$R^3$ in the formula (I).]

In other words, the pyrrolo[3,2-d]pyrimidine derivative (Ib-D2) can be synthesized by reacting the pyrrolo[3,2-d] pyrimidine derivative (Ib-D1) with, for example, a boric acid derivative represented by $R^{2D2}$-$B(OR)_2$ [in which $R^{2D2}$ is as defined above in the synthesis (D), and R is a hydrogen atom or an alkyl group]. The reaction with the boric acid derivative is performed under general Suzuki reaction conditions, for example, at a temperature in a range of 0°C to 150°C using a solvent such as 2-propanol and/or water in the presence of an inorganic base such as sodium carbonate, by using a catalyst such as palladium acetate, and adding a ligand such as triphenylphosphine.

Among the pyrrolo[3,2-d]pyrimidine derivatives of Formula (Ib) or (Ib-CN) prepared in the synthesis (B1), a pyrrolo[3,2-d]pyrimidine derivative of formula (Ib-E2) can be synthesized from the pyrrolo[3,2-d]pyrimidine derivative of formula (Ib-E1) in the following manner shown in Synthesis (E):

[Synthesis (E)]

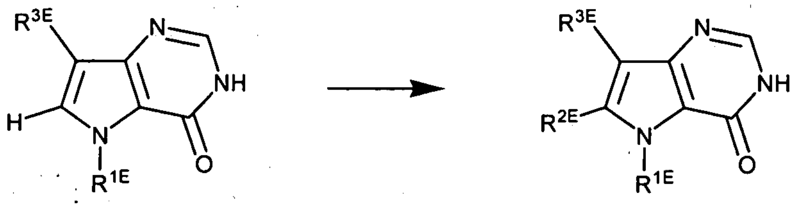

(Ib-E1) (Ib-E2)

[wherein $R^{1E}$ represents a group capable of withstanding a conversion reaction among groups defined to be represented by $A^1$-$A^2$-$G^1$-$A^3$-$A^4$-$G^2$ in the formula (I). $R^{2E}$ is a chlorine atom, a bromine atom or an iodine atom. $R^{3E}$ is a cyano group or a group capable of withstanding a conversion reaction among groups defined to be represented by $A^6$-$R^3$.]

In other words, the pyrrolo[3,2-d]pyrimidine derivative (Ib-E2) can be synthesized by halogenation of the pyrrolo [3,2-d]pyrimidine derivative (Ib-E1). The halogenation is performed using a halogenation reagent such as N-chlorosuccinic imide or N-bromosuccinic imide in the presence of a solvent such as dimethylformamide, dioxane or tetrahydrofuran at a temperature in a range of -20°C to 150°C.

Among the pyrrolo[3,2-d]pyrimidine derivatives of Formula (Ib) or (Ib-CN) prepared in the synthesis (B1), a pyrrolo[3,2-d]pyrimidine derivative of formula (1b-F) given below.can be synthesized from the pyrrol derivative of formula (IV-F) in the following manner shown in Synthesis (F):

[Synthesis (F)]

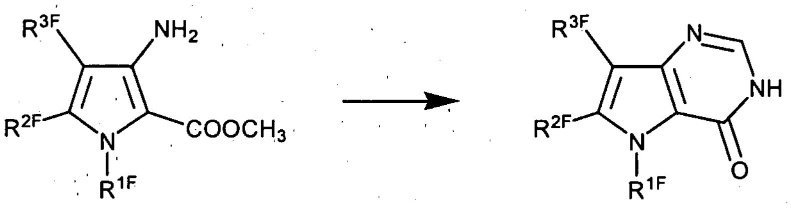

(IV-F) (Ib-F)

[wherein $R^{1F}$ represents a group capable of withstanding a conversion reaction among groups defined to be represented by $A^1$-$A^2$-$G^1$-$A^3$-$A^4$-$G^2$ in the formula (I). $R^{2F}$ represents, among the groups defined for $R^2$ in the formula (I), groups excluding a fluorine atom, a chlorine atom, a bromine atom, an iodine atom, and a substituted or unsubstituted heterocyclic group that is bonded with a carbon atom and a nitrogen atom of a pyrrole ring to which $R^{2F}$ is bonded, and having 1 to 4 atoms selected from the group consisting of an oxygen atom, a nitrogen atom and a sulfur atom, in the ring. $R^{3F}$ is a cyano group or a group capable of withstanding a conversion reaction among groups defined to be represented by $A^6$-$R^3$ in formula (I).]

In other words, the pyrrolo[3,2-d]pyrimidine derivative of formula (Ib-F) can be synthesized by performing a cyclization reaction using formamidine or formamide on the pyrrole derivative of formula (IV-F). The cyclization reaction using formamidine can be performed by using formamidine acetate, for example, in a solvent such as 2-propanol at a temperature in a range of 0°C to 150°C. The cyclization reaction using formamide can be performed smoothly by using a base such as formamide or sodium methoxide, in the presence or absence of a solvent such as dimethylsulfoxide or dimethoxyethane in at a temperature in a range of 0°C to 150°C.

Among the pyrrolo[3,2-d]pyrimidine derivatives of formula (II) and (Ib-CN) prepared by the synthesis (B1), a pyrrolo[3,2-d]pyrimidine derivative of formula (II-G) can be synthesized from the pyrrolo[3,2-d]pyrimidine derivative of Formula (Ib-G) by the following synthesis (G).

[Synthesis (G)]

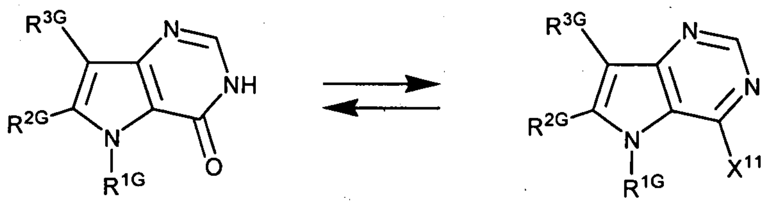

(Ib-G) (II-G)

[wherein $R^{1G}$ represents a group capable of withstanding a conversion reaction among groups defined to be represented by $A^1$-$A^2$-$G^1$-$A^3$-$A^4$-$G^2$ in the formula (I). $R^{2G}$ represents a group capable of withstanding a conversion reaction among groups defined to be represented by $R^2$-$A^5$ in the formula (I). $R^{3G}$ is a cyano group or a group capable of withstanding a conversion reaction among groups defined to be represented by $A^6$-$R^3$ in formula (I). $X^{11}$ is a $C_2$-$C_{10}$ acylthio group or a $C_2$-$C_8$ alkoxymethylthio group.]

In other words, when $X^{11}$ is an acylthio group, the pyrrolo[3,2-d]pyrimidine derivative (II-G) can be synthesized by reacting the pyrrolo[3,2-d]pyrimidine, derivative (Ib-G) with an acyl halide. The acylation reaction using the acyl halide is performed under conventional acylation reaction conditions, for example, in the presence of triethylamine or pyridine, at a temperature in a range of 0°C to 10.0°C.

When $X^{11}$ is an alkoxymethylthio group, the pyrrolo [3,2-d]pyrimidine derivative (II-G) according to the present invention can be synthesized by reacting the pyrrolo [3,2-d]pyrimidine derivative (Ib-G) with an alkoxymethyl halide. The alkoxymethylation reaction using the alkoxymethyl halide is performed under conventional alkoxymethylation conditions, for example, in the presence of triethylamine or pyridine, at a temperature in a range of 0°C to 100°C.

In the thus obtained pyrrolo[3,2-d]pyrimidine derivatives (II-G) according to the present invention, conversion reactions known to one skilled in the art can be performed on $A^1$, $A^2$, $A^3$, $A^4$, $A^5$, $A^6$, $G^1$, $G^2$, $R^2$ and/or $R^3$. Such pyrrolo [3,2-d]pyrimidine derivatives (II-G) can be converted into pyrrolo[3,2-d]pyrimidine derivatives(Ib-G) of the present invention by performing hydrolysis under a neutral or alkaline condition when $X^{11}$ is an acylthio group, or under an acidic condition using, for example, trifluoroacetic acid, when $X^{11}$ is an alkoxymethylthio group.

The pyrrolo[3,2-d]pyrimidine derivatives of formula (Ic) and (Ib-CN) prepared by the synthesis (B1) can be synthesized'from the pyrrolo[3, 2-d]pyrimidine derivatives of formula (I-H) by the following synthesis (H).

[Synthesis (H)]

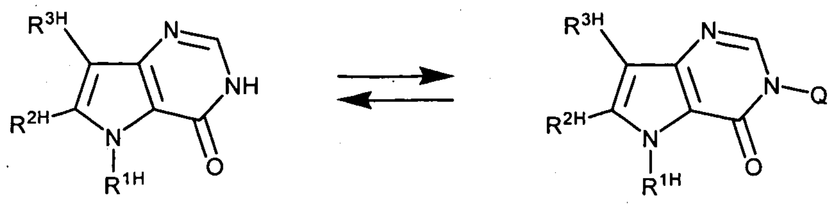

(I-H) (Ic-H)

[wherein $R^{1H}$ represents a group capable of withstanding a conversion reaction among groups defined to be represented by $A^1$-$A^2$-$G^1$-$A^3$-$A^4$-$G^2$ in the formula (I). $R^{2H}$ represents a group capable of withstanding a conversion reaction among groups defined to be represented by $R^2$-$A^5$ in the formula (I). $R^{3H}$ is a cyano group or a group capable of withstanding a conversion reaction among the groups defined to be represented by $A^6$-$R^3$ in the formula (I). Q is an optionally substituted $C_2$-$C_{10}$ acyl group, an optionally substituted $C_2$-$C_{10}$ alkoxymethyl group, or a substituted or unsubstituted benzyl group.]

In other words, when Q is an acyl group, the pyrrolo [3,2-d]pyrimidine derivatives(Ic-H) can be synthesized by reacting the pyrrolo[3,2-d]pyrimidine derivatives(I-H) with an acyl halide. The acylation reaction using the acyl halide is performed under conventional acylation conditions, for example, in the presence of triethylamine or pyridine, at a temperature in a range of 0°C to 100°C.

Also, when Q is an alkoxymethyl or benzyl group, the pyrrolo[3,2-d]pyrimidine derivatives (I-H) of the present invention can be synthesized by reacting the pyrrolo [3,2-d]pyrimidine derivative (I-H) of the present invention with an alkoxymethyl halide or a benzyl halide. The reaction using the alkoxymethyl halide or the benzyl halide is performed in the presence of sodium hydride in a temperature range of 0°C to 100°C.

In the thus obtained pyrrolo[3,2-d]pyrimidine derivatives (Ic-H), conversion reactions known to one skilled in the art can be performed on $A^1$, $A^2$, $A^3$, $A^4$, $A^5$, $A^6$, $G^1$, $G^2$, $R^2$ and/or $R^3$. Such pyrrolo[3,2-d]pyrimidine derivatives (Ic-H) can be converted into pyrrolo[3,2-d]pyrimidine derivatives (I-H) by performing hydrolysis under a neutral or alkaline condition when Q is an acyl group, or under an acidic condition using, for example, trifluoroacetic acid, when Q is an alkoxymethyl group, or by performing a hydrogen addition reaction when $R^3$ is a benzyl group.

When the pyrrolo[3,2-d]pyrimidine derivatives synthesized by the synthesis (A), (B), (C), (D), (E), (F), (G) and (H) have easily convertible substituents, such as an alkoxycarbonyl group, an acyloxy group, an aromatic nitro group, they can be easily converted into pyrrolo[3,2-d]pyrimidine derivatives respectively having a carboxy group, a hydroxy group, and an amino group by performing reactions known to one skilled in the art.

When the pyrrolo[3,2-d]pyrimidine derivatives synthesized by the synthesis (A), (B), (C), (D), (E), (F), (G) and (H) have a carboxy group, they can be converted into pyrrolo[3,2-d]pyrimidine derivatives having an alkoxycarbonyl group, a carbamoyl group, an N-alkylcarbamoyl group by a condensation reaction known to one skilled in the art.

When the pyrrolo[3,2-d]pyrimidine derivatives synthesized by the synthesis (A), (B), (C), (D), (E), (F), (G) and (H) have an amino group, they can be converted into pyrrolo [3,2-d]pyrimidine derivatives having an acylamino group or an alkylsulfonylamino group by a condensation reaction well known to one skilled in the art.

Also, when they have an amino group, they can also be converted into pyrrolo[3,2-d]pyrimidine derivatives having a monoalkylamino or a dialkylamino group by a reductive alkylation reaction known to one skilled in the art.

When the pyrrolo[3,2-d]pyrimidine derivatives synthesized by the synthesis (A), (B), (C), (D), (E), (F), (G) and (H) have a hydroxy group, they can be converted into pyrrolo[3,2-d]pyrimidine derivatives having an acyloxy group by a condensation reaction known to one skilled in the art.

When the pyrrolo[3,2-d]pyrimidine derivatives synthesized by the synthesis (A), (B), (C), (D), (E), (F), (G) and (H) have a formyl group, they can be converted into pyrrolo [3,2-d]pyrimidine derivatives having an alkylaminomethyl group by a reductive alkylation reaction known to one skilled in the art.

In the synthesis of the pyrrolo[3,2-d]pyrimidine derivative of formula (I), the pyrrole derivatives of formula (IV-F) used as starting materials can be prepared from a 3-alkoxypropene nitrile derivative of formula (VI-J) by the following synthesis (J).

[Synthesis (J)]

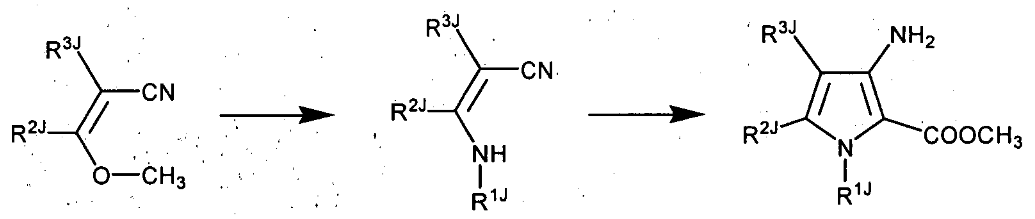

(VI-J) (V-J) (IV-J)

[wherein $R^{1J}$ represents a group capable of withstanding a conversion reaction among groups defined to be represented by $A^1$-$A^2$-$G^1$-$A^3$-$A^4$-$G^2$ in the formula (I). $R^{2J}$ represents, among the groups defined for $R^2$-$A^5$ in the formula (I), groups excluding a fluorine'atom, a chlorine atom, a bromine atom, an iodine atom, and a substituted or unsubstituted heterocyclic group that is bonded with a carbon atom and a nitrogen atom of a pyrrole ring to which $R^{2J}$ is bonded, and having 1 to 4 atoms selected from the group consisting of an oxygen atom, a nitrogen atom and a sulfur atom, in the ring. $R^{3J}$ is a cyano group or a group capable of withstanding a conversion reaction among groups defined to be represented by $A^6$-$R^3$ in formula (I).]

In other words, aminopropenitrile derivatives (V-J) can by synthesized by reacting alkoxypropene nitriles (VI-J) with a primary amine (represented by $R^1$-$NH_2$ in which $R^1$ is as defined above for $R^1$ prepared by the synthesis (J)). The pyrrole derivatives (IV-J) can be synthesized through a reaction between the aminopropenenitrile derivatives (V-J) and methyl bromoacetate in the presence of a base, or through a cyclization reaction.

The reaction between the alkoxypropene nitrile derivatives (V-J) and the primary amine is performed using a solvent such as methanol, ethanol or 2-propanol at a temperature in a range of 0°C to 100°C.

The reaction between the alkoxypropenenitrile derivatives (VI-J) and methyl bromoacetate is performed in the presence of a base such as sodium carbonate using a solvent such as acetonitrile at a temperature in a range of 0°C to 150°C.

In the synthesis of the pyrrolo[3,2-d]pyrimidine derivative of formula (I), among the pyrrole derivatives of formula (IV-F) used as starting materials, a pyrrole derivative having a hydrogen atom as $R^{2F}$ can be prepared from 3-oxopropanenitrile derivatives of formula (VII-K) by the following synthesis (K)

[Synthesis (K)]

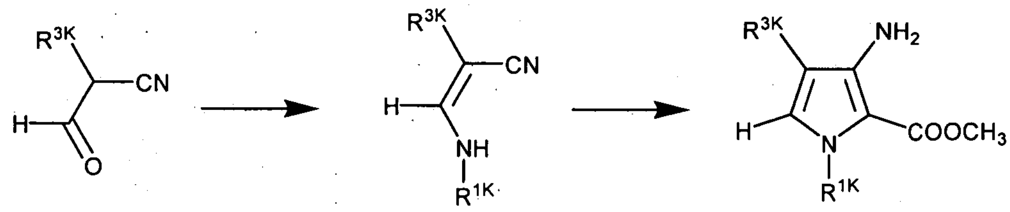

(VII-K) (V-K) (IV-K)

[wherein $R^{1K}$ represents a group which can be converted to $A^1$-$A^2$-$G^1$-$A^3$-$A^4$-$G^2$ in the formula (I), and a group capable of withstanding a conversion reaction. $R^{3K}$ is a cyano group or a group capable of withstanding a conversion reaction among groups defined to be represented by $A^6$-$R^3$ in the formula (I).]

In other words, the aminopropenenitrile derivative (V-K) can be Synthesized by reacting the 3-oxopropanenitrile derivative (VII-k) with a primary amine ($R^1$-$NH_2$ in which $R^1$ is as defined above for $R^1$ prepared by the synthesis (K)). The pyrrole derivatives (IV-K) can be synthesized through a reaction between the aminopropenitrile derivatives (V-K) and methyl bromoacetate in the presence of a base, or through a cyclization reaction.

The reaction between the 3-oxopropanenitrile derivative (VII-K) and the primary amine is performed using a solvent such as methanol, ethanol or 2-propanol at a temperature in a range of 0°C to 100°C.

The reaction between the aminopropenitrile derivative (VI-K) and methyl bromoacetate is performed in the presence of a base such as sodium carbonate using a solvent such as acetonitrile in a temperature range of 0°C to 150°C.

Alternatively, in the syntheses of the pyrrolo [3,2-d]pyrimidine derivative of formula (I), among the pyrrole derivatives of formula (IV-F) used as starting materials, a pyrrole derivative of formula (IV-F) having a hydrogen atom as $R^{2F}$ can also be prepared by the following synthesis (L):

[Synthesis (L)]

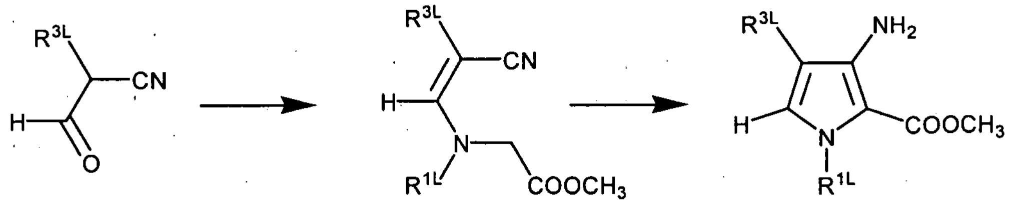

(VII-L) (V-L) (IV-L)

[wherein $R^{1L}$ represents a group which can be converted to $A^1$-$A^2$-$G^1$-$A^3$-$A^4$-$G^2$ in the formula (I), and a group capable of withstanding a conversion reaction. $R^{3L}$ is a cyano or a group capable of withstanding a conversion reaction among groups defined to be represented by $A^6$-$R^3$ in formula (I).]

In other words, the aminopropenitrile derivative (V-L) can by synthesized by reacting the 3-oxopropanenitrile

derivative (VII-L) and a glycinemethylester derivative ($R^1$-NH-$CH_2$-$COOCH_3$ having $R^1$ on a nitrogen atom in which $R^1$ is as defined above for $R^1$ prepared by the synthesis (L)). The pyrrole derivative (IV-L) can be synthesized by performing cyclization of the aminopropenitrile derivative (V-L) in the presence of a base.

The reaction between the 3-oxopropanenitrile derivative (VII-L) and the glycinemethylester derivative is performed using a solvent such as acetic acid at a temperature in a range of 0°C to 150°C.

The cyclization reaction of the aminopropenitrile derivative (V-L) is performed using a solvent such as acetonitrile or ethylene glycol dimethyl ether in the presence of a base such as 1,8-diazabicyclo[5,4,0]-7-undecene or cesium carbonate at a temperature in a range of 0°C to 150°C.

The thus obtained pyrrolo[3,2-d]pyrimidine derivatives of formula (I) have an inhibitory effect of GSK-3 activity, and can be advantageously used as preventive and/or therapeutic agents which are clinically applicable GSK-3 inhibitors. Diseases that can be treated by the GSK-3 activity inhibitor include diabetes, diabetic complications, atherosclerosis, hypertension, obesity, syndrome X, Alzheimer's disease, neurodegenerative diseases (AIDS encephalophy, Huntington's disease, Parkinson's disease, or ischemic attack), manic depressive psychosis, traumatic cerebrospinal injury, alopecia, inflammatory response syndrome, cancer and immunodeficiency.

Also, the pyrrolo[3,2-d]pyrimidine derivatives of formula (I) and its pharmaceutically acceptable salts may be formed as pharmaceutical compositions together with pharmacologically acceptable carriers and/or diluents. The compositions of the present invention may be formed as various kinds of formulations to be administered orally or parenterally. The term "parenteral" as used herein includes intravenous, subcutaneous, intramuscular, percutaneous, and rectal injection or infusion techniques.

For oral administration, examples of the formulation include tablets, pills, granules, powder, solutions, suspensions, syrups, and so on.

Here, the tablet formulations can be formed by conventional methods using a pharmaceutically acceptable carrier such as a vehicle, a binding agent, a disintegrating agent, and the like. The pills, granules and powder can also be formed by conventional methods using a vehicle or the like, like the tablets. The formulations in the form of solutions, suspensions and syrups can be prepared by general methods using glycerine esters, alcohols, water, vegetable oils, and so on. The capsule formulations can be formed by filling capsules of gelatin with granules, powder or solutions.

Among formulations for parenteral administration, intravenous, subcutaneous, and intramuscular administration can take forms of injectable formulations. For injection, the compounds of the invention may be formulated in aqueous solutions such as physiological saline or in nonaqueous solutions including organic esters such as propylene glycol, polyethylene glycol, or vegetable oils.

For transdermal administration, formulations can be used in the form of ointment or cream. Ointments can be used in combination with oils or vaselin, for example. Creams can be prepared in combination with emulsifying agents, for example.

When required, these formulations can be further provided with pharmaceutically acceptable carriers such as an isotonic, a preservative, an antiseptic, a wetting agent, a buffering agent, an emulsifying agent, a dispersing agent, or a stabilizer.

Also, such a variety of formulations can be sterilized through appropriate treatments, for example, filtration using a bacteria retaining filter or combination of disinfectants.

The amount of the pyrrolo[3,2-d]pyrimidine derivative of formula (I) and its pharmaceutically acceptable salt that may be administered may vary depending upon the kind of a disease, administration route, symptom, age, sex, body weight, and so on of the patient. Generally, a dosage for oral administration is between 0.1 and 500 mg/day/patient. A dosage for parenteral application, including intravenous, subcutaneous, intramuscular, and percutaneous injection is between 0.1 and 100 mg/day/patient.

## EXAMPLES

The present invention will now be described in more detail through the following examples. However, the present invention is not limited to these examples. In the following examples, compound numbers labeled for the respective compounds correspond to the compound numbers labeled for the compounds listed in the above Table 1 as specific examples.

Note that, with regard to data for compounds synthesized in the following examples, the term "HPLC retention time" refers to a retention time (unit: min) associated with a particular compound in HPLC analysis performed under the following analysis condition.

HPLC (High performance liquid chromatography) Analysis Condition

System: Hewlett-Packard 1100 HPLC

Column: Cadenza CD-C18 (manufactured by Imtakt Co.) 100 mm × 4.6 mmϕ

Solvent A:
$H_2O$/acetonitrile=95/5 (0.05% trifluoroacetic acid)
Solvent B:
$H_2O$/acetonitrile=5/95 (0.05% trifluoroacetic acid)
Flow rate: 1.0 mL/min
Gradient:
0-1 min Solvent B: 10%, Solvent A: 90%
1-14 min Solvent B: 10% → 100%, Solvent A: 90% → 0%
14-16 min Solvent B: 100%, Solvent A: 0%
Calculation of the purity: Area percentage at UV absorption (254 nm)

Reference Example 1. Synthesis of (cyclopropylhydroxymethylene)methane-1,1-dicarbonitrile

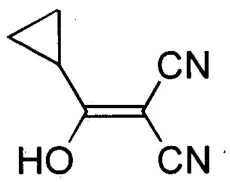

A tetrahydrofuran (150 mL) suspension of sodium hydride (11.49 g) was cooled to 0°C. To the cooled suspension was added dropwise a tetrahydrofuran (50 mL) solution of malononitrile (15.8 g) over an hour. The reaction mixture was stirred at room temperature for 1 hour and cooled to 0°C. To the reaction mixture was added dropwise over 80 minutes a tetrahydrofuran (50 mL) solution of.cyclopropylcarbonyl chloride (25.0 g). The reaction mixture was stirred at room temperature for 49 hours, followed by adding water (50 mL) to the reaction solution. The solvent was distilled off under reduced pressure. To the residue were added ethyl acetate (200 mL) and hydrochloric acid (270 mL, 1 mol/L), which was extracted with ethyl acetate. The organic layer was washed with saturated brine and dried over anhydrous sodium sulfate, and then the solvent was distilled off under reduced pressure to obtain a crude product (40.9 g) of the title compound. The NMR data of the compound is given below.
$^1$H-NMR(400 MHz, $CDCl_3$)δ(ppm): 1.10-1.22(m, 4H), 2.10-2.22(m, 1H), 4.27(s, 3H).

In a similar manner as described above, [(3-chloro(2-thienyl))hydroxymethylene]methane-1,1-dicarbonitrile was prepared from malononitrile and 3-chlorothiophene-2-carbonylchloride. The NMR data and ESI/MS data of the compound are given below. $^1$H-NMR(400 MHz, $CD_3OD$)δ(ppm): 6.92(d,J=5.1,1H), 7.51(d,J=5.4,1H)
ESI/MS m/e: ($M^+$+H, $C_8H_3ClN_2OS$)

Reference Example 2. Synthesis of (cyclopropylmethoxymethylene)methane-1,1-dicarbonitrile

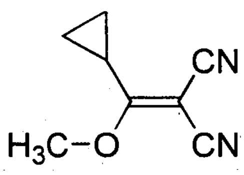

A tetrahydrofuran (100 mL) suspension of sodium hydride (2.6 g) was cooled to 0°C. To the cooled suspension was added dropwise a tetrahydrofuran (60 mL) solution of crude (1-hydroxy-2-phenylmethylidene)methane-1,1-dicarbonitrile (14.5 g) over 30 minutes. The reaction mixture was stirred at room temperature for 20 minutes and cooled to 0°C. To the reaction mixture was added dropwise a tetrahydrofuran solution (40 mL) of dimethyl sulfate (13.7 g) over 1

hour. After heating for 21 hours to reflux, the reaction mixture was cooled to room temperature, and the solvent was distilled off under reduced pressure. To the residue were added ethyl acetate (100 mL) and saturated sodium hydrogen carbonate solution (100 mL), and extraction with ethyl acetate was performed. The organic layer was washed with saturated brine and dried over anhydrous sodium sulfate, and then the solvent was distilled off under reduced pressure. The obtained crude product was purified by column chromatography on silica gel using hexane/ethyl acetate=1/3 as an eluent to obtain the title compound (6.8 g, 54%) as a light yellow solid. NMR data of the compound is given below.
$^1$H-NMR(400MHz, $CDCl_3$)δ(ppm): 1.10-1.22(m,4H), 2.10-2.22(m,1H), 4.27(s,3H).

Reference Example 3. Synthesis of methyl 3-amino-1-{2-[(t-butoxy)carbonylamino]ethyl}-4-cyano-5-cyclopropylpyrrole-2-carboxylate

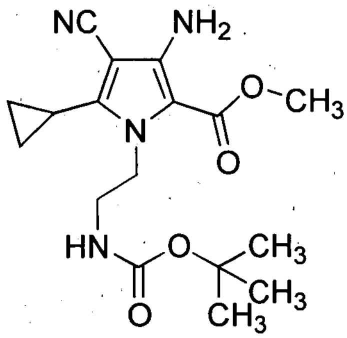

To an acetonitrile (150 mL) solution of (methoxycyclopropylmethylene)methane-1,1-dicarbonitrile (8.7 g) was added N-(2-aminoethyl) t-butyl carbaminic acid (16.3 g) and stirred at room temperature for 10 minutes. To the resultant product were added anhydrous cesium carbonate (38.5 g) and methyl bromoacetate (11.2 mL), followed by heating for 6 hours to reflux. The reaction product was cooled to room temperature and allowed to stand. Then, the supernatant was separated by decantation and the solvent was distilled off under reduced pressure. The concentrated residue and a solid remaining after decantation were collected and ethyl acetate and water were added thereto, followed by extracting 3 times with ethyl acetate. The organic phase was washed with water and saturated brine, and dried over anhydrous magnesium sulfate. After magnesium sulfate was removed by filtration, the solvent was distilled off under reduced pressure. The residue was purified by column chromatography on silica gel (hexane/ethyl acetate=2/1) to obtain the title compound (17.5 g, yield 85%). The ESI/MS data of the compound are given below. ESI/MS m/e: 349.1 ($M^+$+H, $C_{17}H_{24}N_4O_4$)

Methyl-3-amino-1-{2-[(t-butoxy)carbonylamino}ethyl}-5-(3-chloro(2-thienyl))-4-cyanopyrrole-2-carboxylate was synthesized from (3-chloro(2-thienyl))hydroxymethylene] methane-1,1-dicarbonitrile used as a starting material in a similar manner to that in Reference Examples 2 and 3. The ESI/MS data of the compound are given below.
ESI/MS m/e: 425.2($M^+$+H, $C_{18}H_{21}ClN_4O_4S$)

Reference Example 4. Synthesis of (t-butoxy)-N-[2-(7-cyano-4- oxo-6-cyclopropyl(3-hydropyrrolo[3,2-, d]pyrimidin-5-yl))ethyl]carboxyamide

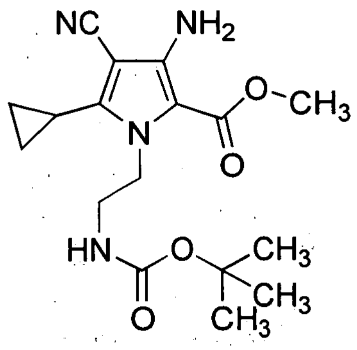

Methyl-3-amino-1-{2-[(t-butoxy)carbonylamino]ethyl}-4-cyano-5-cyclopropylpyrrole-2-carboxylate (17.4 g) and formamidine acetate (104.1 g) were added to 2-propanol (360 mL) and heated for 45 hours to reflux. The reaction mixture was cooled to room temperature, and the solvent was distilled off under reduced pressure. To the residue was added water, and the obtained solid was isolated by filtration and sufficiently washed with water. The resulting solid was recrystallized (ethanol/ethyl acetate/hexane=1/2/1) to obtain the title compound (9.8 g, yield 57%) as a white solid. The ESI/MS data of the compound are given below.
ESI/MS m/e: 362.1($M^{+}$+H, $C_{17}H_{21}N_5O_3$)

(t-butoxy)-N-{2-[6-(3-chloro(2-thienyl))-7-cyano-4-oxo(3-hydropyrrolo[3,2-d]pyrimidin-5-yl)]ethyl}carboxyamide was prepared from methyl-3-amino-1-{2-[(t-butoxy) carbonylamino]ethyl}-5-(3-chloro(2-thienyl))-4-cyanopyrrole-2-carboxylate in a similar manner to that described above. ESI/MS data of the compound are given below.
ESI/MS m/e: 420.2 ($M^{+}$+H, $C_{18}H_{18}ClN_5O_3S$)

Reference Example 5. Synthesis of 5-{2-[(t-butoxy)carbonylamino]ethyl}-6-(3-chloro(2-thienyl))-4-oxo-3-hydropyrrolo

[3,2-d]pyrimidin-7-carboxyamide

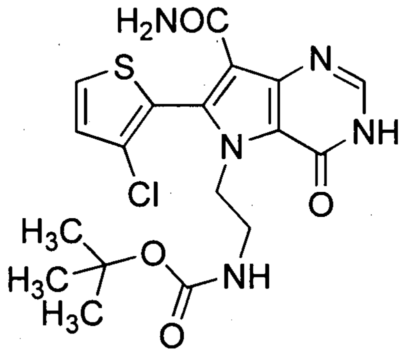

(t-butoxy)-N-{2-[6-(3-chloro(2-thienyl))-7-cyano-4-oxo(3-hydropyrrolo[3,2-d]pyrimidin-5-yl)]ethyl}carboxyamide (3.0 g) was dissolved in ethanol (100 mL), and a 5M aqueous sodium hydroxide solution (20 mL) was added thereto. A 30% hydrogen peroxide solution (30 mL) was added to the reaction mixture over 20 minutes with stirring. After stirring at 45 to 50°C for 24 hours, 30% hydrogen peroxide solution (20 mL) was added to the reaction solution, stirred at 45 to 50°C for 24 hours, concentrated and neutralized with 1 M hydrochloric acid, to obtain a white precipitate. The precipitate was filtered, washed, and dried under reduced pressure to obtain the title compound (2.68 g, yield 86%). The HPLC retention time, NMR data and ESI/MS data of the compound are given below. HPLC retention time=7.2(min)
$^{1}$H-NMR (270 MHz, DMSO-$d_6$)δ(ppm): 1.26(s,9H), 3.2-3.5(m,2H), 3.8-4.0(m,1H), 4.4-4.6(m,2H),6.5-6.6(m,1H), 7.17 (d,1H,J=4.6Hz), 7.2-7.3(m,1H), 7.91(d,1H,J=5.4Hz), 8.0-8.1(m,1H), 12.4-12.5(m,1H).
ESI/MS m/e: 438.3($M^{+}$+H, $C_{18}H_{20}ClN_5O_4S$

Reference Example 6. Synthesis of 5-{2-[(t-butoxy) carbonylamino]ethyl}-6-cyclopropyl-4-oxo-3-hydropyrrolo[3,2-d]pyrimidin-7-carboxyamide

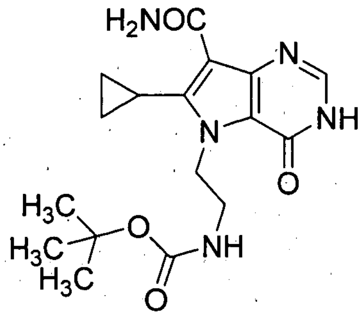

The title compound was prepared from (t-butoxy)-N-{2-[6- cyclopropyl-7-cyano-4-oxo(3-hydropyrrolo[3,2-d] pyrimidin-5-yl)]ethyl}carboxyamide in a similar manner to that described in Reference Example 5. The ESI/MS data of the compound are given below.
ESI/MS:m/e 362.1($M^{+}$+H, $C_{17}H_{23}N_5O_4$)

Reference Example 7. Synthesis of N-{2-{7-amino-6-(3-chloro (2-thienyl))-4-oxo(3-hydropyrrolo[3,2-d]pyrimidin-5-yl)] ethyl}(t-butoxy)carboxyamide

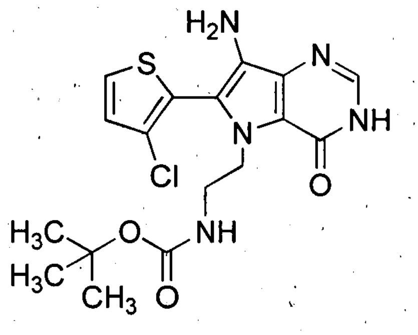

5-{2-[(t-butoxy) carbonylamino] ethyl}-6-(3-chloro (2-thienyl)) 4-oxo-3-hydropyrrolo [3,2-d] pyrimidine-7-carboxyamide (110 mg) was suspended in a 1 M aqueous sodium hydroxide solution (7.5 mL), and benzyltrimethylammonium tribromide (135 mg) was added thereto and the mixture was stirred for 1.5 hours. 1 M hydrochloric acid was added to the reaction mixture to acidify the reaction system, and then washed with ethyl acetate. The aqueous layer was made alkaline with sodium hydrogen carbonate and extracted with ethyl acetate. The organic layer was washed with saturated brine, dried over anhydrous sodium sulfate, filtered, concentrated, and purified by column chromatography on silica gel (15 g) using 1:1 to 0:1 hexane:ethyl acetate as an eluent to obtain the title compound (72 mg, yield 70%). The NMR data and ESI/MS data of the compound are given below. HPLC retention time=6.4(min)
$^{1}$H-NMR(270MHz;$CDCl_3$)δ(ppm) : 1.32(s,9H), 3.3-3.5(m,2H), 4.3-4.5(m,2H), 4.9-5.0(m,1H), 7.11(d,1H,J=5.4Hz), 7.54 (d,1H,J=5.4Hz), 7.79 (brs,1H), 10.0-10.1(m,1H).
ESI/MS m/e: 410.3 ($M^{+}$+H, $C_{17}H_{20}ClN_5O_3S$)

Reference Example 8. Synthesis of N-{2-[7-amino-6-cyclopropyl-4-oxo(3-hydropyrrolo[3,2-d]pyrimidin-5-yl)]ethyl}(t-butoxy)carboxyamide

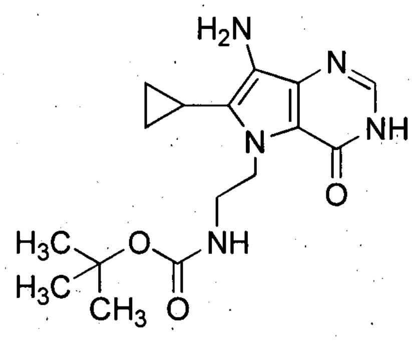

The title compound was prepared from 5-{2-[(t-butoxy) carbonylamino]ethyl}-6-cyclopropyl-4-oxo-3-hydropyrrolo[3,2-d]pyrimidin-7-carboxyamide in a similar manner to that described in Reference Example 7. The NMR data and ESI/MS data of the compound are given below. $^1$H-NMR(270MHz,$CDCl_3$)δ(ppm): 1.34(s,9H),0.8-1.2(m,5H), 3.4(brs, 2H), 3.5-3.6(m,3H), 4.5-4.6(m,2H), 5.6 (brs,1H), 7.8 (brs,1H)
ESI/MS m/e: 334.1($M^+$+H, $C_{16}H_{23}N_5O_3$)

Reference Example 9. Synthesis of (t-butoxy)-N-{2-[7-bromo- 6-(3-chloro(2-thienyl))-4-oxo(3-hydropyrrolo[3,2-d]pyrimidin-5-yl)]ethyl}carboxyamide

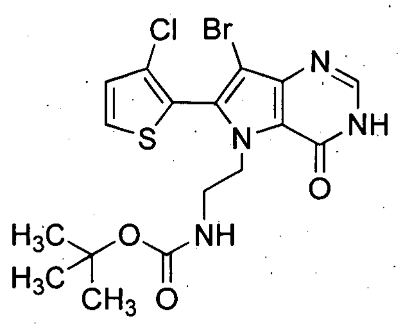

To 30 mg of N-{2-[7-amino-6-(3-chloro(2-thienyl))-4-oxo(3-hydrpyrrolo[3,2-d]pyrimidin-5-yl)]ethyl}(t-butoxy) carboxyamide, 1 ml of bromoform was added, followed by adding 50 μl of isoamylnitrite. After stirring at 70°C for 4 hours, saturated brine was added to the reaction solution, which was extracted with ethyl acetate. The organic layer was dried over anhydrous sodium sulfate, filtered, and concentrated, followed by subjecting to thin layer chromatography on silica gel using ethyl acetate as a developing solvent to obtain the title compound (15 mg, yield 43%). The HPLC retention time, NMR data and ESI/MS data of the compound are given below.
HPLC retention time=9.5(min)
$^1$H-NMR(270MHz,$CDCl_3$) δ (ppm): 1.31(s,9H), 3.3-3.5(m,2H), 4.2-4.3(m,1H), 4.5-4.7(m,1H), 4.7-4.9(m,1H), 7.13(d,1H, J=5.7Hz), 7.60(d,1H,J=5.4Hz), 7.94(d,1H,J=3.0Hz).
ESI/MS m/e 475.2 ($M^+$+H, $C_{17}H_{18}BrClN_4O_3S$)

Reference Example 10. Synthesis of (t-butoxy)-N-{2-[7-bromo- 6-cyclopropyl-4-oxo(3-hydropyrrolo[3,2-d]pyrimidin-5-yl)]ethyl}carboxyamide

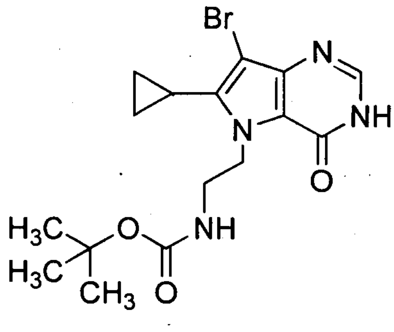

The title compound was prepared from N-{2-[7-amino-6- cyclopropyl-4-oxo(3-hydropyrrolo[3,2-d]pyrimidin-5-yl)]
ethyl}(t-butoxy)carboxyamide in a similar manner to that described in Reference Example 9. The HPLC retention time, NMR data and ESI/MS data of the compound are given below.
HPLC retention time=8.5(min)
$^1$H-NMR(270MHz,$CDCl_3$) δ (ppm): 1.31(s,9H), 1.1-1.3(m,5H), 3.5-3.9(m,3H), 4.5-4.9(m,2H), 5.1-5.3 (brs,1H), 7.9(s,1H)
ESI/MS m/e: 397.1 ($M^+$+H, $C_{16}H_{21}BrN_4O_2S$)

Reference Example 11. Synthesis of (t-butoxy)-N-{2-[6-(3-chloro(2-thienyl))-7-iodo-4-oxo(3-hydropyrrolo[3,2-d]pyrimidin-5-yl)]ethyl}carboxyamide

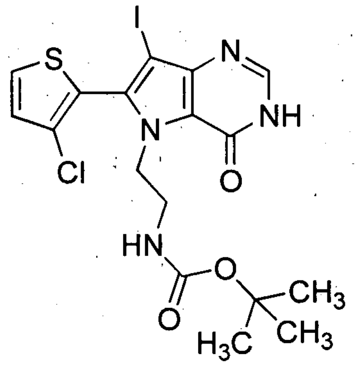

To 1 g of N-{2-[7-amino-6-(3-chloro(2-thienyl))-4-oxo (3-hydropyrrolo[3,2-d]pyrimidin-5-yl)]ethyl}(t-butoxy)carboxyamide, 7 mL of diiodomethane was added, followed by adding 822 μl of isoamyl nitrite. After stirring at 70°C for 4 hours, saturated brine was added to the reaction solution, which was extracted with ethyl acetate. The organic layer was dried over anhydrous sodium sulfate, filtered, and concentrated, followed by subjecting to chromatography on silica gel (using 5:1 to 0:1 hexane/ethyl acetate as an eluent), to yield the 694 mg of title compound (yield: 55%). NMR data and ESI/MS data of the compound are given below. HPLC retention time=9.6(min)
$^1$H-NMR(270MHz,$CDCl_3$) δ (ppm):1.32(s,9H), 3.3-3.5(m,2H), 4.2-4.35(m,1H), 4.6-4.75(m,1H), 4.8-5.0(m,1H), 7.14(d, 1H,J=5.4Hz), 7.59(d,1H,J=5.7Hz), 7.95-8.05 (m,1H).
ESI/MS m/e 521.3 ($M^+$+H, $C_{17}H_{18}ClIN_4O_3S$

Reference Example 12. Synthesis of (t-butoxy-N-{2-[7-iodo-6-cyclopropyl-4-oxo(3-hydropyrrolo[3,2-d]pyrimidin-5-yl)} ethyl)carboxyamide

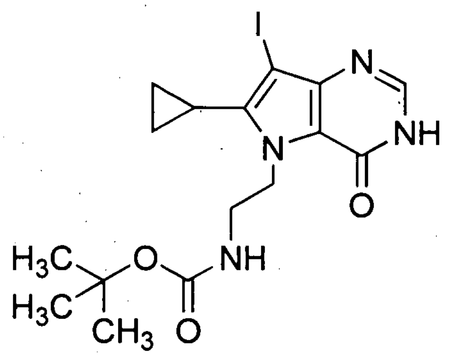

The title compound was prepared from N-(2-[7-amino-6-cyclopropyl]-4-oxo(3-hydropyrrolo[3,2-d]pyrimidin-5-yl)}ethyl(t-butoxy)carboxyamide in a similar manner to that described in Reference Example 11. The HPLC retention time, NMR data and ESI/MS data of the compound are given below.
HPLC retention time=8.7(min)
$^1$H-NMR(270MHz,$CDCl_3$) δ (ppm): 1.31(s,9H), 1.0-1.3(m,5H), 3.5-3.8(m,3H), 4.7-4.9(m,2H), 5.2-5.3 (brs,IH), 7.9(s,1H)
ESI/MS m/e: 445.4 ($M^+$+H, $C_{16}H_{21}IN_4O_3$)

Reference Example 13. Synthesis of (t-butoxy)-N-{2-[7-chloro-6-(3-chloro(2-thienyl))-4-oxo(3-hydropyrrolo[3,2-d]pyrimidin-5-yl)]ethyl}carboxyamide

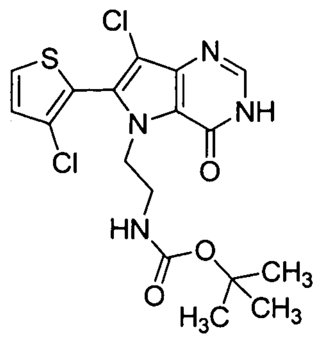

To 20 mg of N-{2-[7-amino-6-(3-chloro(2-thienyl))-4-oxo(3-hydropyrrolo[3,2-d]pyrimidin-5-yl)]ethyl}(t-butoxy) carboxyamide, 2 ml of carbon tetrachloride was added, followed by adding 34 μl of isoamyl nitrite. After refluxing for 40 hours, saturated brine was added to the reaction solution, which was extracted with ethyl acetate. The organic layer was dried over anhydrous sodium sulfate, filtered, and concentrated, followed by subjecting to thin layer chromatography on silica gel (using ethyl acetate as a developing solvent) to obtain 5 mg of the title compound (yield: 24%). The HPLC retention time, NMR data and ESI/MS data of the compound are given below.
HPLC retention time=9.4(min)
$^1$H-NMR (270MHz, $CDCl_3$) δ (ppm): 1.32 (s, 9H), 3.3-3.5(m,2H), 4.15-4.4(m,1H), 4.6-4.75(m,1H), 4.8-4.95(m,1H), 7.13 (d,1H,J=5.4Hz), 7.60(d,1H,J=5.4Hz), 7.98 (brs,1H).
ESI/MS m/e 429.4 ($M^+$+H, $C_{17}H_{18}Cl_2N_4O_3S$)

Reference Example 14. Synthesis of 5-(2-aminoethyl)-6-(3-chloro(2-thienyl))-7-iodo-3-hydropyrrolo[3,2-d]pyrimidin-4-one hydrochloride

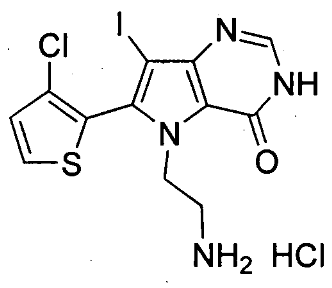

(t-butoxy)-N-{2-[6-(3-chloro(2-thienyl))-7-iodo-4-oxo(3-hydropyrrolo[3,2-d]pyrimidin-5-yl)]ethyl}carboxyamide (331 mg) was dissolved in a mixed solution of methanol(0.5 mL) and 1,4-dioxane(5.0 mL), and hydrochloric acid/1,4-dioxane solution (4 mol/L, 0.64 mL) was added and the mixture was stirred at 60°C for 2 hours. The reaction mixture was cooled to room temperature, and the solvent was distilled off under reduced pressure. The residue was dried in vacuo to obtain the title compound (334 mg in a quantitative yield) as a colorless solid. The ESI/MS data of the compound are given below.
ESI/MS m/e: 421.2($M^{+}$+H, $C_{12}H_{10}ClN_4OS$ HC1)

Reference Example 15. Synthesis of 5-(2-aminoethyl)-7-bromo- 6-(3-chloro(2-thienyl))-3-hydropyrrolo[3,2-d]pyrimidin-4-one hydrochloride

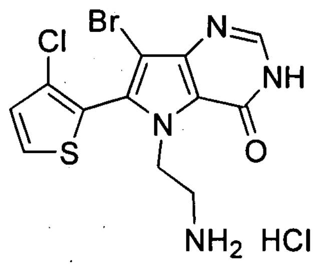

The title compound was prepared from (t-butoxy)-N-{2- [7-bromo-6-(3-chloro(2-thienyl))-4-oxo(3-hydropyrrolo [3,2-d]pyrimidin-5-yl)]ethyl}carboxyamide in a similar manner to that described in Reference Example 14. The ESI/MS data of the compound are given below.
ESI/MS m/e: 375.0($M^{+}$+H, $C_{12}H_{10}BrClN_4OS$ HCl)

Reference Example 16. Synthesis of 5-(2-aminoethyl)-7-chloro- 6-(3-chloro(2-thienyl))-3-hydropyrrolo[3,2-d]pyrimidin-4-one hydrochloride

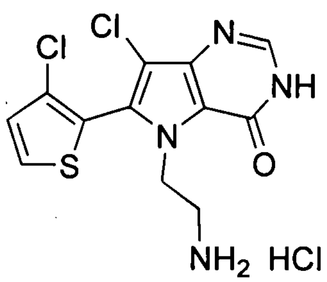

The title compound was prepared from (t-butoxy)-N-{2-[7-chloro-6-(3-chloro(2-thienyl))-4-oxo(3-hydropyrrolo [3,2-d]pyrimidin-5-yl)]ethyl}carboxyamide in a similar manner to that described in Reference Example 14. The ESI/MS data of the compound are given below. ESI/MS m/e: 329.4 ($M^{+}$+H, $C_{12}H_{10}Cl_2N_4OS$ HCl)

Reference Example 17. Synthesis of N-{2-[6-(3-chloro(2-thienyl))-7-iodo-4-oxo(3-hydropyrrolo[3,2-d]pyrimidin-5-yl)] ethyl}-2,2,2-trifluoroacetamide

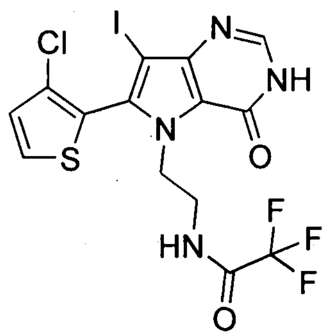

To a tetrahydrofuran (5.0 mL) solution of 5-(2-aminoethyl)-6-(3-chloro(2-thienyl))-7-iodo-3-hydropyrrolo[3,2-d] pyrimidin-4-one hydrochloride(259 mg) was added trifluoroacetic anhydride (595 mg), and triethylamine (1.2 mL) was added slowly dropwise. The reaction mixture was stirred at room temperature for 2 hours, and methanol was added to stop the reaction. The solvent was distilled off under reduced pressure. To the residue were added water and ethyl acetate, which was extracted 3 times with ethyl acetate. The organic layer was washed with saturated brine, and dried over anhydrous magnesium sulfate. After anhydrous magnesium sulfate was filtered off, the solvent was distilled off under reduced pressure. The residue was dried in vacuo to obtain a crude product (365 mg) of the title compound as a light yellow solid. The HPLC retention time, NMR data and ESI/MS data of the compound are given below.
HPLC retention time=9.1(min)
$^1$H-NMR(400 MHz, DMSO-$d_6$) δ (ppm): 3.45(m,2H), 4.16(m,1H), 4.61(m,1H), 7.30(m,1H), 7.93(m,1H), 8.02(m,1H), 9.37 (m,1H), 12.29 (brs,1H).
ESI/MS m/e: 517.2 ($M^+$+H, $C_{19}H_9ClF_3IN_4O_2S$)

Reference Example 18. Synthesis of N-{2-[7-bromo-6-(3-chloro(2-thienyl))-4-oxo(3-hydropyrrolo[3,2-d]pyrimidin-5-yl)] ethyl}-2,2,2-trifluoroacetamide

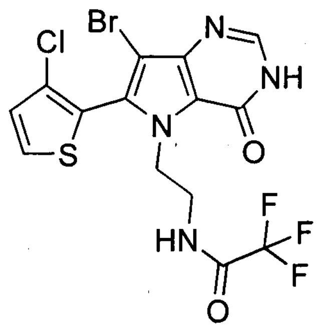

The title compound was prepared from 5-(2-aminoethyl)- 7-bromo-6-(3-chloro(2-thienyl))-3-hydropyrrolo[3,2-d]pyrimidin-4-one hydrochloride in a similar manner to that described in Reference Example 17. The ESI/MS data of the compound are given below.
ESI/MS m/e: 471.1 ($M^+$+H, $C_{14}H_9BrClF_3N_4O_2S$)

Reference Example 19. Synthesis of N-{2-[7-chloro-6-(3-chloro(2-thienyl))-4-oxo(3-hydropyrrolo[3,2-d]pyrimidin-5-yl)] ethyl}-2,2,2-trifluoroacetamide

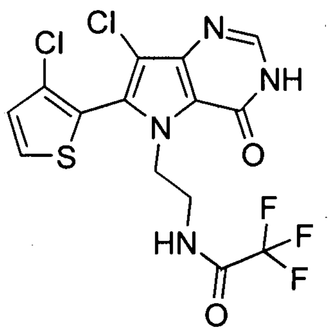

The title compound was prepared from 5-(2-aminoethyl)- 7-chloro-6-(3-chloro(2-thienyl))-3-hydropyrrolo[3,2-d] pyrimidin-4-one hydrochloride in a similar manner to that described in Reference Example 17. The ESI/MS data of the compound are given below.
ESI/MS m/e: 425.4 ($M^{+}$+H, $C_{14}H_9Cl_2F_3N_4O_2S$)

Reference Example 20. Synthesis of N-(5-{2-[(t-butoxy) carbonylamino]ethyl}-6-(3-chloro(2-thienyl))-4-oxo(3-hydropyrrolo[3,2-d]pyrimidin-7-yl))-2,2,2-trifluoroacetamide

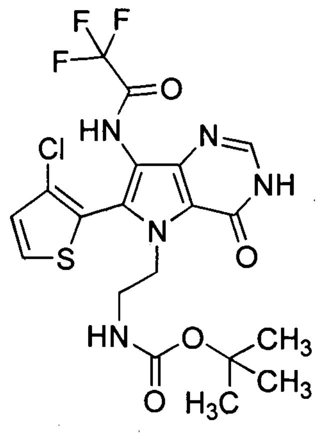

To a tetrahydrofuran solution (39 mL) of N-{2-[7-amino-6-(3-chloro(2-thienyl))-4-oxo(3-hydropyrrolo [3,2-d]pyrimidin-5-yl)]ethyl}(t-butoxy)carboxyamide (1.60 g) was added triethylamine (2.7 mL), and cooled to 0°C, followed by adding trifluoroacetic anhydride (1.35 mL) slowly dropwise. The reaction mixture was stirred at room temperature for 1 hour, and saturated brine was added dropwise to stop the reaction. Ethyl acetate was added to the reaction solution for extraction. The organic layer was washed with saturated brine, dried over anhydrous magnesium sulfate and filtered. The solvent was distilled off under reduced pressure to obtain the title compound (1.97 g, a quantitative yield) as a light yellow solid. The HPLC retention time, NMR data and ESI/MS data of the compound are given below. HPLC retention time=8.7(min)
$^{1}$H-NMR(400 MHz, DMSO-$d_6$) δ(ppm): 1.26(s,9H), 3.13-3.26(m,2H), 4.11 (brs,1H), 4.49 (brs,1H),6.60-6.73(m,1H), 7.23 (d,J=5.4,1H), 7.88(s,1H), 7.95(d,J=5.4,1H), 10.86(s,1H), 12.21 (brs,1H).
ESI/MS m/e: 506.4 ($M^{+}$+H, $C_{19}H_{19}ClF_3N_5O_4S$)

Reference Example 21. Synthesis of N-[5-(2-aminoethyl)-6-(3- chloro(2-thienyl))-4-oxo(3-hydropyrrolo[3,2-d]pyrimidin-7-yl)]-2,2,2-trifluoroacetamide hydrochloride

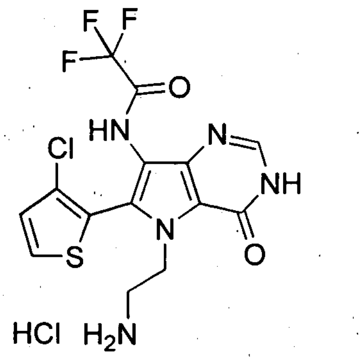

A methanol solution (13 mL) of N-(5-{2-[(t-butoxy) carbonylamino]ethyl}-6-(3-chloro(2-thienyl))-4-oxo(3-hydropyrrolo[3,2-d]pyrimidin-7-yl))-2,2,2-trifluoroacetamide (1.97 g) was cooled to 0°C and added was 4 mol/L hydrochloric acid/1,4-dioxane solution (26 mL), followed by stirring at room temperature for 4 hours. The solvent was distilled off under reduced pressure, yielding a crude product (1.73 g) of the title compound as a brown solid. The ESI/MS data of the compound are given below.
ESI/MS m/e: 406.3 ($M^{+}$+H, $C_{14}H_{11}F_{3}N_{5}O_{2}S{\cdot}HCl$)

Reference Example 22. Synthesis of N-(6-(3-chloro(2-thienyl))-5-{2-[(4-fluorophenyl)carbonylamino]ethyl}-4-oxo(3-hydropyrrolo[3,2-d]pyrimidin-7-yl))-2,2,2-trifluoroacetamide

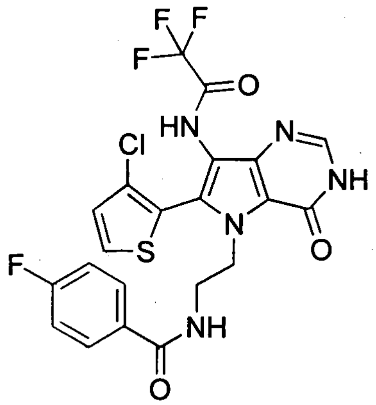

N-[5-(2-aminoethyl)-6-(3-chloro(2-thienyl))-4-oxo(3-hydropyrrolo[3,2-d]pyrimidin-7-yl)]-2,2,2-trifluoroacetamide crude product (1.73g) in pyridine (39 mL) was cooled to 0°C, and 4-fluorobenzoylchloride (0.92 mL) was added thereto, followed by stirring at'room temperature for 1 hour. To the reaction solution was added water (40 mL), stirred at room temperature for 1 hour, and to stop the reaction. Brine was added until the reaction solution was saturated, and extraction with ethyl acetate was performed. The organic layer was washed with a 9:1 mixed solution of saturated brine and hydrochloric acid (1 mol/L), dried over anhydrous sodium sulfate and filtered. The solvent was distilled off under reduced pressure and purified by column chromatography on silica gel using 1/2 hexane/ethyl acetate and then using ethyl acetate only as eluents to obtain the title compound (1.60 g, 78% for 2 steps) as a white solid. The HPLC retention time, NMR data and ESI/MS data of the compound are given below.
HPLC retention time=8.3(min) $^{1}$H-NMR(400 MHz, DMSO-$d_6$) δ (ppm): 3.49-3.58(m,2H), 4.34(brs,1H), 4.66(brs,1H), 7.16 (d,J=5.4,1H), 7.19(t,J=7.1,2H), 7.65-7.75(m,2H), 7.83(d,J=5.4,1H), 7.88(s,1H), 8.40-8.50(m,1H), 10.85(s,1H), 12.25 (brs,1H).
ESI/MS m/e: 528.4 ($M^{+}$+H, $C_{21}H_{14}ClF_{4}N_{5}O_{3}S$)

Reference Example 23. Synthesis of N-(2-{7-[(1E)-1-aza-2-(dimethylamino)vinyl]-4-chloro-6-(3-chloro(2-thienyl)) pyrrolo[3,2-d]pyrimidin-5-yl}ethyl)(4-fluorophenyl) carboxyamide

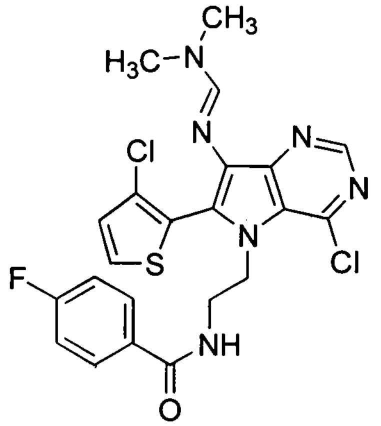

N-(6-(3-chloro(2-thienyl))-5-{2-[(4-fluorophenyl) carbonylamino]ethyl}-4-oxo(3-hydropyrrolo[3,2-d]pyrimidin-7-yl))-2,2,2-trifluoroacetamide (1.09 g) was dissolved in DMF(0.16 g) and phosphorus oxychloride (30 mL), and the reaction solution was stirred at 100°C for 1 hour. The reaction mixture was cooled to room temperature, and excess phosphorus oxychloride was distilled off under reduced pressure. To the residue were added ethyl acetate and aqueous saturated sodium hydrogen carbonate solution, and the mixture was stirred at room temperature for 30 minutes. The organic layer was separated, and the aqueous layer was extracted with ethyl acetate. The combined organic layer was washed with saturated brine, dried over anhydrous sodium sulfate and filtered. The solvent was distilled off under reduced pressure, yielding a crude product (0.87 g) of the title compound. NMR data and ESI/MS data of the compound are given below.
$^1$H-NMR(400 MHz, DMSO-$d_6$) δ (ppm): 2.81(brs,1H), 2.97(brs,1H), 3.28(s,2H), 3.37-3.45(m,2H), 7.17-7.27(m,3H), 7.55-7.68(m,2H), 7.86(d,J=5.4,1H), 8.31-8.38(m,1H), 8.61(s,1H), 8.75(s,1H).
ESI/MS m/e: 505.4($M^+$+H, $C_{22}H_{19}Cl_2FN_6OS$)

Reference Example 24. Synthesis of (t-butoxy)-N-{2-[6-(3-chloro(2-thienyl))-4-oxo-7-(phenylcarbonylamino)(3-hydropyrrolo[3,2-d]pyrimidin-5-yl)]ethyl}carboxyamide

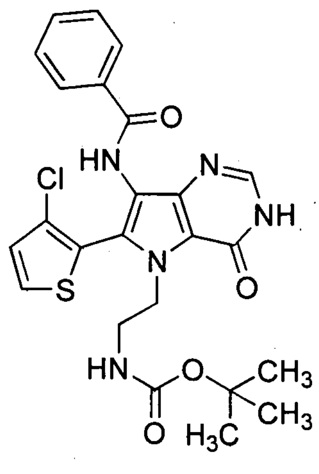

To a tetrahydrofuran solution (10 mL) of N-{2-[7-amino-6-(3-chloro(2-thienyl))-4-oxo(3-hydropyrrolo[3,2-d]pyrimidin-5-yl)]ethyl}(t-butoxy)carboxyamide (0.41 g) was added triethylamine (0.69 mL), and the reaction mixture was cooled to 0°C and benzoylchloride (0.29 mL) was added slowly dropwise. The reaction mixture was stirred at room temperature for 1 hour, and an aqueous sodium hydroxide solution (2 mol/L, 2.0 mL) was added dropwise and stirred for 18 hours to stop the reaction. Hydrochloric acid (1 mol/L, 4.0 mL) was added to the reaction solution for neutralization, brine was added thereto until the reaction solution was saturated, and extraction with ethyl acetate was performed. The organic layer was washed with saturated brine, dried over anhydrous sodium sulfate and filtered. The solvent was distilled off under reduced pressure, and purified by column chromatography was performed on silica gel using ethyl acetate only to obtain the title compound (0.51 g, a quantitative yield) as a light yellow solid. The ESI/MS data of the compound

are given below.
ESI/MS m/e: 514.4($M^{+}$+H, $C_{24}H_{24}ClN_5O_4S$)

Reference Example 25. Synthesis of N-[5-(2-aminoethyl)-6-(3- chloro(2-thienyl))-4-oxo(3-hydropyrrolo[3,2-d]pyrimidin-7-yl)]benzamide hydrochloride

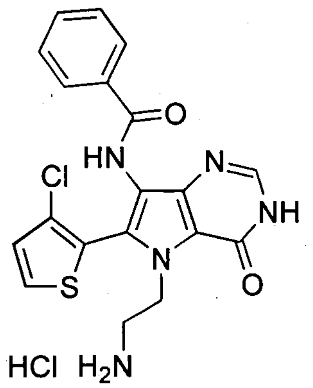

To a methanol solution (3.3 mL) of (t-butoxy)-N-{2-[6-(3-chloro(2-thienyl))-4-oxo-7-(phenylcarbonylamino)(3-hydropyrrolo[3,2-d]pyrimidin-5-yl)]ethyl}carboxyamide (0.51 g) was added 4 mol/L hydrochloric acid/1,4-dioxane solution (6.6 mL) and the mixture was stirred at room temperature for 5 hours. The solvent was distilled off under reduced pressure, yielding a crude product (0.47 g) of the title compound as a brown solid. The ESI/MS data of the compound are given below.
ESI/MS m/e: 414.3 ($M^{+}$+H, $C_{19}H_{16}ClN_5O_2 \cdot HCl$)

Reference Example 26. Synthesis of N-{6-(3-chloro(2-thienyl))-4-oxo-5-[2-(quinazolin-4-ylamino)ethyl](3-hydropyrrolo [3,2-d]pyrimidin-7-yl)}benzamide

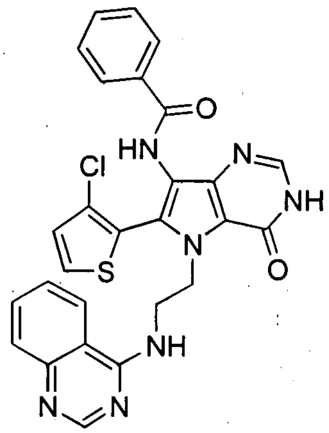

N-[5-(aminoethyl)-6-(3-chloro(2-thienyl))-4-oxo(3-hydropyrrolo[3,2-d]pyrimidin-7-yl)]benzamide (45 mg) and 4-chloroquinazoline (16.5 mg) were dissolved in dimethylacetamide (2.9 mL), and triethylamine (27.7 μL) was added thereto and the mixture was stirred at 70°C for 3 hours. Triethylamine (13.9 μL) was further added to the reaction mixture and the solution was stirred at 70°C for 5 hours. The reaction solution was cooled to room temperature and purified by fraction HPLC, to obtain the title compound (44.3 mg, 82%) as a white solid. The HPLC retention time, NMR data and ESI/MS data of the compound are given below.
HPLC retention time=6.4(min)
$^{1}$H-NMR(400 MHz, DMSO-$d_6$) δ(ppm): 3.95-4.05(m,2H), 4.52(brs,1H), 5.00(brs,1H), 7.05(d,J=5.4,1H), 7.43(t,J=7.6,2H),

7.52(t,J=7.3,1H), 7.65(d,J=5.4,1H), 7.70-7.85(m,5H), 8.02(t,J=7.8,1H), 8.23(d,J=8.5,1H), 8.61(s,1H),9.66(s,1H), 10.17 (m,1H), 12.10(brs,1H).
ESI/MS m/e: 542.4 ($M^{+}$+H, $C_{27}H_{20}ClN_{7}O_{2}S$)

Reference Example 27. Synthesis of N-{2-[7-pyridine-3-yl-6- (3-chloro(2-thienyl))-4-oxo(3-hydropyrrolo[3,2-d]pyrimidin-5-yl)ethyl](tert-butoxy)carboxyamide

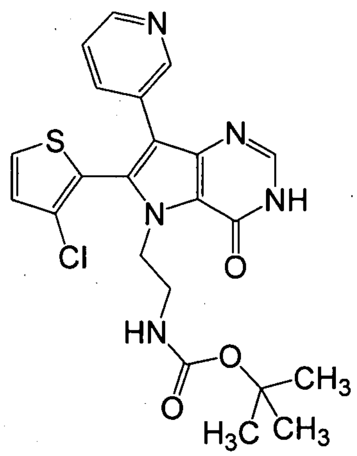

A round-bottom flask was charged with 2.6 g of (tert-butoxy)-N-{2-[6-(3-chloro(2-thienyl))-4-oxo(-7-iodo(3-hydropyrrolo[3,2-d]pyrimidin-5-yl)]ethyl} carboxyamide, 224 mg of palladium acetate, 524 mg of triphenyl phosphine, 2.65 g of sodium carbonate, and 1.23 g of 3-pyridyl borate, substituted with nitrogen, and then added with 100 ml of dimethylformamide: water = 2:1 solution. The reaction mixture was stirred for 24 hours at 80°C. A saturated brine was added thereto, followed by extraction with ethyl acetate, the organic layer was concentrated, and then purified by column chromatography on silica gel (developing solvent: ethyl acetate, and then ethyl acetate:methanol = 20:1), the ethyl acetate/methanol dissolved portion was concentrated to obtain the title compound (1.68 g, yield of 71%). NSI/MS data of the compound is given below.
ESI/MS m/e: 472.0(M+$H^{+}$, $C_{22}H_{22}ClN_{5}O_{3}S$)

Reference Example 28. Synthesis of 5-(2-aminoethyl)-6-(3-chlorothiophen-2-yl)-7-pyridin-3-yl-3-hydropyrrolo[3,2-d] pyrimidin-4-onedihydrochloride

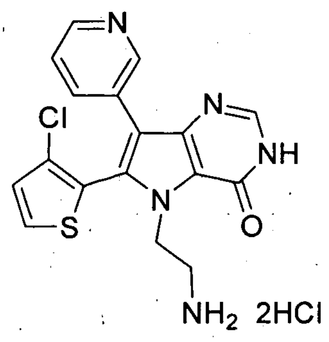

(t-butoxy)-N-{2-[6-(3-chlorothiophen-2-yl)-7-pyridin-3-yl-4-oxo-3-hydropyrrolo[3,2-d]pyrimidin-5-yl]-ethyl} carboxyamide(1.68 g) was dissolved in methanol(10.0 mL), followed by adding 4 mol/L hydrochloric acid/1,4-dioxane solution (2.0 mL) thereto and stirring at 60°C for 12 hours. The reaction mixture was cooled to room temperature, and the solvent was distilled off under reduced pressure. The residue was dried in vacuo to obtain the title compound (1.64 g, a quantitative yield) as a colorless solid. The ESI/MS data of the compound are given below.

ESI/MS m/e: 371.9($M^+$+H, $C_{17}H_{14}ClN_5OS$ 2HCl)

Example 1. Synthesis of 5-(2-aminoethyl)-6-(3-chloro(2-thienyl))-7-iodo-3-hydropyrrolo[3,2-d]pyrimidin-4-thione trifluoroacetate (Compound No. 41)

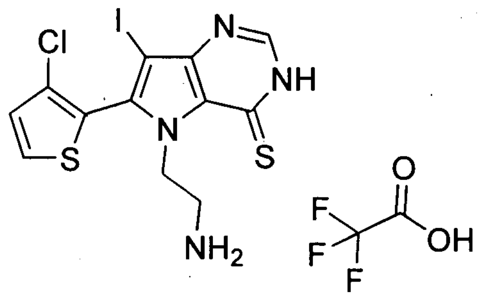

To a 1,4-dioxane (5.0 mL) and 2-propanol(1.0 mL) solution of a crude product of N-{2-[4-chloro-6-(3-chloro(2-thienyl))-7-iodopyrrolo[3,2-d]pyrimidin-5-yl]ethyl}-2,2,2-trifluoroacetamide, was added a thiourea (98 mg), and the mixture was stirred at 80°C for 30 minutes. The reaction mixture was cooled to room temperature, and water was added and extracted 3 times with ethyl acetate. The organic phase was washed with saturated brine, and dried over anhydrous magnesium sulfate. After anhydrous magnesium sulfate was filtered off, the solvent was distilled off under reduced pressure. A brown oily compound obtained by drying the residue in vacuo was dissolved in 1,4-dioxane (4.0 mL). An aqueous sodium hydroxide solution (5 mol/L, 0.2 mL) was added to the resulting solution and stirred at room temperature for 1 hour, followed by further adding an aqueous sodium hydroxide solution (5 mol/L, 0.3 mL) and stirring at room temperature for 2 hours. To the reaction mixture was added acetic acid for neutralization. The solvent was distilled off under reduced pressure, and the residue was purified by fraction HPLC to obtain the title compound (180 mg, 51% yield of Example 17) as a light brown solid. The HPLC retention time, NMR data and ESI/MS data of the compound are given below.
HPLC retention time=6.4(min)
$^1$H-NMR(400 MHz, DMSO-$d_6$) δ(ppm): 3.10(brs,2H), 4.68(m,1H), 5.17(m,1H), 7.37(d,J=5.4,1H), 7.91(brs,2H), 8.09(d, J=5.4,1H), 8.15(s,1H), 13.73(brs,1H).
ESI/MS m/e: 437.2($M^+$+H, $C_{12}H_{10}ClIN_4S_2$ $C_2HF_3O_2$)

Example 2. Synthesis of 5-(2-aminoethyl)-7-bromo-6-(3-chloro(2-thienyl))-3-hydropyrrolo[3,2-d]pyrimidin-4-thione trifluoroacetate (Compound No. 40)

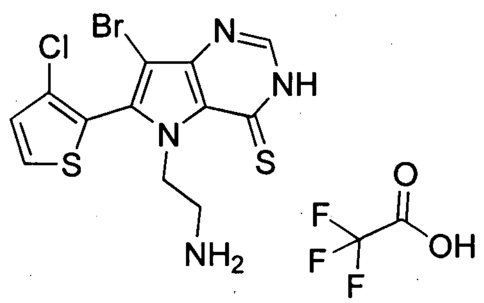

The title compound was prepared from a crude product of N-{2-[7-bromo-4-chloro-6-(3-chloro(2-thienyl))pyrrolo [3,2-d]pyrimidin-5-yl]ethyl}-2,2,2-trifluoroacetamide in a similar manner to that described in Example 1. The ESI/MS data of the compound are given below.
ESI/MS m/e: 391.1 ($M^+$+H, $C_{12}H_{10}BrClN_4S_2$ $C_2HF_3O_2$)

Example 3. Synthesis of 5-(2-aminoethyl)-7-chloro-6-(3-chloro(2-thienyl))-3-hydropyrrolo[3,2-d]pyrimidin-4-thione trifluoroacetate (Compound No. 39)

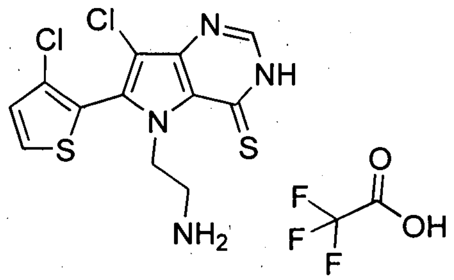

The title compound was prepared from a crude product of N-{2-[4,7-dichloro-6-(3-chloro(2-thienyl))pyrrolo[3,2-d]pyrimidin-5-yl]ethyl}-2,2,2-trifluoroacetamide in a similar manner to that described in Example 1. The ESI/MS data of the compound are given below.
ESI/MS m/e: 347.2 ($M^{+}$+H, $C_{12}H_{10}Cl_2N_4S_2$ $C_2HF_3O_2$)

Example 4. Synthesis of N-{2-[6-(3-chloro(2-thienyl))-7-iodo-4-thioxo(3-hydropyrrolo[3,2-d]pyrimidin-5-yl)]ethyl}(4-fluorophenyl)carboxyamide (Compound No. 1)

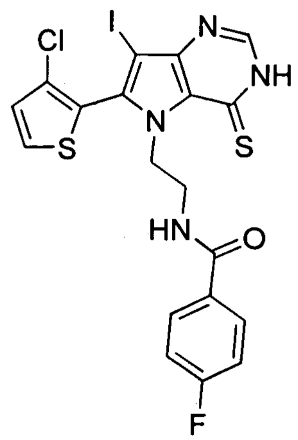

To an N,N-dimethylacetamide (2.0 mL) solution of 5-(2-aminoethyl)-6-(3-chloro(2-thienyl))-7-iodo-3-hydropyrrolo[3,2-d]pyrimidin-4-thione trifluoroacetic acid (37 mg) was added 4-fluorobenzoylchloride (21 mg), and the mixture was stirred at room temperature for a short time, followed by adding triethylamine (0.2 mL) and stirring at room temperature for 3 hours. To the reaction solution was added water (0.2 mL), and the solution was stirred again at room temperature overnight. The solvent was distilled off under reduced pressure, and the residue was purified by fraction HPLC to obtain the title compound (18 mg, yield 47%) as a colorless solid. The HPLC retention time, NMR data and ESI/MS data of the compound are given below.
HPLC retention time=10.4(min)
$^1$H-NMR(400 MHz, DMSO-$d_6$) δ(ppm): 3.57(m,2H), 4.51(m,1H), 5.36(m,1H), 7.21-7.25(m,3H), 7.70-7.73(m,2H), 7.92 (d,J=5.4, 1H), 8.09(m,1H), 8.39 (m, 1H), 13.59 (brs, 1H).
ESI/MS m/e: 559.2 ($M^{+}$+H, $C_{19}H_{13}ClFIN_4OS_2$)

Example 5. Synthesis of 6-(3-chloro(2-thienyl))-7-iodo-5-[2-(quinazolin-4-ylamino)ethyl]-3-hydropyrrolo[3,2-d] pyrimidin-4-thione (Compound No. 37)

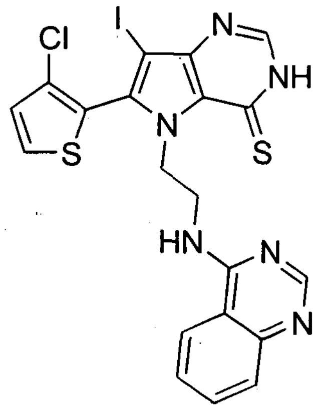

To an N,N-dimethylacetamide(2.0 mL) solution of 5-(2-aminoethyl)-6-(3-chloro(2-thienyl))-7-iodo-3-hydropyrrolo[3,2-d]pyrimidin-4-thione trifluoroacetic acid (40 mg) and 4-chloroquinazoline (12 mg) was added triethylamine (15 mg), and the mixture was stirred at 70°C for 1 hour. Triethylamine (15 mg) was further added to the reaction mixture and stirred at 70°C for 1 hour. The reaction mixture was cooled to room temperature and purified by fraction HPLC to obtain the title compound (18 mg, yield 43%) as a colorless solid. The HPLC retention time, NMR data and ESI/MS data of the compound are given below.
HPLC retention time=7.7(min) $^1$H-NMR(400 MHz, DMSO-$d_6$) δ(ppm): 4.00(m,2H), 4.80(m,1H), 5.58(m,1H), 7.12(d, J=5.4,1H), 7.70-7.77(m,2H), 7.81(d,J=5.4,1H), 8.00(t,J=7.7,1H), 8.07(d,J=2.9,1H), 8.19(d,J=8.3,1H), 8.55(s,1H), 9.81 (brs,1H), 13.62(brs,1H).
ESI/MS m/e: 565.2 ($M^+$+H, $C_{20}H_{14}ClIN_6S_2$)

Example 6. Synthesis of N-{2-[6-(3-chloro(2-thienyl))-7-iodo-4-thioxo(3-hydropyrrolo[3,2-d]pyrimidin-5-yl)]ethyl} (1-ethyl-3-methylpyrazol-5-yl)carboxyamide (Compound No. 234)

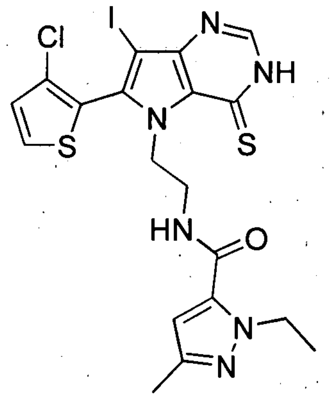

To a N,N-dimethylacetamide (2.0 mL) solution of 5-(2-aminoethyl)-6-(3-chloro(2-thienyl))-7-iodo-3-hydropyrrolo [3,2-d]pyrimidin-4-thione trifluoroacetic acid (36 mg), 1-ethyl-3-methyl-1H-pyrazol-5-carboxylic acid (20 mg), 1-ethyl-3-(3'-dimethylaminopropyl)carbodiimide hydrochloride (50 mg) and N-hydroxybenzotriazole (9 mg) was added triethylamine (0.2 mL), and the mixture was stirred at room temperature for 3 hours. To the reaction solution was further added water (0.2 mL) and stirred at room temperature for 1 hour. The reaction mixture was purified by fraction HPLC to obtain the title compound (13 mg, yield 34%) as a colorless solid. The HPLC retention time, NMR data and ESI/MS data of the compound are given below.
HPLC retention time=9.8(min)
$^1$H-NMR(400 MHz, DMSO-$d_6$) δ (ppm): 1.18(t,J=6.8,3H), 2.12(s,3H), 3.51(m,2H), 4.21(q,J=7.0,2H), 4.42(m,1H), 5.39 (m,1H), 6.39(s,1H), 7.26(d,J=5.4,1H), 7.96(d,J=5.4,1H), 8.10(s,1H), 8.21(m,1H), 13.54(brs,1H).
ESI/MS m/e: 573.2($M^+$+H, $C_{19}H_{18}ClIN_6OS_2$)

Example 7. Synthesis of N-(6-(3-chloro(2-thienyl))-5-{2-[(4-fluorophenyl)carbonylamino]ethyl}-4-thioxo(3-hydropyrrolo [3,2-d]pyrimidin-7-yl))-2,2,2-trifluoroacetamide (Compound No. 182)

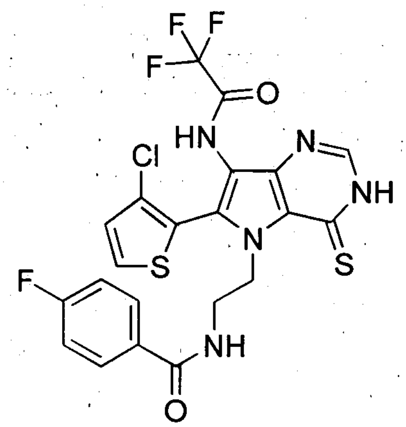

N-(6-(3-chloro(2-thienyl))-5-{2-[(4-fluorophenyl) carbonylamino]ethyl}-4-oxo(3-hydropyrrolo[3,2-d]pyrimidin-7-yl))-2,2,2-trifluoroacetamide(1.60 g) was dissolved in phosphorus oxychloride(30 mL) and the mixture was stirred at 100°C for 1 hour. The reaction mixture was cooled to room temperature, and excess phosphorus oxychloride was distilled off under reduced pressure. To the residue were added ethyl acetate and aqueous saturated sodium hydrogen carbonate solution and stirred for 30 minutes. The organic layer was separated and the aqueous layer was extracted with ethyl acetate. The combined organic layer was washed with saturated brine, dried over anhydrous sodium sulfate and filtered. The residue was dissolved in 2-propanol (30 mL), and thiourea (0.23 g) was added thereto and stirred at 100°C for 1 hour. The reaction mixture was cooled to room temperature, added with ethyl acetate and brine, and extracted with the ethyl acetate. The organic layer was washed with saturated brine, dried over anhydrous sodium sulfate and filtered. The solvent was distilled off under reduced pressure, yielding a crude product (1.65 g) of the title compound as a reddish brown solid. The crude product was used for the subsequent reaction without further purification, and a portion of the crude product was purified by fraction HPLC to be used as a sample to be evaluated. The HPLC retention time and ESI/MS data of the compound are given below. HPLC retention time=9.4(min)
ESI/MS m/e: 544.3($M^{+}$+H, $C_{21}H_{14}ClF_4N_5O_2S_2$)

Example 8. Synthesis of N-{2-[7-amino-6-(3-chloro(2-thienyl))-4-thioxo(3-hydropyrrolo[3,2-d]pyrimidin-5-yl)] ethyl} (4-fluorophenyl)carboxyamide (Compound No. 160)

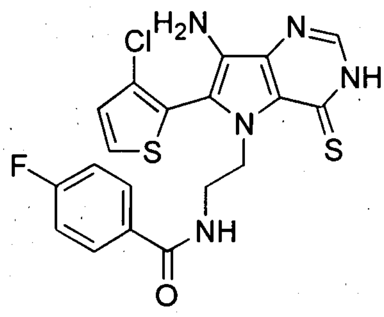

To a 1,4-dioxane solution (20 mL) of N-(6-(3-chloro(2-thienyl))-5-{2-[(4-fluorophenyl)carbonylamino]ethyl}-4-thioxo(3-hydropyrrolo[3,2-d]pyrimidin-7-yl))-2,2,2-trifluoroacetamide crude product (1.65 g) was added 2 mol/L aqueous sodium hydroxide solution (10 mL) dropwise, and the mixtures was stirred at room temperature for 24 hours. To the reaction mixture was added an aqueous hydrochloric acid (1 mol/L) for neutralization. Salt was added until the reaction solution was saturated, and extraction with ethyl acetate was performed. The organic layer was washed with saturated brine and dried over anhydrous sodium sulfate and filtered. The solvent was distilled off under reduced pressure to yield

the title compound (1.36 g, a quantitative yield) as a reddish brown solid. The HPLC retention time, NMR data and ESI/MS data of the compound are given below.
HPLC retention time=7.5(min)
$^1$H-NMR(400 MHz, DMSO-$d_6$) δ (ppm): 3.44(brs,2H), 4.17(brs,2H), 4.52(brs,1H), 5.21(brs,1H), 7.15-7.25(m,3H), 7.68-7.77(m,2H), 7.85(d,J=5.4,1H), 7.93(s,1H), 8.29-8.35(m,1H), 13.22(brs,1H).
ESI/MS m/e: 448.4($M^+$+H, $C_{19}H_{15}ClFN_5OS_2$)

Example 9. Synthesis of N-{2-[6-(3-chloro(2-thienyl))-7-(phenylcarboriylamino)-4-thioxo(3-hydropyrrolo[3,2-d] pyrimidin-5-yl)]ethyl}(4-fluorophenyl)carboxyamide (Compound No. 193)

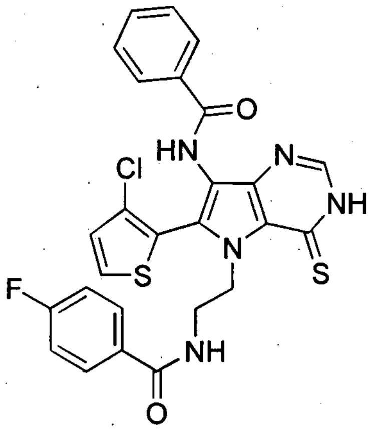

To a dimethylacetamide solution (500 μL) of N-{2-[7-amino-6-(3-chloro(2-thienyl))-4-thioxo(3-hydropyrrolo[3,2-d]pyrimidin-5-yl)]ethyl}(4-fluorophenyl) carboxyamide (22.4 mg) were added benzoylchloride (14.5 μL) and triethylamine (20.8 μL) and the mixture was stirred at room temperature for 1 hour. To the reaction solution was added an aqueous sodium hydroxide solution (2 mol/L, 500 μL) and the solution was stirred at room temperature for 2 hours, to stop the reaction. Hydrochloric acid (1 mol/L, 1.0 mL) was added to the reaction solution for neutralization, and extraction with ethyl acetate was performed. The organic layer was washed with saturated brine, dried over anhydrous sodium sulfate and filtered. The solvent was distilled off under reduced pressure and purification by fraction HPLC was performed to obtain the title compound (7.87 mg, yield 29%) as a white solid. The HPLC retention time, NMR data and ESI/MS data of the compound are given below. HPLC retention time=9.2(min)
$^1$H-NMR(400 MHz, DMSO-$d_6$) δ (ppm): 3.52-3.62(m,2H), 4.55(brs,1H), 5.50(brs,1H), 7.13(d,J=5.4,1H), 7.21(t,J=9.0,2H), 7.44(t,J=7.6,2H), 7.53(t,J=6.8,1H), 7.70-7.78(m,3H), 7.84(d,J=7.8,2H), 8.04(d,J=3.2,1H), 8.38(t,J=5.4,1H),9.81(s,1H), 13.50(brs,1H).
ESI/MS m/e: 552.4 ($M^+$+H), $C_{26}H_{19}ClFN_5O_2S_2$)

Example 10. Synthesis of N-{2-[6-(3-chloro(2-thienyl))-7-(benzylamino)-4-thioxo(3-hydropyrrolo[3,2-d]pyrimidin-5-yl)] ethyl}(4-fluorophenyl)carboxyamide (Compound No. 165)

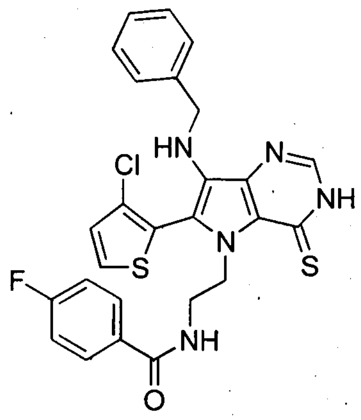

N-{2-[7-amino-6-(3-chloro(2-thienyl))-4-thioxo(3-hydropyrrolo[3,2-d]pyrimidin-5-yl)]ethyl}(4-fluorophenyl) carboxyamide (22.4 mg) was dissolved in a mixed solvent (500 μL) of chloroform and acetic acid (9:1), and benzaldehyde (7.6 μL) was added thereto, followed by stirring at 70°C overnight. The reaction solution was cooled to room temperature, sodium triacetoxy borohydride (21.2 mg) was added thereto and further reacted at 70°C for 4 hours. The reaction solution was cooled to room temperature, and aqueous saturated sodium chloride solution was added thereto to stop the reaction. The organic layer was separated, and the aqueous layer was extracted with dichloromethane. The organic layer was dried over anhydrous sodium sulfate and filtered. The solvent was distilled off under reduced pressure and purification by fraction HPLC was performed to obtain the title compound (2.56 mg, 10%) as a white solid. The HPLC retention time and ESI/MS data of the compound are given below.
HPLC retention time=12.2(min)
ESI/MS m/e: 538.4 ($M^+$+H, $C_{26}H_{21}ClFN_5OS_2$)

Example 11. Synthesis of N-{2-[6-(3-chloro(2-thienyl))-7-[(phenylamino)carbonylamino]-4-thioxo(3-hydropyrrolo[3,2-d]pyrimidin-5-yl)]ethyl}(4-fluorophenyl)carboxyamide (Compound No. 168)

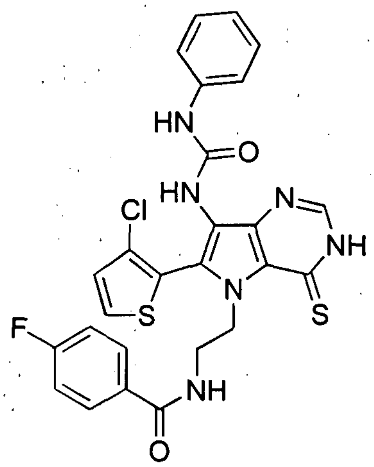

To a dimethylacetamide solution (500 μL) of N-{2-[7-amino-6-(3-chloro(2-thienyl))-4-thioxo(3-hydropyrrolo[3,2-d]pyrimidin-5-yl)]ethyl}(4-fluorophenyl) carboxyamide (22.4 mg), were added phenylisocyanate (8.1 μL) and triethylamine (13.9 μL) and the mixture was stirred at room temperature for 24 hours. To the reaction solution was added an aqueous sodium hydroxide solution (2 mol/L, 500 μL) and the solution was stirred at room temperature for 15 hours to stop the reaction. Hydrochloric acid (1 mol/L, 1.0 mL) was added to the reaction solution for neutralization, and extraction with ethyl acetate was performed. The organic layer was washed with saturated brine, dried over anhydrous sodium sulfate and filtered. The solvent was distilled off under reduced pressure; and purified by fraction HPLC was performed to obtain the title compound (7.04 mg, yield 25%) as a white solid. The HPLC retention time and ESI/MS data of the compound are given below.

HPLC retention time=9.6(min)
ESI/MS m/e: 567.3 ($M^{+}$+H, $C_{26}H_{20}ClFN_6O_2S_2$)

Example 12. Synthesis of N-(2-{7-[(1E)-1-aza-2-(dimethylamino)vinyl]-6-(3-chloro(2-thienyl))-4-thioxo(3-hydropyrrolo[3,2-d]pyrimidin-5-yl)}ethyl)(4-fluorophenyl) carboxyamide (Compound No. 163)

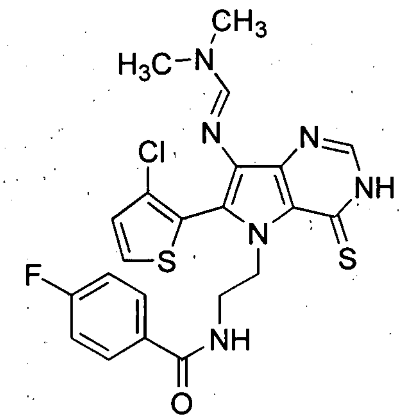

N-(2-{7-[(1E)-1-aza-2-(dimethylamino)vinyl]-4-chloro-6-(3-chloro(2-thienyl))pyrrolo[3,2-d]pyrimidin-5-yl}ethyl)(4-fluorophenyl)carboxyamide (0.87 g) was dissolved in 2-propanol (17 mL), thiourea (0.13 g) was added thereto and the mixture was stirred at 100°C for 3 hours. Thiourea (0.13 g) was further added to the reaction solution and the solution was stirred at 100°C for 3 hours. Thiourea (0.26 g) was further added to the resultant solution and the mixture was stirred at 100°C for 42 hours. The reaction mixture was cooled to room temperature, and ethyl acetate and brine were added thereto, followed by extracting with ethyl acetate. The organic layer was washed with saturated brine, and dried over anhydrous sodium sulfate and filtered. The solvent was distilled off under reduced pressure, yielding a crude product (0.28 g) of the title compound as a reddish brown solid. The crude product was purified by fraction HPLC to be used as sample to be evaluated. The HPLC retention time and ESI/MS data of the compound are given below.
HPLC retention time=7.0(min)
ESI/MS m/e: 503.4($M^{+}$+H, $C_{22}H_{20}ClFN_6OS_2$)

Example 13. Synthesis of N-{6-(3-chloro(2-thienyl))-5-[2-(quinazolin-4-ylamino)ethyl]-4-thioxo(3-hydropyrrolo[3,2-d]pyrimidin-7-yl)}benzamide (Compound No. 38)

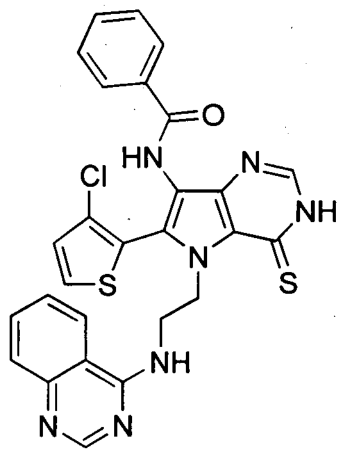

N-{6-(3-chloro(2-thienyl))-4-oxo-5-[2-(quinazolin-4-ylamino)ethyl](3-hydropyrrolo[3,2-d]pyrimidin-7-yl)} benzamide (25.5 mg) was dissolved in phosphorus oxychloride (500 μL) and the mixture was stirred at 100°C for 5 hours. The

reaction mixture was cooled to room temperature, and excess phosphorus oxychloride was distilled off under reduced pressure. The residue was dissolved in 2-propanol (500 μL), and thiourea (5.4 mg) was added thereto and the solution was stirred at 100°C for 1 hour. The reaction mixture was cooled to room temperature, and the solvent was distilled off under reduced pressure. The residue was purified by fraction HPLC to obtain the title compound (3.93 mg, yield 15%) as a white solid. The HPLC retention time and ESI/MS data of the compound are given below.
HPLC retention time=7.1(min) ESI/MS m/e: 558.4 ($M^+$+H, $C_{27}H_{20}ClN_7OS_2$

Example 14. Synthesis of N-{2-[7-pyridine-3-yl-6-(3-chloro(2-thienyl))-4-oxo(3-hydropyrrolo[3,2-d]pyrimidin-5-yl)] ethyl}-2,2,2-trifluoroacetamide (Compound No. 123)

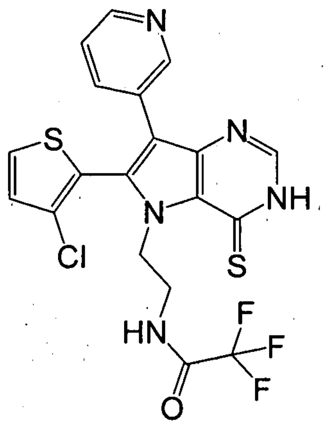

To 5-(2-aminoethyl)-6-(3-chloro-thiophene-2-yl)-7-pyridine-3-yl-3-hydropyrrolo[3,2-d]pyrimidin-4-one dichloride (51 mg) was added triethylamine (0.174 ml) and the mixture was stirred at room temperature for 2 hours. Water was added to the reaction solution and extracted with ethyl acetate. The organic layer was washed with saturated brine, and dried over anhydrous magnesium sulfate. After anhydrous magnesium sulfate was filtered off, the solvent was distilled off under reduced pressure, and the residue was dried in vacuo. A phosphorus oxychloride (2.0 ml) was added to the obtained residue, and stirred at 110°C for 1 hour. The reaction solution was cooled to room temperature, excess phosphorus oxychloride was distilled off under reduced pressure, and the residue was dried in vacuo. A thiourea (19 mg) was added to 1.4-dioxane (2 ml) and dimethylacetamide (1.5 ml), and stirred at 80 for 2 hours. The solvent was distilled off from the reaction mixture under reduced pressure, and the residue was purified by fraction HPLC to obtain the title compound (19.6 mg, 33% yield). The HPLC retention time, and ESI/MS data of the compound are given below.
HPLC retention time=9.7(min)
ESI/MS m/e: 484.2($M^+$+H, $C_{19}H_{13}ClF_3N_5OS_2$)

Examples 15-54.

The following compounds of the present invention were synthesized according to any method of Example 1 to Example 14 by using corresponding starting materials and reaction agents. The ESI/MS data in the HPLC/mass spectrum analysis, the retention time of the compounds in the HPLC analysis and purity under the following analysis conditions, and compounds numbers corresponding to executed syntheses were summarized in Table 2.

Table 2

| Ex. no. | Comp. no. | Formula | ESI/MS m/e | HPLC min | Purity (%) | Synthesis |
|---|---|---|---|---|---|---|
| 15 | 35 | $C_{20}H_{14}Cl_2N_6S_2$ | 473.4 | 7.5 | 88 | Ex. 5 |
| 16 | 36 | $C_{20}H_{14}BrClN_6S_2$ | 519.2 | 7.6 | 99 | Ex. 5 |
| 17 | 157 | $C_{19}H_{13}Cl_2FN_4OS_2$ | 467.4 | 10.2 | 100 | Ex. 4 |
| 18 | 158 | $C_{19}H_{13}BrClFN_4OS_2$ | 513.3 | 10.2 | 99 | Ex. 4 |
| 19 | 181 | $C_{21}H_{17}ClFN_5O_2S_2$ | 490.19 | 7.5 | 93 | Ex. 9 |

Table continued

| Ex. no. | Comp. no. | Formula | ESI/MS m/e | HPLC min | Purity (%) | Synthesis |
|---|---|---|---|---|---|---|
| 20 | 183 | $C_{21} H_{17} Cl F N_5 O_3 S_2$ | 506.18 | 7.1 | 95 | Ex. 9 |
| 21 | 184 | $C_{22} H_{19} Cl F N_5 O_3 S_2$ | 520.22 | 7.8 | 99 | Ex. 9 |
| 22 | 185 | $C_{23} H_{22} Cl F N_6 O_2 S_2$ | 533.22 | 6.5 | 87 | Ex. 9 |
| 23 | 186 | $C_{23} H_{19} Cl F N_5 O_2 S_2$ | 516.19 | 8.3 | 97 | Ex. 9 |
| 24 | 187 | $C_{25} H_{23} Cl F N_5 O_2 S_2$ | 544.27 | 9.4 | 89 | Ex. 9 |
| 25 | 188 | $C_{26} H_{25} Cl F N_5 O_2 S_2$ | 558.24 | 9.9 | 93 | Ex. 9 |
| 26 | 189 | $C_{30} H_{21} Cl F N_5 O_2 S_2$ | 602.25 | 10.4 | 99 | Ex. 9 |
| 27 | 190 | $C_{30} H_{21} Cl F N_5 O_2 S_2$ | 602.25 | 10.1 | 97 | Ex. 9 |
| 28 | 191 | $C_{24} H_{17} Cl F N_5 O_3 S_2$ | 542.19 | 8.5 | 97 | Ex. 9 |
| 29 | 192 | $C_{24} H_{17} Cl F N_5 O_2 S_3$ | 558.18 | 9.1 | 99 | Ex. 9 |
| 30 | 194 | $C_{25} H_{18} Cl F N_6 O_2 S_2$ | 553.24 | 9.1 | 95 | Ex. 9 |
| 31 | 195 | $C_{25} H_{18} Cl F N_6 O_2 S_2$ | 553.24 | 7.0 | 96 | Ex. 9 |
| 32 | 196 | $C_{25} H_{18} Cl F N_6 O_2 S_2$ | 553.17 | 6.9 | 98 | Ex. 9 |
| 33 | 197 | $C_{27} H_{21} Cl F N_5 O_2 S_2$ | 566.24 | 9.3 | 99 | Ex. 9 |
| 34 | 198 | $C_{28} H_{23} Cl F N_5 O_2 S_2$ | 580.28 | 9.9 | 99 | Ex. 9 |
| 35 | 263 | $C_{22} H_{27} N_5 O S$ | 410.3 | 6.2 | 100 | Ex. 14 |
| 36 | 363 | $C_{26} H_{21} Cl F N_5 S_3$ | 554.08 | 12.2 | 95 | Ex. 10 |
| 37 | 364 | $C_{26} H_{20} Cl F N_6 O S_3$ | 583.14 | 10.7 | 98 | Ex. 11 |
| 38 | 365 | $C_{21} H_{14} Cl F_4 N_5 O S_3$ | 560.19 | 10.9 | 97 | Ex. 7 |
| 39 | 366 | $C_{21} H_{17} Cl F N_5 O_2 S_3$ | 522.17 | 8.7 | 97 | Ex. 9 |
| 40 | 367 | $C_{22} H_{19} Cl F N_5 O_2 S_3$ | 536.21 | 9.6 | 99 | Ex. 9 |
| 41 | 368 | $C_{23} H_{22} Cl F N_6 O S_3$ | 549.21 | 7.8 | 94 | Ex. 9 |
| 42 | 369 | $C_{23} H_{19} Cl F N_5 O S_3$ | 532.18 | 10.0 | 100 | Ex. 9 |
| 43 | 370 | $C_{25} H_{23} Cl F N_5 O S_3$ | 560.26 | 11.0 | 99 | Ex. 9 |
| 44 | 371 | $C_{26} H_{25} Cl F N_5 O S_3$ | 574.23 | 11.4 | 100 | Ex. 9 |
| 45 | 372' | $C_{30} H_{21} Cl F N_5 O S_3$ | 618.25 | 11.8 | 95 | Ex. 9 |
| 46 | 373 | $C_{30} H_{21} Cl F N_5 O S_3$ | 618.25 | 11.5 | 98 | Ex. 9 |
| 47 | 374 | $C_{24} H_{17} Cl F N_5 O_2 S_3$ | 558.18 | 10.1 | 99 | Ex. 9 |
| 48 | 375 | $C_{24} H_{17} Cl F N_5 O S_4$ | 574.17 | 10.6 | 99 | Ex. 9 |
| 49 | 376 | $C_{26} H_{19} Cl F N_5 O S_3$ | 568.21 | 10.8 | 99 | Ex. 9 |
| 50 | 377 | $C_{25} H_{18} Cl F N_6 O S_3$ | 569.16 | 10.8 | 99 | Ex. 9 |
| 51 | 378 | $C_{25} H_{18} Cl F N_6 O S_3$ | 569.23 | 8.5 | 99 | Ex. 9 |
| 52 | 379 | $C_{25} H_{18} Cl F N_6 O S_3$ | 569.16 | 8.3 | 96 | Ex. 9 |
| 53 | 380 | $C_{27} H_{21} Cl F N_5 O S_3$ | 582.23 | 10.8 | 99 | Ex. 9 |
| 54 | 381 | $C_{28} H_{23} Cl F N_5 O S_3$ | 596.27 | 11.3 | 96 | Ex. 9 |

Example 55. Synthesis of N-{2-[4-chloro-6-(3-chloro(2-thienyl))-7-iodopyrrolo[3,2-d]pyrimidin-5-yl]ethyl}-2,2,2-trifluoroacetamide

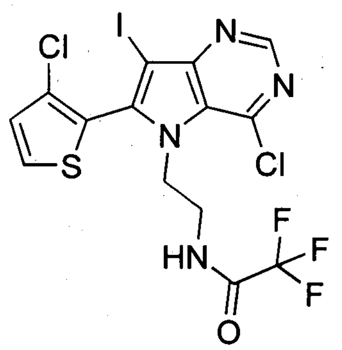

A phosphorus oxychloride (3.0 mL) solution of N-{2-[6-(3-chloro(2-thienyl))-7-iodo-4-oxo(3-hydropyrrolo [3,2-d]pyrimidin-5-yl)]ethyl}-2,2,2-trifluoroacetamide (333 mg) was stirred at 110°C for 2 hours. The reaction mixture was cooled to room temperature, and excess phosphorus oxychloride was distilled off under reduced pressure. The residue was dried in vacuo to obtain a crude product of the title compound as a brown oily compound, which was used for the subsequent reaction without further purification. The ESI/MS data of the compound are given below.
ESI/MS m/e: 535.2 ($M^{+}$+H, $C_{14}H_{8}Cl_{2}F_{3}IN_{4}OS$)

Example 56. Synthesis of N-{2-[7-bromo-4-chloro-6-(3-chloro(2-thienyl))pyrrolo[3,2-d]pyrimidin-5-yl]ethyl}-2,2,2-trifluoroacetamide

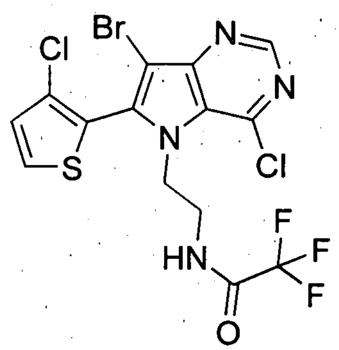

A crude product of the title compound was prepared from N-{2-[7-bromo-6-(3-chloro(2-thienyl))-4-oxo(3-hydropyrrolo [3,2-d]pyrimidin-5-yl)]ethyl}-2,2,2-trifluoroacetamide in a similar manner to that described in Example 17. The ESI/MS data of the compound are given below.
ESI/MS m/e: 489.0 ($M^{+}$+H, $C_{14}H_{8}BrCl_{2}F_{3}N_{4}OS$)

Example 57. Synthesis of N-{2-[4,7-dichloro-6-(3-chloro(2- thienyl))pyrrolo[3,2-d]pyrimidin-5-yl]ethyl}-2,2,2-trifluoroacetamide

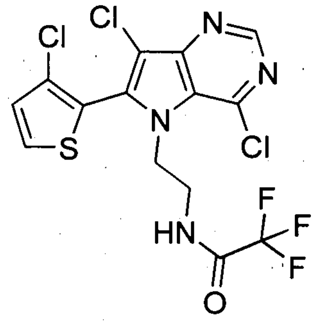

A crude product of the title compound was prepared from N-{2-[7-chloro-6-(3-chloro(2-thienyl))-4-oxo(3-hydropyrrolo[3,2-d]pyrimidin-5-yl)]ethyl}-2,2,2-trifluoroacetamide in a similar manner to that described in Example 55. The ESI/MS data of the compound are given below.
ESI/MS m/e: 443.4($M^+$+H, $C_{14}H_8Cl_3F_3N_4OS$)

Example 58. $^1$H-NMR(400 MHz, DMSO-$d_6$) of the compounds according to the present invention were measured. Data of chemical shift values (δ: ppm) and coupling constant (J: Hz) are shown in Table 3. Compound numbers in Table 3 designate compounds in Table 1 listed as preferred specific examples, and example numbers in Table 3 denote examples of corresponding syntheses.

Table 3

| Ex. no. | Comp. no. | NMR data δ(ppm) |
|---|---|---|
| 16 | 36 | 4.02(m, 2H), 4.83(m, 1H), 5.59(m, 1H), 7.11(d, J=5.4, 1H), 7.73-7.80(m, 2H), 7.84(d, J=5.4, 1H), 8.03(m, 1H), 8.10(m, 1H), 8.20(d, J=8.3, 1H), 8.60(s, 1H), 9.93(brs, 1H), 13.69 (brs, 1H). |
| 18 | 158 | 3.57(m, 2H), 4.50(m, 1H), 5.38(m, 1H), 7.19-7.26(m, 3H), 7.68-7.72(m, 2H), 7.93(m, 1H), 8.12(m, 1H), 8.38(m, 1H), 13.64 (brs, 1H). |

Example 59. Determination of inhibition of GSK-3 activity

The reaction was initiated by adding 25 μL of phospho-glycogen synthase peptide-2 substrate solution [containing 6 μM phospho-glycogen synthase peptide-2, 20 μM ATP, 16 mM MOPS buffer (pH 7.0), 0.2 mM EDTA, 20 mM magnesium acetate, 0.1 μ Ci[γ-$^{33}$P]ATP(relative activity: approximately 110 TBq/mmol)] to 5μL of each test compound using 5% dimethylsulfoxide as a solvent, and further adding 20 μL of GSK-3β enzyme solution [containing 10 mU recombinant human GSK-3β, 20 mM MOPS buffer (pH 7.0), 1 mM EDTA, 0.1% polyoxyethylene lauryl ether(23 Lauryl Ether; Brij 35), 5% glycerol, and 0.1% β-mercaptoethanol. After 20 minutes at room temperature, the reaction was terminated by the addition of the equivalent amount of 200 mM phosphoric acid solution. 90 μL of the reaction product was spotted onto a multiscreen PH plate (manufactured by Millipore) and washed with 100 mM phosphoric acid solution. The plate was dried, and 30 μL of MicroScint-O (manufactured by Packard BioScience) was added thereto. To evaluate inhibitory activity, cpm was counted using a scintillation counter. Here, Phospho GS Peptide 2 is an amino acid peptide having the following sequence: Tyr-Arg-Arg-Ala-Ala-Val-Pro-Pro-Ser-Pro-Ser-Leu-Ser-Arg-His-Ser-Ser-Pro-His-Gln-Ser(P)-Glu-Asp-Glu-Glu-Glu.

GSK-3 inhibitor activity values ($IC_{50}$ values) of the compounds according to the present invention were measured by the method described above. As a result, inhibition activity of $IC_{50}$<100 nM was confirmed in compounds of compound numbers 1, 35, 36, 37, 123, 157, 158, 160, 193, 234, and 263.

Also, the inhibition activity of 100nM≤$IC_{50}$<1 μM was confirmed in compounds of compound numbers 165, 182, 363, and 365.

Compound numbers designate compounds in Table 1 listed as preferred specific examples.

As described above, the pyrrolopyrimidine derivatives according to the present invention exhibit strong inhibitory activity against GSK-3. Therefore, the pyrrolopyrimidine derivatives according to the present invention have been found to be inhibitors of GSK-3 activity to be used in prevention and/or treatment of various diseases associated with GSK-3, which are clinically applicable compounds.

Example 60. Preparation of tablets

Tablets each comprising the following ingredients were prepared.

| | |
|---|---|
| Compound (Example 1) | 50 mg |
| Lactose | 230 mg |
| Potato starch | 80 mg |
| Polyvinylpyrrolidone | 11 mg |
| Magnesium stearate | 5 mg |

The compound of the present invention (the compound prepared in Example 1), lactose and potato starch were mixed, homogenously wetted with 20% ethanol solution of polyvinylpyrrolidone, passed through a 20 mesh sieve, dried at 45°C, and passed through again a 15 mesh sieve to obtain granules. The thus obtained granules were mixed with magnesium stearate and compressed into tablets.

INDUSTRIAL APPLICABILITY

The pyrrolopyrimidine-thione derivatives of Formula (I) according to the present invention and its pharmaceutically acceptable salts have GSK-3 inhibitory activity and used as valid components as pharmaceutical product. Therefore, pharmaceutical agents containing these compounds as effective components are expected as promising therapeutic drugs or preventive drugs in GSK-3 mediated diseases including diabetes, diabetic complications, Alzheimer's disease, neurodegenerative diseases manic depression, traumatic cerebrospinal injury, alopecia, inflammatory diseases, cancer and immunodeficiency.

## Claims

**1.** A compound represented by the formula (I) or a pharmaceutically acceptable salt thereof:

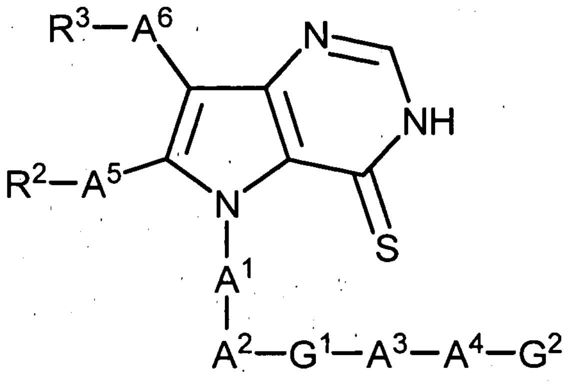

(I)

wherein
$A^1$ is a bond representing a single bond, or an acyclic aliphatic hydrocarbon group having 1 to 6 carbon atoms that links a nitrogen atom bonded to $A^1$ with $A^2$ on the same or different carbon atom;
$A^2$ represents a single bond or represents a group that links $A^1$ with $G^1$ in the form of $A^1$ -C(=O)-$G^1$, $A^1$-C(=O)-O-$G^1$; $A^1$-C(=O)-$NR^{101}$-$G^1$, $A^1$-C(=S)-$NR^{102}$-$G^1$, $A^1$-C(=$NR^{103}$)-$G^1$, $A^1$-O-$G^1$, $A^1$-O-C(=O)-$G^1$, $A^1$-$NR^{104}$-$G^1$, $A^1$-$NR^{105}$-C(=O)-$G^1$, $A^1$-$NR^{106}$-$S(=O)_2$-$G^1$, $A^1$-$NR^{107}$-C(=O)-O-$G^1$, $A^1$-$NR^{108}$-C(=O)-$NR^{109}$-$G^1$, $A^1$-$NR^{110}$C(=S)-$G^1$, $A^1$-$NR^{111}$-C(=S)-$NR^{112}$-$G^1$, $A^1$-S-$G^1$, $A^1$-S(=O)-$G^1$, $A^1$-$S(=O)_2$-$G^1$, $A^1$-$S(=O)_2$-$NR^{113}$-$G^1$, $A^1$-$CR^{114}$=CH-$G^1$, $A^1$-$CR^{115}$=CF-$G^1$, $A^1$-CH=$CR^{116}$-$G^1$, or $A^1$-CF=$CR^{117}$-$G^1$;
$G^1$ represents a single bond or represents a divalent group which is obtained by removing two hydrogen atoms from any one of an optionally substituted alicyclic hydrocarbon having 3 to 10 carbon atoms, an optionally substituted aromatic hydrocarbon having 6 to 14 carbon atoms, and an optionally substituted heterocyclic compound having 1 to 4 atoms selected from the group consisting of an oxygen atom, a nitrogen atom and a sulfur atom, in the ring,
$A^3$ represents a single bond or represents an optionally substituted divalent acyclic aliphatic hydrocarbon group

having 1 to 10 carbon atoms that links $G^1$ with $A^4$ on the same or different carbon atom;
$A^4$ represents a single bond or represents a group that links $A^3$ with $G^2$ in the form of $A^3$ -C(=O)-$G^2$, $A^3$-C(=O)-O-$G^2$, $A^3$-C(=O)-$NR^{121}$-$G^2$, $A^3$-C(=S)-$NR^{122}$-$G^2$, $A^3$-C(=$NR^{123}$)-$G^2$, $A^3$-O-$G^2$, $A^3$-O-C(=O)-$G^2$, $A^3$-$NR^{124}$-$G^2$, $A^3$-$NR^{125}$-C(=O)-$G^2$, $A^3$-$NR^{126}$-$S(=O)_2G^2$, $A^3$-$NR^{127}$-C(=O)-O-$G^2$, $A^3$-$NR^{128}$-C(=O)-$NR^{129}$-$G^2$, $A^3$-$NR^{130}$-C(=S)-$G^2$, $A^3$-$NR^{131}$-C(=S)-$NR^{132}$-$G^2$, $A^3$-S-$G^2$, $A^3$-S(=O)-$G^2$, $A^3$-$S(=O)_2$-$G^2$, $A^3$-$S(=O)_2$-$NR^{133}$-$G^2$ or $A^3$-$S(=O)_2$-O-$G^2$;
$G^2$ is a hydrogen atom, an optionally substituted acyclic aliphatic hydrocarbon'group having 1 to 10 carbon atoms, an optionally substituted alicyclic hydrocarbon group having 3 to 10 carbon atoms, an optionally substituted aromatic hydrocarbon group having 6 to 14 carbon atoms, or an optionally substituted heterocyclic group having 1 to 4 atoms selected from the group consisting of an oxygen atom, a nitrogen atom and a sulfur atom, in the ring;
$A^5$ represents a single bond or -$NR^{201}$-;
$R^2$ is a hydrogen atom, a fluorine atom, a chlorine atom, a bromine atom, an iodine atom, an optionally substituted acyclic aliphatic hydrocarbon group having 1 to 10 carbon atoms, an optionally substituted alicyclic hydrocarbon group having 3 to 8 carbon atoms, an optionally substituted aromatic hydrocarbon group having 6 to 14 carbon atoms, or an optionally substituted heterocyclic group having 1 to 4 atoms selected from the group consisting of an oxygen atom, a nitrogen atom and a sulfur atom, in the ring;
$A^6$ represents a single bond or represents a group that links $R^3$ with a carbon atom of a pyrrole ring to which $A^6$ is bonded, in the form of $R^3$-$NR^{301}$-pyrrole ring, $R^3$-C(=O)-pyrrole ring, $R^3$-$NR^{302}$-C(=O)-pyrrole ring, $R^3$-$NR^{303}$-C(=S)-pyrrole ring, $R^3$-$NR^{304}$-C(=O)-$NR^{305}$-pyrrole ring, $R^3$-C(=O)-$NR^{306}$-pyrrole ring, $R^3$-$NR^{307}$-CH=N-pyrrole ring, $R^3$-C(=O)-O-pyrrole ring, $R^3$-O-C(=O)-pyrrole ring, $R^3$-O-pyrrole ring, $R^3$-S-pyrrole ring, $R^3$-S(=O)-pyrrole ring, $R^3$-$S(=O)_2$-pyrrole ring, $R^3$-$CR^{308}$=$CR^{309}$-pyrrole ring, $R^3$-C≡C-pyrrole ring, or $R^3$-$S(=O)_2$-C≡C-pyrrole ring;
$R^3$ is a hydrogen atom, a fluorine atom, a chlorine atom, a bromine atom, an iodine atom, a nitro group, an optionally substituted saturated acyclic aliphatic hydrocarbon group having 1 to 10 carbon atoms, an optionally substituted alicyclic hydrocarbon group having 3 to 8 carbon atoms, an optionally substituted aromatic hydrocarbon group having 6 to 14 carbon atoms, or an optionally substituted heterocyclic group having 1 to 4 atoms selected from the group consisting of an oxygen atom, a nitrogen atom and a sulfur atom, in the ring;
$A^6$-$R^3$ may be a combination wherein $A^6$ represents a group that links a carbon atom of a pyrrole ring to which $A^6$ is bonded, with $R^3$ in the form of $R^3$-$CR^{308}$=$CR^{309}$-pyrrole ring or $R^3$-C≡C-pyrrole ring, and $R^3$ represents a trimethylsilyl group, a formyl group, an optionally substituted $C_2$-$C_7$ acyl group, a carboxyl group, a $C_2$-$C_7$ alkoxycarbonyl group, a carbamoyl group, an optionally substituted $C_2$-$C_7$ alkylcarbamoyl group, or a cyano group;
$R^{101}$-$R^{117}$, $R^{121}$-$R^{133}$, $R^{201}$ and $R^{301}$-$R^{309}$ are each independently a hydrogen atom or an acyclic aliphatic hydrocarbon group having 1 to 4 carbon atoms;
where when both of $A^1$ and $A^3$ represent the acyclic aliphatic hydrocarbon group, at least either one of $A^2$ or $G^1$ is not a single bond.

**2.** The compound or a pharmaceutically acceptable salt thereof according to claim 1, wherein $A^1$ represents a divalent acyclic aliphatic hydrocarbon group having 1 to 6 carbon atoms in formula (I).

**3.** The compound or a pharmaceutically acceptable salt thereof according to claim 1, wherein $A^1$ is $-(CH_2)_2-$ or $(CH_2)_3-$ in formula (I).

**4.** The compound or a pharmaceutically acceptable salt thereof according to claim 2 or 3,wherein $A^2$ represents something other than the single bond.

**5.** The compound or a pharmaceutically acceptable salt thereof according to claim 2 or 3, wherein $A^2$ represents -C(=O)-, -C(=O)-O-, -C(=O)-NH-, -C(=O)-NMe-, -NH-, -NH-C(=O)-, -NH-C(=O)-O-, -NH-C(=O)-NH-, -NH-C(=O)-NMe-, or -NH-C(=S)- in formula (I).

**6.** The compound or a pharmaceutically acceptable salt thereof according to claim 2 or 3, wherein $A^2$ represents -C(=O)-NH-, -NH-, -NH-C(=O)-, -NH-C(=O)-O-, or -NH-C(=O)-NH- in formula (I).

**7.** The compound or a pharmaceutically acceptable salt thereof according to claim 1, wherein both of $A^1$ and $A^2$ represent a single bond in formula (I).

**8.** The compound or a pharmaceutically acceptable salt thereof according to claim any one of claims 1 to 6, wherein combination of $G^1$, $A^3$, $A^4$, and $G^2$ is any of the combinations of 1 to 10 in the following table in formula (I).

| Combination | $G^1$ | $A^3$ | $A^4$ | $G^2$ |
|---|---|---|---|---|
| 1 | Group other than single bond | Single bond | Single bond | Hydrogen atom |
| 2 | Single bond | Group other than single bond | Single bond | Hydrogen atom |
| 3 | Group other than single bond | Single bond | Single bond | Group other than hydrogen atom |
| 4 | Single bond | Group other than single bond | Single bond | Group other than hydrogen atom |
| 5 | Group other than single bond | Single bond | Group other than single bond | Group other than hydrogen atom |
| 6 | Single bond | Group other than single bond | Group other than single bond | Group other than hydrogen atom |
| 7 | Group other than single bond | Group other than single bond | Single bond | Group other than hydrogen atom |
| 8 | Group other than single bond | Group other than single bond | Group other than single bond | Group other than hydrogen atom |
| 9 | Group other than single bond | Group other than single bond | Group other than single bond | Hydrogen atom |
| 10 | Single bond | Single bond | Single bond | Hydrogen atom |

**9.** The compound or a pharmaceutically acceptable salt thereof according to any one of claims 1 to 7, wherein $G^1$ represents a group other than a single bond, $A^3$ and $A^4$ represent a single bond, and $G^2$ represents a hydrogen atom in formula (I).

**10.** The compound or a pharmaceutically acceptable salt thereof according to any one of claims 1 to 7, wherein $G^1$ and $A^4$ represent a single bond, $A^3$ represents a group other than the single bond, and $G^2$ represents a hydrogen atom in formula (I).

**11.** The compound or a pharmaceutically acceptable salt thereof according to any one of claims 1 to 7, wherein $G^1$ represents a group other than a single bond, $A^3$ and $A^4$ represent a single bond, and $G^2$ represents a group other than the hydrogen atom in formula (I).

**12.** The compound or a pharmaceutically acceptable salt thereof according to any one of claims 1 to 7, wherein $G^1$ and $A^4$ represent a single bond, $A^3$ represents a group other than the single bond, and $G^2$ represents a group other than the hydrogen atom in formula (I).

**13.** The compound or a pharmaceutically acceptable salt thereof according to claim 12, wherein $A^3$ represent a $C_1$-$C_3$ alkylene group in formula (I).

**14.** The compound or a pharmaceutically acceptable salt thereof according to any one of claims 1 to 7, wherein $G^1$ and $A^4$ represent a group other than the single bond, $A^3$ represents a single bond, and $G^2$ represents a group other than the hydrogen atom in formula (I).

**15.** The compound or a pharmaceutically acceptable salt thereof according to claim 14, wherein $A^4$ represents -C(=O)-, -C(=O)-NH-, -O-, or -NH-C(=O)- in formula (I).

**16.** The compound or a pharmaceutically acceptable salt thereof according to any one of claims 1 to 7, wherein $G^1$ represents a single bond, $A^3$ and $A^4$ represent a group other than the single bond, and $G^2$ represents a group other than the hydrogen atom in formula (I).

**17.** The compound or a pharmaceutically acceptable salt thereof according to any one of claims 1 to 7, wherein $G^1$ and $A^3$ represent a group other than the single bond, $A^4$ represents a single bond, and $G^2$ represents a group other than the hydrogen atom in formula (I).

**18.** The compound or a pharmaceutically acceptable salt thereof according to claim 17, wherein $A^3$ represent a $C_1$-$C_3$ alkylene group in formula (I).

**19.** The compound or a pharmaceutically acceptable salt thereof according to any one of claims 1 to 7, wherein $G^1$, $A^3$ and $A^4$ represent a group other than the single bond, and $G^2$ represents a group other than the hydrogen atom in formula (I).

**20.** The compound or a pharmaceutically acceptable salt thereof according to any one of claims 1 to 7, wherein $A^4$ represents -O- in formula (I).

**21.** The compound or a pharmaceutically acceptable salt thereof according to any one of claims 1 to 7, wherein $G^1$, $A^3$ and $A^4$ represent a single bond, and $G^2$ represents a hydrogen atom in formula (I).

**22.** The compound or a pharmaceutically acceptable salt thereof according to any one of claims 1 to 7, wherein $G^1$, $A^3$ and $A^4$ represent a single bond, and $G^2$ represents a hydrogen atom in formula (I).

**23.** The compound or a pharmaceutically acceptable salt thereof according to claim 3, wherein $A^2$ represents -NH-(C=O)- or -NH-(C=O)-NH-, $G^1$ represents a single bond, and $A^3$ represents a divalent acyclic aliphatic hydrocarbon group having 1 to 10 carbon atoms in formula (I).

**24.** The compound or a pharmaceutically acceptable salt thereof according to claim 3, wherein $A^2$ represents -NH-(C=O)-, -NH-(C=O)-NH-, -NH- or -C(=O)-NH-, and $G^1$ represents a group other than the single bond in formula (I).

**25.** The compound or a pharmaceutically acceptable salt thereof according to claim 2 or 3, wherein: in formula (I), $A^2$ represents a single bond, and $G^1$ represents an optionally substituted heterocyclic group, in which a 5-6 membered monocyclic heterocyclic group of $G^1$ is substituted or $A^3$-$G^2$ portion represents those other than the hydrogen atom where the heterocyclic group of $G^1$ is 5-6 membered monocyclic.

**26.** The compound or a pharmaceutically acceptable salt thereof according to any one of claims 4 to 6, wherein: in formula (I), $G^1$ represents an optionally substituted aromatic hydrocarbon group, an optionally substituted alicyclic hydrocarbon group having 7 to 10 carbon atoms, or an optionally substituted heterocyclic group, in which, where the aromatic hydrocarbon group of $G^1$ is a phenyl group, or the heterocyclic group of $G^1$ is 5-6 membered monocyclic ring, the phenyl group of $G^1$ or the 5-6 membered monocyclic heterocyclic group is substituted, or $A^3$-$G^2$ portion represents those other than the hydrogen atom.

**27.** The compound or a pharmaceutically acceptable salt thereof according to any one of claims 4 to 6, wherein $G^1$ and $A^4$ represent a single bond, $A^3$ represents an optionally substituted acyclic aliphatic hydrocarbon group haying 1 to 10 carbon atoms, $G^2$ represents an optionally substituted alicyclic hydrocarbon group having 5 to 10 carbon atoms, an optionally substituted aromatic hydrocarbon group or an optionally substituted heterocyclic group in formula (I).

**28.** The compound or a pharmaceutically acceptable salt thereof according to any one of claims 4 to 6, wherein $G^1$ represents a single bond, $A^3$ represents an optionally substituted acyclic aliphatic hydrocarbon group having 1 to 10 carbon atoms, and $A^4$ represents -C(=O)-, -C(=O)-$NR^{121}$-, -C(=S)-$NR^{122}$-, -C(=$NR^{123}$)-, -O-C(=O)-, -$NR^{125}$-C(=O)-, -$NR^{126}$-$S(=O)_2$-, -$NR^{127}$-C(=O)-O-, -$NR^{128}$-C(=O)-$NR^{129}$-, -$NR^{130}$-C (=S) -, -$NR^{131}$-C(=S)-$NR^{132}$-, -S-, -S (=O)-, -$S(=O)_2$-, -$S(=O)_2$-$NR^{133}$-, or -$S(=O)_2$-O-.

**29.** The compound or a pharmaceutically acceptable salt thereof according to any one of claims 1 to 28, wherein $A^5$ represents a single bond in formula (I).

**30.** The compound or a pharmaceutically acceptable salt thereof according to claim 29, wherein $R^2$ represents an optionally substituted acyclic aliphatic hydrocarbon group having 1 to 10 carbon atoms, optionally substituted alicyclic hydrocarbon group having 3 to 8 carbon atoms, an optionally substituted aromatic hydrocarbon group having 6 to 14 carbon atoms, or an optionally substituted heterocyclic group in formula (I).

**31.** The compound or a pharmaceutically acceptable salt thereof according to claim 29, wherein $R^2$ represents an acyclic aliphatic hydrocarbon group having 1 to 10 carbon atoms, alicyclic hydrocarbon group having 3 to 8 carbon atoms, an optionally substituted phenyl group, or an optionally substituted heterocyclic group having 1 or 2 atoms selected from the group consisting of an oxygen atom, a nitrogen atom, or a sulfur atom, in the ring, in formula (I).

**32.** The compound or a pharmaceutically acceptable salt thereof according to claim 29, wherein $R^2$ represents a cyclopropyl group, cyclobutyl group, cyclopropylmethyl group, methyl group, ethyl group, vinyl group, isopropyl group, or 2-methyl-1-propenyl group in formula (I).

**33.** The compound or a pharmaceutically acceptable salt thereof according to claim 29, wherein $R^2$ represents a thienyl group, a pyridyl group, a furyl group, a pyrrolyl group, a pyrazolyl group or phenyl group which can be substituted by any or more of a $C_1$-$C_4$ alkyl group, a $C_1$-$C_4$ alkoxy group, a $C_2$-$C_4$ acyl group, a hydroxy group, a carboxyl group, an alkoxycarbonyl group, a fluorine atom, or a chlorine atom in formula (I).

**34.** The compound or a pharmaceutically acceptable salt thereof according to any one of claims 1 to 33, wherein $A^3$ represents a single bond in formula (I).

**35.** The compound or a pharmaceutically acceptable salt thereof according to claim 34, wherein $R^3$ represents an optionally substituted aromatic hydrocarbon group having 6 to 14 carbon atoms in formula (I).

**36.** The compound or a pharmaceutically acceptable salt thereof according to claim 34, wherein $R^3$ represents a thienyl group, a pyridyl group, a furyl group, a pyrrolyl group, a pyrazolyl group or phenyl group which can be substituted by one or more of a $C_1$-$C_4$ alkyl group in formula (I).

**37.** The compound or a pharmaceutically acceptable salt thereof according to claim 34, wherein $R^3$ represents a pyridyl group or 1-oxypyridyl group, or a pyrazolyl group or N-methylpyrazolyl group which can be substituted by one $C_1$-$C_4$ alkyl group or one halogen atom in formula (I) .

**38.** The compound or a pharmaceutically acceptable salt thereof according to claim 29, wherein $A^6$ represents a single bond in formula (I).

**39.** The compound or a pharmaceutically acceptable salt thereof according to claim 32, wherein $A^6$ represents a single bond, and $R^3$ represents a pyridyl group or 1-oxypyridyl group, or a pyrazolyl group or N-methylpyrazolyl group which can be substituted by one $C_{1-}C_4$ alkyl group or one halogen atom in formula (I).

**40.** The compound or a pharmaceutically acceptable salt thereof according to claim 33, wherein $A^6$ represents a single bond, and $R^3$ represents a pyridyl group or 1-oxypyridyl group, or a pyrazolyl group or N-methylpyrazolyl group which can be substituted by one $C_{1-}C_4$ alkyl group or one halogen atom in formula (I).

**41.** The compound or a pharmaceutically acceptable salt thereof according to any one of claims 23 to 28, wherein both of $A^5$ and $A^6$ represent a single bond in formula (I).

**42.** The compound or a pharmaceutically acceptable salt thereof according to claim 41, wherein $R^2$ represents an optionally substituted acyclic aliphatic hydrocarbon group having 1 to 10 carbon atoms, optionally substituted alicyclic hydrocarbon group having 3 to 8 carbon atoms, an optionally substituted aromatic hydrocarbon group having 6 to 14 carbon atoms, or an optionally substituted heterocyclic group, and $R^3$ represents an optionally substituted aromatic hydrocarbon group having 6 to 14 carbon atoms or optionally substituted heterocyclic group in formula (I).

**43.** The compound or a pharmaceutically acceptable salt thereof according to claim 41, wherein $R^2$ represents an optionally substituted acyclic aliphatic hydrocarbon group having 1 to 10 carbon atoms, alicyclic hydrocarbon group having 3 to 8 carbon atoms, an optionally substituted phenyl group or an optionally substituted heterocyclic group having 1 or 2 atoms selected from the group consisting of an oxygen atom, a nitrogen atom, and a sulfur atom, in the ring, and $R^3$ represents a thienyl group, a pyridyl group, a furyl group, a pyrrolyl group, a pyrazolyl group or phenyl group which can be substituted by one or more of a $C_1$-$C_4$ alkyl group in formula (I).

**44.** The compound or a pharmaceutically acceptable salt thereof according to claim 41, wherein $R^2$ represents a cyclopropyl group, methyl group, ethyl group, vinyl group, isopropyl group, isobutyl group or 2-methyl-1-propenyl group, and $R^3$ represents a pyridyl group or 1-oxypyridyl group, or pyrazolyl group or N-methylpyrazolyl group which can

be substituted by one $C_1$-$C_4$ alkyl group or one halogen atom in formula (I).

**45.** The compound or a pharmaceutically acceptable salt thereof according to claim 41, wherein $R^2$ represents a thienyl group, a pyridyl group, a furyl group, a pyrrolyl group, a pyrazolyl group or phenyl group which can be substituted by one or more of a $C_1$-$C_4$ alkyl group, a $C_1$-$C_4$ alkoxy group or a chlorine atom and $R^3$ represents a pyridyl group or 1-oxypyridyl group, or pyrazolyl group or N-methylpyrazolyl group which can be substituted by one $C_1$-$C_4$ alkyl group or one halogen atom in formula (I).

**46.** A pharmaceutical composition comprising the compound or a pharmaceutically acceptable salt thereof according to any one of claims 1 to 45; and a pharmaceutically acceptable carrier.

**47.** A GSK-3 inhibitor comprising the compound or a pharmaceutically acceptable salt thereof according to any one of claims 1 to 45.

**48.** An agent for treating or preventing a GSK-3-mediated disease, comprising the compound or a pharmaceutically acceptable salt thereof according to any one of claims 1 to 45.

**49.** The agent for treating or preventing according to claim 48, wherein the GSK-3-mediated disease is selected from the group consisting of diabetes, diabetic complications, Alzheimer's disease, neurodegenerative disease, manic depressive psychosis, traumatic brain injury, alopecia, inflammatory disease, cancer, and immunodeficiency.

**50.** A compound represented by the formula (II):

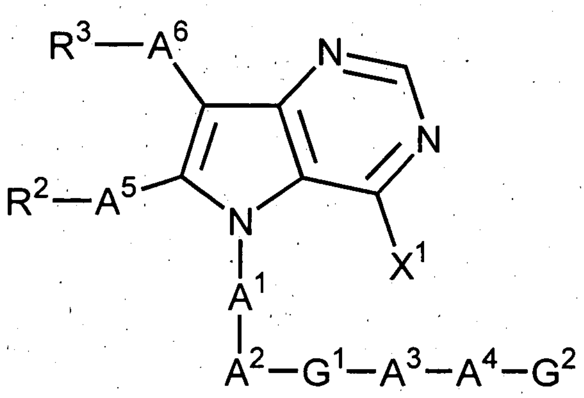

(II)

wherein $A^1$, $A^2$, $A^3$, $A^4$, $A^5$, $A^6$, $G^1$, $G^2$, $R^2$ and $R^3$ are as defined in the formula (I); and $X^1$ is a chlorine atom, a bromine atom, an iodine atom, an acylthio group having 2 to 10 carbon atoms, an alkoxymethylthio group having 2 to 8 carbon atoms, an alkyl group having 1 to 8 carbon atoms, or an arylsulfonyloxy group having 1 to 8 carbon atoms.

**51.** A compound represented by the formula (Ic):

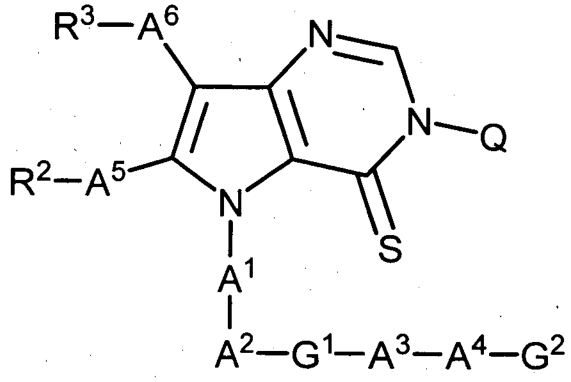

(Ic)

wherein $A^1$, $A^2$, $A^3$, $A^4$, $A^5$, $A^6$, $G^1$, $G^2$ $R^2$, $R^3$, and X are as defined in the formula (I); and Q represents an optionally

substituted acyl group having 2 to 10 carbon atoms, an optionally substituted alkoxymethyl group having 2 to 10 carbon atoms or an optionally substituted benzyl group.

**INTERNATIONAL SEARCH REPORT**

International application No.
PCT/JP2004/012690

A. CLASSIFICATION OF SUBJECT MATTER
Int.Cl[7] C07D487/04, A61K31/519, A61P3/10, 17/14, 25/24, 25/28, 29/00, 35/00, 37/02, 43/00

According to International Patent Classification (IPC) or to both national classification and IPC

B. FIELDS SEARCHED

Minimum documentation searched (classification system followed by classification symbols)
Int.Cl[7] C07D487/04, A61K31/519

Documentation searched other than minimum documentation to the extent that such documents are included in the fields searched

Electronic data base consulted during the international search (name of data base and, where practicable, search terms used)
CAS ONLINE

C. DOCUMENTS CONSIDERED TO BE RELEVANT

| Category* | Citation of document, with indication, where appropriate, of the relevant passages | Relevant to claim No. |
|---|---|---|
| P,X | WO 2004/018496 A1 (ALBERT EINSTEIN COLLEGE OF MEDICINE OF YESHIVA UNVERSITY), 04 March, 2004 (04.03.04), Full text (Family: none) | 1,7,8,22,29, 34,35,38,46, 50 |
| P,X | WO 03/100009 A2 (BIOCRYST PHARMACEUTICALS, INC.), 04 December, 2003 (04.12.03), Full text (Family: none) | 50 |

[X] Further documents are listed in the continuation of Box C. [ ] See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 17 November, 2004 (17.11.04) | 07 December, 2004 (07.12.04) |
| Name and mailing address of the ISA/ Japanese Patent Office | Authorized officer |
| Facsimile No. | Telephone No. |

Form PCT/ISA/210 (second sheet) (January 2004)

**INTERNATIONAL SEARCH REPORT**

International application No.
PCT/JP2004/012690

C (Continuation). DOCUMENTS CONSIDERED TO BE RELEVANT

| Category* | Citation of document, with indication, where appropriate, of the relevant passages | Relevant to claim No. |
|---|---|---|
| X | EVANS, Gary B. et al., Exploring Structure-Activity Relationships of Transition State Analogues of Human Purine Nucleoside Phosphorylase, Journal of Medicinal Chemistry, 2003, Vol.46, No.15, pages 3412 to 3423 | 1,7,8,22,29, 34,35,38,46 |
| X | US 2003/0114466 A1 (BIOCRYST PHARMACEUTICALS, INC.), 19 June, 2003 (19.06.03), Full text (Family: none) | 50 |
| X | US 6458799 B1 (BIOCRYST PHARMACEUTICALS, INC.), 01 October, 2002 (01.10.02), Full text (Family: none) | 50 |
| X | WO 02/18371 A1 (INDUSTRIAL RESEARCH LTD.), 07 March, 2002 (07.03.02), Full text & JP 2004-507534 A & US 2004/053944 A1 | 50 |
| X | JP 2001-233855 A (BASF AG.), 28 August, 2001 (28.08.01), Full text & EP 1127874 A2 & US 2001/037031 A1 | 50 |
| X | WO 97/49706 A1 (NOVARTIS AG.), 31 December, 1997 (31.12.97), Example 1 & AU 9731762 A1 | 50 |
| X | JP 5-43578 A (Marion Merrell Dow Inc.), 23 February, 1993 (23.02.93), Full text & EP 475413 A2 & US 5470857 A & US 5605903 A & US 5624930 A | 50 |
| X | JP 4-270282 A (Marion Merrell Dow Inc.), 25 September, 1992 (25.09.92), Full text & EP 475411 A1 & US 5244896 A & US 5514688 A & US 5631258 A | 50 |
| X | LAFON, Stephen W. et al., 6-Thiopurine riboside analogs: their toxicity and metabolism in Leishmania donovani and mammalian cells, Advances in Experimental Medicine and Biology, 1986, vol.195B (Purine Pyrimidine Metabolism Man 5, Pt.B), pages 565 to 571 | 1,7,8,22,29, 34,35,38 |

Form PCT/ISA/210 (continuation of second sheet) (January 2004)

**INTERNATIONAL SEARCH REPORT**

International application No.
PCT/JP2004/012690

C (Continuation). DOCUMENTS CONSIDERED TO BE RELEVANT

| Category* | Citation of document, with indication, where appropriate, of the relevant passages | Relevant to claim No. |
|---|---|---|
| X | JP 58-26888 A (Sloan Kettering Institute for Cancer Research), 17 February, 1983 (17.02.83), Full text & EP 71227 A1 & US 4584369 A | 1,7,8,22,29, 34,35,38, 46-49 |
| X | WO 02/85909 A1 (VERTEX PHARMACEUTICALS INC.), 31 October, 2002 (31.10.02), Full text & EP 1383771 A1 & US 2003/096813 A1 | 1-49 |
| P,A | WO 03/70729 A1 (Teijin Ltd.), 28 August, 2003 (28.08.03), Claims & CA 2477116 A | 1-51 |

Form PCT/ISA/210 (continuation of second sheet) (January 2004)